# EUROPEAN PATENT SPECIFICATION

(11) **EP 3 502 134 B1**
(45) Date of publication and mention of the grant of the patent: **16.12.2020**
(21) Application number: 18212794.4
(22) Date of filing: 10.03.2015
(51) Int. Cl.: C07K 16/08, C07K 16/20, C07K 16/28, C07K 16/30, C07K 16/32

(54) **PROTEINS COMPRISING BINDING REGIONS, SHIGA TOXIN A SUBUNIT EFFECTOR REGIONS, AND CARBOXY-TERMINAL, ENDOPLASMIC RETICULUM LOCALIZATION SIGNAL MOTIFS**
PROTEINE MIT BINDUNGSREGIONEN, SHIGA-TOXIN UND EFFEKTORREGIONEN EINER UNTEREINHEIT UND CARBOXYTERMINALE, ENDOPLASMATISCHE RETIKULUM-LOKALISIERUNGSSIGNALMOTIVE
PROTÉINES COMPORTANT DES RÉGIONS DE LIAISON, DES RÉGIONS EFFECTRICES À SOUS-MOTIFS A DE SHIGA-TOXINE ET DES MOTIFS DE SIGNAL DE LOCALISATION DU RÉTICULUM ENDOPLASMIQUE CARBOXY-TERMINAUX

(30) Priority: 11.03.2014 US 201461951110 P
(43) Date of publication of application: 26.06.2019
(62) Divisional of application: 15716202.5
(73) Proprietor: Molecular Templates, Inc., Austin, TX 78729 (US)
(72) Inventor: POMA, Eric, New York, NY 10014 (US); WILLERT, Erin, Round Rock, TX 78681 (US); KIM, Jason, Austin, TX 8759 (US); HIGGINS, Jack, Georgetown, Texas 78626 (US)
(74) Representative: Griffin, Philippa Jane

(56) References cited:
- WO-A2-2014/164693
- AL-JAUFY AHMED Y ET AL: "Cytotoxicity of a shiga toxin a subunit-CD4 fusion protein to human immunodeficiency virus-infected cells", INFECTION AND IMMUNITY, vol. 62, no. 3, 1994, pages 956-960, XP002740867, ISSN: 0019-9567
- HABIBI ROUDKENAR M ET AL: "Selective cytotoxicity of recombinant STXA1-GM-CSF protein in hematopoetic cancer cells", CELL BIOLOGY AND TOXICOLOGY, KLUWER ACADEMIC PUBLISHERS, DO, vol. 22, no. 3, 1 May 2006 (2006-05-01), pages 213-219, XP019392870, ISSN: 1573-6822, DOI: 10.1007/S10565-006-0051-Y
- PASTAN IRA ET AL: "Immunotoxin treatment of cancer", ANNUAL REVIEW OF MEDICINE : SELECTED TOPICS IN THE CLINICALSCIENCES, ANNUAL REVIEWS INC, US, vol. 58, 1 January 2007 (2007-01-01), pages 221-237, XP009134786, ISSN: 0066-4219
- JOHANNES L ET AL: "RETROGRADE TRANSPORT OF KDEL-BEARING B-FRAGMENT OF SHIGA TOXIN", JOURNAL OF BIOLOGICAL CHEMISTRY, AMERICAN SOCIETY FOR BIOCHEMISTRY AND MOLECULAR BIOLOGY, US, vol. 272, no. 31, 1 August 1997 (1997-08-01), pages 19554-19561, XP002061696, ISSN: 0021-9258, DOI: 10.1074/JBC.272.31.19554
- JACKSON MICHELLE E ET AL: "The KDEL retrieval system is exploited by Pseudomonas exotoxin A, but not by Shiga-like toxin-1, during retrograde transport from the Golgi complex to the endoplasmic reticulum", JOURNAL OF CELL SCIENCE, vol. 112, no. 4, February 1999 (1999-02), pages 467-475, XP002741013, ISSN: 0021-9533
- WALES RICHARD ET AL: "Addition of an endoplasmic reticulum retrieval sequence to ricin A chain significantly increases its cytotoxicity to mammalian cells", JOURNAL OF BIOLOGICAL CHEMISTRY, vol. 268, no. 32, 1993, pages 23986-23990, XP002741014, ISSN: 0021-9258

### SEQUENCE LISTING

<110> MOLECULAR TEMPLATES, INC.
<120> PROTEINS COMPRISING BINDING REGIONS, SHIGA TOXIN A SUBUNIT EFFECTOR REGIONS, AND CARBOXY-TERMINAL, ENDOPLASMIC RETICULUM LOCALIZATION SIGNAL MOTIFS
<130> P45254EP-D1-PCT
<140> EP15716202.5
   <141> 2015-03-10
<150> 61/951,110
   <151> 2014-03-11
<160> 110
<170> PatentIn version 3.5
<210> 1
   <211> 293
   <212> PRT
   <213> Escherichia coli
<400> 1
   Lys Glu Phe Thr Leu Asp Phe Ser Thr Ala Lys Thr Tyr Val Asp Ser
   1               5                   10                  15
   Leu Asn Val Ile Arg Ser Ala Ile Gly Thr Pro Leu Gln Thr Ile Ser
               20                  25                  30
   Ser Gly Gly Thr Ser Leu Leu Met Ile Asp Ser Gly Ser Gly Asp Asn
           35                  40                  45
   Leu Phe Ala Val Asp Val Arg Gly Ile Asp Pro Glu Glu Gly Arg Phe
       50                  55                  60
   Asn Asn Leu Arg Leu Ile Val Glu Arg Asn Asn Leu Tyr Val Thr Gly
   65                  70                  75                  80
   Phe Val Asn Arg Thr Asn Asn Val Phe Tyr Arg Phe Ala Asp Phe Ser
                   85                  90                  95
   His Val Thr Phe Pro Gly Thr Thr Ala Val Thr Leu Ser Gly Asp Ser
               100                 105                 110
   Ser Tyr Thr Thr Leu Gln Arg Val Ala Gly Ile Ser Arg Thr Gly Met
           115                 120                 125
   Gln Ile Asn Arg His Ser Leu Thr Thr Ser Tyr Leu Asp Leu Met Ser
       130                 135                 140
   His Ser Gly Thr Ser Leu Thr Gln Ser Val Ala Arg Ala Met Leu Arg
   145                 150                 155                 160
   Phe Val Thr Val Thr Ala Glu Ala Leu Arg Phe Arg Gln Ile Gln Arg
                   165                 170                 175
   Gly Phe Arg Thr Thr Leu Asp Asp Leu Ser Gly Arg Ser Tyr Val Met
               180                 185                 190
   Thr Ala Glu Asp Val Asp Leu Thr Leu Asn Trp Gly Arg Leu Ser Ser
           195                 200                 205
   Val Leu Pro Asp Tyr His Gly Gln Asp Ser Val Arg Val Gly Arg Ile
       210                 215                 220
   Ser Phe Gly Ser Ile Asn Ala Ile Leu Gly Ser Val Ala Leu Ile Leu
   225                 230                 235                 240
   Asn Cys His His His Ala Ser Arg Val Ala Arg Met Ala Ser Asp Glu
                   245                 250                 255
   Phe Pro Ser Met Cys Pro Ala Asp Gly Arg Val Arg Gly Ile Thr His
               260                 265                 270
   Asn Lys Ile Leu Trp Asp Ser Ser Thr Leu Gly Ala Ile Leu Met Arg
           275                 280                 285
   Arg Thr Ile Ser Ser
       290
<210> 2
   <211> 293
   <212> PRT
   <213> Shigella dysenteriae
<400> 2
   Lys Glu Phe Thr Leu Asp Phe Ser Thr Ala Lys Thr Tyr Val Asp Ser
   1               5                   10                  15
   Leu Asn Val Ile Arg Ser Ala Ile Gly Thr Pro Leu Gln Thr Ile Ser
               20                  25                  30
   Ser Gly Gly Thr Ser Leu Leu Met Ile Asp Ser Gly Thr Gly Asp Asn
           35                  40                  45
   Leu Phe Ala Val Asp Val Arg Gly Ile Asp Pro Glu Glu Gly Arg Phe
       50                  55                  60
   Asn Asn Leu Arg Leu Ile Val Glu Arg Asn Asn Leu Tyr Val Thr Gly
   65                  70                  75                  80
   Phe Val Asn Arg Thr Asn Asn Val Phe Tyr Arg Phe Ala Asp Phe Ser
                   85                  90                  95
   His Val Thr Phe Pro Gly Thr Thr Ala Val Thr Leu Ser Gly Asp Ser
               100                 105                 110
   Ser Tyr Thr Thr Leu Gln Arg Val Ala Gly Ile Ser Arg Thr Gly Met
           115                 120                 125
   Gln Ile Asn Arg His Ser Leu Thr Thr Ser Tyr Leu Asp Leu Met Ser
       130                 135                 140
   His Ser Gly Thr Ser Leu Thr Gln Ser Val Ala Arg Ala Met Leu Arg
   145                 150                 155                 160
   Phe Val Thr Val Thr Ala Glu Ala Leu Arg Phe Arg Gln Ile Gln Arg
                   165                 170                 175
   Gly Phe Arg Thr Thr Leu Asp Asp Leu Ser Gly Arg Ser Tyr Val Met
               180                 185                 190
   Thr Ala Glu Asp Val Asp Leu Thr Leu Asn Trp Gly Arg Leu Ser Ser
           195                 200                 205
   Val Leu Pro Asp Tyr His Gly Gln Asp Ser Val Arg Val Gly Arg Ile
       210                 215                 220
   Ser Phe Gly Ser Ile Asn Ala Ile Leu Gly Ser Val Ala Leu Ile Leu
   225                 230                 235                 240
   Asn Cys His His His Ala Ser Arg Val Ala Arg Met Ala Ser Asp Glu
                   245                 250                 255
   Phe Pro Ser Met Cys Pro Ala Asp Gly Arg Val Arg Gly Ile Thr His
               260                 265                 270
   Asn Lys Ile Leu Trp Asp Ser Ser Thr Leu Gly Ala Ile Leu Met Arg
           275                 280                 285
   Arg Thr Ile Ser Ser
       290
<210> 3
   <211> 297
   <212> PRT
   <213> Escherichia coli
<400> 3
   Asp Glu Phe Thr Val Asp Phe Ser Ser Gln Lys Ser Tyr Val Asp Ser
   1               5                   10                  15
   Leu Asn Ser Ile Arg Ser Ala Ile Ser Thr Pro Leu Gly Asn Ile Ser
               20                  25                  30
   Gln Gly Gly Val Ser Val Ser Val Ile Asn His Val Leu Gly Gly Asn
           35                  40                  45
   Tyr Ile Ser Leu Asn Val Arg Gly Leu Asp Pro Tyr Ser Glu Arg Phe
       50                  55                  60
   Asn His Leu Arg Leu Ile Met Glu Arg Asn Asn Leu Tyr Val Ala Gly
   65                  70                  75                  80
   Phe Ile Asn Thr Glu Thr Asn Ile Phe Tyr Arg Phe Ser Asp Phe Ser
                   85                  90                  95
   His Ile Ser Val Pro Asp Val Ile Thr Val Ser Met Thr Thr Asp Ser
               100                 105                 110
   Ser Tyr Ser Ser Leu Gln Arg Ile Ala Asp Leu Glu Arg Thr Gly Met
           115                 120                 125
   Gln Ile Gly Arg His Ser Leu Val Gly Ser Tyr Leu Asp Leu Met Glu
       130                 135                 140
   Phe Arg Gly Arg Ser Met Thr Arg Ala Ser Ser Arg Ala Met Leu Arg
   145                 150                 155                 160
   Phe Val Thr Val Ile Ala Glu Ala Leu Arg Phe Arg Gln Ile Gln Arg
                   165                 170                 175
   Gly Phe Arg Pro Ala Leu Ser Glu Ala Ser Pro Leu Tyr Thr Met Thr
               180                 185                 190
   Ala Gln Asp Val Asp Leu Thr Leu Asn Trp Gly Arg Ile Ser Asn Val
           195                 200                 205
   Leu Pro Glu Tyr Arg Gly Glu Glu Gly Val Arg Ile Gly Arg Ile Ser
       210                 215                 220
   Phe Asn Ser Leu Ser Ala Ile Leu Gly Ser Val Ala Val Ile Leu Asn
   225                 230                 235                 240
   Cys His Ser Thr Gly Ser Tyr Ser Val Arg Ser Val Ser Gln Lys Gln
                   245                 250                 255
   Lys Thr Glu Cys Gln Ile Val Gly Asp Arg Ala Ala Ile Lys Val Asn
   260 265 270
   Asn Val Leu Trp Glu Ala Asn Thr Ile Ala Ala Leu Leu Asn Arg Lys
   275 280 285
   Pro Gln Asp Leu Thr Glu Pro Asn Gln
   290 295
<210> 4
   <211> 516
   <212> PRT
   <213> Artificial Sequence
<220>
   <221> source
   <223> /note="Description of Artificial Sequence: Synthetic polypeptide"
<400> 4
   Met Asp Ile Gln Met Thr Gln Ser Pro Ser Ser Leu Ser Ala Ser Val
   1 5 10 15
   Gly Asp Arg Val Thr Ile Thr Cys Arg Ala Ser Gln Asp Val Asn Thr
   20 25 30
   Ala Val Ala Trp Tyr Gln Gln Lys Pro Gly Lys Ala Pro Lys Leu Leu
   35 40 45
   Ile Tyr Ser Ala Ser Phe Leu Tyr Ser Gly Val Pro Ser Arg Phe Ser
   50 55 60
   Gly Ser Arg Ser Gly Thr Asp Phe Thr Leu Thr Ile Ser Ser Leu Gln
   65 70 75 80
   Pro Glu Asp Phe Ala Thr Tyr Tyr Cys Gln Gln His Tyr Thr Thr Pro
   85 90 95
   Pro Thr Phe Gly Gln Gly Thr Lys Val Glu Ile Lys Arg Thr Gly Ser
   100 105 110
   Thr Ser Gly Ser Gly Lys Pro Gly Ser Gly Glu Gly Ser Glu Val Gln
   115 120 125
   Leu Val Glu Ser Gly Gly Gly Leu Val Gln Pro Gly Gly Ser Leu Arg
   130 135 140
   Leu Ser Cys Ala Ala Ser Gly Phe Asn Ile Lys Asp Thr Tyr Ile His
   145 150 155 160
   Trp Val Arg Gln Ala Pro Gly Lys Gly Leu Glu Trp Val Ala Arg Ile
   165 170 175
   Tyr Pro Thr Asn Gly Tyr Thr Arg Tyr Ala Asp Ser Val Lys Gly Arg
   180 185 190
   Phe Thr Ile Ser Ala Asp Thr Ser Lys Asn Thr Ala Tyr Leu Gln Met
   195 200 205
   Asn Ser Leu Arg Ala Glu Asp Thr Ala Val Tyr Tyr Cys Ser Arg Trp
   210 215 220
   Gly Gly Asp Gly Phe Tyr Ala Met Asp Val Trp Gly Gln Gly Thr Leu
   225 230 235 240
   Val Thr Val Ser Ser Glu Phe Pro Lys Pro Ser Thr Pro Pro Gly Ser
   245 250 255
   Ser Gly Gly Ala Pro Lys Glu Phe Thr Leu Asp Phe Ser Thr Ala Lys
   260 265 270
   Thr Tyr Val Asp Ser Leu Asn Val Ile Arg Ser Ala Ile Gly Thr Pro
   275 280 285
   Leu Gln Thr Ile Ser Ser Gly Gly Thr Ser Leu Leu Met Ile Asp Ser
   290 295 300
   Gly Ser Gly Asp Asn Leu Phe Ala Val Asp Val Arg Gly Ile Asp Pro
   305 310 315 320
   Glu Glu Gly Arg Phe Asn Asn Leu Arg Leu Ile Val Glu Arg Asn Asn
   325 330 335
   Leu Tyr Val Thr Gly Phe Val Asn Arg Thr Asn Asn Val Phe Tyr Arg
   340 345 350
   Phe Ala Asp Phe Ser His Val Thr Phe Pro Gly Thr Thr Ala Val Thr
   355 360 365
   Leu Ser Gly Asp Ser Ser Tyr Thr Thr Leu Gln Arg Val Ala Gly Ile
   370 375 380
   Ser Arg Thr Gly Met Gln Ile Asn Arg His Ser Leu Thr Thr Ser Tyr
   385 390 395 400
   Leu Asp Leu Met Ser His Ser Gly Thr Ser Leu Thr Gln Ser Val Ala
   405 410 415
   Arg Ala Met Leu Arg Phe Val Thr Val Thr Ala Glu Ala Leu Arg Phe
   420 425 430
   Arg Gln Ile Gln Arg Gly Phe Arg Thr Thr Leu Asp Asp Leu Ser Gly
   435 440 445
   Arg Ser Tyr Val Met Thr Ala Glu Asp Val Asp Leu Thr Leu Asn Trp
   450 455 460
   Gly Arg Leu Ser Ser Val Leu Pro Asp Tyr His Gly Gln Asp Ser Val
   465 470 475 480
   Arg Val Gly Arg Ile Ser Phe Gly Ser Ile Asn Ala Ile Leu Gly Ser
   485 490 495
   Val Ala Leu Ile Leu Asn Cys His His His Ala Ser Arg Val Ala Arg
   500 505 510
   Lys Asp Glu Leu
   515
<210> 5
   <211> 525
   <212> PRT
   <213> Artificial Sequence
<220>
   <221> source
   <223> /note="Description of Artificial Sequence: Synthetic polypeptide"
<400> 5
   Met Asp Ile Gln Met Thr Gln Ser Pro Ser Ser Leu Ser Ala Ser Val
   1               5                   10                  15
   Gly Asp Arg Val Thr Ile Thr Cys Arg Ala Ser Gln Asp Val Asn Thr
               20                  25                  30
   Ala Val Ala Trp Tyr Gln Gln Lys Pro Gly Lys Ala Pro Lys Leu Leu
           35                  40                  45
   Ile Tyr Ser Ala Ser Phe Leu Tyr Ser Gly Val Pro Ser Arg Phe Ser
       50                  55                  60
   Gly Ser Arg Ser Gly Thr Asp Phe Thr Leu Thr Ile Ser Ser Leu Gln
   65                  70                  75                  80
   Pro Glu Asp Phe Ala Thr Tyr Tyr Cys Gln Gln His Tyr Thr Thr Pro
                   85                  90                  95
   Pro Thr Phe Gly Gln Gly Thr Lys Val Glu Ile Lys Arg Thr Gly Ser
               100                 105                 110
   Thr Ser Gly Ser Gly Lys Pro Gly Ser Gly Glu Gly Ser Glu Val Gln
           115                 120                 125
   Leu Val Glu Ser Gly Gly Gly Leu Val Gln Pro Gly Gly Ser Leu Arg
       130                 135                 140
   Leu Ser Cys Ala Ala Ser Gly Phe Asn Ile Lys Asp Thr Tyr Ile His
   145                 150                 155                 160
   Trp Val Arg Gln Ala Pro Gly Lys Gly Leu Glu Trp Val Ala Arg Ile
                   165                 170                 175
   Tyr Pro Thr Asn Gly Tyr Thr Arg Tyr Ala Asp Ser Val Lys Gly Arg
               180                 185                 190
   Phe Thr Ile Ser Ala Asp Thr Ser Lys Asn Thr Ala Tyr Leu Gln Met
           195                 200                 205
   Asn Ser Leu Arg Ala Glu Asp Thr Ala Val Tyr Tyr Cys Ser Arg Trp
       210                 215                 220
   Gly Gly Asp Gly Phe Tyr Ala Met Asp Val Trp Gly Gln Gly Thr Leu
   225                 230                 235                 240
   Val Thr Val Ser Ser Glu Phe Pro Lys Pro Ser Thr Pro Pro Gly Ser
                   245                 250                 255
   Ser Gly Gly Ala Pro Gly Ile Leu Gly Phe Val Phe Thr Leu Lys Glu
               260                 265                 270
   Phe Thr Leu Asp Phe Ser Thr Ala Lys Thr Tyr Val Asp Ser Leu Asn
           275                 280                 285
   Val Ile Arg Ser Ala Ile Gly Thr Pro Leu Gln Thr Ile Ser Ser Gly
       290                 295                 300
   Gly Thr Ser Leu Leu Met Ile Asp Ser Gly Ser Gly Asp Asn Leu Phe
   305                 310                 315                 320
   Ala Val Asp Val Arg Gly Ile Asp Pro Glu Glu Gly Arg Phe Asn Asn
                   325                 330                 335
   Leu Arg Leu Ile Val Glu Arg Asn Asn Leu Tyr Val Thr Gly Phe Val
   340 345 350
   Asn Arg Thr Asn Asn Val Phe Tyr Arg Phe Ala Asp Phe Ser His Val
   355 360 365
   Thr Phe Pro Gly Thr Thr Ala Val Thr Leu Ser Gly Asp Ser Ser Tyr
   370 375 380
   Thr Thr Leu Gln Arg Val Ala Gly Ile Ser Arg Thr Gly Met Gln Ile
   385 390 395 400
   Asn Arg His Ser Leu Thr Thr Ser Tyr Leu Asp Leu Met Ser His Ser
   405 410 415
   Gly Thr Ser Leu Thr Gln Ser Val Ala Arg Ala Met Leu Arg Phe Val
   420 425 430
   Thr Val Thr Ala Glu Ala Leu Arg Phe Arg Gln Ile Gln Arg Gly Phe
   435 440 445
   Arg Thr Thr Leu Asp Asp Leu Ser Gly Arg Ser Tyr Val Met Thr Ala
   450 455 460
   Glu Asp Val Asp Leu Thr Leu Asn Trp Gly Arg Leu Ser Ser Val Leu
   465 470 475 480
   Pro Asp Tyr His Gly Gln Asp Ser Val Arg Val Gly Arg Ile Ser Phe
   485 490 495
   Gly Ser Ile Asn Ala Ile Leu Gly Ser Val Ala Leu Ile Leu Asn Cys
   500 505 510
   His His His Ala Ser Arg Val Ala Arg Lys Asp Glu Leu
   515 520 525
<210> 6
   <211> 516
   <212> PRT
   <213> Artificial Sequence
<220>
   <221> source
   <223> /note="Description of Artificial Sequence: Synthetic polypeptide"
<400> 6
   Met Asp Ile Gln Met Thr Gln Ser Pro Ser Ser Leu Ser Ala Ser Val
   1 5 10 15
   Gly Asp Arg Val Thr Ile Thr Cys Arg Ala Ser Gln Asp Val Asn Thr
               20                  25                  30
   Ala Val Ala Trp Tyr Gln Gln Lys Pro Gly Lys Ala Pro Lys Leu Leu
           35                  40                  45
   Ile Tyr Ser Ala Ser Phe Leu Tyr Ser Gly Val Pro Ser Arg Phe Ser
       50                  55                  60
   Gly Ser Arg Ser Gly Thr Asp Phe Thr Leu Thr Ile Ser Ser Leu Gln
   65                  70                  75                  80
   Pro Glu Asp Phe Ala Thr Tyr Tyr Cys Gln Gln His Tyr Thr Thr Pro
                   85                  90                  95
   Pro Thr Phe Gly Gln Gly Thr Lys Val Glu Ile Lys Arg Thr Gly Ser
               100                 105                 110
   Thr Ser Gly Ser Gly Lys Pro Gly Ser Gly Glu Gly Ser Glu Val Gln
           115                 120                 125
   Leu Val Glu Ser Gly Gly Gly Leu Val Gln Pro Gly Gly Ser Leu Arg
       130                 135                 140
   Leu Ser Cys Ala Ala Ser Gly Phe Asn Ile Lys Asp Thr Tyr Ile His
   145                 150                 155                 160
   Trp Val Arg Gln Ala Pro Gly Lys Gly Leu Glu Trp Val Ala Arg Ile
                   165                 170                 175
   Tyr Pro Thr Asn Gly Tyr Thr Arg Tyr Ala Asp Ser Val Lys Gly Arg
               180                 185                 190
   Phe Thr Ile Ser Ala Asp Thr Ser Lys Asn Thr Ala Tyr Leu Gln Met
           195                 200                 205
   Asn Ser Leu Arg Ala Glu Asp Thr Ala Val Tyr Tyr Cys Ser Arg Trp
       210                 215                 220
   Gly Gly Asp Gly Phe Tyr Ala Met Asp Val Trp Gly Gln Gly Thr Leu
   225                 230                 235                 240
   Val Thr Val Ser Ser Glu Phe Pro Lys Pro Ser Thr Pro Pro Gly Ser
                   245                 250                 255
   Ser Gly Gly Ala Pro Lys Glu Phe Thr Leu Asp Phe Ser Thr Ala Lys
               260                 265                 270
   Thr Tyr Val Asp Ser Leu Asn Val Ile Arg Ser Ala Ile Gly Thr Pro
           275                 280                 285
   Leu Gln Thr Ile Ser Ser Gly Gly Thr Ser Leu Leu Met Ile Asp Ser
       290                 295                 300
   Gly Ser Gly Asp Asn Leu Phe Ala Val Asp Val Arg Gly Ile Asp Pro
   305                 310                 315                 320
   Glu Glu Gly Arg Phe Asn Asn Leu Arg Leu Ile Val Glu Arg Asn Asn
                   325                 330                 335
   Leu Tyr Val Thr Gly Phe Val Asn Arg Thr Asn Asn Val Phe Tyr Arg
               340                 345                 350
   Phe Ala Asp Phe Ser His Val Thr Phe Pro Gly Thr Thr Ala Val Thr
           355                 360                 365
   Leu Ser Gly Asp Ser Ser Tyr Thr Thr Leu Gln Arg Val Ala Gly Ile
       370                 375                 380
   Ser Arg Thr Gly Met Gln Ile Asn Arg His Ser Leu Thr Thr Ser Tyr
   385                 390                 395                 400
   Leu Asp Leu Met Ser His Ser Gly Thr Ser Leu Thr Gln Ser Val Ala
                   405                 410                 415
   Arg Ala Met Leu Arg Phe Val Thr Val Thr Ala Glu Ala Leu Arg Phe
               420                 425                 430
   Arg Gln Ile Gln Arg Gly Phe Arg Thr Thr Leu Asp Asp Leu Ser Gly
           435                 440                 445
   Arg Ser Tyr Val Met Thr Ala Glu Asp Val Asp Leu Thr Leu Asn Trp
       450                 455                 460
   Gly Arg Leu Ser Ser Val Leu Pro Asp Tyr His Gly Gln Asp Ser Val
   465                 470                 475                 480
   Arg Val Gly Arg Ile Ser Phe Gly Ser Ile Asn Ala Ile Leu Gly Ser
                   485                 490                 495
   Val Ala Leu Ile Leu Asn Cys His His His Ala Ser Arg Val Ala Arg
               500                 505                 510
   Lys Asp Glu Leu
           515
<210> 7
   <211> 525
   <212> PRT
   <213> Artificial Sequence
<220>
   <221> source
   <223> /note="Description of Artificial Sequence: Synthetic polypeptide"
<400> 7
   Met Asp Ile Gln Met Thr Gln Ser Pro Ser Ser Leu Ser Ala Ser Val
   1               5                   10                  15
   Gly Asp Arg Val Thr Ile Thr Cys Arg Ala Ser Gln Asp Val Asn Thr
               20                  25                  30
   Ala Val Ala Trp Tyr Gln Gln Lys Pro Gly Lys Ala Pro Lys Leu Leu
           35                  40                  45
   Ile Tyr Ser Ala Ser Phe Leu Tyr Ser Gly Val Pro Ser Arg Phe Ser
       50                  55                  60
   Gly Ser Arg Ser Gly Thr Asp Phe Thr Leu Thr Ile Ser Ser Leu Gln
   65                  70                  75                  80
   Pro Glu Asp Phe Ala Thr Tyr Tyr Cys Gln Gln His Tyr Thr Thr Pro
                   85                  90                  95
   Pro Thr Phe Gly Gln Gly Thr Lys Val Glu Ile Lys Arg Thr Gly Ser
               100                 105                 110
   Thr Ser Gly Ser Gly Lys Pro Gly Ser Gly Glu Gly Ser Glu Val Gln
           115                 120                 125
   Leu Val Glu Ser Gly Gly Gly Leu Val Gln Pro Gly Gly Ser Leu Arg
       130                 135                 140
   Leu Ser Cys Ala Ala Ser Gly Phe Asn Ile Lys Asp Thr Tyr Ile His
   145                 150                 155                 160
   Trp Val Arg Gln Ala Pro Gly Lys Gly Leu Glu Trp Val Ala Arg Ile
                   165                 170                 175
   Tyr Pro Thr Asn Gly Tyr Thr Arg Tyr Ala Asp Ser Val Lys Gly Arg
               180                 185                 190
   Phe Thr Ile Ser Ala Asp Thr Ser Lys Asn Thr Ala Tyr Leu Gln Met
           195                 200                 205
   Asn Ser Leu Arg Ala Glu Asp Thr Ala Val Tyr Tyr Cys Ser Arg Trp
       210                 215                 220
   Gly Gly Asp Gly Phe Tyr Ala Met Asp Val Trp Gly Gln Gly Thr Leu
   225                 230                 235                 240
   Val Thr Val Ser Ser Glu Phe Pro Lys Pro Ser Thr Pro Pro Gly Ser
                   245                 250                 255
   Ser Gly Gly Ala Pro Gly Ile Leu Gly Phe Val Phe Thr Leu Lys Glu
               260                 265                 270
   Phe Thr Leu Asp Phe Ser Thr Ala Lys Thr Tyr Val Asp Ser Leu Asn
           275                 280                 285
   Val Ile Arg Ser Ala Ile Gly Thr Pro Leu Gln Thr Ile Ser Ser Gly
       290                 295                 300
   Gly Thr Ser Leu Leu Met Ile Asp Ser Gly Ser Gly Asp Asn Leu Phe
   305                 310                 315                 320
   Ala Val Asp Val Arg Gly Ile Asp Pro Glu Glu Gly Arg Phe Asn Asn
                   325                 330                 335
   Leu Arg Leu Ile Val Glu Arg Asn Asn Leu Tyr Val Thr Gly Phe Val
               340                 345                 350
   Asn Arg Thr Asn Asn Val Phe Tyr Arg Phe Ala Asp Phe Ser His Val
           355                 360                 365
   Thr Phe Pro Gly Thr Thr Ala Val Thr Leu Ser Gly Asp Ser Ser Tyr
       370                 375                 380
   Thr Thr Leu Gln Arg Val Ala Gly Ile Ser Arg Thr Gly Met Gln Ile
   385                 390                 395                 400
   Asn Arg His Ser Leu Thr Thr Ser Tyr Leu Asp Leu Met Ser His Ser
                   405                 410                 415
   Gly Thr Ser Leu Thr Gln Ser Val Ala Arg Ala Met Leu Arg Phe Val
               420                 425                 430
   Thr Val Thr Ala Glu Ala Leu Arg Phe Arg Gln Ile Gln Arg Gly Phe
           435                 440                 445
   Arg Thr Thr Leu Asp Asp Leu Ser Gly Arg Ser Tyr Val Met Thr Ala
   450 455 460
   Glu Asp Val Asp Leu Thr Leu Asn Trp Gly Arg Leu Ser Ser Val Leu
   465 470 475 480
   Pro Asp Tyr His Gly Gln Asp Ser Val Arg Val Gly Arg Ile Ser Phe
   485 490 495
   Gly Ser Ile Asn Ala Ile Leu Gly Ser Val Ala Leu Ile Leu Asn Cys
   500 505 510
   His His His Ala Ser Arg Val Ala Arg Lys Asp Glu Leu
   515 520 525
<210> 8
   <211> 512
   <212> PRT
   <213> Artificial Sequence
<220>
   <221> source
   <223> /note="Description of Artificial Sequence: Synthetic polypeptide"
<400> 8
   Met Asp Ile Glu Leu Thr Gln Ser Pro Ser Ser Phe Ser Val Ser Leu
   1 5 10 15
   Gly Asp Arg Val Thr Ile Thr Cys Lys Ala Ser Glu Asp Ile Tyr Asn
   20 25 30
   Arg Leu Ala Trp Tyr Gln Gln Lys Pro Gly Asn Ala Pro Arg Leu Leu
   35 40 45
   Ile Ser Gly Ala Thr Ser Leu Glu Thr Gly Val Pro Ser Arg Phe Ser
   50 55 60
   Gly Ser Gly Ser Gly Lys Asp Tyr Thr Leu Ser Ile Thr Ser Leu Gln
   65 70 75 80
   Thr Glu Asp Val Ala Thr Tyr Tyr Cys Gln Gln Tyr Trp Ser Thr Pro
   85 90 95
   Thr Phe Gly Gly Gly Thr Lys Leu Glu Ile Lys Gly Ser Thr Ser Gly
   100 105 110
   Ser Gly Lys Pro Gly Ser Gly Glu Gly Ser Lys Val Gln Leu Gln Glu
   115 120 125
   Ser Gly Pro Ser Leu Val Gln Pro Ser Gln Arg Leu Ser Ile Thr Cys
   130 135 140
   Thr Val Ser Gly Phe Ser Leu Ile Ser Tyr Gly Val His Trp Val Arg
   145 150 155 160
   Gln Ser Pro Gly Lys Gly Leu Glu Trp Leu Gly Val Ile Trp Arg Gly
   165 170 175
   Gly Ser Thr Asp Tyr Asn Ala Ala Phe Met Ser Arg Leu Ser Ile Thr
   180 185 190
   Lys Asp Asn Ser Lys Ser Gln Val Phe Phe Lys Met Asn Ser Leu Gln
   195 200 205
   Ala Asp Asp Thr Ala Ile Tyr Phe Cys Ala Lys Thr Leu Ile Thr Thr
   210 215 220
   Gly Tyr Ala Met Asp Tyr Trp Gly Gln Gly Thr Thr Val Thr Val Ser
   225 230 235 240
   Ser Glu Phe Pro Lys Pro Ser Thr Pro Pro Gly Ser Ser Gly Gly Ala
   245 250 255
   Pro Lys Glu Phe Thr Leu Asp Phe Ser Thr Ala Lys Thr Tyr Val Asp
   260 265 270
   Ser Leu Asn Val Ile Arg Ser Ala Ile Gly Thr Pro Leu Gln Thr Ile
   275 280 285
   Ser Ser Gly Gly Thr Ser Leu Leu Met Ile Asp Ser Gly Ser Gly Asp
   290 295 300
   Asn Leu Phe Ala Val Asp Val Arg Gly Ile Asp Pro Glu Glu Gly Arg
   305 310 315 320
   Phe Asn Asn Leu Arg Leu Ile Val Glu Arg Asn Asn Leu Tyr Val Thr
   325 330 335
   Gly Phe Val Asn Arg Thr Asn Asn Val Phe Tyr Arg Phe Ala Asp Phe
   340 345 350
   Ser His Val Thr Phe Pro Gly Thr Thr Ala Val Thr Leu Ser Gly Asp
   355 360 365
   Ser Ser Tyr Thr Thr Leu Gln Arg Val Ala Gly Ile Ser Arg Thr Gly
   370 375 380
   Met Gln Ile Asn Arg His Ser Leu Thr Thr Ser Tyr Leu Asp Leu Met
   385 390 395 400
   Ser His Ser Gly Thr Ser Leu Thr Gln Ser Val Ala Arg Ala Met Leu
   405 410 415
   Arg Phe Val Thr Val Thr Ala Glu Ala Leu Arg Phe Arg Gln Ile Gln
   420 425 430
   Arg Gly Phe Arg Thr Thr Leu Asp Asp Leu Ser Gly Arg Ser Tyr Val
   435 440 445
   Met Thr Ala Glu Asp Val Asp Leu Thr Leu Asn Trp Gly Arg Leu Ser
   450 455 460
   Ser Val Leu Pro Asp Tyr His Gly Gln Asp Ser Val Arg Val Gly Arg
   465 470 475 480
   Ile Ser Phe Gly Ser Ile Asn Ala Ile Leu Gly Ser Val Ala Leu Ile
   485 490 495
   Leu Asn Cys His His His Ala Ser Arg Val Ala Arg Lys Asp Glu Leu
   500 505 510
<210> 9
   <211> 521
   <212> PRT
   <213> Artificial Sequence
<220>
   <221> source
   <223> /note="Description of Artificial Sequence: Synthetic polypeptide"
<400> 9
   Met Asp Ile Glu Leu Thr Gln Ser Pro Ser Ser Phe Ser Val Ser Leu
   1 5 10 15
   Gly Asp Arg Val Thr Ile Thr Cys Lys Ala Ser Glu Asp Ile Tyr Asn
   20 25 30
   Arg Leu Ala Trp Tyr Gln Gln Lys Pro Gly Asn Ala Pro Arg Leu Leu
   35 40 45
   Ile Ser Gly Ala Thr Ser Leu Glu Thr Gly Val Pro Ser Arg Phe Ser
   50 55 60
   Gly Ser Gly Ser Gly Lys Asp Tyr Thr Leu Ser Ile Thr Ser Leu Gln
   65 70 75 80
   Thr Glu Asp Val Ala Thr Tyr Tyr Cys Gln Gln Tyr Trp Ser Thr Pro
   85 90 95
   Thr Phe Gly Gly Gly Thr Lys Leu Glu Ile Lys Gly Ser Thr Ser Gly
   100 105 110
   Ser Gly Lys Pro Gly Ser Gly Glu Gly Ser Lys Val Gln Leu Gln Glu
   115 120 125
   Ser Gly Pro Ser Leu Val Gln Pro Ser Gln Arg Leu Ser Ile Thr Cys
   130 135 140
   Thr Val Ser Gly Phe Ser Leu Ile Ser Tyr Gly Val His Trp Val Arg
   145 150 155 160
   Gln Ser Pro Gly Lys Gly Leu Glu Trp Leu Gly Val Ile Trp Arg Gly
   165 170 175
   Gly Ser Thr Asp Tyr Asn Ala Ala Phe Met Ser Arg Leu Ser Ile Thr
   180 185 190
   Lys Asp Asn Ser Lys Ser Gln Val Phe Phe Lys Met Asn Ser Leu Gln
   195 200 205
   Ala Asp Asp Thr Ala Ile Tyr Phe Cys Ala Lys Thr Leu Ile Thr Thr
   210 215 220
   Gly Tyr Ala Met Asp Tyr Trp Gly Gln Gly Thr Thr Val Thr Val Ser
   225 230 235 240
   Ser Glu Phe Pro Lys Pro Ser Thr Pro Pro Gly Ser Ser Gly Gly Ala
   245 250 255
   Pro Gly Ile Leu Gly Phe Val Phe Thr Leu Lys Glu Phe Thr Leu Asp
   260 265 270
   Phe Ser Thr Ala Lys Thr Tyr Val Asp Ser Leu Asn Val Ile Arg Ser
   275 280 285
   Ala Ile Gly Thr Pro Leu Gln Thr Ile Ser Ser Gly Gly Thr Ser Leu
   290 295 300
   Leu Met Ile Asp Ser Gly Ser Gly Asp Asn Leu Phe Ala Val Asp Val
   305 310 315 320
   Arg Gly Ile Asp Pro Glu Glu Gly Arg Phe Asn Asn Leu Arg Leu Ile
   325 330 335
   Val Glu Arg Asn Asn Leu Tyr Val Thr Gly Phe Val Asn Arg Thr Asn
   340 345 350
   Asn Val Phe Tyr Arg Phe Ala Asp Phe Ser His Val Thr Phe Pro Gly
   355 360 365
   Thr Thr Ala Val Thr Leu Ser Gly Asp Ser Ser Tyr Thr Thr Leu Gln
   370 375 380
   Arg Val Ala Gly Ile Ser Arg Thr Gly Met Gln Ile Asn Arg His Ser
   385 390 395 400
   Leu Thr Thr Ser Tyr Leu Asp Leu Met Ser His Ser Gly Thr Ser Leu
   405 410 415
   Thr Gln Ser Val Ala Arg Ala Met Leu Arg Phe Val Thr Val Thr Ala
   420 425 430
   Glu Ala Leu Arg Phe Arg Gln Ile Gln Arg Gly Phe Arg Thr Thr Leu
   435 440 445
   Asp Asp Leu Ser Gly Arg Ser Tyr Val Met Thr Ala Glu Asp Val Asp
   450 455 460
   Leu Thr Leu Asn Trp Gly Arg Leu Ser Ser Val Leu Pro Asp Tyr His
   465 470 475 480
   Gly Gln Asp Ser Val Arg Val Gly Arg Ile Ser Phe Gly Ser Ile Asn
   485 490 495
   Ala Ile Leu Gly Ser Val Ala Leu Ile Leu Asn Cys His His His Ala
   500 505 510
   Ser Arg Val Ala Arg Lys Asp Glu Leu
   515 520
<210> 10
   <211> 512
   <212> PRT
   <213> Artificial Sequence
<220>
   <221> source
   <223> /note="Description of Artificial Sequence: Synthetic polypeptide"
<400> 10
   Met Asp Ile Glu Leu Thr Gln Ser Pro Ser Ser Phe Ser Val Ser Leu
   1 5 10 15
   Gly Asp Arg Val Thr Ile Thr Cys Lys Ala Ser Glu Asp Ile Tyr Asn
               20                  25                  30
   Arg Leu Ala Trp Tyr Gln Gln Lys Pro Gly Asn Ala Pro Arg Leu Leu
           35                  40                  45
   Ile Ser Gly Ala Thr Ser Leu Glu Thr Gly Val Pro Ser Arg Phe Ser
       50                  55                  60
   Gly Ser Gly Ser Gly Lys Asp Tyr Thr Leu Ser Ile Thr Ser Leu Gln
   65                  70                  75                  80
   Thr Glu Asp Val Ala Thr Tyr Tyr Cys Gln Gln Tyr Trp Ser Thr Pro
                   85                  90                  95
   Thr Phe Gly Gly Gly Thr Lys Leu Glu Ile Lys Gly Ser Thr Ser Gly
               100                 105                 110
   Ser Gly Lys Pro Gly Ser Gly Glu Gly Ser Lys Val Gln Leu Gln Glu
           115                 120                 125
   Ser Gly Pro Ser Leu Val Gln Pro Ser Gln Arg Leu Ser Ile Thr Cys
       130                 135                 140
   Thr Val Ser Gly Phe Ser Leu Ile Ser Tyr Gly Val His Trp Val Arg
   145                 150                 155                 160
   Gln Ser Pro Gly Lys Gly Leu Glu Trp Leu Gly Val Ile Trp Arg Gly
                   165                 170                 175
   Gly Ser Thr Asp Tyr Asn Ala Ala Phe Met Ser Arg Leu Ser Ile Thr
               180                 185                 190
   Lys Asp Asn Ser Lys Ser Gln Val Phe Phe Lys Met Asn Ser Leu Gln
           195                 200                 205
   Ala Asp Asp Thr Ala Ile Tyr Phe Cys Ala Lys Thr Leu Ile Thr Thr
       210                 215                 220
   Gly Tyr Ala Met Asp Tyr Trp Gly Gln Gly Thr Thr Val Thr Val Ser
   225                 230                 235                 240
   Ser Glu Phe Pro Lys Pro Ser Thr Pro Pro Gly Ser Ser Gly Gly Ala
                   245                 250                 255
   Pro Lys Glu Phe Thr Leu Asp Phe Ser Thr Ala Lys Thr Tyr Val Asp
               260                 265                 270
   Ser Leu Asn Val Ile Arg Ser Ala Ile Gly Thr Pro Leu Gln Thr Ile
           275                 280                 285
   Ser Ser Gly Gly Thr Ser Leu Leu Met Ile Asp Ser Gly Ser Gly Asp
       290                 295                 300
   Asn Leu Phe Ala Val Asp Val Arg Gly Ile Asp Pro Glu Glu Gly Arg
   305                 310                 315                 320
   Phe Asn Asn Leu Arg Leu Ile Val Glu Arg Asn Asn Leu Tyr Val Thr
                   325                 330                 335
   Gly Phe Val Asn Arg Thr Asn Asn Val Phe Tyr Arg Phe Ala Asp Phe
               340                 345                 350
   Ser His Val Thr Phe Pro Gly Thr Thr Ala Val Thr Leu Ser Gly Asp
           355                 360                 365
   Ser Ser Tyr Thr Thr Leu Gln Arg Val Ala Gly Ile Ser Arg Thr Gly
       370                 375                 380
   Met Gln Ile Asn Arg His Ser Leu Thr Thr Ser Tyr Leu Asp Leu Met
   385                 390                 395                 400
   Ser His Ser Gly Thr Ser Leu Thr Gln Ser Val Ala Arg Ala Met Leu
                   405                 410                 415
   Arg Phe Val Thr Val Thr Ala Glu Ala Leu Arg Phe Arg Gln Ile Gln
               420                 425                 430
   Arg Gly Phe Arg Thr Thr Leu Asp Asp Leu Ser Gly Arg Ser Tyr Val
           435                 440                 445
   Met Thr Ala Glu Asp Val Asp Leu Thr Leu Asn Trp Gly Arg Leu Ser
       450                 455                 460
   Ser Val Leu Pro Asp Tyr His Gly Gln Asp Ser Val Arg Val Gly Arg
   465                 470                 475                 480
   Ile Ser Phe Gly Ser Ile Asn Ala Ile Leu Gly Ser Val Ala Leu Ile
                   485                 490                 495
   Leu Asn Cys His His His Ala Ser Arg Val Ala Arg Lys Asp Glu Leu
               500                 505                 510
<210> 11
   <211> 521
   <212> PRT
   <213> Artificial Sequence
<220>
   <221> source
   <223> /note="Description of Artificial Sequence: Synthetic polypeptide"
<400> 11
   Met Asp Ile Glu Leu Thr Gln Ser Pro Ser Ser Phe Ser Val Ser Leu
   1               5                   10                  15
   Gly Asp Arg Val Thr Ile Thr Cys Lys Ala Ser Glu Asp Ile Tyr Asn
               20                  25                  30
   Arg Leu Ala Trp Tyr Gln Gln Lys Pro Gly Asn Ala Pro Arg Leu Leu
           35                  40                  45
   Ile Ser Gly Ala Thr Ser Leu Glu Thr Gly Val Pro Ser Arg Phe Ser
       50                  55                  60
   Gly Ser Gly Ser Gly Lys Asp Tyr Thr Leu Ser Ile Thr Ser Leu Gln
   65                  70                  75                  80
   Thr Glu Asp Val Ala Thr Tyr Tyr Cys Gln Gln Tyr Trp Ser Thr Pro
                   85                  90                  95
   Thr Phe Gly Gly Gly Thr Lys Leu Glu Ile Lys Gly Ser Thr Ser Gly
               100                 105                 110
   Ser Gly Lys Pro Gly Ser Gly Glu Gly Ser Lys Val Gln Leu Gln Glu
           115                 120                 125
   Ser Gly Pro Ser Leu Val Gln Pro Ser Gln Arg Leu Ser Ile Thr Cys
       130                 135                 140
   Thr Val Ser Gly Phe Ser Leu Ile Ser Tyr Gly Val His Trp Val Arg
   145                 150                 155                 160
   Gln Ser Pro Gly Lys Gly Leu Glu Trp Leu Gly Val Ile Trp Arg Gly
                   165                 170                 175
   Gly Ser Thr Asp Tyr Asn Ala Ala Phe Met Ser Arg Leu Ser Ile Thr
               180                 185                 190
   Lys Asp Asn Ser Lys Ser Gln Val Phe Phe Lys Met Asn Ser Leu Gln
           195                 200                 205
   Ala Asp Asp Thr Ala Ile Tyr Phe Cys Ala Lys Thr Leu Ile Thr Thr
               210                 215                 220
   Gly Tyr Ala Met Asp Tyr Trp Gly Gln Gly Thr Thr Val Thr Val Ser
   225                 230                 235                 240
   Ser Glu Phe Pro Lys Pro Ser Thr Pro Pro Gly Ser Ser Gly Gly Ala
                   245                 250                 255
   Pro Gly Ile Leu Gly Phe Val Phe Thr Leu Lys Glu Phe Thr Leu Asp
               260                 265                 270
   Phe Ser Thr Ala Lys Thr Tyr Val Asp Ser Leu Asn Val Ile Arg Ser
           275                 280                 285
   Ala Ile Gly Thr Pro Leu Gln Thr Ile Ser Ser Gly Gly Thr Ser Leu
       290                 295                 300
   Leu Met Ile Asp Ser Gly Ser Gly Asp Asn Leu Phe Ala Val Asp Val
   305                 310                 315                 320
   Arg Gly Ile Asp Pro Glu Glu Gly Arg Phe Asn Asn Leu Arg Leu Ile
                   325                 330                 335
   Val Glu Arg Asn Asn Leu Tyr Val Thr Gly Phe Val Asn Arg Thr Asn
               340                 345                 350
   Asn Val Phe Tyr Arg Phe Ala Asp Phe Ser His Val Thr Phe Pro Gly
           355                 360                 365
   Thr Thr Ala Val Thr Leu Ser Gly Asp Ser Ser Tyr Thr Thr Leu Gln
       370                 375                 380
   Arg Val Ala Gly Ile Ser Arg Thr Gly Met Gln Ile Asn Arg His Ser
   385                 390                 395                 400
   Leu Thr Thr Ser Tyr Leu Asp Leu Met Ser His Ser Gly Thr Ser Leu
                   405                 410                 415
   Thr Gln Ser Val Ala Arg Ala Met Leu Arg Phe Val Thr Val Thr Ala
               420                 425                 430
   Glu Ala Leu Arg Phe Arg Gln Ile Gln Arg Gly Phe Arg Thr Thr Leu
           435                 440                 445
   Asp Asp Leu Ser Gly Arg Ser Tyr Val Met Thr Ala Glu Asp Val Asp
       450                 455                 460
   Leu Thr Leu Asn Trp Gly Arg Leu Ser Ser Val Leu Pro Asp Tyr His
   465 470 475 480
   Gly Gln Asp Ser Val Arg Val Gly Arg Ile Ser Phe Gly Ser Ile Asn
   485 490 495
   Ala Ile Leu Gly Ser Val Ala Leu Ile Leu Asn Cys His His His Ala
   500 505 510
   Ser Arg Val Ala Arg Lys Asp Glu Leu
   515 520
<210> 12
   <211> 521
   <212> PRT
   <213> Artificial Sequence
<220>
   <221> source
   <223> /note="Description of Artificial Sequence: Synthetic polypeptide"
<400> 12
   Met Asp Ile Val Met Thr Gln Ala Ala Pro Ser Ile Pro Val Thr Pro
   1 5 10 15
   Gly Glu Ser Val Ser Ile Ser Cys Arg Ser Ser Lys Ser Leu Leu Asn
   20 25 30
   Ser Asn Gly Asn Thr Tyr Leu Tyr Trp Phe Leu Gln Arg Pro Gly Gln
   35 40 45
   Ser Pro Gln Leu Leu Ile Tyr Arg Met Ser Asn Leu Ala Ser Gly Val
   50 55 60
   Pro Asp Arg Phe Ser Gly Ser Gly Ser Gly Thr Ala Phe Thr Leu Arg
   65 70 75 80
   Ile Ser Arg Val Glu Ala Glu Asp Val Gly Val Tyr Tyr Cys Met Gln
   85 90 95
   His Leu Glu Tyr Pro Phe Thr Phe Gly Ala Gly Thr Lys Leu Glu Leu
   100 105 110
   Lys Gly Ser Thr Ser Gly Ser Gly Lys Pro Gly Ser Gly Glu Gly Ser
   115 120 125
   Glu Val Gln Leu Gln Gln Ser Gly Pro Glu Leu Ile Lys Pro Gly Ala
   130 135 140
   Ser Val Lys Met Ser Cys Lys Ala Ser Gly Tyr Thr Phe Thr Ser Tyr
   145 150 155 160
   Val Met His Trp Val Lys Gln Lys Pro Gly Gln Gly Leu Glu Trp Ile
   165 170 175
   Gly Tyr Ile Asn Pro Tyr Asn Asp Gly Thr Lys Tyr Asn Glu Lys Phe
   180 185 190
   Lys Gly Lys Ala Thr Leu Thr Ser Asp Lys Ser Ser Ser Thr Ala Tyr
   195 200 205
   Met Glu Leu Ser Ser Leu Thr Ser Glu Asp Ser Ala Val Tyr Tyr Cys
   210 215 220
   Ala Arg Gly Thr Tyr Tyr Tyr Gly Ser Arg Val Phe Asp Tyr Trp Gly
   225 230 235 240
   Gln Gly Thr Thr Leu Thr Val Ser Ser Ala Glu Phe Pro Lys Pro Ser
   245 250 255
   Thr Pro Pro Gly Ser Ser Gly Gly Ala Pro Lys Glu Phe Thr Leu Asp
   260 265 270
   Phe Ser Thr Ala Lys Thr Tyr Val Asp Ser Leu Asn Val Ile Arg Ser
   275 280 285
   Ala Ile Gly Thr Pro Leu Gln Thr Ile Ser Ser Gly Gly Thr Ser Leu
   290 295 300
   Leu Met Ile Asp Ser Gly Ser Gly Asp Asn Leu Phe Ala Val Asp Val
   305 310 315 320
   Arg Gly Ile Asp Pro Glu Glu Gly Arg Phe Asn Asn Leu Arg Leu Ile
   325 330 335
   Val Glu Arg Asn Asn Leu Tyr Val Thr Gly Phe Val Asn Arg Thr Asn
   340 345 350
   Asn Val Phe Tyr Arg Phe Ala Asp Phe Ser His Val Thr Phe Pro Gly
   355 360 365
   Thr Thr Ala Val Thr Leu Ser Gly Asp Ser Ser Tyr Thr Thr Leu Gln
   370 375 380
   Arg Val Ala Gly Ile Ser Arg Thr Gly Met Gln Ile Asn Arg His Ser
   385 390 395 400
   Leu Thr Thr Ser Tyr Leu Asp Leu Met Ser His Ser Gly Thr Ser Leu
                   405                 410                 415
   Thr Gln Ser Val Ala Arg Ala Met Leu Arg Phe Val Thr Val Thr Ala
               420                 425                 430
   Glu Ala Leu Arg Phe Arg Gln Ile Gln Arg Gly Phe Arg Thr Thr Leu
           435                 440                 445
   Asp Asp Leu Ser Gly Arg Ser Tyr Val Met Thr Ala Glu Asp Val Asp
       450                 455                 460
   Leu Thr Leu Asn Trp Gly Arg Leu Ser Ser Val Leu Pro Asp Tyr His
   465                 470                 475                 480
   Gly Gln Asp Ser Val Arg Val Gly Arg Ile Ser Phe Gly Ser Ile Asn
                   485                 490                 495
   Ala Ile Leu Gly Ser Val Ala Leu Ile Leu Asn Cys His His His Ala
               500                 505                 510
   Ser Arg Val Ala Arg Lys Asp Glu Leu
           515                 520
<210> 13
   <211> 530
   <212> PRT
   <213> Artificial Sequence
<220>
   <221> source
   <223> /note="Description of Artificial Sequence: Synthetic polypeptide"
<400> 13
   Met Asp Ile Val Met Thr Gln Ala Ala Pro Ser Ile Pro Val Thr Pro
   1               5                   10                  15
   Gly Glu Ser Val Ser Ile Ser Cys Arg Ser Ser Lys Ser Leu Leu Asn
               20                  25                  30
   Ser Asn Gly Asn Thr Tyr Leu Tyr Trp Phe Leu Gln Arg Pro Gly Gln
           35                  40                  45
   Ser Pro Gln Leu Leu Ile Tyr Arg Met Ser Asn Leu Ala Ser Gly Val
       50                  55                  60
   Pro Asp Arg Phe Ser Gly Ser Gly Ser Gly Thr Ala Phe Thr Leu Arg
   65                  70                  75                  80
   Ile Ser Arg Val Glu Ala Glu Asp Val Gly Val Tyr Tyr Cys Met Gln
   85 90 95
   His Leu Glu Tyr Pro Phe Thr Phe Gly Ala Gly Thr Lys Leu Glu Leu
   100 105 110
   Lys Gly Ser Thr Ser Gly Ser Gly Lys Pro Gly Ser Gly Glu Gly Ser
   115 120 125
   Glu Val Gln Leu Gln Gln Ser Gly Pro Glu Leu Ile Lys Pro Gly Ala
   130 135 140
   Ser Val Lys Met Ser Cys Lys Ala Ser Gly Tyr Thr Phe Thr Ser Tyr
   145 150 155 160
   Val Met His Trp Val Lys Gln Lys Pro Gly Gln Gly Leu Glu Trp Ile
   165 170 175
   Gly Tyr Ile Asn Pro Tyr Asn Asp Gly Thr Lys Tyr Asn Glu Lys Phe
   180 185 190
   Lys Gly Lys Ala Thr Leu Thr Ser Asp Lys Ser Ser Ser Thr Ala Tyr
   195 200 205
   Met Glu Leu Ser Ser Leu Thr Ser Glu Asp Ser Ala Val Tyr Tyr Cys
   210 215 220
   Ala Arg Gly Thr Tyr Tyr Tyr Gly Ser Arg Val Phe Asp Tyr Trp Gly
   225 230 235 240
   Gln Gly Thr Thr Leu Thr Val Ser Ser Ala Glu Phe Pro Lys Pro Ser
   245 250 255
   Thr Pro Pro Gly Ser Ser Gly Gly Ala Pro Gly Ile Leu Gly Phe Val
   260 265 270
   Phe Thr Leu Lys Glu Phe Thr Leu Asp Phe Ser Thr Ala Lys Thr Tyr
   275 280 285
   Val Asp Ser Leu Asn Val Ile Arg Ser Ala Ile Gly Thr Pro Leu Gln
   290 295 300
   Thr Ile Ser Ser Gly Gly Thr Ser Leu Leu Met Ile Asp Ser Gly Ser
   305 310 315 320
   Gly Asp Asn Leu Phe Ala Val Asp Val Arg Gly Ile Asp Pro Glu Glu
   325 330 335
   Gly Arg Phe Asn Asn Leu Arg Leu Ile Val Glu Arg Asn Asn Leu Tyr
   340 345 350
   Val Thr Gly Phe Val Asn Arg Thr Asn Asn Val Phe Tyr Arg Phe Ala
   355 360 365
   Asp Phe Ser His Val Thr Phe Pro Gly Thr Thr Ala Val Thr Leu Ser
   370 375 380
   Gly Asp Ser Ser Tyr Thr Thr Leu Gln Arg Val Ala Gly Ile Ser Arg
   385 390 395 400
   Thr Gly Met Gln Ile Asn Arg His Ser Leu Thr Thr Ser Tyr Leu Asp
   405 410 415
   Leu Met Ser His Ser Gly Thr Ser Leu Thr Gln Ser Val Ala Arg Ala
   420 425 430
   Met Leu Arg Phe Val Thr Val Thr Ala Glu Ala Leu Arg Phe Arg Gln
   435 440 445
   Ile Gln Arg Gly Phe Arg Thr Thr Leu Asp Asp Leu Ser Gly Arg Ser
   450 455 460
   Tyr Val Met Thr Ala Glu Asp Val Asp Leu Thr Leu Asn Trp Gly Arg
   465 470 475 480
   Leu Ser Ser Val Leu Pro Asp Tyr His Gly Gln Asp Ser Val Arg Val
   485 490 495
   Gly Arg Ile Ser Phe Gly Ser Ile Asn Ala Ile Leu Gly Ser Val Ala
   500 505 510
   Leu Ile Leu Asn Cys His His His Ala Ser Arg Val Ala Arg Lys Asp
   515 520 525
   Glu Leu
   530
<210> 14
   <211> 521
   <212> PRT
   <213> Artificial Sequence
<220>
   <221> source
   <223> /note="Description of Artificial Sequence: Synthetic polypeptide"
<400> 14
   Met Asp Ile Val Met Thr Gln Ala Ala Pro Ser Ile Pro Val Thr Pro
   1               5                   10                  15
   Gly Glu Ser Val Ser Ile Ser Cys Arg Ser Ser Lys Ser Leu Leu Asn
               20                  25                  30
   Ser Asn Gly Asn Thr Tyr Leu Tyr Trp Phe Leu Gln Arg Pro Gly Gln
           35                  40                  45
   Ser Pro Gln Leu Leu Ile Tyr Arg Met Ser Asn Leu Ala Ser Gly Val
       50                  55                  60
   Pro Asp Arg Phe Ser Gly Ser Gly Ser Gly Thr Ala Phe Thr Leu Arg
   65                  70                  75                  80
   Ile Ser Arg Val Glu Ala Glu Asp Val Gly Val Tyr Tyr Cys Met Gln
                   85                  90                  95
   His Leu Glu Tyr Pro Phe Thr Phe Gly Ala Gly Thr Lys Leu Glu Leu
               100                 105                 110
   Lys Gly Ser Thr Ser Gly Ser Gly Lys Pro Gly Ser Gly Glu Gly Ser
           115                 120                 125
   Glu Val Gln Leu Gln Gln Ser Gly Pro Glu Leu Ile Lys Pro Gly Ala
       130                 135                 140
   Ser Val Lys Met Ser Cys Lys Ala Ser Gly Tyr Thr Phe Thr Ser Tyr
   145                 150                 155                 160
   Val Met His Trp Val Lys Gln Lys Pro Gly Gln Gly Leu Glu Trp Ile
                   165                 170                 175
   Gly Tyr Ile Asn Pro Tyr Asn Asp Gly Thr Lys Tyr Asn Glu Lys Phe
               180                 185                 190
   Lys Gly Lys Ala Thr Leu Thr Ser Asp Lys Ser Ser Ser Thr Ala Tyr
           195                 200                 205
   Met Glu Leu Ser Ser Leu Thr Ser Glu Asp Ser Ala Val Tyr Tyr Cys
       210                 215                 220
   Ala Arg Gly Thr Tyr Tyr Tyr Gly Ser Arg Val Phe Asp Tyr Trp Gly
   225                 230                 235                 240
   Gln Gly Thr Thr Leu Thr Val Ser Ser Ala Glu Phe Pro Lys Pro Ser
   245 250 255
   Thr Pro Pro Gly Ser Ser Gly Gly Ala Pro Lys Glu Phe Thr Leu Asp
   260 265 270
   Phe Ser Thr Ala Lys Thr Tyr Val Asp Ser Leu Asn Val Ile Arg Ser
   275 280 285
   Ala Ile Gly Thr Pro Leu Gln Thr Ile Ser Ser Gly Gly Thr Ser Leu
   290 295 300
   Leu Met Ile Asp Ser Gly Ser Gly Asp Asn Leu Phe Ala Val Asp Val
   305 310 315 320
   Arg Gly Ile Asp Pro Glu Glu Gly Arg Phe Asn Asn Leu Arg Leu Ile
   325 330 335
   Val Glu Arg Asn Asn Leu Tyr Val Thr Gly Phe Val Asn Arg Thr Asn
   340 345 350
   Asn Val Phe Tyr Arg Phe Ala Asp Phe Ser His Val Thr Phe Pro Gly
   355 360 365
   Thr Thr Ala Val Thr Leu Ser Gly Asp Ser Ser Tyr Thr Thr Leu Gln
   370 375 380
   Arg Val Ala Gly Ile Ser Arg Thr Gly Met Gln Ile Asn Arg His Ser
   385 390 395 400
   Leu Thr Thr Ser Tyr Leu Asp Leu Met Ser His Ser Gly Thr Ser Leu
   405 410 415
   Thr Gln Ser Val Ala Arg Ala Met Leu Arg Phe Val Thr Val Thr Ala
   420 425 430
   Glu Ala Leu Arg Phe Arg Gln Ile Gln Arg Gly Phe Arg Thr Thr Leu
   435 440 445
   Asp Asp Leu Ser Gly Arg Ser Tyr Val Met Thr Ala Glu Asp Val Asp
   450 455 460
   Leu Thr Leu Asn Trp Gly Arg Leu Ser Ser Val Leu Pro Asp Tyr His
   465 470 475 480
   Gly Gln Asp Ser Val Arg Val Gly Arg Ile Ser Phe Gly Ser Ile Asn
   485 490 495
   Ala Ile Leu Gly Ser Val Ala Leu Ile Leu Asn Cys His His His Ala
   500 505 510
   Ser Arg Val Ala Arg Lys Asp Glu Leu
   515 520
<210> 15
   <211> 530
   <212> PRT
   <213> Artificial Sequence
<220>
   <221> source
   <223> /note="Description of Artificial Sequence: Synthetic polypeptide"
<400> 15
   Met Asp Ile Val Met Thr Gln Ala Ala Pro Ser Ile Pro Val Thr Pro
   1 5 10 15
   Gly Glu Ser Val Ser Ile Ser Cys Arg Ser Ser Lys Ser Leu Leu Asn
   20 25 30
   Ser Asn Gly Asn Thr Tyr Leu Tyr Trp Phe Leu Gln Arg Pro Gly Gln
   35 40 45
   Ser Pro Gln Leu Leu Ile Tyr Arg Met Ser Asn Leu Ala Ser Gly Val
   50 55 60
   Pro Asp Arg Phe Ser Gly Ser Gly Ser Gly Thr Ala Phe Thr Leu Arg
   65 70 75 80
   Ile Ser Arg Val Glu Ala Glu Asp Val Gly Val Tyr Tyr Cys Met Gln
   85 90 95
   His Leu Glu Tyr Pro Phe Thr Phe Gly Ala Gly Thr Lys Leu Glu Leu
   100 105 110
   Lys Gly Ser Thr Ser Gly Ser Gly Lys Pro Gly Ser Gly Glu Gly Ser
   115 120 125
   Glu Val Gln Leu Gln Gln Ser Gly Pro Glu Leu Ile Lys Pro Gly Ala
   130 135 140
   Ser Val Lys Met Ser Cys Lys Ala Ser Gly Tyr Thr Phe Thr Ser Tyr
   145 150 155 160
   Val Met His Trp Val Lys Gln Lys Pro Gly Gln Gly Leu Glu Trp Ile
   165 170 175
   Gly Tyr Ile Asn Pro Tyr Asn Asp Gly Thr Lys Tyr Asn Glu Lys Phe
               180                 185                 190
   Lys Gly Lys Ala Thr Leu Thr Ser Asp Lys Ser Ser Ser Thr Ala Tyr
           195                 200                 205
   Met Glu Leu Ser Ser Leu Thr Ser Glu Asp Ser Ala Val Tyr Tyr Cys
       210                 215                 220
   Ala Arg Gly Thr Tyr Tyr Tyr Gly Ser Arg Val Phe Asp Tyr Trp Gly
   225                 230                 235                 240
   Gln Gly Thr Thr Leu Thr Val Ser Ser Ala Glu Phe Pro Lys Pro Ser
                   245                 250                 255
   Thr Pro Pro Gly Ser Ser Gly Gly Ala Pro Gly Ile Leu Gly Phe Val
               260                 265                 270
   Phe Thr Leu Lys Glu Phe Thr Leu Asp Phe Ser Thr Ala Lys Thr Tyr
           275                 280                 285
   Val Asp Ser Leu Asn Val Ile Arg Ser Ala Ile Gly Thr Pro Leu Gln
       290                 295                 300
   Thr Ile Ser Ser Gly Gly Thr Ser Leu Leu Met Ile Asp Ser Gly Ser
   305                 310                 315                 320
   Gly Asp Asn Leu Phe Ala Val Asp Val Arg Gly Ile Asp Pro Glu Glu
                   325                 330                 335
   Gly Arg Phe Asn Asn Leu Arg Leu Ile Val Glu Arg Asn Asn Leu Tyr
               340                 345                 350
   Val Thr Gly Phe Val Asn Arg Thr Asn Asn Val Phe Tyr Arg Phe Ala
           355                 360                 365
   Asp Phe Ser His Val Thr Phe Pro Gly Thr Thr Ala Val Thr Leu Ser
       370                 375                 380
   Gly Asp Ser Ser Tyr Thr Thr Leu Gln Arg Val Ala Gly Ile Ser Arg
   385                 390                 395                 400
   Thr Gly Met Gln Ile Asn Arg His Ser Leu Thr Thr Ser Tyr Leu Asp
                   405                 410                 415
   Leu Met Ser His Ser Gly Thr Ser Leu Thr Gln Ser Val Ala Arg Ala
               420                 425                 430
   Met Leu Arg Phe Val Thr Val Thr Ala Glu Ala Leu Arg Phe Arg Gln
   435 440 445
   Ile Gln Arg Gly Phe Arg Thr Thr Leu Asp Asp Leu Ser Gly Arg Ser
   450 455 460
   Tyr Val Met Thr Ala Glu Asp Val Asp Leu Thr Leu Asn Trp Gly Arg
   465 470 475 480
   Leu Ser Ser Val Leu Pro Asp Tyr His Gly Gln Asp Ser Val Arg Val
   485 490 495
   Gly Arg Ile Ser Phe Gly Ser Ile Asn Ala Ile Leu Gly Ser Val Ala
   500 505 510
   Leu Ile Leu Asn Cys His His His Ala Ser Arg Val Ala Arg Lys Asp
   515 520 525
   Glu Leu
   530
<210> 16
   <211> 522
   <212> PRT
   <213> Artificial Sequence
<220>
   <221> source
   <223> /note="Description of Artificial Sequence: Synthetic polypeptide"
<400> 16
   Met Asp Ile Gln Leu Thr Gln Ser Pro Leu Ser Leu Pro Val Thr Leu
   1 5 10 15
   Gly Gln Pro Ala Ser Ile Ser Cys Arg Ser Ser Gln Ser Leu Val His
   20 25 30
   Arg Asn Gly Asn Thr Tyr Leu His Trp Phe Gln Gln Arg Pro Gly Gln
   35 40 45
   Ser Pro Arg Leu Leu Ile Tyr Thr Val Ser Asn Arg Phe Ser Gly Val
   50 55 60
   Pro Asp Arg Phe Ser Gly Ser Gly Ser Gly Thr Asp Phe Thr Leu Lys
   65 70 75 80
   Ile Ser Arg Val Glu Ala Glu Asp Val Gly Val Tyr Phe Cys Ser Gln
   85 90 95
   Ser Ser His Val Pro Pro Thr Phe Gly Ala Gly Thr Arg Leu Glu Ile
               100                 105                 110
   Lys Gly Ser Thr Ser Gly Ser Gly Lys Pro Gly Ser Gly Glu Gly Ser
           115                 120                 125
   Thr Lys Gly Gln Val Gln Leu Gln Gln Ser Gly Ser Glu Leu Lys Lys
       130                 135                 140
   Pro Gly Ala Ser Val Lys Val Ser Cys Lys Ala Ser Gly Tyr Thr Phe
   145                 150                 155                 160
   Thr Asn Tyr Gly Val Asn Trp Ile Lys Gln Ala Pro Gly Gln Gly Leu
                   165                 170                 175
   Gln Trp Met Gly Trp Ile Asn Pro Asn Thr Gly Glu Pro Thr Phe Asp
               180                 185                 190
   Asp Asp Phe Lys Gly Arg Phe Ala Phe Ser Leu Asp Thr Ser Val Ser
           195                 200                 205
   Thr Ala Tyr Leu Gln Ile Ser Ser Leu Lys Ala Asp Asp Thr Ala Val
       210                 215                 220
   Tyr Phe Cys Ser Arg Ser Arg Gly Lys Asn Glu Ala Trp Phe Ala Tyr
   225                 230                 235                 240
   Trp Gly Gln Gly Thr Leu Val Thr Val Ser Ser Glu Phe Pro Lys Pro
                   245                 250                 255
   Ser Thr Pro Pro Gly Ser Ser Gly Gly Ala Pro Lys Glu Phe Thr Leu
               260                 265                 270
   Asp Phe Ser Thr Ala Lys Thr Tyr Val Asp Ser Leu Asn Val Ile Arg
           275                 280                 285
   Ser Ala Ile Gly Thr Pro Leu Gln Thr Ile Ser Ser Gly Gly Thr Ser
       290                 295                 300
   Leu Leu Met Ile Asp Ser Gly Ser Gly Asp Asn Leu Phe Ala Val Asp
   305                 310                 315                 320
   Val Arg Gly Ile Asp Pro Glu Glu Gly Arg Phe Asn Asn Leu Arg Leu
                   325                 330                 335
   Ile Val Glu Arg Asn Asn Leu Tyr Val Thr Gly Phe Val Asn Arg Thr
               340                 345                 350
   Asn Asn Val Phe Tyr Arg Phe Ala Asp Phe Ser His Val Thr Phe Pro
   355 360 365
   Gly Thr Thr Ala Val Thr Leu Ser Gly Asp Ser Ser Tyr Thr Thr Leu
   370 375 380
   Gln Arg Val Ala Gly Ile Ser Arg Thr Gly Met Gln Ile Asn Arg His
   385 390 395 400
   Ser Leu Thr Thr Ser Tyr Leu Asp Leu Met Ser His Ser Gly Thr Ser
   405 410 415
   Leu Thr Gln Ser Val Ala Arg Ala Met Leu Arg Phe Val Thr Val Thr
   420 425 430
   Ala Glu Ala Leu Arg Phe Arg Gln Ile Gln Arg Gly Phe Arg Thr Thr
   435 440 445
   Leu Asp Asp Leu Ser Gly Arg Ser Tyr Val Met Thr Ala Glu Asp Val
   450 455 460
   Asp Leu Thr Leu Asn Trp Gly Arg Leu Ser Ser Val Leu Pro Asp Tyr
   465 470 475 480
   His Gly Gln Asp Ser Val Arg Val Gly Arg Ile Ser Phe Gly Ser Ile
   485 490 495
   Asn Ala Ile Leu Gly Ser Val Ala Leu Ile Leu Asn Cys His His His
   500 505 510
   Ala Ser Arg Val Ala Arg Lys Asp Glu Leu
   515 520
<210> 17
   <211> 531
   <212> PRT
   <213> Artificial Sequence
<220>
   <221> source
   <223> /note="Description of Artificial Sequence: Synthetic polypeptide"
<400> 17
   Met Asp Ile Gln Leu Thr Gln Ser Pro Leu Ser Leu Pro Val Thr Leu
   1 5 10 15
   Gly Gln Pro Ala Ser Ile Ser Cys Arg Ser Ser Gln Ser Leu Val His
   20 25 30
   Arg Asn Gly Asn Thr Tyr Leu His Trp Phe Gln Gln Arg Pro Gly Gln
   35 40 45
   Ser Pro Arg Leu Leu Ile Tyr Thr Val Ser Asn Arg Phe Ser Gly Val
   50 55 60
   Pro Asp Arg Phe Ser Gly Ser Gly Ser Gly Thr Asp Phe Thr Leu Lys
   65 70 75 80
   Ile Ser Arg Val Glu Ala Glu Asp Val Gly Val Tyr Phe Cys Ser Gln
   85 90 95
   Ser Ser His Val Pro Pro Thr Phe Gly Ala Gly Thr Arg Leu Glu Ile
   100 105 110
   Lys Gly Ser Thr Ser Gly Ser Gly Lys Pro Gly Ser Gly Glu Gly Ser
   115 120 125
   Thr Lys Gly Gln Val Gln Leu Gln Gln Ser Gly Ser Glu Leu Lys Lys
   130 135 140
   Pro Gly Ala Ser Val Lys Val Ser Cys Lys Ala Ser Gly Tyr Thr Phe
   145 150 155 160
   Thr Asn Tyr Gly Val Asn Trp Ile Lys Gln Ala Pro Gly Gln Gly Leu
   165 170 175
   Gln Trp Met Gly Trp Ile Asn Pro Asn Thr Gly Glu Pro Thr Phe Asp
   180 185 190
   Asp Asp Phe Lys Gly Arg Phe Ala Phe Ser Leu Asp Thr Ser Val Ser
   195 200 205
   Thr Ala Tyr Leu Gln Ile Ser Ser Leu Lys Ala Asp Asp Thr Ala Val
   210 215 220
   Tyr Phe Cys Ser Arg Ser Arg Gly Lys Asn Glu Ala Trp Phe Ala Tyr
   225 230 235 240
   Trp Gly Gln Gly Thr Leu Val Thr Val Ser Ser Glu Phe Pro Lys Pro
   245 250 255
   Ser Thr Pro Pro Gly Ser Ser Gly Gly Ala Pro Gly Ile Leu Gly Phe
   260 265 270
   Val Phe Thr Leu Lys Glu Phe Thr Leu Asp Phe Ser Thr Ala Lys Thr
           275                 280                 285
   Tyr Val Asp Ser Leu Asn Val Ile Arg Ser Ala Ile Gly Thr Pro Leu
       290                 295                 300
   Gln Thr Ile Ser Ser Gly Gly Thr Ser Leu Leu Met Ile Asp Ser Gly
   305                 310                 315                 320
   Ser Gly Asp Asn Leu Phe Ala Val Asp Val Arg Gly Ile Asp Pro Glu
                   325                 330                 335
   Glu Gly Arg Phe Asn Asn Leu Arg Leu Ile Val Glu Arg Asn Asn Leu
               340                 345                 350
   Tyr Val Thr Gly Phe Val Asn Arg Thr Asn Asn Val Phe Tyr Arg Phe
           355                 360                 365
   Ala Asp Phe Ser His Val Thr Phe Pro Gly Thr Thr Ala Val Thr Leu
       370                 375                 380
   Ser Gly Asp Ser Ser Tyr Thr Thr Leu Gln Arg Val Ala Gly Ile Ser
   385                 390                 395                 400
   Arg Thr Gly Met Gln Ile Asn Arg His Ser Leu Thr Thr Ser Tyr Leu
                   405                 410                 415
   Asp Leu Met Ser His Ser Gly Thr Ser Leu Thr Gln Ser Val Ala Arg
               420                 425                 430
   Ala Met Leu Arg Phe Val Thr Val Thr Ala Glu Ala Leu Arg Phe Arg
           435                 440                 445
   Gln Ile Gln Arg Gly Phe Arg Thr Thr Leu Asp Asp Leu Ser Gly Arg
       450                 455                 460
   Ser Tyr Val Met Thr Ala Glu Asp Val Asp Leu Thr Leu Asn Trp Gly
   465                 470                 475                 480
   Arg Leu Ser Ser Val Leu Pro Asp Tyr His Gly Gln Asp Ser Val Arg
                   485                 490                 495
   Val Gly Arg Ile Ser Phe Gly Ser Ile Asn Ala Ile Leu Gly Ser Val
               500                 505                 510
   Ala Leu Ile Leu Asn Cys His His His Ala Ser Arg Val Ala Arg Lys
           515                 520                 525
   Asp Glu Leu
   530
<210> 18
   <211> 522
   <212> PRT
   <213> Artificial Sequence
<220>
   <221> source
   <223> /note="Description of Artificial Sequence: Synthetic polypeptide"
<400> 18
   Met Asp Ile Gln Leu Thr Gln Ser Pro Leu Ser Leu Pro Val Thr Leu
   1 5 10 15
   Gly Gln Pro Ala Ser Ile Ser Cys Arg Ser Ser Gln Ser Leu Val His
   20 25 30
   Arg Asn Gly Asn Thr Tyr Leu His Trp Phe Gln Gln Arg Pro Gly Gln
   35 40 45
   Ser Pro Arg Leu Leu Ile Tyr Thr Val Ser Asn Arg Phe Ser Gly Val
   50 55 60
   Pro Asp Arg Phe Ser Gly Ser Gly Ser Gly Thr Asp Phe Thr Leu Lys
   65 70 75 80
   Ile Ser Arg Val Glu Ala Glu Asp Val Gly Val Tyr Phe Cys Ser Gln
   85 90 95
   Ser Ser His Val Pro Pro Thr Phe Gly Ala Gly Thr Arg Leu Glu Ile
   100 105 110
   Lys Gly Ser Thr Ser Gly Ser Gly Lys Pro Gly Ser Gly Glu Gly Ser
   115 120 125
   Thr Lys Gly Gln Val Gln Leu Gln Gln Ser Gly Ser Glu Leu Lys Lys
   130 135 140
   Pro Gly Ala Ser Val Lys Val Ser Cys Lys Ala Ser Gly Tyr Thr Phe
   145 150 155 160
   Thr Asn Tyr Gly Val Asn Trp Ile Lys Gln Ala Pro Gly Gln Gly Leu
   165 170 175
   Gln Trp Met Gly Trp Ile Asn Pro Asn Thr Gly Glu Pro Thr Phe Asp
   180 185 190
   Asp Asp Phe Lys Gly Arg Phe Ala Phe Ser Leu Asp Thr Ser Val Ser
           195                 200                 205
   Thr Ala Tyr Leu Gln Ile Ser Ser Leu Lys Ala Asp Asp Thr Ala Val
       210                 215                 220
   Tyr Phe Cys Ser Arg Ser Arg Gly Lys Asn Glu Ala Trp Phe Ala Tyr
   225                 230                 235                 240
   Trp Gly Gln Gly Thr Leu Val Thr Val Ser Ser Glu Phe Pro Lys Pro
                   245                 250                 255
   Ser Thr Pro Pro Gly Ser Ser Gly Gly Ala Pro Lys Glu Phe Thr Leu
               260                 265                 270
   Asp Phe Ser Thr Ala Lys Thr Tyr Val Asp Ser Leu Asn Val Ile Arg
           275                 280                 285
   Ser Ala Ile Gly Thr Pro Leu Gln Thr Ile Ser Ser Gly Gly Thr Ser
       290                 295                 300
   Leu Leu Met Ile Asp Ser Gly Ser Gly Asp Asn Leu Phe Ala Val Asp
   305                 310                 315                 320
   Val Arg Gly Ile Asp Pro Glu Glu Gly Arg Phe Asn Asn Leu Arg Leu
                   325                 330                 335
   Ile Val Glu Arg Asn Asn Leu Tyr Val Thr Gly Phe Val Asn Arg Thr
               340                 345                 350
   Asn Asn Val Phe Tyr Arg Phe Ala Asp Phe Ser His Val Thr Phe Pro
           355                 360                 365
   Gly Thr Thr Ala Val Thr Leu Ser Gly Asp Ser Ser Tyr Thr Thr Leu
       370                 375                 380
   Gln Arg Val Ala Gly Ile Ser Arg Thr Gly Met Gln Ile Asn Arg His
   385                 390                 395                 400
   Ser Leu Thr Thr Ser Tyr Leu Asp Leu Met Ser His Ser Gly Thr Ser
                   405                 410                 415
   Leu Thr Gln Ser Val Ala Arg Ala Met Leu Arg Phe Val Thr Val Thr
               420                 425                 430
   Ala Glu Ala Leu Arg Phe Arg Gln Ile Gln Arg Gly Phe Arg Thr Thr
           435                 440                 445
   Leu Asp Asp Leu Ser Gly Arg Ser Tyr Val Met Thr Ala Glu Asp Val
   450 455 460
   Asp Leu Thr Leu Asn Trp Gly Arg Leu Ser Ser Val Leu Pro Asp Tyr
   465 470 475 480
   His Gly Gln Asp Ser Val Arg Val Gly Arg Ile Ser Phe Gly Ser Ile
   485 490 495
   Asn Ala Ile Leu Gly Ser Val Ala Leu Ile Leu Asn Cys His His His
   500 505 510
   Ala Ser Arg Val Ala Arg Lys Asp Glu Leu
   515 520
<210> 19
   <211> 531
   <212> PRT
   <213> Artificial Sequence
<220>
   <221> source
   <223> /note="Description of Artificial Sequence: Synthetic polypeptide"
<400> 19
   Met Asp Ile Gln Leu Thr Gln Ser Pro Leu Ser Leu Pro Val Thr Leu
   1 5 10 15
   Gly Gln Pro Ala Ser Ile Ser Cys Arg Ser Ser Gln Ser Leu Val His
   20 25 30
   Arg Asn Gly Asn Thr Tyr Leu His Trp Phe Gln Gln Arg Pro Gly Gln
   35 40 45
   Ser Pro Arg Leu Leu Ile Tyr Thr Val Ser Asn Arg Phe Ser Gly Val
   50 55 60
   Pro Asp Arg Phe Ser Gly Ser Gly Ser Gly Thr Asp Phe Thr Leu Lys
   65 70 75 80
   Ile Ser Arg Val Glu Ala Glu Asp Val Gly Val Tyr Phe Cys Ser Gln
   85 90 95
   Ser Ser His Val Pro Pro Thr Phe Gly Ala Gly Thr Arg Leu Glu Ile
   100 105 110
   Lys Gly Ser Thr Ser Gly Ser Gly Lys Pro Gly Ser Gly Glu Gly Ser
   115 120 125
   Thr Lys Gly Gln Val Gln Leu Gln Gln Ser Gly Ser Glu Leu Lys Lys
       130                 135                 140
   Pro Gly Ala Ser Val Lys Val Ser Cys Lys Ala Ser Gly Tyr Thr Phe
   145                 150                 155                 160
   Thr Asn Tyr Gly Val Asn Trp Ile Lys Gln Ala Pro Gly Gln Gly Leu
                   165                 170                 175
   Gln Trp Met Gly Trp Ile Asn Pro Asn Thr Gly Glu Pro Thr Phe Asp
               180                 185                 190
   Asp Asp Phe Lys Gly Arg Phe Ala Phe Ser Leu Asp Thr Ser Val Ser
           195                 200                 205
   Thr Ala Tyr Leu Gln Ile Ser Ser Leu Lys Ala Asp Asp Thr Ala Val
       210                 215                 220
   Tyr Phe Cys Ser Arg Ser Arg Gly Lys Asn Glu Ala Trp Phe Ala Tyr
   225                 230                 235                 240
   Trp Gly Gln Gly Thr Leu Val Thr Val Ser Ser Glu Phe Pro Lys Pro
                   245                 250                 255
   Ser Thr Pro Pro Gly Ser Ser Gly Gly Ala Pro Gly Ile Leu Gly Phe
               260                 265                 270
   Val Phe Thr Leu Lys Glu Phe Thr Leu Asp Phe Ser Thr Ala Lys Thr
           275                 280                 285
   Tyr Val Asp Ser Leu Asn Val Ile Arg Ser Ala Ile Gly Thr Pro Leu
       290                 295                 300
   Gln Thr Ile Ser Ser Gly Gly Thr Ser Leu Leu Met Ile Asp Ser Gly
   305                 310                 315                 320
   Ser Gly Asp Asn Leu Phe Ala Val Asp Val Arg Gly Ile Asp Pro Glu
                   325                 330                 335
   Glu Gly Arg Phe Asn Asn Leu Arg Leu Ile Val Glu Arg Asn Asn Leu
               340                 345                 350
   Tyr Val Thr Gly Phe Val Asn Arg Thr Asn Asn Val Phe Tyr Arg Phe
           355                 360                 365
   Ala Asp Phe Ser His Val Thr Phe Pro Gly Thr Thr Ala Val Thr Leu
       370                 375                 380
   Ser Gly Asp Ser Ser Tyr Thr Thr Leu Gln Arg Val Ala Gly Ile Ser
   385 390 395 400
   Arg Thr Gly Met Gln Ile Asn Arg His Ser Leu Thr Thr Ser Tyr Leu
   405 410 415
   Asp Leu Met Ser His Ser Gly Thr Ser Leu Thr Gln Ser Val Ala Arg
   420 425 430
   Ala Met Leu Arg Phe Val Thr Val Thr Ala Glu Ala Leu Arg Phe Arg
   435 440 445
   Gln Ile Gln Arg Gly Phe Arg Thr Thr Leu Asp Asp Leu Ser Gly Arg
   450 455 460
   Ser Tyr Val Met Thr Ala Glu Asp Val Asp Leu Thr Leu Asn Trp Gly
   465 470 475 480
   Arg Leu Ser Ser Val Leu Pro Asp Tyr His Gly Gln Asp Ser Val Arg
   485 490 495
   Val Gly Arg Ile Ser Phe Gly Ser Ile Asn Ala Ile Leu Gly Ser Val
   500 505 510
   Ala Leu Ile Leu Asn Cys His His His Ala Ser Arg Val Ala Arg Lys
   515 520 525
   Asp Glu Leu
   530
<210> 20
   <211> 389
   <212> PRT
   <213> Artificial Sequence
<220>
   <221> source
   <223> /note="Description of Artificial Sequence: Synthetic polypeptide"
<400> 20
   Met Glu Val Gln Leu Val Glu Ser Gly Gly Gly Leu Val Gln Ala Gly
   1 5 10 15
   Gly Ser Leu Arg Leu Ser Cys Ala Ala Ser Gly Ile Thr Phe Ser Ile
   20 25 30
   Asn Thr Met Gly Trp Tyr Arg Gln Ala Pro Gly Lys Gln Arg Glu Leu
   35 40 45
   Val Ala Leu Ile Ser Ser Ile Gly Asp Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Lys Arg Phe Arg Thr Ala Ala Gln Gly Thr Asp Tyr Trp Gly Gln Gly
               100                 105                 110
   Thr Gln Val Thr Val Ser Ser Ala His His Ser Glu Asp Pro Ser Ser
           115                 120                 125
   Lys Ala Pro Lys Ala Pro Lys Glu Phe Thr Leu Asp Phe Ser Thr Ala
       130                 135                 140
   Lys Thr Tyr Val Asp Ser Leu Asn Val Ile Arg Ser Ala Ile Gly Thr
   145                 150                 155                 160
   Pro Leu Gln Thr Ile Ser Ser Gly Gly Thr Ser Leu Leu Met Ile Asp
                   165                 170                 175
   Ser Gly Ser Gly Asp Asn Leu Phe Ala Val Asp Val Arg Gly Ile Asp
               180                 185                 190
   Pro Glu Glu Gly Arg Phe Asn Asn Leu Arg Leu Ile Val Glu Arg Asn
           195                 200                 205
   Asn Leu Tyr Val Thr Gly Phe Val Asn Arg Thr Asn Asn Val Phe Tyr
       210                 215                 220
   Arg Phe Ala Asp Phe Ser His Val Thr Phe Pro Gly Thr Thr Ala Val
   225                 230                 235                 240
   Thr Leu Ser Gly Asp Ser Ser Tyr Thr Thr Leu Gln Arg Val Ala Gly
                   245                 250                 255
   Ile Ser Arg Thr Gly Met Gln Ile Asn Arg His Ser Leu Thr Thr Ser
               260                 265                 270
   Tyr Leu Asp Leu Met Ser His Ser Gly Thr Ser Leu Thr Gln Ser Val
           275                 280                 285
   Ala Arg Ala Met Leu Arg Phe Val Thr Val Thr Ala Glu Ala Leu Arg
       290                 295                 300
   Phe Arg Gln Ile Gln Arg Gly Phe Arg Thr Thr Leu Asp Asp Leu Ser
   305                 310                 315                 320
   Gly Arg Ser Tyr Val Met Thr Ala Glu Asp Val Asp Leu Thr Leu Asn
                   325                 330                 335
   Trp Gly Arg Leu Ser Ser Val Leu Pro Asp Tyr His Gly Gln Asp Ser
               340                 345                 350
   Val Arg Val Gly Arg Ile Ser Phe Gly Ser Ile Asn Ala Ile Leu Gly
           355                 360                 365
   Ser Val Ala Leu Ile Leu Asn Cys His His His Ala Ser Arg Val Ala
       370                 375                 380
   Arg Lys Asp Glu Leu
   385
<210> 21
   <211> 390
   <212> PRT
   <213> Artificial Sequence
<220>
   <221> source
   <223> /note="Description of Artificial Sequence: Synthetic polypeptide"
<400> 21
   Met Glu Val Gln Leu Val Glu Ser Gly Gly Gly Leu Val Gln Ala Gly
   1               5                   10                  15
   Gly Ser Leu Arg Leu Ser Cys Ala Ala Ser Gly Ile Thr Phe Ser Ile
               20                  25                  30
   Asn Thr Met Gly Trp Tyr Arg Gln Ala Pro Gly Lys Gln Arg Glu Leu
           35                  40                  45
   Val Ala Leu Ile Ser Ser Ile Gly Asp Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Lys Arg Phe Arg Thr Ala Ala Gln Gly Thr Asp Tyr Trp Gly Gln Gly
               100                 105                 110
   Thr Gln Val Thr Val Ser Ser Glu Phe Pro Lys Pro Ser Thr Pro Pro
           115                 120                 125
   Gly Ser Ser Gly Gly Ala Pro Lys Glu Phe Thr Leu Asp Phe Ser Thr
       130                 135                 140
   Ala Lys Thr Tyr Val Asp Ser Leu Asn Val Ile Arg Ser Ala Ile Gly
   145                 150                 155                 160
   Thr Pro Leu Gln Thr Ile Ser Ser Gly Gly Thr Ser Leu Leu Met Ile
                   165                 170                 175
   Asp Ser Gly Ser Gly Asp Asn Leu Phe Ala Val Asp Val Arg Gly Ile
               180                 185                 190
   Asp Pro Glu Glu Gly Arg Phe Asn Asn Leu Arg Leu Ile Val Glu Arg
           195                 200                 205
   Asn Asn Leu Tyr Val Thr Gly Phe Val Asn Arg Thr Asn Asn Val Phe
       210                 215                 220
   Tyr Arg Phe Ala Asp Phe Ser His Val Thr Phe Pro Gly Thr Thr Ala
   225                 230                 235                 240
   Val Thr Leu Ser Gly Asp Ser Ser Tyr Thr Thr Leu Gln Arg Val Ala
                   245                 250                 255
   Gly Ile Ser Arg Thr Gly Met Gln Ile Asn Arg His Ser Leu Thr Thr
               260                 265                 270
   Ser Tyr Leu Asp Leu Met Ser His Ser Gly Thr Ser Leu Thr Gln Ser
           275                 280                 285
   Val Ala Arg Ala Met Leu Arg Phe Val Thr Val Thr Ala Glu Ala Leu
       290                 295                 300
   Arg Phe Arg Gln Ile Gln Arg Gly Phe Arg Thr Thr Leu Asp Asp Leu
   305                 310                 315                 320
   Ser Gly Arg Ser Tyr Val Met Thr Ala Glu Asp Val Asp Leu Thr Leu
                   325                 330                 335
   Asn Trp Gly Arg Leu Ser Ser Val Leu Pro Asp Tyr His Gly Gln Asp
               340                 345                 350
   Ser Val Arg Val Gly Arg Ile Ser Phe Gly Ser Ile Asn Ala Ile Leu
   355 360 365
   Gly Ser Val Ala Leu Ile Leu Asn Cys His His His Ala Ser Arg Val
   370 375 380
   Ala Arg Lys Asp Glu Leu
   385 390
<210> 22
   <211> 398
   <212> PRT
   <213> Artificial Sequence
<220>
   <221> source
   <223> /note="Description of Artificial Sequence: Synthetic polypeptide"
<400> 22
   Met Glu Val Gln Leu Val Glu Ser Gly Gly Gly Leu Val Gln Ala Gly
   1 5 10 15
   Gly Ser Leu Arg Leu Ser Cys Ala Ala Ser Gly Ile Thr Phe Ser Ile
   20 25 30
   Asn Thr Met Gly Trp Tyr Arg Gln Ala Pro Gly Lys Gln Arg Glu Leu
   35 40 45
   Val Ala Leu Ile Ser Ser Ile Gly Asp Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Lys Arg Phe Arg Thr Ala Ala Gln Gly Thr Asp Tyr Trp Gly Gln Gly
   100 105 110
   Thr Gln Val Thr Val Ser Ser Ala His His Ser Glu Asp Pro Ser Ser
   115 120 125
   Lys Ala Pro Lys Ala Pro Gly Ile Leu Gly Phe Val Phe Thr Leu Lys
   130 135 140
   Glu Phe Thr Leu Asp Phe Ser Thr Ala Lys Thr Tyr Val Asp Ser Leu
   145 150 155 160
   Asn Val Ile Arg Ser Ala Ile Gly Thr Pro Leu Gln Thr Ile Ser Ser
                   165                 170                 175
   Gly Gly Thr Ser Leu Leu Met Ile Asp Ser Gly Ser Gly Asp Asn Leu
               180                 185                 190
   Phe Ala Val Asp Val Arg Gly Ile Asp Pro Glu Glu Gly Arg Phe Asn
           195                 200                 205
   Asn Leu Arg Leu Ile Val Glu Arg Asn Asn Leu Tyr Val Thr Gly Phe
       210                 215                 220
   Val Asn Arg Thr Asn Asn Val Phe Tyr Arg Phe Ala Asp Phe Ser His
   225                 230                 235                 240
   Val Thr Phe Pro Gly Thr Thr Ala Val Thr Leu Ser Gly Asp Ser Ser
                   245                 250                 255
   Tyr Thr Thr Leu Gln Arg Val Ala Gly Ile Ser Arg Thr Gly Met Gln
               260                 265                 270
   Ile Asn Arg His Ser Leu Thr Thr Ser Tyr Leu Asp Leu Met Ser His
           275                 280                 285
   Ser Gly Thr Ser Leu Thr Gln Ser Val Ala Arg Ala Met Leu Arg Phe
       290                 295                 300
   Val Thr Val Thr Ala Glu Ala Leu Arg Phe Arg Gln Ile Gln Arg Gly
   305                 310                 315                 320
   Phe Arg Thr Thr Leu Asp Asp Leu Ser Gly Arg Ser Tyr Val Met Thr
                   325                 330                 335
   Ala Glu Asp Val Asp Leu Thr Leu Asn Trp Gly Arg Leu Ser Ser Val
               340                 345                 350
   Leu Pro Asp Tyr His Gly Gln Asp Ser Val Arg Val Gly Arg Ile Ser
           355                 360                 365
   Phe Gly Ser Ile Asn Ala Ile Leu Gly Ser Val Ala Leu Ile Leu Asn
       370                 375                 380
   Cys His His His Ala Ser Arg Val Ala Arg Lys Asp Glu Leu
   385                 390                 395
<210> 23
   <211> 399
   <212> PRT
   <213> Artificial Sequence
<220>
   <221> source
   <223> /note="Description of Artificial Sequence: Synthetic polypeptide"
<400> 23
   Met Glu Val Gln Leu Val Glu Ser Gly Gly Gly Leu Val Gln Ala Gly
   1               5                   10                  15
   Gly Ser Leu Arg Leu Ser Cys Ala Ala Ser Gly Ile Thr Phe Ser Ile
               20                  25                  30
   Asn Thr Met Gly Trp Tyr Arg Gln Ala Pro Gly Lys Gln Arg Glu Leu
           35                  40                  45
   Val Ala Leu Ile Ser Ser Ile Gly Asp Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Lys Arg Phe Arg Thr Ala Ala Gln Gly Thr Asp Tyr Trp Gly Gln Gly
               100                 105                 110
   Thr Gln Val Thr Val Ser Ser Glu Phe Pro Lys Pro Ser Thr Pro Pro
           115                 120                 125
   Gly Ser Ser Gly Gly Ala Pro Gly Ile Leu Gly Phe Val Phe Thr Leu
       130                 135                 140
   Lys Glu Phe Thr Leu Asp Phe Ser Thr Ala Lys Thr Tyr Val Asp Ser
   145                 150                 155                 160
   Leu Asn Val Ile Arg Ser Ala Ile Gly Thr Pro Leu Gln Thr Ile Ser
                   165                 170                 175
   Ser Gly Gly Thr Ser Leu Leu Met Ile Asp Ser Gly Ser Gly Asp Asn
               180                 185                 190
   Leu Phe Ala Val Asp Val Arg Gly Ile Asp Pro Glu Glu Gly Arg Phe
           195                 200                 205
   Asn Asn Leu Arg Leu Ile Val Glu Arg Asn Asn Leu Tyr Val Thr Gly
       210                 215                 220
   Phe Val Asn Arg Thr Asn Asn Val Phe Tyr Arg Phe Ala Asp Phe Ser
   225 230 235 240
   His Val Thr Phe Pro Gly Thr Thr Ala Val Thr Leu Ser Gly Asp Ser
   245 250 255
   Ser Tyr Thr Thr Leu Gln Arg Val Ala Gly Ile Ser Arg Thr Gly Met
   260 265 270
   Gln Ile Asn Arg His Ser Leu Thr Thr Ser Tyr Leu Asp Leu Met Ser
   275 280 285
   His Ser Gly Thr Ser Leu Thr Gln Ser Val Ala Arg Ala Met Leu Arg
   290 295 300
   Phe Val Thr Val Thr Ala Glu Ala Leu Arg Phe Arg Gln Ile Gln Arg
   305 310 315 320
   Gly Phe Arg Thr Thr Leu Asp Asp Leu Ser Gly Arg Ser Tyr Val Met
   325 330 335
   Thr Ala Glu Asp Val Asp Leu Thr Leu Asn Trp Gly Arg Leu Ser Ser
   340 345 350
   Val Leu Pro Asp Tyr His Gly Gln Asp Ser Val Arg Val Gly Arg Ile
   355 360 365
   Ser Phe Gly Ser Ile Asn Ala Ile Leu Gly Ser Val Ala Leu Ile Leu
   370 375 380
   Asn Cys His His His Ala Ser Arg Val Ala Arg Lys Asp Glu Leu
   385 390 395
<210> 24
   <211> 389
   <212> PRT
   <213> Artificial Sequence
<220>
   <221> source
   <223> /note="Description of Artificial Sequence: Synthetic polypeptide"
<400> 24
   Met Glu Val Gln Leu Val Glu Ser Gly Gly Gly Leu Val Gln Ala Gly
   1 5 10 15
   Gly Ser Leu Arg Leu Ser Cys Ala Ala Ser Gly Ile Thr Phe Ser Ile
   20 25 30
   Asn Thr Met Gly Trp Tyr Arg Gln Ala Pro Gly Lys Gln Arg Glu Leu
           35                  40                  45
   Val Ala Leu Ile Ser Ser Ile Gly Asp Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Lys Arg Phe Arg Thr Ala Ala Gln Gly Thr Asp Tyr Trp Gly Gln Gly
               100                 105                 110
   Thr Gln Val Thr Val Ser Ser Ala His His Ser Glu Asp Pro Ser Ser
           115                 120                 125
   Lys Ala Pro Lys Ala Pro Lys Glu Phe Thr Leu Asp Phe Ser Thr Ala
       130                 135                 140
   Lys Thr Tyr Val Asp Ser Leu Asn Val Ile Arg Ser Ala Ile Gly Thr
   145                 150                 155                 160
   Pro Leu Gln Thr Ile Ser Ser Gly Gly Thr Ser Leu Leu Met Ile Asp
                   165                 170                 175
   Ser Gly Thr Gly Asp Asn Leu Phe Ala Val Asp Val Arg Gly Ile Asp
               180                 185                 190
   Pro Glu Glu Gly Arg Phe Asn Asn Leu Arg Leu Ile Val Glu Arg Asn
           195                 200                 205
   Asn Leu Tyr Val Thr Gly Phe Val Asn Arg Thr Asn Asn Val Phe Tyr
       210                 215                 220
   Arg Phe Ala Asp Phe Ser His Val Thr Phe Pro Gly Thr Thr Ala Val
   225                 230                 235                 240
   Thr Leu Ser Gly Asp Ser Ser Tyr Thr Thr Leu Gln Arg Val Ala Gly
                   245                 250                 255
   Ile Ser Arg Thr Gly Met Gln Ile Asn Arg His Ser Leu Thr Thr Ser
               260                 265                 270
   Tyr Leu Asp Leu Met Ser His Ser Gly Thr Ser Leu Thr Gln Ser Val
           275                 280                 285
   Ala Arg Ala Met Leu Arg Phe Val Thr Val Thr Ala Glu Ala Leu Arg
       290                 295                 300
   Phe Arg Gln Ile Gln Arg Gly Phe Arg Thr Thr Leu Asp Asp Leu Ser
   305                 310                 315                 320
   Gly Arg Ser Tyr Val Met Thr Ala Glu Asp Val Asp Leu Thr Leu Asn
                   325                 330                 335
   Trp Gly Arg Leu Ser Ser Val Leu Pro Asp Tyr His Gly Gln Asp Ser
               340                 345                 350
   Val Arg Val Gly Arg Ile Ser Phe Gly Ser Ile Asn Ala Ile Leu Gly
           355                 360                 365
   Ser Val Ala Leu Ile Leu Asn Ser His His His Ala Ser Arg Val Ala
       370                 375                 380
   Arg Lys Asp Glu Leu
   385
<210> 25
   <211> 390
   <212> PRT
   <213> Artificial Sequence
<220>
   <221> source
   <223> /note="Description of Artificial Sequence: Synthetic polypeptide"
<400> 25
   Met Glu Val Gln Leu Val Glu Ser Gly Gly Gly Leu Val Gln Ala Gly
   1               5                   10                  15
   Gly Ser Leu Arg Leu Ser Cys Ala Ala Ser Gly Ile Thr Phe Ser Ile
               20                  25                  30
   Asn Thr Met Gly Trp Tyr Arg Gln Ala Pro Gly Lys Gln Arg Glu Leu
           35                  40                  45
   Val Ala Leu Ile Ser Ser Ile Gly Asp Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Lys Arg Phe Arg Thr Ala Ala Gln Gly Thr Asp Tyr Trp Gly Gln Gly
               100                 105                 110
   Thr Gln Val Thr Val Ser Ser Glu Phe Pro Lys Pro Ser Thr Pro Pro
           115                 120                 125
   Gly Ser Ser Gly Gly Ala Pro Lys Glu Phe Thr Leu Asp Phe Ser Thr
       130                 135                 140
   Ala Lys Thr Tyr Val Asp Ser Leu Asn Val Ile Arg Ser Ala Ile Gly
   145                 150                 155                 160
   Thr Pro Leu Gln Thr Ile Ser Ser Gly Gly Thr Ser Leu Leu Met Ile
                   165                 170                 175
   Asp Ser Gly Thr Gly Asp Asn Leu Phe Ala Val Asp Val Arg Gly Ile
               180                 185                 190
   Asp Pro Glu Glu Gly Arg Phe Asn Asn Leu Arg Leu Ile Val Glu Arg
           195                 200                 205
   Asn Asn Leu Tyr Val Thr Gly Phe Val Asn Arg Thr Asn Asn Val Phe
       210                 215                 220
   Tyr Arg Phe Ala Asp Phe Ser His Val Thr Phe Pro Gly Thr Thr Ala
   225                 230                 235                 240
   Val Thr Leu Ser Gly Asp Ser Ser Tyr Thr Thr Leu Gln Arg Val Ala
                   245                 250                 255
   Gly Ile Ser Arg Thr Gly Met Gln Ile Asn Arg His Ser Leu Thr Thr
               260                 265                 270
   Ser Tyr Leu Asp Leu Met Ser His Ser Gly Thr Ser Leu Thr Gln Ser
           275                 280                 285
   Val Ala Arg Ala Met Leu Arg Phe Val Thr Val Thr Ala Glu Ala Leu
       290                 295                 300
   Arg Phe Arg Gln Ile Gln Arg Gly Phe Arg Thr Thr Leu Asp Asp Leu
   305                 310                 315                 320
   Ser Gly Arg Ser Tyr Val Met Thr Ala Glu Asp Val Asp Leu Thr Leu
                   325                 330                 335
   Asn Trp Gly Arg Leu Ser Ser Val Leu Pro Asp Tyr His Gly Gln Asp
   340 345 350
   Ser Val Arg Val Gly Arg Ile Ser Phe Gly Ser Ile Asn Ala Ile Leu
   355 360 365
   Gly Ser Val Ala Leu Ile Leu Asn Cys His His His Ala Ser Arg Val
   370 375 380
   Ala Arg Lys Asp Glu Leu
   385 390
<210> 26
   <211> 407
   <212> PRT
   <213> Artificial Sequence
<220>
   <221> source
   <223> /note="Description of Artificial Sequence: Synthetic polypeptide"
<400> 26
   Met Glu Val Gln Leu Val Glu Ser Gly Gly Gly Leu Val Gln Ala Gly
   1               5                   10                  15
   Gly Ser Leu Arg Leu Ser Cys Ala Ala Ser Gly Ile Thr Phe Ser Ile
               20                  25                  30
   Asn Thr Met Gly Trp Tyr Arg Gln Ala Pro Gly Lys Gln Arg Glu Leu
           35                  40                  45
   Val Ala Leu Ile Ser Ser Ile Gly Asp Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Lys Arg Phe Arg Thr Ala Ala Gln Gly Thr Asp Tyr Trp Gly Gln Gly
               100                 105                 110
   Thr Gln Val Thr Val Ser Ser Ala His His Ser Glu Asp Pro Ser Ser
           115                 120                 125
   Lys Ala Pro Lys Ala Pro Gly Ile Leu Gly Phe Val Phe Thr Leu Gly
       130                 135                 140
   Ile Leu Gly Phe Val Phe Thr Leu Lys Glu Phe Thr Leu Asp Phe Ser
   145 150 155 160
   Thr Ala Lys Thr Tyr Val Asp Ser Leu Asn Val Ile Arg Ser Ala Ile
   165 170 175
   Gly Thr Pro Leu Gln Thr Ile Ser Ser Gly Gly Thr Ser Leu Leu Met
   180 185 190
   Ile Asp Ser Gly Thr Gly Asp Asn Leu Phe Ala Val Asp Val Arg Gly
   195 200 205
   Ile Asp Pro Glu Glu Gly Arg Phe Asn Asn Leu Arg Leu Ile Val Glu
   210 215 220
   Arg Asn Asn Leu Tyr Val Thr Gly Phe Val Asn Arg Thr Asn Asn Val
   225 230 235 240
   Phe Tyr Arg Phe Ala Asp Phe Ser His Val Thr Phe Pro Gly Thr Thr
   245 250 255
   Ala Val Thr Leu Ser Gly Asp Ser Ser Tyr Thr Thr Leu Gln Arg Val
   260 265 270
   Ala Gly Ile Ser Arg Thr Gly Met Gln Ile Asn Arg His Ser Leu Thr
   275 280 285
   Thr Ser Tyr Leu Asp Leu Met Ser His Ser Gly Thr Ser Leu Thr Gln
   290 295 300
   Ser Val Ala Arg Ala Met Leu Arg Phe Val Thr Val Thr Ala Glu Ala
   305 310 315 320
   Leu Arg Phe Arg Gln Ile Gln Arg Gly Phe Arg Thr Thr Leu Asp Asp
   325 330 335
   Leu Ser Gly Arg Ser Tyr Val Met Thr Ala Glu Asp Val Asp Leu Thr
   340 345 350
   Leu Asn Trp Gly Arg Leu Ser Ser Val Leu Pro Asp Tyr His Gly Gln
   355 360 365
   Asp Ser Val Arg Val Gly Arg Ile Ser Phe Gly Ser Ile Asn Ala Ile
   370 375 380
   Leu Gly Ser Val Ala Leu Ile Leu Asn Cys His His His Ala Ser Arg
   385 390 395 400
   Val Ala Arg Lys Asp Glu Leu
   405
<210> 27
   <211> 408
   <212> PRT
   <213> Artificial Sequence
<220>
   <221> source
   <223> /note="Description of Artificial Sequence: Synthetic polypeptide"
<400> 27
   Met Glu Val Gln Leu Val Glu Ser Gly Gly Gly Leu Val Gln Ala Gly
   1               5                   10                  15
   Gly Ser Leu Arg Leu Ser Cys Ala Ala Ser Gly Ile Thr Phe Ser Ile
               20                  25                  30
   Asn Thr Met Gly Trp Tyr Arg Gln Ala Pro Gly Lys Gln Arg Glu Leu
           35                  40                  45
   Val Ala Leu Ile Ser Ser Ile Gly Asp Thr Tyr Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Lys Arg Phe Arg Thr Ala Ala Gln Gly Thr Asp Tyr Trp Gly Gln Gly
               100                 105                 110
   Thr Gln Val Thr Val Ser Ser Glu Phe Pro Lys Pro Ser Thr Pro Pro
           115                 120                 125
   Gly Ser Ser Gly Gly Ala Pro Gly Ile Leu Gly Phe Val Phe Thr Leu
       130                 135                 140
   Gly Ile Leu Gly Phe Val Phe Thr Leu Lys Glu Phe Thr Leu Asp Phe
   145                 150                 155                 160
   Ser Thr Ala Lys Thr Tyr Val Asp Ser Leu Asn Val Ile Arg Ser Ala
                   165                 170                 175
   Ile Gly Thr Pro Leu Gln Thr Ile Ser Ser Gly Gly Thr Ser Leu Leu
               180                 185                 190
   Met Ile Asp Ser Gly Thr Gly Asp Asn Leu Phe Ala Val Asp Val Arg
   195 200 205
   Gly Ile Asp Pro Glu Glu Gly Arg Phe Asn Asn Leu Arg Leu Ile Val
   210 215 220
   Glu Arg Asn Asn Leu Tyr Val Thr Gly Phe Val Asn Arg Thr Asn Asn
   225 230 235 240
   Val Phe Tyr Arg Phe Ala Asp Phe Ser His Val Thr Phe Pro Gly Thr
   245 250 255
   Thr Ala Val Thr Leu Ser Gly Asp Ser Ser Tyr Thr Thr Leu Gln Arg
   260 265 270
   Val Ala Gly Ile Ser Arg Thr Gly Met Gln Ile Asn Arg His Ser Leu
   275 280 285
   Thr Thr Ser Tyr Leu Asp Leu Met Ser His Ser Gly Thr Ser Leu Thr
   290 295 300
   Gln Ser Val Ala Arg Ala Met Leu Arg Phe Val Thr Val Thr Ala Glu
   305 310 315 320
   Ala Leu Arg Phe Arg Gln Ile Gln Arg Gly Phe Arg Thr Thr Leu Asp
   325 330 335
   Asp Leu Ser Gly Arg Ser Tyr Val Met Thr Ala Glu Asp Val Asp Leu
   340 345 350
   Thr Leu Asn Trp Gly Arg Leu Ser Ser Val Leu Pro Asp Tyr His Gly
   355 360 365
   Gln Asp Ser Val Arg Val Gly Arg Ile Ser Phe Gly Ser Ile Asn Ala
   370 375 380
   Ile Leu Gly Ser Val Ala Leu Ile Leu Asn Ser His His His Ala Ser
   385 390 395 400
   Arg Val Ala Arg Lys Asp Glu Leu
   405
<210> 28
   <211> 370
   <212> PRT
   <213> Artificial Sequence
<220>
   <221> source
   <223> /note="Description of Artificial Sequence: Synthetic polypeptide"
<400> 28
   Met Ala Ser Val Ser Asp Val Pro Arg Asp Leu Glu Val Val Ala Ala
   1               5                   10                  15
   Thr Pro Thr Ser Leu Leu Ile Ser Trp Cys Arg Gln Arg Cys Ala Asp
               20                  25                  30
   Ser Tyr Arg Ile Thr Tyr Gly Glu Thr Gly Gly Asn Ser Pro Val Gln
           35                  40                  45
   Glu Phe Thr Val Pro Gly Ser Trp Lys Thr Ala Thr Ile Ser Gly Leu
       50                  55                  60
   Lys Pro Gly Val Asp Tyr Thr Ile Thr Val Tyr Val Val Thr His Tyr
   65                  70                  75                  80
   Tyr Gly Trp Asp Arg Tyr Ser His Pro Ile Ser Ile Asn Tyr Arg Thr
                   85                  90                  95
   Gly Ser Met Glu Phe Pro Lys Pro Ser Thr Pro Pro Gly Ser Ser Gly
               100                 105                 110
   Gly Ala Pro Lys Glu Phe Thr Leu Asp Phe Ser Thr Ala Lys Thr Tyr
           115                 120                 125
   Val Asp Ser Leu Asn Val Ile Arg Ser Ala Ile Gly Thr Pro Leu Gln
       130                 135                 140
   Thr Ile Ser Ser Gly Gly Thr Ser Leu Leu Met Ile Asp Ser Gly Ser
   145                 150                 155                 160
   Gly Asp Asn Leu Phe Ala Val Asp Val Arg Gly Ile Asp Pro Glu Glu
                   165                 170                 175
   Gly Arg Phe Asn Asn Leu Arg Leu Ile Val Glu Arg Asn Asn Leu Tyr
               180                 185                 190
   Val Thr Gly Phe Val Asn Arg Thr Asn Asn Val Phe Tyr Arg Phe Ala
           195                 200                 205
   Asp Phe Ser His Val Thr Phe Pro Gly Thr Thr Ala Val Thr Leu Ser
       210                 215                 220
   Gly Asp Ser Ser Tyr Thr Thr Leu Gln Arg Val Ala Gly Ile Ser Arg
   225                 230                 235                 240
   Thr Gly Met Gln Ile Asn Arg His Ser Leu Thr Thr Ser Tyr Leu Asp
                   245                 250                 255
   Leu Met Ser His Ser Gly Thr Ser Leu Thr Gln Ser Val Ala Arg Ala
               260                 265                 270
   Met Leu Arg Phe Val Thr Val Thr Ala Glu Ala Leu Arg Phe Arg Gln
           275                 280                 285
   Ile Gln Arg Gly Phe Arg Thr Thr Leu Asp Asp Leu Ser Gly Arg Ser
       290                 295                 300
   Tyr Val Met Thr Ala Glu Asp Val Asp Leu Thr Leu Asn Trp Gly Arg
   305                 310                 315                 320
   Leu Ser Ser Val Leu Pro Asp Tyr His Gly Gln Asp Ser Val Arg Val
                   325                 330                 335
   Gly Arg Ile Ser Phe Gly Ser Ile Asn Ala Ile Leu Gly Ser Val Ala
               340                 345                 350
   Leu Ile Leu Asn Ser His His His Ala Ser Arg Val Ala Arg Lys Asp
           355                 360                 365
   Glu Leu
       370
<210> 29
   <211> 369
   <212> PRT
   <213> Artificial Sequence
<220>
   <221> source
   <223> /note="Description of Artificial Sequence: Synthetic polypeptide"
<400> 29
   Met Lys Glu Phe Thr Leu Asp Phe Ser Thr Ala Lys Thr Tyr Val Asp
   1               5                   10                  15
   Ser Leu Asn Val Ile Arg Ser Ala Ile Gly Thr Pro Leu Gln Thr Ile
               20                  25                  30
   Ser Ser Gly Gly Thr Ser Leu Leu Met Ile Asp Ser Gly Thr Gly Asp
           35                  40                  45
   Asn Leu Phe Ala Val Asp Val Arg Gly Ile Asp Pro Glu Glu Gly Arg
       50                  55                  60
   Phe Asn Asn Leu Arg Leu Ile Val Glu Arg Asn Asn Leu Tyr Val Thr
   65                  70                  75                  80
   Gly Phe Val Asn Arg Thr Asn Asn Val Phe Tyr Arg Phe Ala Asp Phe
                   85                  90                  95
   Ser His Val Thr Phe Pro Gly Thr Thr Ala Val Thr Leu Ser Gly Asp
               100                 105                 110
   Ser Ser Tyr Thr Thr Leu Gln Arg Val Ala Gly Ile Ser Arg Thr Gly
           115                 120                 125
   Met Gln Ile Asn Arg His Ser Leu Thr Thr Ser Tyr Leu Asp Leu Met
       130                 135                 140
   Ser His Ser Gly Thr Ser Leu Thr Gln Ser Val Ala Arg Ala Met Leu
   145                 150                 155                 160
   Arg Phe Val Thr Val Thr Ala Glu Ala Leu Arg Phe Arg Gln Ile Gln
                   165                 170                 175
   Arg Gly Phe Arg Thr Thr Leu Asp Asp Leu Ser Gly Arg Ser Tyr Val
               180                 185                 190
   Met Thr Ala Glu Asp Val Asp Leu Thr Leu Asn Trp Gly Arg Leu Ser
           195                 200                 205
   Ser Val Leu Pro Asp Tyr His Gly Gln Asp Ser Val Arg Val Gly Arg
       210                 215                 220
   Ile Ser Phe Gly Ser Ile Asn Ala Ile Leu Gly Ser Val Ala Leu Ile
   225                 230                 235                 240
   Leu Asn Cys His His His Ala Ser Arg Val Ala Arg Glu Phe Pro Lys
                   245                 250                 255
   Pro Ser Thr Pro Pro Gly Ser Ser Gly Gly Ala Pro Ala Ser Val Ser
               260                 265                 270
   Asp Val Pro Arg Asp Leu Glu Val Val Ala Ala Thr Pro Thr Ser Leu
           275                 280                 285
   Leu Ile Ser Trp Cys Arg Gln Arg Cys Ala Asp Ser Tyr Arg Ile Thr
       290                 295                 300
   Tyr Gly Glu Thr Gly Gly Asn Ser Pro Val Gln Glu Phe Thr Val Pro
   305                 310                 315                 320
   Gly Ser Trp Lys Thr Ala Thr Ile Ser Gly Leu Lys Pro Gly Val Asp
   325 330 335
   Tyr Thr Ile Thr Val Tyr Val Val Thr His Tyr Tyr Gly Trp Asp Arg
   340 345 350
   Tyr Ser His Pro Ile Ser Ile Asn Tyr Arg Thr Gly Ser Lys Asp Glu
   355 360 365
   Leu
<210> 30
   <211> 405
   <212> PRT
   <213> Artificial Sequence
<220>
   <221> source
   <223> /note="Description of Artificial Sequence: Synthetic polypeptide"
<400> 30
   Met Ala Pro Thr Ser Ser Ser Thr Lys Lys Thr Gln Leu Gln Leu Glu
   1 5 10 15
   His Leu Leu Leu Asp Leu Gln Met Ile Leu Asn Gly Ile Asn Asn Tyr
   20 25 30
   Lys Asn Pro Lys Leu Thr Arg Met Leu Thr Phe Lys Phe Tyr Met Pro
   35 40 45
   Lys Lys Ala Thr Glu Leu Lys His Leu Gln Cys Leu Glu Glu Glu Leu
   50 55 60
   Lys Pro Leu Glu Glu Val Leu Asn Leu Ala Gln Ser Lys Asn Phe His
   65 70 75 80
   Leu Arg Pro Arg Asp Leu Ile Ser Asn Ile Asn Val Ile Val Leu Glu
   85 90 95
   Leu Lys Gly Ser Glu Thr Thr Phe Met Cys Glu Tyr Ala Asp Glu Thr
   100 105 110
   Ala Thr Ile Val Glu Phe Leu Asn Arg Trp Ile Thr Phe Cys Gln Ser
   115 120 125
   Ile Ile Ser Thr Leu Thr Glu Phe Pro Lys Pro Ser Thr Pro Pro Gly
   130 135 140
   Ser Ser Gly Gly Ala Pro Lys Glu Phe Thr Leu Asp Phe Ser Thr Ala
   145 150 155 160
   Lys Thr Tyr Val Asp Ser Leu Asn Val Ile Arg Ser Ala Ile Gly Thr
   165 170 175
   Pro Leu Gln Thr Ile Ser Ser Gly Gly Thr Ser Leu Leu Met Ile Asp
   180 185 190
   Ser Gly Ser Gly Asp Asn Leu Phe Ala Val Asp Val Arg Gly Ile Asp
   195 200 205
   Pro Glu Glu Gly Arg Phe Asn Asn Leu Arg Leu Ile Val Glu Arg Asn
   210 215 220
   Asn Leu Tyr Val Thr Gly Phe Val Asn Arg Thr Asn Asn Val Phe Tyr
   225 230 235 240
   Arg Phe Ala Asp Phe Ser His Val Thr Phe Pro Gly Thr Thr Ala Val
   245 250 255
   Thr Leu Ser Gly Asp Ser Ser Tyr Thr Thr Leu Gln Arg Val Ala Gly
   260 265 270
   Ile Ser Arg Thr Gly Met Gln Ile Asn Arg His Ser Leu Thr Thr Ser
   275 280 285
   Tyr Leu Asp Leu Met Ser His Ser Gly Thr Ser Leu Thr Gln Ser Val
   290 295 300
   Ala Arg Ala Met Leu Arg Phe Val Thr Val Thr Ala Glu Ala Leu Arg
   305 310 315 320
   Phe Arg Gln Ile Gln Arg Gly Phe Arg Thr Thr Leu Asp Asp Leu Ser
   325 330 335
   Gly Arg Ser Tyr Val Met Thr Ala Glu Asp Val Asp Leu Thr Leu Asn
   340 345 350
   Trp Gly Arg Leu Ser Ser Val Leu Pro Asp Tyr His Gly Gln Asp Ser
   355 360 365
   Val Arg Val Gly Arg Ile Ser Phe Gly Ser Ile Asn Ala Ile Leu Gly
   370 375 380
   Ser Val Ala Leu Ile Leu Asn Cys His His His Ala Ser Arg Val Ala
   385 390 395 400
   Arg Lys Asp Glu Leu
   405
<210> 31
   <211> 414
   <212> PRT
   <213> Artificial Sequence
<220>
   <221> source
   <223> /note="Description of Artificial Sequence: Synthetic polypeptide"
<400> 31
   Met Ala Pro Thr Ser Ser Ser Thr Lys Lys Thr Gln Leu Gln Leu Glu
   1               5                   10                  15
   His Leu Leu Leu Asp Leu Gln Met Ile Leu Asn Gly Ile Asn Asn Tyr
               20                  25                  30
   Lys Asn Pro Lys Leu Thr Arg Met Leu Thr Phe Lys Phe Tyr Met Pro
           35                  40                  45
   Lys Lys Ala Thr Glu Leu Lys His Leu Gln Cys Leu Glu Glu Glu Leu
       50                  55                  60
   Lys Pro Leu Glu Glu Val Leu Asn Leu Ala Gln Ser Lys Asn Phe His
   65                  70                  75                  80
   Leu Arg Pro Arg Asp Leu Ile Ser Asn Ile Asn Val Ile Val Leu Glu
                   85                  90                  95
   Leu Lys Gly Ser Glu Thr Thr Phe Met Cys Glu Tyr Ala Asp Glu Thr
               100                 105                 110
   Ala Thr Ile Val Glu Phe Leu Asn Arg Trp Ile Thr Phe Cys Gln Ser
           115                 120                 125
   Ile Ile Ser Thr Leu Thr Glu Phe Pro Lys Pro Ser Thr Pro Pro Gly
       130                 135                 140
   Ser Ser Gly Gly Ala Pro Gly Ile Leu Gly Phe Val Phe Thr Leu Lys
   145                 150                 155                 160
   Glu Phe Thr Leu Asp Phe Ser Thr Ala Lys Thr Tyr Val Asp Ser Leu
                   165                 170                 175
   Asn Val Ile Arg Ser Ala Ile Gly Thr Pro Leu Gln Thr Ile Ser Ser
   180 185 190
   Gly Gly Thr Ser Leu Leu Met Ile Asp Ser Gly Ser Gly Asp Asn Leu
   195 200 205
   Phe Ala Val Asp Val Arg Gly Ile Asp Pro Glu Glu Gly Arg Phe Asn
   210 215 220
   Asn Leu Arg Leu Ile Val Glu Arg Asn Asn Leu Tyr Val Thr Gly Phe
   225 230 235 240
   Val Asn Arg Thr Asn Asn Val Phe Tyr Arg Phe Ala Asp Phe Ser His
   245 250 255
   Val Thr Phe Pro Gly Thr Thr Ala Val Thr Leu Ser Gly Asp Ser Ser
   260 265 270
   Tyr Thr Thr Leu Gln Arg Val Ala Gly Ile Ser Arg Thr Gly Met Gln
   275 280 285
   Ile Asn Arg His Ser Leu Thr Thr Ser Tyr Leu Asp Leu Met Ser His
   290 295 300
   Ser Gly Thr Ser Leu Thr Gln Ser Val Ala Arg Ala Met Leu Arg Phe
   305 310 315 320
   Val Thr Val Thr Ala Glu Ala Leu Arg Phe Arg Gln Ile Gln Arg Gly
   325 330 335
   Phe Arg Thr Thr Leu Asp Asp Leu Ser Gly Arg Ser Tyr Val Met Thr
   340 345 350
   Ala Glu Asp Val Asp Leu Thr Leu Asn Trp Gly Arg Leu Ser Ser Val
   355 360 365
   Leu Pro Asp Tyr His Gly Gln Asp Ser Val Arg Val Gly Arg Ile Ser
   370 375 380
   Phe Gly Ser Ile Asn Ala Ile Leu Gly Ser Val Ala Leu Ile Leu Asn
   385 390 395 400
   Cys His His His Ala Ser Arg Val Ala Arg Lys Asp Glu Leu
   405 410
<210> 32
   <211> 405
   <212> PRT
   <213> Artificial Sequence
<220>
   <221> source
   <223> /note="Description of Artificial Sequence: Synthetic polypeptide"
<400> 32
   Met Ala Pro Thr Ser Ser Ser Thr Lys Lys Thr Gln Leu Gln Leu Glu
   1               5                   10                  15
   His Leu Leu Leu Asp Leu Gln Met Ile Leu Asn Gly Ile Asn Asn Tyr
               20                  25                  30
   Lys Asn Pro Lys Leu Thr Arg Met Leu Thr Phe Lys Phe Tyr Met Pro
           35                  40                  45
   Lys Lys Ala Thr Glu Leu Lys His Leu Gln Cys Leu Glu Glu Glu Leu
       50                  55                  60
   Lys Pro Leu Glu Glu Val Leu Asn Leu Ala Gln Ser Lys Asn Phe His
   65                  70                  75                  80
   Leu Arg Pro Arg Asp Leu Ile Ser Asn Ile Asn Val Ile Val Leu Glu
                   85                  90                  95
   Leu Lys Gly Ser Glu Thr Thr Phe Met Cys Glu Tyr Ala Asp Glu Thr
               100                 105                 110
   Ala Thr Ile Val Glu Phe Leu Asn Arg Trp Ile Thr Phe Cys Gln Ser
           115                 120                 125
   Ile Ile Ser Thr Leu Thr Glu Phe Pro Lys Pro Ser Thr Pro Pro Gly
       130                 135                 140
   Ser Ser Gly Gly Ala Pro Lys Glu Phe Thr Leu Asp Phe Ser Thr Ala
   145                 150                 155                 160
   Lys Thr Tyr Val Asp Ser Leu Asn Val Ile Arg Ser Ala Ile Gly Thr
                   165                 170                 175
   Pro Leu Gln Thr Ile Ser Ser Gly Gly Thr Ser Leu Leu Met Ile Asp
               180                 185                 190
   Ser Gly Thr Gly Asp Asn Leu Phe Ala Val Asp Val Arg Gly Ile Asp
           195                 200                 205
   Pro Glu Glu Gly Arg Phe Asn Asn Leu Arg Leu Ile Val Glu Arg Asn
       210                 215                 220
   Asn Leu Tyr Val Thr Gly Phe Val Asn Arg Thr Asn Asn Val Phe Tyr
   225 230 235 240
   Arg Phe Ala Asp Phe Ser His Val Thr Phe Pro Gly Thr Thr Ala Val
   245 250 255
   Thr Leu Ser Gly Asp Ser Ser Tyr Thr Thr Leu Gln Arg Val Ala Gly
   260 265 270
   Ile Ser Arg Thr Gly Met Gln Ile Asn Arg His Ser Leu Thr Thr Ser
   275 280 285
   Tyr Leu Asp Leu Met Ser His Ser Gly Thr Ser Leu Thr Gln Ser Val
   290 295 300
   Ala Arg Ala Met Leu Arg Phe Val Thr Val Thr Ala Glu Ala Leu Arg
   305 310 315 320
   Phe Arg Gln Ile Gln Arg Gly Phe Arg Thr Thr Leu Asp Asp Leu Ser
   325 330 335
   Gly Arg Ser Tyr Val Met Thr Ala Glu Asp Val Asp Leu Thr Leu Asn
   340 345 350
   Trp Gly Arg Leu Ser Ser Val Leu Pro Asp Tyr His Gly Gln Asp Ser
   355 360 365
   Val Arg Val Gly Arg Ile Ser Phe Gly Ser Ile Asn Ala Ile Leu Gly
   370 375 380
   Ser Val Ala Leu Ile Leu Asn Cys His His His Ala Ser Arg Val Ala
   385 390 395 400
   Arg Lys Asp Glu Leu
   405
<210> 33
   <211> 414
   <212> PRT
   <213> Artificial Sequence
<220>
   <221> source
   <223> /note="Description of Artificial Sequence: Synthetic polypeptide"
<400> 33
   Met Ala Pro Thr Ser Ser Ser Thr Lys Lys Thr Gln Leu Gln Leu Glu
   1 5 10 15
   His Leu Leu Leu Asp Leu Gln Met Ile Leu Asn Gly Ile Asn Asn Tyr
               20                  25                  30
   Lys Asn Pro Lys Leu Thr Arg Met Leu Thr Phe Lys Phe Tyr Met Pro
           35                  40                  45
   Lys Lys Ala Thr Glu Leu Lys His Leu Gln Cys Leu Glu Glu Glu Leu
       50                  55                  60
   Lys Pro Leu Glu Glu Val Leu Asn Leu Ala Gln Ser Lys Asn Phe His
   65                  70                  75                  80
   Leu Arg Pro Arg Asp Leu Ile Ser Asn Ile Asn Val Ile Val Leu Glu
                   85                  90                  95
   Leu Lys Gly Ser Glu Thr Thr Phe Met Cys Glu Tyr Ala Asp Glu Thr
               100                 105                 110
   Ala Thr Ile Val Glu Phe Leu Asn Arg Trp Ile Thr Phe Cys Gln Ser
           115                 120                 125
   Ile Ile Ser Thr Leu Thr Glu Phe Pro Lys Pro Ser Thr Pro Pro Gly
       130                 135                 140
   Ser Ser Gly Gly Ala Pro Gly Ile Leu Gly Phe Val Phe Thr Leu Lys
   145                 150                 155                 160
   Glu Phe Thr Leu Asp Phe Ser Thr Ala Lys Thr Tyr Val Asp Ser Leu
                   165                 170                 175
   Asn Val Ile Arg Ser Ala Ile Gly Thr Pro Leu Gln Thr Ile Ser Ser
               180                 185                 190
   Gly Gly Thr Ser Leu Leu Met Ile Asp Ser Gly Thr Gly Asp Asn Leu
           195                 200                 205
   Phe Ala Val Asp Val Arg Gly Ile Asp Pro Glu Glu Gly Arg Phe Asn
       210                 215                 220
   Asn Leu Arg Leu Ile Val Glu Arg Asn Asn Leu Tyr Val Thr Gly Phe
   225                 230                 235                 240
   Val Asn Arg Thr Asn Asn Val Phe Tyr Arg Phe Ala Asp Phe Ser His
                   245                 250                 255
   Val Thr Phe Pro Gly Thr Thr Ala Val Thr Leu Ser Gly Asp Ser Ser
               260                 265                 270
   Tyr Thr Thr Leu Gln Arg Val Ala Gly Ile Ser Arg Thr Gly Met Gln
   275 280 285
   Ile Asn Arg His Ser Leu Thr Thr Ser Tyr Leu Asp Leu Met Ser His
   290 295 300
   Ser Gly Thr Ser Leu Thr Gln Ser Val Ala Arg Ala Met Leu Arg Phe
   305 310 315 320
   Val Thr Val Thr Ala Glu Ala Leu Arg Phe Arg Gln Ile Gln Arg Gly
   325 330 335
   Phe Arg Thr Thr Leu Asp Asp Leu Ser Gly Arg Ser Tyr Val Met Thr
   340 345 350
   Ala Glu Asp Val Asp Leu Thr Leu Asn Trp Gly Arg Leu Ser Ser Val
   355 360 365
   Leu Pro Asp Tyr His Gly Gln Asp Ser Val Arg Val Gly Arg Ile Ser
   370 375 380
   Phe Gly Ser Ile Asn Ala Ile Leu Gly Ser Val Ala Leu Ile Leu Asn
   385 390 395 400
   Ser His His His Ala Ser Arg Val Ala Arg Lys Asp Glu Leu
   405 410
<210> 34
   <211> 4
   <212> PRT
   <213> Artificial Sequence
<220>
   <221> source
   <223> /note="Description of Artificial Sequence: Synthetic peptide"
<400> 34
   Lys Asp Glu Leu
   1
<210> 35
   <211> 4
   <212> PRT
   <213> Artificial Sequence
<220>
   <221> source
   <223> /note="Description of Artificial Sequence: Synthetic peptide"
<400> 35
   His Asp Glu Phe
   1
<210> 36
   <211> 4
   <212> PRT
   <213> Artificial Sequence
<220>
   <221> source
   <223> /note="Description of Artificial Sequence: Synthetic peptide"
<400> 36
   His Asp Glu Leu
   1
<210> 37
   <211> 4
   <212> PRT
   <213> Artificial Sequence
<220>
   <221> source
   <223> /note="Description of Artificial Sequence: Synthetic peptide"
<400> 37
   Arg Asp Glu Phe
   1
<210> 38
   <211> 4
   <212> PRT
   <213> Artificial Sequence
<220>
   <221> source
   <223> /note="Description of Artificial Sequence: Synthetic peptide"
<400> 38
   Arg Asp Glu Leu
   1
<210> 39
   <211> 4
   <212> PRT
   <213> Artificial Sequence
<220>
   <221> source
   <223> /note="Description of Artificial Sequence: Synthetic peptide"
<400> 39
   Trp Asp Glu Leu
   1
<210> 40
   <211> 4
   <212> PRT
   <213> Artificial Sequence
<220>
   <221> source
   <223> /note="Description of Artificial Sequence: Synthetic peptide"
<400> 40
   Tyr Asp Glu Leu
   1
<210> 41
   <211> 4
   <212> PRT
   <213> Artificial Sequence
<220>
   <221> source
   <223> /note="Description of Artificial Sequence: Synthetic peptide"
<400> 41
   His Glu Glu Phe
   1
<210> 42
   <211> 4
   <212> PRT
   <213> Artificial Sequence
<220>
   <221> source
   <223> /note="Description of Artificial Sequence: Synthetic peptide"
<400> 42
   His Glu Glu Leu
   1
<210> 43
   <211> 4
   <212> PRT
   <213> Artificial Sequence
<220>
   <221> source
   <223> /note="Description of Artificial Sequence: Synthetic peptide"
<400> 43
   Lys Glu Glu Leu
   1
<210> 44
   <211> 4
   <212> PRT
   <213> Artificial Sequence
<220>
   <221> source
   <223> /note="Description of Artificial Sequence: Synthetic peptide"
<400> 44
   Arg Glu Glu Leu
   1
<210> 45
   <211> 4
   <212> PRT
   <213> Artificial Sequence
<220>
   <221> source
   <223> /note="Description of Artificial Sequence: Synthetic peptide"
<400> 45
   Lys Ala Glu Leu
   1
<210> 46
   <211> 4
   <212> PRT
   <213> Artificial Sequence
<220>
   <221> source
   <223> /note="Description of Artificial Sequence: Synthetic peptide"
<400> 46
   Lys Cys Glu Leu
   1
<210> 47
   <211> 4
   <212> PRT
   <213> Artificial Sequence
<220>
   <221> source
   <223> /note="Description of Artificial Sequence: Synthetic peptide"
<400> 47
   Lys Phe Glu Leu
   1
<210> 48
   <211> 4
   <212> PRT
   <213> Artificial Sequence
<220>
   <221> source
   <223> /note="Description of Artificial Sequence: Synthetic peptide"
<400> 48
   Lys Gly Glu Leu
   1
<210> 49
   <211> 4
   <212> PRT
   <213> Artificial Sequence
<220>
   <221> source
   <223> /note="Description of Artificial Sequence: Synthetic peptide"
<400> 49
   Lys His Glu Leu
   1
<210> 50
   <211> 4
   <212> PRT
   <213> Artificial Sequence
<220>
   <221> source
   <223> /note="Description of Artificial Sequence: Synthetic peptide"
<400> 50
   Lys Leu Glu Leu
   1
<210> 51
   <211> 4
   <212> PRT
   <213> Artificial Sequence
<220>
   <221> source
   <223> /note="Description of Artificial Sequence: Synthetic peptide"
<400> 51
   Lys Asn Glu Leu
   1
<210> 52
   <211> 4
   <212> PRT
   <213> Artificial Sequence
<220>
   <221> source
   <223> /note="Description of Artificial Sequence: Synthetic peptide"
<400> 52
   Lys Gln Glu Leu
   1
<210> 53
   <211> 4
   <212> PRT
   <213> Artificial Sequence
<220>
   <221> source
   <223> /note="Description of Artificial Sequence: Synthetic peptide"
<400> 53
   Lys Arg Glu Leu
   1
<210> 54
   <211> 4
   <212> PRT
   <213> Artificial Sequence
<220>
   <221> source
   <223> /note="Description of Artificial Sequence: Synthetic peptide"
<400> 54
   Lys Ser Glu Leu
   1
<210> 55
   <211> 4
   <212> PRT
   <213> Artificial Sequence
<220>
   <221> source
   <223> /note="Description of Artificial Sequence: Synthetic peptide"
<400> 55
   Lys Val Glu Leu
   1
<210> 56
   <211> 4
   <212> PRT
   <213> Artificial Sequence
<220>
   <221> source
   <223> /note="Description of Artificial Sequence: Synthetic peptide"
<400> 56
   Lys Trp Glu Leu
   1
<210> 57
   <211> 4
   <212> PRT
   <213> Artificial Sequence
<220>
   <221> source
   <223> /note="Description of Artificial Sequence: Synthetic peptide"
<400> 57
   Lys Tyr Glu Leu
   1
<210> 58
   <211> 4
   <212> PRT
   <213> Artificial Sequence
<220>
   <221> source
   <223> /note="Description of Artificial Sequence: Synthetic peptide"
<400> 58
   Lys Glu Asp Leu
   1
<210> 59
   <211> 4
   <212> PRT
   <213> Artificial Sequence
<220>
   <221> source
   <223> /note="Description of Artificial Sequence: Synthetic peptide"
<400> 59
   Lys Ile Glu Leu
   1
<210> 60
   <211> 4
   <212> PRT
   <213> Artificial Sequence
<220>
   <221> source
   <223> /note="Description of Artificial Sequence: Synthetic peptide"
<400> 60
   Asp Lys Glu Leu
   1
<210> 61
   <211> 4
   <212> PRT
   <213> Artificial Sequence
<220>
   <221> source
   <223> /note="Description of Artificial Sequence: Synthetic peptide"
<400> 61
   Phe Asp Glu Leu
   1
<210> 62
   <211> 4
   <212> PRT
   <213> Artificial Sequence
<220>
   <221> source
   <223> /note="Description of Artificial Sequence: Synthetic peptide"
<400> 62
   Lys Asp Glu Phe
   1
<210> 63
   <211> 4
   <212> PRT
   <213> Artificial Sequence
<220>
   <221> source
   <223> /note="Description of Artificial Sequence: Synthetic peptide"
<400> 63
   Lys Lys Glu Leu
   1
<210> 64
   <211> 4
   <212> PRT
   <213> Artificial Sequence
<220>
   <221> source
   <223> /note="Description of Artificial Sequence: Synthetic peptide"
<400> 64
   His Ala Asp Leu
   1
<210> 65
   <211> 4
   <212> PRT
   <213> Artificial Sequence
<220>
   <221> source
   <223> /note="Description of Artificial Sequence: Synthetic peptide"
<400> 65
   His Ala Glu Leu
   1
<210> 66
   <211> 4
   <212> PRT
   <213> Artificial Sequence
<220>
   <221> source
   <223> /note="Description of Artificial Sequence: Synthetic peptide"
<400> 66
   His Ile Glu Leu
   1
<210> 67
   <211> 4
   <212> PRT
   <213> Artificial Sequence
<220>
   <221> source
   <223> /note="Description of Artificial Sequence: Synthetic peptide"
<400> 67
   His Asn Glu Leu
   1
<210> 68
   <211> 4
   <212> PRT
   <213> Artificial Sequence
<220>
   <221> source
   <223> /note="Description of Artificial Sequence: Synthetic peptide"
<400> 68
   His Thr Glu Leu
   1
<210> 69
   <211> 4
   <212> PRT
   <213> Artificial Sequence
<220>
   <221> source
   <223> /note="Description of Artificial Sequence: Synthetic peptide"
<400> 69
   Lys Thr Glu Leu
   1
<210> 70
   <211> 4
   <212> PRT
   <213> Artificial Sequence
<220>
   <221> source
   <223> /note="Description of Artificial Sequence: Synthetic peptide"
<400> 70
   His Val Glu Leu
   1
<210> 71
   <211> 4
   <212> PRT
   <213> Artificial Sequence
<220>
   <221> source
   <223> /note="Description of Artificial Sequence: Synthetic peptide"
<400> 71
   Asn Asp Glu Leu
   1
<210> 72
   <211> 4
   <212> PRT
   <213> Artificial Sequence
<220>
   <221> source
   <223> /note="Description of Artificial Sequence: Synthetic peptide"
<400> 72
   Gln Asp Glu Leu
   1
<210> 73
   <211> 4
   <212> PRT
   <213> Artificial Sequence
<220>
   <221> source
   <223> /note="Description of Artificial Sequence: Synthetic peptide"
<400> 73
   Arg Glu Asp Leu
   1
<210> 74
   <211> 4
   <212> PRT
   <213> Artificial Sequence
<220>
   <221> source
   <223> /note="Description of Artificial Sequence: Synthetic peptide"
<400> 74
   Arg Asn Glu Leu
   1
<210> 75
   <211> 4
   <212> PRT
   <213> Artificial Sequence
<220>
   <221> source
   <223> /note="Description of Artificial Sequence: Synthetic peptide"
<400> 75
   Arg Thr Asp Leu
   1
<210> 76
   <211> 4
   <212> PRT
   <213> Artificial Sequence
<220>
   <221> source
   <223> /note="Description of Artificial Sequence: Synthetic peptide"
<400> 76
   Arg Thr Glu Leu
   1
<210> 77
   <211> 4
   <212> PRT
   <213> Artificial Sequence
<220>
   <221> source
   <223> /note="Description of Artificial Sequence: Synthetic peptide"
<400> 77
   Ser Asp Glu Leu
   1
<210> 78
   <211> 4
   <212> PRT
   <213> Artificial Sequence
<220>
   <221> source
   <223> /note="Description of Artificial Sequence: Synthetic peptide"
<400> 78
   Thr Asp Glu Leu
   1
<210> 79
   <211> 4
   <212> PRT
   <213> Artificial Sequence
<220>
   <221> source
   <223> /note="Description of Artificial Sequence: Synthetic peptide"
<400> 79
   Ser Lys Glu Leu
   1
<210> 80
   <211> 4
   <212> PRT
   <213> Artificial Sequence
<220>
   <221> source
   <223> /note="Description of Artificial Sequence: Synthetic peptide"
<400> 80
   Ser Thr Glu Leu
   1
<210> 81
   <211> 4
   <212> PRT
   <213> Artificial Sequence
<220>
   <221> source
   <223> /note="Description of Artificial Sequence: Synthetic peptide"
<400> 81
   Glu Asp Glu Leu
   1
<210> 82
   <211> 6
   <212> PRT
   <213> Artificial Sequence
<220>
   <221> source
   <223> /note="Description of Artificial Sequence: Synthetic peptide"
<400> 82
   Ala Leu Glu Asp Glu Leu
   1 5
<210> 83
   <211> 6
   <212> PRT
   <213> Artificial Sequence
<220>
   <221> source
   <223> /note="Description of Artificial Sequence: Synthetic peptide"
<400> 83
   His Ala Glu Asp Glu Leu
   1 5
<210> 84
   <211> 6
   <212> PRT
   <213> Artificial Sequence
<220>
   <221> source
   <223> /note="Description of Artificial Sequence: Synthetic peptide"
<400> 84
   His Leu Glu Asp Glu Leu
   1 5
<210> 85
   <211> 6
   <212> PRT
   <213> Artificial Sequence
<220>
   <221> source
   <223> /note="Description of Artificial Sequence: Synthetic peptide"
<400> 85
   Lys Leu Glu Asp Glu Leu
   1 5
<210> 86
   <211> 6
   <212> PRT
   <213> Artificial Sequence
<220>
   <221> source
   <223> /note="Description of Artificial Sequence: Synthetic peptide"
<400> 86
   Ile Arg Ser Asp Glu Leu
   1 5
<210> 87
   <211> 6
   <212> PRT
   <213> Artificial Sequence
<220>
   <221> source
   <223> /note="Description of Artificial Sequence: Synthetic peptide"
<400> 87
   Glu Arg Ser Thr Glu Leu
   1 5
<210> 88
   <211> 6
   <212> PRT
   <213> Artificial Sequence
<220>
   <221> source
   <223> /note="Description of Artificial Sequence: Synthetic peptide"
<400> 88
   Arg Pro Ser Thr Glu Leu
   1 5
<210> 89
   <211> 6
   <212> PRT
   <213> Artificial Sequence
<220>
   <221> source
   <223> /note="Description of Artificial Sequence: Synthetic peptide"
<400> 89
   Ala Ser Gly Gly Pro Glu
   1 5
<210> 90
   <211> 54
   <212> PRT
   <213> Artificial Sequence
<220>
   <221> source
   <223> /note="Description of Artificial Sequence: Synthetic polypeptide"
<220>
   <221> VARIANT
   <222> (5)..(6)
   <223> /replace=" "
<220>
   <221> VARIANT
   <222> (10)..(11)
   <223> /replace=" "
<220>
   <221> VARIANT
   <222> (15)..(16)
   <223> /replace=" "
<220>
   <221> VARIANT
   <222> (20)..(21)
   <223> /replace=" "
<220>
   <221> VARIANT
   <222> (25)..(26)
   <223> /replace=" "
<220>
   <221> VARIANT
   <222> (30)..(31)
   <223> /replace=" "
<220>
   <221> VARIANT
   <222> (35)..(36)
   <223> /replace=" "
<220>
   <221> VARIANT
   <222> (40)..(41)
   <223> /replace=" "
<220>
   <221> VARIANT
   <222> (45)..(46)
   <223> /replace=" "
<220>
   <221> VARIANT
   <222> (50)..(51)
   <223> /replace=" "
<220>
   <221> MISC_FEATURE
   <222> (1)..(54)
   <223> /note="Variant residues given in the sequence have no preference with respect to those in the annotations for variant positions"
<220>
   <221> MISC_FEATURE
   <222> (3)..(52)
   <223> /note="This region may encompass 1 to 10 'Gly Gly Gly Gly Ser' repeating units, wherein some positions may be absent"
<220>
   <221> source
   <223> /note="See specification as filed for detailed description of substitutions and preferred embodiments"
<400> 90
   Ala Met Gly Gly Gly Gly Ser Gly Gly Gly Gly Ser Gly Gly Gly Gly
   1               5                   10                  15
   Ser Gly Gly Gly Gly Ser Gly Gly Gly Gly Ser Gly Gly Gly Gly Ser
               20                  25                  30
   Gly Gly Gly Gly Ser Gly Gly Gly Gly Ser Gly Gly Gly Gly Ser Gly
           35                  40                  45
   Gly Gly Gly Ser Ala Met
       50
<210> 91
   <211> 210
   <212> PRT
   <213> Artificial Sequence
<220>
   <221> source
   <223> /note="Description of Artificial Sequence: Synthetic polypeptide"
<220>
   <221> VARIANT
   <222> (2)..(6)
   <223> /replace=" "
<220>
   <221> VARIANT
   <222> (9)..(13)
   <223> /replace=" "
<220>
   <221> VARIANT
   <222> (16)..(20)
   <223> /replace=" "
<220>
   <221> VARIANT
   <222> (23)..(27)
   <223> /replace=" "
<220>
   <221> VARIANT
   <222> (30)..(34)
   <223> /replace=" "
<220>
   <221> VARIANT
   <222> (37)..(41)
   <223> /replace=" "
<220>
   <221> VARIANT
   <222> (44)..(48)
   <223> /replace=" "
<220>
   <221> VARIANT
   <222> (51)..(55)
   <223> /replace=" "
<220>
   <221> VARIANT
   <222> (58)..(62)
   <223> /replace=" "
<220>
   <221> VARIANT
   <222> (65)..(69)
   <223> /replace=" "
<220>
   <221> VARIANT
   <222> (72)..(76)
   <223> /replace=" "
<220>
   <221> VARIANT
   <222> (79)..(83)
   <223> /replace=" "
<220>
   <221> VARIANT
   <222> (86)..(90)
   <223> /replace=" "
<220>
   <221> VARIANT
   <222> (93)..(97)
   <223> /replace=" "
<220>
   <221> VARIANT
   <222> (100)..(104)
   <223> /replace=" "
<220>
   <221> VARIANT
   <222> (107)..(111)
   <223> /replace=" "
<220>
   <221> VARIANT
   <222> (114)..(118)
   <223> /replace=" "
<220>
   <221> VARIANT
   <222> (121)..(125)
   <223> /replace=" "
<220>
   <221> VARIANT
   <222> (128)..(132)
   <223> /replace=" "
<220>
   <221> VARIANT
   <222> (135)..(139)
   <223> /replace=" "
<220>
   <221> VARIANT
   <222> (142)..(146)
   <223> /replace=" "
<220>
   <221> VARIANT
   <222> (149)..(153)
   <223> /replace=" "
<220>
   <221> VARIANT
   <222> (156)..(160)
   <223> /replace=" "
<220>
   <221> VARIANT
   <222> (163)..(167)
   <223> /replace=" "
<220>
   <221> VARIANT
   <222> (170)..(174)
   <223> /replace=" "
<220>
   <221> VARIANT
   <222> (177)..(181)
   <223> /replace=" "
<220>
   <221> VARIANT
   <222> (184)..(188)
   <223> /replace=" "
<220>
   <221> VARIANT
   <222> (191)..(195)
   <223> /replace=" "
<220>
   <221> VARIANT
   <222> (198)..(202)
   <223> /replace=" "
<220>
   <221> VARIANT
   <222> (205)..(209)
   <223> /replace=" "
<220>
   <221> MISC_FEATURE
   <222> (1)..(210)
   <223> /note="This sequence may encompass 1 to 30 'Gly Gly Gly Gly Gly Gly Ser' repeating units, wherein some positions may be absent"
<220>
   <221> MISC_FEATURE
   <222> (1)..(210)
   <223> /note="Variant residues given in the sequence have no preference with respect to those in the annotations for variant positions"
<220>
   <221> source
   <223> /note="See specification as filed for detailed description of substitutions and preferred embodiments"
<400> 91
   Gly Gly Gly Gly Gly Gly Ser Gly Gly Gly Gly Gly Gly Ser Gly Gly
   1               5                   10                  15
   Gly Gly Gly Gly Ser Gly Gly Gly Gly Gly Gly Ser Gly Gly Gly Gly
               20                  25                  30
   Gly Gly Ser Gly Gly Gly Gly Gly Gly Ser Gly Gly Gly Gly Gly Gly
           35                  40                  45
   Ser Gly Gly Gly Gly Gly Gly Ser Gly Gly Gly Gly Gly Gly Ser Gly
       50                  55                  60
   Gly Gly Gly Gly Gly Ser Gly Gly Gly Gly Gly Gly Ser Gly Gly Gly
   65                  70                  75                      80
   Gly Gly Gly Ser Gly Gly Gly Gly Gly Gly Ser Gly Gly Gly Gly Gly
                   85                  90                  95
   Gly Ser Gly Gly Gly Gly Gly Gly Ser Gly Gly Gly Gly Gly Gly Ser
               100                 105                 110
   Gly Gly Gly Gly Gly Gly Ser Gly Gly Gly Gly Gly Gly Ser Gly Gly
           115                 120                 125
   Gly Gly Gly Gly Ser Gly Gly Gly Gly Gly Gly Ser Gly Gly Gly Gly
       130                 135                 140
   Gly Gly Ser Gly Gly Gly Gly Gly Gly Ser Gly Gly Gly Gly Gly Gly
   145                 150                 155                     160
   Ser Gly Gly Gly Gly Gly Gly Ser Gly Gly Gly Gly Gly Gly Ser Gly
                   165                 170                 175
   Gly Gly Gly Gly Gly Ser Gly Gly Gly Gly Gly Gly Ser Gly Gly Gly
               180                 185                 190
   Gly Gly Gly Ser Gly Gly Gly Gly Gly Gly Ser Gly Gly Gly Gly Gly
           195                 200                 205
   Gly Ser
       210
<210> 92
   <211> 210
   <212> PRT
   <213> Artificial Sequence
<220>
   <221> source
   <223> /note="Description of Artificial Sequence: Synthetic polypeptide"
<220>
   <221> VARIANT
   <222> (2)..(6)
   <223> /replace=" "
<220>
   <221> VARIANT
   <222> (9)..(13)
   <223> /replace=" "
<220>
   <221> VARIANT
   <222> (16)..(20)
   <223> /replace=" "
<220>
   <221> VARIANT
   <222> (23)..(27)
   <223> /replace=" "
<220>
   <221> VARIANT
   <222> (30)..(34)
   <223> /replace=" "
<220>
   <221> VARIANT
   <222> (37)..(41)
   <223> /replace=" "
<220>
   <221> VARIANT
   <222> (44)..(48)
   <223> /replace=" "
<220>
   <221> VARIANT
   <222> (51)..(55)
   <223> /replace=" "
<220>
   <221> VARIANT
   <222> (58)..(62)
   <223> /replace=" "
<220>
   <221> VARIANT
   <222> (65)..(69)
   <223> /replace=" "
<220>
   <221> VARIANT
   <222> (72)..(76)
   <223> /replace=" "
<220>
   <221> VARIANT
   <222> (79)..(83)
   <223> /replace=" "
<220>
   <221> VARIANT
   <222> (86)..(90)
   <223> /replace=" "
<220>
   <221> VARIANT
   <222> (93)..(97)
   <223> /replace=" "
<220>
   <221> VARIANT
   <222> (100)..(104)
   <223> /replace=" "
<220>
   <221> VARIANT
   <222> (107)..(111)
   <223> /replace=" "
<220>
   <221> VARIANT
   <222> (114)..(118)
   <223> /replace=" "
<220>
   <221> VARIANT
   <222> (121)..(125)
   <223> /replace=" "
<220>
   <221> VARIANT
   <222> (128)..(132)
   <223> /replace=" "
<220>
   <221> VARIANT
   <222> (135)..(139)
   <223> /replace=" "
<220>
   <221> VARIANT
   <222> (142)..(146)
   <223> /replace=" "
<220>
   <221> VARIANT
   <222> (149)..(153)
   <223> /replace=" "
<220>
   <221> VARIANT
   <222> (156)..(160)
   <223> /replace=" "
<220>
   <221> VARIANT
   <222> (163)..(167)
   <223> /replace=" "
<220>
   <221> VARIANT
   <222> (170)..(174)
   <223> /replace=" "
<220>
   <221> VARIANT
   <222> (177)..(181)
   <223> /replace=" "
<220>
   <221> VARIANT
   <222> (184)..(188)
   <223> /replace=" "
<220>
   <221> VARIANT
   <222> (191)..(195)
   <223> /replace=" "
<220>
   <221> VARIANT
   <222> (198)..(202)
   <223> /replace=" "
<220>
   <221> VARIANT
   <222> (205)..(209)
   <223> /replace=" "
<220>
   <221> MISC_FEATURE
   <222> (1)..(210)
   <223> /note="This sequence may encompass 1 to 30 'Ser Ser Ser Ser Ser Ser Gly' repeating units, wherein some positions may be absent"
<220>
   <221> MISC_FEATURE
   <222> (1)..(210)
   <223> /note="Variant residues given in the sequence have no preference with respect to those in the annotations for variant positions"
<220>
   <221> source
   <223> /note="See specification as filed for detailed description of substitutions and preferred embodiments"
<400> 92
   Ser Ser Ser Ser Ser Ser Gly Ser Ser Ser Ser Ser Ser Gly Ser Ser
   1               5                   10                  15
   Ser Ser Ser Ser Gly Ser Ser Ser Ser Ser Ser Gly Ser Ser Ser Ser
               20                  25                  30
   Ser Ser Gly Ser Ser Ser Ser Ser Ser Gly Ser Ser Ser Ser Ser Ser
           35                  40                  45
   Gly Ser Ser Ser Ser Ser Ser Gly Ser Ser Ser Ser Ser Ser Gly Ser
       50                  55                  60
   Ser Ser Ser Ser Ser Gly Ser Ser Ser Ser Ser Ser Gly Ser Ser Ser
   65                  70                  75                  80
   Ser Ser Ser Gly Ser Ser Ser Ser Ser Ser Gly Ser Ser Ser Ser Ser
                   85                  90                  95
   Ser Gly Ser Ser Ser Ser Ser Ser Gly Ser Ser Ser Ser Ser Ser Gly
               100                 105                 110
   Ser Ser Ser Ser Ser Ser Gly Ser Ser Ser Ser Ser Ser Gly Ser Ser
           115                 120                 125
   Ser Ser Ser Ser Gly Ser Ser Ser Ser Ser Ser Gly Ser Ser Ser Ser
       130                 135                 140
   Ser Ser Gly Ser Ser Ser Ser Ser Ser Gly Ser Ser Ser Ser Ser Ser
   145                 150                 155                 160
   Gly Ser Ser Ser Ser Ser Ser Gly Ser Ser Ser Ser Ser Ser Gly Ser
                   165                 170                 175
   Ser Ser Ser Ser Ser Gly Ser Ser Ser Ser Ser Ser Gly Ser Ser Ser
               180                 185                 190
   Ser Ser Ser Gly Ser Ser Ser Ser Ser Ser Gly Ser Ser Ser Ser Ser
           195                 200                 205
   Ser Gly
       210
<210> 93
   <211> 150
   <212> PRT
   <213> Artificial Sequence
<220>
   <221> source
   <223> /note="Description of Artificial Sequence: Synthetic polypeptide"
<220>
   <221> VARIANT
   <222> (6)..(150)
   <223> /replace=" "
<220>
   <221> MISC_FEATURE
   <222> (1)..(150)
   <223> /note="This sequence may encompass 1 to 30 'Gly Gly Gly Gly Ser' repeating units, wherein some positions may be absent"
<220>
   <221> MISC_FEATURE
   <222> (1)..(150)
   <223> /note="Variant residues given in the sequence have no preference with respect to those in the annotations for variant positions"
<220>
   <221> source
   <223> /note="See specification as filed for detailed description of substitutions and preferred embodiments"
<400> 93
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser Gly Gly
               20                  25                  30
   Gly Gly Ser Gly Gly Gly Gly Ser Gly Gly Gly Gly Ser Gly Gly Gly
           35                  40                  45
   Gly Ser Gly Gly Gly Gly Ser Gly Gly Gly Gly Ser Gly Gly Gly Gly
       50                  55                  60
   Ser Gly Gly Gly Gly Ser Gly Gly Gly Gly Ser Gly Gly Gly Gly Ser
   65                  70                  75                  80
   Gly Gly Gly Gly Ser Gly Gly Gly Gly Ser Gly Gly Gly Gly Ser Gly
                   85                  90                  95
   Gly Gly Gly Ser Gly Gly Gly Gly Ser Gly Gly Gly Gly Ser Gly Gly
               100                 105                 110
   Gly Gly Ser Gly Gly Gly Gly Ser Gly Gly Gly Gly Ser Gly Gly Gly
           115                 120                 125
   Gly Ser Gly Gly Gly Gly Ser Gly Gly Gly Gly Ser Gly Gly Gly Gly
       130                 135                 140
   Ser Gly Gly Gly Gly Ser
   145                 150
<210> 94
   <211> 30
   <212> PRT
   <213> Artificial Sequence
<220>
   <221> source
   <223> /note="Description of Artificial Sequence: Synthetic polypeptide"
<220>
   <221> VARIANT
   <222> (2)..(30)
   <223> /replace=" "
<220>
   <221> MISC_FEATURE
   <222> (1)..(30)
   <223> /note="This sequence may encompass 1 to 30 residues, wherein some positions may be absent"
<220>
   <221> MISC_FEATURE
   <222> (1)..(30)
   <223> /note="Variant residues given in the sequence have no preference with respect to those in the annotations for variant positions"
<220>
   <221> source
   <223> /note="See specification as filed for detailed description of substitutions and preferred embodiments"
<400> 94
   Gly Gly Gly Gly Gly Gly Gly Gly Gly Gly Gly Gly Gly Gly Gly Gly
   1               5                   10                  15
   Gly Gly Gly Gly Gly Gly Gly Gly Gly Gly Gly Gly Gly Gly
               20                  25                  30
<210> 95
   <211> 12
   <212> PRT
   <213> Artificial Sequence
<220>
   <221> source
   <223> /note="Description of Artificial Sequence: Synthetic peptide"
<400> 95
   Gly Lys Ser Ser Gly Ser Gly Ser Glu Ser Lys Ser
   1 5 10
<210> 96
   <211> 14
   <212> PRT
   <213> Artificial Sequence
<220>
   <221> source
   <223> /note="Description of Artificial Sequence: Synthetic peptide"
<400> 96
   Gly Ser Thr Ser Gly Ser Gly Lys Ser Ser Glu Gly Lys Gly
   1 5 10
<210> 97
   <211> 18
   <212> PRT
   <213> Artificial Sequence
<220>
   <221> source
   <223> /note="Description of Artificial Sequence: Synthetic peptide"
<400> 97 Lys Gly
<210> 98
   <211> 14
   <212> PRT
   <213> Artificial Sequence
<220>
   <221> source
   <223> /note="Description of Artificial Sequence: Synthetic peptide"
<400> 98
   Gly Ser Thr Ser Gly Ser Gly Lys Ser Ser Glu Gly Lys Gly
   1 5 10
<210> 99
   <211> 18
   <212> PRT
   <213> Artificial Sequence
<220>
   <221> source
   <223> /note="Description of Artificial Sequence: Synthetic peptide"
<400> 99 Lys Gly
<210> 100
   <211> 14
   <212> PRT
   <213> Artificial Sequence
<220>
   <221> source
   <223> /note="Description of Artificial Sequence: Synthetic peptide"
<400> 100
   Glu Gly Lys Ser Ser Gly Ser Gly Ser Glu Ser Lys Glu Phe
   1 5 10
<210> 101
   <211> 5
   <212> PRT
   <213> Artificial Sequence
<220>
   <221> source
   <223> /note="Description of Artificial Sequence: Synthetic peptide"
<400> 101
   Ser Arg Ser Ser Gly
   1 5
<210> 102
   <211> 5
   <212> PRT
   <213> Artificial Sequence
<220>
   <221> source
   <223> /note="Description of Artificial Sequence: Synthetic peptide"
<400> 102
   Ser Gly Ser Ser Cys
   1 5
<210> 103
   <211> 28
   <212> PRT
   <213> Artificial Sequence
<220>
   <221> source
   <223> /note="Description of Artificial Sequence: Synthetic peptide"
<400> 103
   Ala Met Gly Arg Ser Gly Gly Gly Cys Ala Gly Asn Arg Val Gly Ser
   1               5                   10                  15
   Ser Leu Ser Cys Gly Gly Leu Asn Leu Gln Ala Met
               20                  25
<210> 104
   <211> 15
   <212> PRT
   <213> Artificial Sequence
<220>
   <221> source
   <223> /note="Description of Artificial Sequence: Synthetic peptide"
<400> 104
   Gly Gly Gly Gly Ser Gly Gly Gly Gly Ser Gly Gly Gly Gly Ser
   1               5                   10                  15
<210> 105
   <211> 4
   <212> PRT
   <213> Artificial Sequence
<220>
   <221> source
   <223> /note="Description of Artificial Sequence: Synthetic peptide"
<400> 105
   Gly Gly Gly Ser
   1
<210> 106
   <211> 5
   <212> PRT
   <213> Artificial Sequence
<220>
   <221> source
   <223> /note="Description of Artificial Sequence: Synthetic peptide"
<400> 106
   Gly Gly Gly Gly Ser
   1               5
<210> 107
   <211> 8
   <212> PRT
   <213> Artificial Sequence
<220>
   <221> source
   <223> /note="Description of Artificial Sequence: Synthetic peptide"
<400> 107
   Gly Gly Gly Gly Ser Gly Gly Gly
   1               5
<210> 108
   <211> 7
   <212> PRT
   <213> Artificial Sequence
<220>
   <221> source
   <223> /note="Description of Artificial Sequence: Synthetic peptide"
<400> 108
   Gly Gly Ser Gly Gly Gly Gly
   1               5
<210> 109
   <211> 18
   <212> PRT
   <213> Artificial Sequence
<220>
   <221> source
   <223> /note="Description of Artificial Sequence: Synthetic peptide"
<400> 109
   Gly Ser Thr Ser Gly Gly Gly Ser Gly Gly Gly Ser Gly Gly Gly Gly
   1               5                   10                  15
   Ser Ser
<210> 110
   <211> 18
   <212> PRT
   <213> Artificial Sequence
<220>
   <221> source
   <223> /note="Description of Artificial Sequence: Synthetic peptide"
<400> 110
   Gly Ser Thr Ser Gly Ser Gly Lys Pro Gly Ser Ser Glu Gly Ser Thr
   1               5                   10                  15
   Lys Gly

## Description

### FIELD OF THE INVENTION

The present invention relates to proteins comprising binding regions for mediating cell targeting, Shiga toxin effector regions, and carboxy-terminal endoplasmic reticulum-localization signal motifs, which are linked to an additional exogenous material, as defined in the claims. The proteins of this invention have uses, *e.g.,* for the selective killing of specific cell types, delivering exogenous materials inside target cells, labeling subcellular compartments of target cells, and as therapeutic molecules for the treatment of a variety of diseases, disorders, and conditions, including cancers, tumors, immune disorders, and microbial infections.

### BACKGROUND

The development of synthetic, fusion proteins from toxins that are effective as therapeutics has challenged scientists for decades (Pastan I et al., Annu Rev Med 58: 221-37 (2007)). One hurdle is that the enzymatic toxin moieties of synthetic cytotoxic proteins derived from bacterial and plant toxins must reach their cytosolic target substrates in order to kill cells. For many recombinant cytotoxic proteins, the potency of cytotoxicity depends on the molecule's efficiency in intracellular routing (Pirie C et al., J Biol Chem 286: 4165-72 (2011)); however, understanding how toxins direct their own intracellular transport from endosomes to the cytosol remains a challenge to scientific inquiry (Antignani A, Fitzgerald D, Toxins 5: 1486-502 (2013)).

Naturally occurring toxins or truncated toxin fragments have been linked or fused to immunoglobulin domains or receptor ligands through chemical conjugation or recombinant protein engineering techniques with the hope of creating cell-targeted therapeutic molecules (Moolten F, Cooperband S, Science 169: 68-70 (1970); Thorpe P et al., Nature 271: 752-5 (1978); Krolick K et al., Proc Natl Acad Sci USA 77: 5419-23 (1980); Krolick K et al., Cancer Immunol Immunother 12: 39-41 (1981); Blythman H et al., Nature 290: 145-46 (1981); Chaudhary V et al., Nature 339: 394-7 (1989); Strom T et al., Semin Immunol 2: 467-79 (1990); Pastan I et al., Annu Rev Biochem 61: 331-54 (1992); Foss F et al., Curr Top Microbiol Immunol 234: 63-81 (1998)). One aim of such molecular engineering is to design chimeric molecules with the dual functionality of: 1) delivering toxins to specific cell types or places within an organism after systemic administration; and 2) effectuating a targeted cytotoxicity to specific cells using potent cytotoxicity mechanisms effective in eukaryotic cells.

The Shiga toxin family of related protein toxins, notably toxins isolated from *S. dysenteriae* and *E*. *coli,* is composed of various naturally occurring toxins which are structurally and functionally related (Johannes L, Römer W, Nat Rev Microbiol 8: 105-16 (2010)). For example, the Shiga toxin family encompasses true Shiga toxin (Stx) isolated from *S. dysenteriae* serotype 1, Shiga-like toxin 1 variants (SLT1 or Stx1 or SLT-1 or Slt-I) isolated from serotypes of enterohemorrhagic *E. coli,* and Shiga-like toxin 2 variants (SLT2 or Stx2 or SLT-2) isolated from serotypes of enterohemorrhagic *E. coli.* SLT1 differs by only one residue from Stx, and both have been referred to as Verocytotoxins or Verotoxins (VTs) (O'Brien A et al., Curr Top Microbiol Immunol 180: 65-94 (1992)). Members of the Shiga toxin family share the same overall structure and mechanism of action (Engedal N et al., Microbial Biotech 4: 32-46 (2011)). For example, Stx, SLT-1 and SLT-2 display indistinguishable enzymatic activity in cell free systems (Head S et al., J Biol Chem 266: 3617-21 (1991); Tesh V et al., Infect Immun 61: 3392-402 (1993); Brigotti M et al., Toxicon 35:1431-1437 (1997)). Members of the Shiga toxin family contain targeting domains that preferentially bind a specific glycosphingolipid present on the surface of some host cells and an enzymatic domain capable of permanently inactivating ribosomes once inside a cell (Johannes, Nat Rev Microbiol 8: 105-16 (2010)).

Members of the Shiga toxin family are employed by bacteria as virulence factors during infection of a host (Johannes, Nat Rev Microbiol 8: 105-16 (2010)). In an infected host, Shiga toxins are cytotoxic because of the toxins' potent ability to inhibit protein synthesis and to trigger apoptotic cell death (Johannes, Nat Rev Microbiol 8: 105-16 (2010)). The potent cytotoxic effects of Shiga toxins on host cells can result in hemorrhagic colitis and hemolytic uremic syndrome (Johannes, Nat Rev Microbiol 8: 105-16 (2010)).

Members of the Shiga toxin family share a common, multimeric, protein structure characterized by an A(B)₅ arrangement of Shiga protein subunits (Johannes, Nat Rev Microbiol 8: 105-16 (2010)). Each Shiga toxin is composed of two protein subunits, A and B, that associate in an A(B)₅ arrangement to form a holotoxin protein complex. The Shiga toxin A Subunit is a 32 kilodalton monomer that contains an enzymatic domain, and the Shiga toxin B Subunit is a 7.7 kilodalton subunit that associates with four other Shiga toxin B Subunits to form a pentamer of Shiga toxin B Subunits. The pentamer of B subunits associates with one A subunit to form the Shiga holotoxin, which is about 70 kilodaltons (O'Brien A, Holmes, R, Microbiol Rev 51: 206-20 (1987)).

Members of the Shiga toxin family share a common process for the intoxication of a host cell that can be divided into five main phases: cell surface binding, endocytosis, retrograde subcellular movement to the endoplasmic reticulum, translocation from the endoplasmic reticulum to the cytosol, and the enzymatic inactivation of ribosomes in the cytosol. First, Shiga holotoxins are directed to the cellular surfaces of specific host cells by the B subunit's ability to specifically bind the glycosphingolipid globotriaosylceramide Gb3, also known as CD77, present on the exoplasmic membrane leaflet (Ling, H et al, Biochemistry 37: 1777-88 (1998); Thorpe C et al., Infect Immun 67: 5985-93 (1999); Soltyk A et al., J Biol Chem 277: 5351-59 (2002)). Second, Shiga holotoxins exploit the host cell's endocytotic machinery to enter into the host cell, where the holotoxins are initially contained within the endosomes (Sandvig K et al., J Cell Biol 108: 1331-43 (1989); Sandvig K et al., Histochem Cell Biol 117: 131-141 (2002)). Third, Shiga holotoxins exploit the host cell's intracellular-transport machinery to reach the endoplasmic reticulum and gain access to the cytosol (Nichols B et al., J Cell Biol 153: 529-41 (2001); Lauvrak S et al., J Cell Sci 117: 2321-31 (2004); Saint-Pol A et al., Dev Cell 6: 525-38 (2004)). Fourth, enzymatically active fragments of the Shiga holotoxins retrotranslocate from the lumen of the endoplasmic reticulum to the cytosol (Yu M, Haslam D, Infect Immun 73: 2524-32 (2005); LaPointe P et al., J Biol Chem 280: 23310-18 (2005); Falguieres T, Johannes L, Biol Cell 98: 125-34 (2006); Tam P, Lingwood C, Microbiology 153: 2700-10 (2007)). Fifth, the cytosolic fraction of Shiga toxin enzymatically active fragments causes cytotoxicity by inactivating host-cell ribosomes (Tam, Microbiology 153: 2700-10 (2007)).

During the Shiga toxin intoxication process, the Shiga toxin A Subunit is proteolytically cleaved between a conserved arginine residue and a methionine residue (*e.g.* Arg251-Met252 in StxA and SLT-1A) by furin, a host cell endoprotease (Garred Ø et al., J Biol Chem 270: 10817-21 (1995)). The amino-terminal fragment of the furin-cleaved, Shiga-toxin A Subunit is called the Shiga toxin "A1 fragment" (or Stxn-A1, SLTn-A1, SLT-nA1). The Shiga toxin A1 fragment is a 28 kilodalton protein that contains the catalytic domain of the Shiga toxin (Fraser M et al., Nat Struct Biol 1: 59-64 (1994)). The mechanism of Shiga toxin cytotoxicity to host cells is predominantly through the A1 fragment's potent catalytic inactivation of eukaryotic ribosomes and cell-wide inhibition of protein synthesis (Johannes, Nat Rev Microbiol 8: 105-16 (2010)).

The Shiga toxin A1 fragment inhibits protein translation by its potent depurination activity towards a universally conserved adenine nucleobase at position 4,324 in the alpha-sarcin-ricin loop of the 28S ribosomal RNA of the eukaryotic ribosome (Johannes, Nat Rev Microbiol 8: 105-16 (2010)). After a threshold number of ribosomes is inactivated, the host cell is predicted to experience sufficient reduction in protein synthesis to induce cell death via apoptosis (Iordanov M et al., Mol Cell Biol 17: 3373-81 (1997); Smith W et al., Infect Immun 71: 1497-504 (2003); Lee S et al., Cell Microbiol 10: 770-80 (2008); Tesh V, Future Microbiol 5: 431-53 (2010)).

The potency of A-B toxins is reported to be extremely high, such that as little as one toxin molecule can kill a cell (Yamaizumi M et al., Cell 15: 245-50 (1978); Antignani A, Fitzgerald D, Toxins 5: 1486-502 (2013)). A ribosome-inactivating A-B toxin can permanently cripple one ribosome after another within the same cell at a rate of approximately 1,500 ribosomes per minute (Endo Y, Tsurugi K, Eur J Biochem 171: 45-50 (1988); Endo Y et al., J Biol Chem 263: 8735-9 (1988)). The catalytic efficiency of this enzymatic reaction (K_{cat}/Km) is near the diffusion limit (Jasheway K et al., Toxins 3: 1233-48 (2011)). It is believed that a single molecule of A-B toxin can irreversibly inactivate 300 ribosomes in 35 minutes and is sufficient to kill a cancer cell *(see* Weldon J, Pastan I, FEBS Journal 278: 4683-700 (2011)). This level of cytotoxic potency is further predicted for the Shiga toxin A Subunit, for which it has been suggested that one molecule translocated into the cytosol would be sufficient to kill a cell (Tarn, Microbiology 153: 2700-10 (2007)).

Holotoxins of the Shiga toxin family are predicted to be too toxic for untargeted use as a therapeutic (Jain, R, Tumor physiology and antibody delivery, Front Radiat Ther Oncol 24: 32-46 (1990)). However, members of the Shiga toxin family have the potential to be synthetically engineered for therapeutic applications by rational alterations to the toxin's structure, characteristics, and biological activities (Johannes, Nat Rev Microbiol 8: 105-16 (2010); Engedal, Microbial Biotech 4: 32-46 (2011)). Shiga holotoxins have a bipartite structure composed of two non-covalently attached, modular parts: an A-moiety containing the enzymatically active A1 fragment and a B-moiety containing binding sites to the cell-surface target Gb3. Because the Shiga toxin subunits are modular, it has been hypothesized that therapeutic compositions may be created based on the separate structures and functions of the A and B moieties (U.S. application 20090156417 A1; Johannes, Nat Rev Microbiol 8: 105-16 (2010); Engedal, Microbial Biotech 4: 32-46 (2011); E.P. application 2402367 A1; U.S. application 20130196928 A1).

The A-moiety of members of the Shiga toxin family is stable, enzymatically active, and cytotoxic independent of any B-moiety (Engedal, Microbial Biotech 4: 32-46 (2011)). The Shiga toxin 1 A Subunit is catalytically active, capable of enzymatically inactivating ribosomes *in vitro,* and cytotoxic even if truncated or fused to other protein domains (Haddad J et al., J Bacteriol 175: 4970-8 (1993); Al-Jaufy A et al., Infect Immun 62: 956-60 (1994); Al-Jaufy A et al., Infect Immun 63: 3073-8 (1995); LaPointe, J Biol Chem 280: 23310-18 (2005); Di R et al., Toxicon 57: 525-39 (2011)). Shiga-like toxin 1 A Subunit truncations are catalytically active, capable of enzymatically inactivating ribosomes *in vitro,* and cytotoxic when expressed within a cell (LaPointe, J Biol Chem 280: 23310-18 (2005)).

Members of the Shiga toxin family take advantage of eukaryotic cellular machinery to enter and reach the cytosol of intoxicated cells, where their cytotoxic effect is exerted. After cell entry, Shiga toxins utilize retrograde transport mechanisms to move from the endocytotic compartment to the lumen of the endoplasmic reticulum (Sandvig K et al., J Cell Biol 126: 53-64 (1994); Johannes L et al., J Biol Chem 272: 19554-61 (1997); Rapak A et al., Proc Natl Acad Sci USA 94: 3783-8 (1997)). Retrograde transport is the intracellular movement of proteins in the reverse direction of the more standard movement of proteins during their production and secretion from eukaryotic cells (Pelham H et al., Trends Cell Biol 2: 183-5 (1992)). In a seminal study, Shiga toxins were observed in endocytotic compartments, the Golgi complex, the endoplasmic reticulum and the cytosol of intoxicated eukaryotic cells (Sandvig K et al., Nature 358: 510-12 (1992)).

Shiga holotoxins travel via retrograde transport from endocytotic compartments to the endoplasmic reticulum, where retrotranslocation of the Shiga toxin enzymatic fragment into the cytosol can occur. First, Shiga toxins exploit host cell endocytotic mechanisms to reach the trans-Golgi network (Sandvig, J Cell Biol 108: 1331-43 (1989); Sandvig K et al., J Cell Biol 113: 553-62 (1991)). Next, Shiga toxins travel from the Golgi complex to the endoplasmic reticulum (Simpson J et al., J Biol Chem 270: 20078-83 (1995)). Finally, Shiga toxins exploit endoplasmic reticulum machinery to penetrate into the cytosolic compartment via retrotranslocation (LaPointe, J Biol Chem 280: 23310-18 (2005); Li S et al., PLoS One 7: e41119 (2012)). Once in the cytosol, a Shiga toxin A1 fragment can irreversibly cripple one eukaryotic ribosome after another via the A1 fragment's potent enzymatic activity (Tam P, Microbiology 153: 2700-10 (2007)).

After endocytosis into a eukaryotic cell, soluble molecules like toxins are hypothesized to be sorted in the early endosomes to be 1) recycled for secretion, 2) degraded in the lysosomal compartment, or 3) retrogradely transported from endocytotic organelles to the Golgi complex (Bonifacino J, Hurley J, Curr Opin Cell Biol 20: 427-36 (2008); Pfeffer S, FEBS Lett 583: 3811-6 (2009); Varkouhi et al., J Control Release 151: 220-8 (2011)). Molecules can be shuttled from the cis-Golgi to the endoplasmic reticulum via vesicles, maturation of cisterns, or tubular connections (Jackson C, J Cell Sci 122: 443-52 (2009)). The exact mechanisms that traffic Shiga holotoxins to Golgi complex are not clear (Sandvig K et al., Toxicon 56: 1181-5 (2010)) but might involve clathrin, retrograde tubules, actin microfilaments, microtubules, and many other factors, such as annexins, dynamin, small GTPases, and syntaxins.

Numerous Shiga toxin constructs have been shown to be cytotoxic, which shows that their enzymatic domains reach the cytosol of intoxicated cells to effectuate cell-kill (Haddad, J Bacteriol 175: 4970-8 (1993); Al-Jaufy, Infect Immun 62: 956-60 (1994); Al-Jaufy, Infect Immun 63: 3073-8 (1995); Su et al., Protein Expr Purif 66: 149-57 (2009); U.S. Patent 7,700,557). These Shiga toxin constructs are capable of self-directing their own intracellular routing to eventually delivery their enzymatic toxin fragments to the cytosol after retrotranslocating from the lumen of the endoplasmic reticulum. For example, a Stx A Subunit fusion protein was shown to be cytotoxic when administered to cells, and the cytotoxicity depending on proper function of the Golgi complex, presumably for retrograde transport (Al-Jaufy, Infect Immun 63: 3073-8 (1995)).

The mechanism(s) for retrograde transport of Shiga toxins neither rely on nor benefit from the KDEL-endoplasmic-reticulum-retention/retrieval system to exert their cytotoxic effects (Johannes L et al., J Biol Chem 272: 19554-61 (1997); Girod A et al., Nat Cell Biol 1: 423-30 (1999); Jackson M et al., J Cell Sci 112: 467-75 (1999); Luna A et al., Mol Biol Cell 13: 866-79 (2002); Kano F et al., J Cell Sci 122: 2218-27 (2009)). The prior art teaches that Shiga toxins do not require any KDEL family signal motif to function as a cytotoxin. Unlike other toxins such as *Pseudomonas* exotoxin and cholera toxin, Shiga toxins reach the endoplasmic reticulum via a retrograde transport pathway that does not involve the use of a signal motif of the KDEL family (Kano F et al., J Cell Sci 122: 2218-27 (2009)). Further, overexpressing KDEL receptors enhanced ricin cytotoxicity but did not alter Shiga toxin cytotoxicity (Jackson, J Cell Sci 112: 467-75 (1999)). Finally, treating cells with antibodies against the KDEL receptor to inhibit the KDEL retrieval system results in protection of cells against *Pseudomonas* intoxication but has no effect on Shiga toxin intoxication (Kreitman R, Pastan I, Biochem J 307: 29-37 (1995)).

Retrograde transport of Shiga toxins from endocytotic compartments to the endoplasmic reticulum is completely independent of KDEL-receptors and not dependent on COPI-coated vesicles. This is in stark contrast to other toxins, such as *Pseudomonas* exotoxin with a REDLK signal motif at its carboxy-terminus, cholera toxin with a KDEL at its carboxy-terminus, and heat-labile toxin with a RDEL signal sequence at its carboxy-terminus (Chaudhary V et al., Proc Natl Acad Sci USA 87: 308-12 (1990); Lencer W et al., J Cell Biol 131: 951-62 (1995)). *Pseudomonas* exotoxin requires the KDEL-endoplasmic-reticulum-retention/retrieval system for intoxication of eukaryotic cells (Jackson, J Cell Sci 112: 467-75 (1999); Bard F et al., J Biol Chem 278, 46601-6 (2003)). *Pseudomonas* exotoxin intoxication requires empty KDEL-receptors in the Golgi complex for retrograde transport and most likely requires the COPI-coated vesicle system as well. In addition, it was demonstrated that *Pseudomonas* exotoxin's cytotoxicity can be increased by mutating its RDEL to KDEL (Kreitman, Biochem J 307: 29-37 (1995)). Similarly, the addition of the KDEL signal motif to the carboxyl-terminus of ricin increase the cytotoxicity of a ricin construct, likely because the KDEL signal motif increases ricin's rate of entry into the cytosol (Wales R et al., J Biol Chem 268: 23986-90 (1993)). However, Shiga toxins differ from toxins that utilize the endoplasmic-reticulum-retention-system via KDEL-type, signal motifs located at their carboxy terminals.

The lack of understanding how Shiga toxins direct intracellular routing inside intoxicated cells makes development of effective Shiga toxin-based therapeutics challenging.

The idea of linking a toxin to a targeting domain to make a chimeric molecule that selectively kills cancer cells is not new (Strebhardt K, Ullrich A, Nat Rev Cancer 8: 473-80 (2008)). For example, since the 1970s immunotoxins have been developed using three, primary, toxin candidates: the bacterial diphtheria toxin, the bacterial *Pseudomonas* exotoxin, and plant toxins exemplified by ricin (Antignani, Toxins 5: 1486-502 (2013)). By 2013, however, these three toxins remained "among the top choices for immunotoxin development" (Antignani, Toxins 5: 1486-502 (2013)). To date, no one has described a cytotoxic protein comprising an amino acid sequence derived from a Shiga-toxin combined with a cell-targeting, binding region and an endoplasmic reticulum retrieval signal motif that was capable of specifically and selectively killing a targeted cell type.

Al-Jaufy et al., Infection and Immunity 62: 956-60 (1994), describes the cytoxicity of a Shiga toxin A subunit-CD4 fusion protein to Human Immunodeficiency Virus-infected cells. Habibi Roudkenar et al., Cell Biology and Toxicology 22: 213-19 (2006), describes the selective cytotoxicity of recombinant STXA1-GM-CSF protein in hematopoetic cancer cells. Pastan et al., Annual Review of Medicine 58: 221-37 (2007), provides a review of the immunotoxin treatment of cancer. WO 2014/164693 describes cytotoxic proteins comprising immunoglobulin-type binding regions for mediating cell-type specific targeting and Shiga toxin effector regions derived from A subunits of members of the Shiga toxin family for effectuating cytotoxicity. Johannes et al., The Journal of Biological Sciences 272: 19554-61 (1997), describes the retrograde transport of a KDEL-bearing B-fragment of Shiga toxin. Jackson et al., Journal of Cell Science 112: 467-75 (1999), describes that the KDEL retrieval system is exploited by Pseudomonas exotoxin A, but not by Shiga-like toxin-1, during retrograde transport from the Golgi complex to the endoplasmic reticulum. Wales et al., The Journal of Biological Chemistry 268: 23986-90 (1993), describes the addition of an endoplasmic reticulum retrieval sequence to ricin A chain significantly increases it cytotoxicity to mammalian cells.

It would be desirable to have cell-targeting cytotoxic proteins comprising Shiga-toxin-Subunit-A derived regions that self-direct their own intracellular routing and display potent cytotoxicity for uses involving the targeted killing of specific cell types and for use as therapeutics in the treatment of a variety of diseases, such as, *e.g.,* cancers, tumors, immune disorders, and microbial infections. Thus, there remains a need in the art for ways of engineering cytotoxic proteins comprising Shiga toxin regions and cell-targeting binding regions that exhibit effective intracellular-routing to provide potent cytotoxicity.

### SUMMARY OF THE INVENTION

The present invention provides a protein comprising 1) a binding region as defined in the claims, such as from immunoglobulins; 2) a Shiga toxin effector region as defined in the claims, such as from SLT-1A; and 3) a carboxy-terminal, endoplasmic reticulum retention/retrieval signal motif of a member of the KDEL family as defined in the claims, such as KDEL (SEQ ID NO:34), wherein the protein is linked to an additional exogenous material, as defined in the claims. The linking of binding regions with Shiga-toxin-Subunit-A-derived polypeptides enabled the engineering of cell-type specific targeting of Shiga toxin cytotoxicity, and the addition of carboxy-terminal signal motifs increased that cytotoxicity. The protein of the invention has uses such as, *e.g.,* for targeted cell-killing, delivering exogenous materials, as diagnostic agents, and as therapeutic molecules for the treatment of a variety of diseases, disorders, and conditions, including cancers, tumors, growth abnormalities, immune disorders, and microbial infections.

A protein of the present invention comprises (a) a binding region capable of specifically binding at least one extracellular target biomolecule, (b) a Shiga toxin A Subunit effector region comprising a polypeptide capable of exhibiting at least one Shiga toxin function selected from the group consisting of: cellular internalization, subcellular routing and cytotoxicity; wherein the polypeptide comprises or consists of a sequence that is at least 85% identical to a sequence selected from: (i) amino acids 75 to 251 of SEQ ID NO: 1, SEQ ID NO: 2 or SEQ ID NO:3; (ii) amino acids 1 to 241 of SEQ ID NO: 1, SEQ ID NO: 2 or SEQ ID NO:3; (iii) amino acids 1 to 251 of SEQ ID NO: 1, SEQ ID NO: 2 or SEQ ID NO:3; or (iv) amino acids 1 to 261 of SEQ ID NO: 1, SEQ ID NO: 2 or SEQ ID NO:3, and (c) a carboxy-terminal endoplasmic reticulum retention/retrieval signal motif of a member of the KDEL family; wherein the protein is linked to an additional exogenous material for delivery into a cell physically coupled with the extracellular target biomolecule bound by said protein, as defined in the claims.

In certain embodiments, the protein of the present invention comprises the binding region comprising a polypeptide selected from the group consisting of: single-domain antibody (sdAb) fragment, nanobody, heavy-chain antibody domain derived from a camelid (V_{H}H fragment), heavy-chain antibody domain derived from a cartilaginous fish, immunoglobulin new antigen receptor (IgNAR), V_{NAR} fragment, single-chain variable fragment (scFv), antibody variable fragment (Fv), a complementary determining region 3 (CDR3) fragment, constrained FR3-CDR3-FR4 (FR3-CDR3-FR4) polypeptide, Fd fragment, small modular immunopharmaceutical (SMIP) domain, antigen-binding fragment (Fab), fibronectin-derived 10^{th} fibronectin type III domain (10Fn3) (*e.g.* monobody), tenascin type III domain (*e.g.* TNfn3), ankyrin repeat motif domain(ARD), low-density-lipoprotein-receptor-derived A-domain (A domain of LDLR or LDLR-A), lipocalin (anticalin), Kunitz domain, Protein-A-derived Z domain, gamma-B crystallin-derived domain, ubiquitin-derived domain, Sac7d-derived polypeptide (affitin), Fyn-derived SH2 domain, miniprotein, C-type lectin-like domain scaffold, engineered antibody mimic, and any genetically manipulated counterparts of any of the foregoing which retain binding functionality.

For certain embodiments, whereby administration of the protein of the present invention to a cell physically coupled with the extracellular target biomolecule of the protein's binding region, the protein is capable of causing death of the cell. In certain further embodiments, upon administration of the protein of the invention to two different populations of cell types which differ with respect to the presence or level of an extracellular target biomolecule, the protein is capable of causing cell death of the cell-types physically coupled with an extracellular target biomolecule of the cytotoxic protein's binding region at a CD₅₀ that is at least three times less than the CD₅₀ observed for cell types which are not physically coupled with an extracellular target biomolecule of the protein's binding region. For certain embodiments, whereby administration of the protein of the invention to a first population of cells whose members are physically coupled to extracellular target biomolecules of the protein's binding region, and a second population of cells whose members are not physically coupled to any extracellular target biomolecule of the binding region, the cytotoxic effect of the cell-targeted molecule to members of said first population of cells relative to members of said second population of cells is at least 3-fold greater. For certain embodiments, whereby administration of the protein of the invention to a first population of cells whose members are physically coupled to a significant amount of the extracellular target biomolecule of the protein's binding region, and a second population of cells whose members are not physically coupled to a significant amount of any extracellular target biomolecule of the binding region, the cytotoxic effect of the cell-targeted molecule to members of said first population of cells relative to members of said second population of cells is at least 3-fold greater. For certain embodiments, whereby administration of the protein of the invention to a first population of target biomolecule positive cells, and a second population of cells whose members do not express a significant amount of a target biomolecule of the protein's binding region at a cellular surface, the cytotoxic effect of the protein to members of the first population of cells relative to members of the second population of cells is at least 3-fold greater.

In certain embodiments, the binding region is designed or selected by its ability to bind the extracellular target biomolecule selected from the group consisting of: CD20, CD22, CD40, CD74, CD79, CD25, CD30, HER2/neu/ErbB2, EGFR, EpCAM, EphB2, prostate-specific membrane antigen, Cripto, CDCP1, endoglin, fibroblast activated protein, Lewis-Y, CD19, CD21, CS1/ SLAMF7, CD33, CD52, CD133, EpCAM, CEA, gpA33, mucin, TAG-72, tyrosine-protein kinase transmembrane receptor (ROR1 or NTRKR1), carbonic anhydrase IX, folate binding protein, ganglioside GD2, ganglioside GD3, ganglioside GM2, ganglioside Lewis-Y2, VEGFR, Alpha Vbeta3, Alpha5beta1, ErbB1/EGFR, Erb3, c-MET, IGF1R, EphA3, TRAIL-R1, TRAIL-R2, RANK, FAP, tenascin, CD64, mesothelin, BRCA1, MART-1/MelanA, gp100, tyrosinase, TRP-1, TRP-2, MAGE-1, MAGE-3, GAGE-1/2, BAGE, RAGE, NY-ESO-1, CDK-4, beta-catenin, MUM-1, caspase-8, KIAA0205, HPVE6, SART-1, PRAME, carcinoembryonic antigen, prostate specific antigen, prostate stem cell antigen, human aspartyl (asparaginyl) beta-hydroxylase, EphA2, HER3/ErbB-3, MUC1, MART-1/MelanA, gp100, tyrosinase associated antigen, HPV-E7, Epstein-Barr virus antigen, Bcr-Abl, alpha-fetoprotein antigen, 17-A1, bladder tumor antigen, CD38, CD15, CD23, CD53, CD88, CD129, CD183, CD191, CD193, CD244, CD294, CD305; C3AR, FceRIa, galectin-9, mrp-14, programmed death-ligand 1 (PD-L1), siglec-8, siglec-10, CD49d, CD13, CD44, CD54, CD63, CD69, CD123, CD193, TLR4, FceRIa, IgE, CD107a, CD203c, CD14, CD15, CD33, CD64, CD68, CD80, CD86, CD105, CD115, F4/80, ILT-3, galectin-3, CD11a-c, GITRL, MHC Class I molecule (optionally complexed with a peptide), MHC Class II molecule (optionally complexed with a peptide), CD284-TLR4, CD107-Mac3, CD195-CCR5, HLA-DR, CD16/32, CD282-TLR2, CD11c, CD123, and any immunogenic fragment of any of the foregoing.

In certain embodiments, the protein of the present invention comprises the Shiga toxin effector region comprising or consisting of amino acids 75 to 251 of SEQ ID NO:1, SEQ ID NO:2, or SEQ ID NO:3. In certain further embodiments, the protein of the present invention comprises the Shiga toxin effector region comprising or consisting of amino acids 1 to 241 of SEQ ID NO: 1, SEQ ID NO:2, or SEQ ID NO:3. In certain further embodiments, the Shiga toxin effector region comprises or consists of amino acids 1 to 251 of SEQ ID NO:1, SEQ ID NO:2, or SEQ ID NO:3. In certain further embodiments, the Shiga toxin effector region comprises or consists of amino acids 1 to 261 of SEQ ID NO:1, SEQ ID NO:2, or SEQ ID NO:3.

In certain embodiments, the protein of the present invention comprises the carboxy-terminal endoplasmic reticulum retention/retrieval signal motif selected from the group consisting of: KDEL (SEQ ID NO: 34), HDEF (SEQ ID NO: 35), HDEL (SEQ ID NO: 36), RDEF (SEQ ID NO: 37), RDEL (SEQ ID NO: 38), WDEL (SEQ ID NO: 39), YDEL (SEQ ID NO: 40), HEEF (SEQ ID NO: 41), HEEL (SEQ ID NO: 42), KEEL (SEQ ID NO: 43), REEL (SEQ ID NO: 44), KAEL (SEQ ID NO: 45), KCEL (SEQ ID NO: 46), KFEL (SEQ ID NO: 47), KGEL (SEQ ID NO: 48), KHEL (SEQ ID NO: 49), KLEL (SEQ ID NO: 50), KNEL (SEQ ID NO: 51), KQEL (SEQ ID NO: 52), KREL (SEQ ID NO: 53), KSEL (SEQ ID NO: 54), KVEL (SEQ ID NO: 55), KWEL (SEQ ID NO: 56), KYEL (SEQ ID NO: 57), KEDL (SEQ ID NO: 58), KIEL (SEQ ID NO: 59), DKEL (SEQ ID NO: 60), FDEL (SEQ ID NO: 61), KDEF (SEQ ID NO: 62), KKEL (SEQ ID NO: 63), HADL (SEQ ID NO: 64), HAEL (SEQ ID NO: 65), HIEL (SEQ ID NO: 66), HNEL (SEQ ID NO: 67), HTEL (SEQ ID NO: 68), KTEL (SEQ ID NO: 69), HVEL (SEQ ID NO: 70), NDEL (SEQ ID NO: 71), QDEL (SEQ ID NO: 72), REDL (SEQ ID NO: 73), RNEL (SEQ ID NO: 74), RTDL (SEQ ID NO: 75), RTEL (SEQ ID NO: 76), SDEL (SEQ ID NO: 77), TDEL (SEQ ID NO: 78), SKEL (SEQ ID NO: 79), STEL (SEQ ID NO: 80), and EDEL (SEQ ID NO: 81).

In certain embodiments, the protein of the present invention comprises the binding region comprising or consisting essentially of amino acids 2-241 of any one of SEQ ID NOs: 4-16.

In certain embodiments, the protein of the present invention comprises or consists essentially of the polypeptide shown in any one SEQ ID NOs: 4-33.

In certain embodiments, the protein of the present invention comprises a Shiga toxin effector region which comprises a mutation relative to a naturally occurring A Subunit of a member of the Shiga toxin family that changes the enzymatic activity of the Shiga toxin effector region. In certain further embodiments, the mutation is selected from at least one amino acid residue deletion, insertion, or substitution that reduces or eliminates cytotoxicity of the Shiga toxin region. In certain further embodiments, the protein comprises a mutation which reduces or eliminates catalytic activity but retains other Shiga toxin effector functions, such as, *e.g*., promoting cellular internalization and/or directing intracellular routing. In certain embodiments, the mutation is selected from at least one amino acid residue substitution, such as, *e.g.,* A231E, R75A, Y77S, Y114S, E167D, R170A, R176K and/or W203A in SEQ ID NO:1, SEQ ID NO:2, or SEQ ID NO:3.

The present invention also provides a pharmaceutical composition comprising a protein of the present invention and at least one pharmaceutically acceptable excipient or carrier as defined in the claims.

Among certain embodiments of the present invention is a diagnostic composition comprising a protein of the invention and a detection promoting agent as defined in the claims. The diagnostic composition may be used for the collection of information, such as diagnostically useful information about a cell type, tissue, organ, disease, disorder, condition, and/or patient.

The disclosure provides polynucleotides capable of encoding a protein described herein, as well as expression vectors which comprise a polynucleotide described herein and host cells comprising an expression vector described herein. Host cells comprising an expression vector may be used, *e.g.,* in methods for producing a protein described herein or a polypeptide component or fragment thereof by recombinant expression.

The disclosure further provides a system for conferring improved cytotoxicity to a protein comprising the step of adding a carboxy-terminal endoplasmic reticulum retention/retrieval signal motif of a member of the KDEL family to a carboxy terminus of the protein or polypeptide component of the protein, wherein the protein comprises (a) a binding region capable of specifically binding at least one extracellular target biomolecule and (b) a Shiga toxin effector region comprising a polypeptide derived from the amino acid sequence of the A Subunit of at least one member of the Shiga toxin family.

Additionally, the present invention provides an *in vitro* method of killing a cell comprising the step of contacting the cell with a protein of the invention, as defined in the claims. In further embodiments of the cell killing methods, the method is capable of selectively killing cell(s) and/or cell types preferentially over other cell(s) and/or cell types when contacting a mixture of cells which differ with respect to the extracellular presence and/or expression level of an extracellular target biomolecule of the binding region of the protein.

The present invention further provides a protein of the invention or a pharmaceutical composition of the invention for use in treating or preventing a disease, disorder, or condition in a patient, as defined in the claims. In certain embodiments, the disease, disorder, or condition is selected from: a cancer, tumor, growth abnormality, immune disorder, or microbial infection. In certain embodiments, the cancer is selected from the group consisting of: bone cancer, breast cancer, central/peripheral nervous system cancer, gastrointestinal cancer, germ cell cancer, glandular cancer, head-neck cancer, hematological cancer, kidney-urinary tract cancer, liver cancer, lung/pleura cancer, prostate cancer, sarcoma, skin cancer, and uterine cancer. In certain embodiments, the immune disorder is an immune disorder associated with a disease selected from the group consisting of: amyloidosis, ankylosing spondylitis, asthma, Crohn's disease, diabetes, graft rejection, graft-versus-host disease, Hashimoto's thyroiditis, hemolytic uremic syndrome, HIV-related disease, lupus erythematosus, multiple sclerosis, polyarteritis nodosa, polyarthritis, psoriasis, psoriatic arthritis, rheumatoid arthritis, scleroderma, septic shock, Sjögren's syndrome, ulcerative colitis, and vasculitis.

The disclosure provides the use of any composition described herein for the treatment or prevention of a cancer, tumor, growth abnormality, and/or immune disorder. Among certain embodiments of the present invention is a protein of the present invention or a pharmaceutical composition of the present invention for use in treating or preventing a cancer, tumor, growth abnormality, immune disorder, and/or microbial infection. Among certain embodiments of the present disclosure is the use of a protein described herein and/or pharmaceutical composition thereof in the manufacture of a medicament for the treatment or prevention of a cancer, tumor, growth abnormality, immune disorder, or microbial infection.

The protein of the invention is linked to an additional exogenous material for delivery into a cell physically coupled with the extracellular target biomolecule bound by the protein of the invention, as defined in the claims. Additionally, the present invention provides an *in vitro* method for delivering an exogenous material to the inside of a cell comprising contacting the cell with a protein of the present invention as defined in the claims, a pharmaceutical composition of the present invention as defined in the claims, or a diagnostic composition of the present invention as defined in the claims; wherein the cell is physically coupled with the extracellular target biomolecule bound by the protein. The present disclosure further provides a method for delivering exogenous material to the inside of a cell(s) in a patient, the method comprising the step of administering to the patient a protein described herein (with or without cytotoxic activity), wherein the target cell(s) is physically coupled with an extracellular target biomolecule of the protein.

The present invention also provides the use of a protein of the present invention, a pharmaceutical composition of the present invention, or a diagnostic composition of the present invention in the *in vitro* diagnosis of a disease, disorder, or condition as defined in the claims. The present invention also provides a protein of the present invention, a pharmaceutical composition of the present invention, or a diagnostic composition of the present invention for use in the *in vivo* diagnosis, prognosis or characterization of a disease, disorder or condition, as defined in the claims. Among certain embodiments of the present disclosure is a method of using a protein described herein comprising a detection promoting agent and/or composition described herein (*e.g.* a diagnostic composition) for the collection of information useful in the diagnosis, prognosis, or characterization of a disease, disorder, or condition. Among certain embodiments of the present disclosure is the method of detecting a cell using a protein and/or diagnostic composition described herein comprising the steps of contacting a cell with the protein and/or diagnostic composition and detecting the presence of said cell-targeted molecule and/or diagnostic composition. In certain embodiments of the disclosure, the step of contacting the cell(s) occurs *in vitro.* In certain embodiments of the disclosure, the step of contacting the cell(s) occurs *in vivo.* In certain embodiments of the disclosure, the step of detecting the cell(s) occurs *in vitro.* In certain embodiments, the step of detecting the cell(s) occurs *in vivo.* In certain further embodiments of the disclosure, the method involves the detection of the location of the protein in an organism using one or more imaging procedures after the administration of the protein to said organism. For example, proteins of the invention which incorporate detection promoting agents as described herein may be used to characterize diseases as potentially treatable by a related pharmaceutical composition of the invention. For example, proteins of the invention, compositions (*e.g*. pharmaceutical compositions and diagnostic compositions) of the invention, and methods of the disclosure may be used to determine if a patient belongs to a group that responds to a pharmaceutical composition described herein.

Among certain embodiments of the present invention is a kit as defined in the claims comprising (i) a protein of the invention, a pharmaceutical composition of the invention or a diagnostic composition of the invention, and (ii) an additional reagent and/or pharmaceutical delivery device; and optionally, instructions for use.

These and other features, aspects and advantages of the present invention will become better understood with regard to the following description, appended claims, and accompanying figures. The aforementioned elements of the invention may be individually combined or removed freely in order to make other embodiments of the invention, without any statement to object to such combination or removal hereinafter.

### BRIEF DESCRIPTION OF THE FIGURES

**Figure 1** shows the general arrangement of the proteins of the invention with "N" and "C" denoting an amino-terminus and carboxy-terminus, respectively, of the protein or a polypeptide component of the protein.
**Figure 2** graphically shows αHER2scFv::SLT-1A::KDEL exhibited improved target cell-type specific cytotoxicity as compared to αHER2scFv::SLT-1A which lacked a KDEL signal motif. The percent viability of cells was plotted over the logarithm to base 10 of the cytotoxic protein concentration.
**Figure 3** shows microscopy images of the subcellular localization of αHER2scFv::SLT-1A and αHER2scFv::SLT-1A::KDEL. The images showed both cytotoxic proteins entered target cells within one hour of administration.
**Figure 4** graphically shows improved target cell-type specific cytotoxicity of the protein αCD38scFv::SLT-1A::KDEL compared to the protein αCD38scFv::SLT-1A which lacked a KDEL signal motif. The percent viability of cells was plotted over the logarithm to base 10 of the cytotoxic protein concentration.

### DETAILED DESCRIPTION

The present invention is as defined in the appended claims.

In order that the present invention may be more readily understood, certain terms are defined below. Additional definitions may be found within the detailed description of the invention.

As used in the specification and the appended claims, the terms "a," "an" and "the" include both singular and the plural referents unless the context clearly dictates otherwise.

As used in the specification and the appended claims, the term "and/or" when referring to two species, A and B, means at least one of A and B. As used in the specification and the appended claims, the term "and/or" when referring to greater than two species, such as A, B, and C, means at least one of A, B, or C, or at least one of any combination of A, B, or C (with each species in singular or multiple possibility).

Throughout this specification, the word "comprise" or variations such as "comprises" or "comprising" will be understood to imply the inclusion of a stated integer (or components) or group of integers (or components), but not the exclusion of any other integer (or components) or group of integers (or components).

Throughout this specification, the term "including" is used to mean "including but not limited to." "Including" and "including but not limited to" are used interchangeably.

The term "amino acid residue" or "amino acid" includes reference to an amino acid that is incorporated into a protein, polypeptide, or peptide. The term "polypeptide" includes any polymer of amino acids or amino acid residues. The term "polypeptide sequence" refers to a series of amino acids or amino acid residues from which a polypeptide is physically composed. A "protein" is a macromolecule comprising one or more polypeptides or polypeptide "chains." A "peptide" is a small polypeptide of sizes less than a total of 15-20 amino acid residues. The term "amino acid sequence" refers to a series of amino acids or amino acid residues which physically comprise a peptide or polypeptide depending on the length. Unless otherwise indicated, polypeptide and protein sequences disclosed herein are written from left to right representing their order from an amino terminus to a carboxy terminus.

The terms "amino acid," "amino acid residue," "amino acid sequence," or polypeptide sequence include naturally occurring amino acids (including L and D isosteriomers) and, unless otherwise limited, also include known analogs of natural amino acids that can function in a similar manner as naturally occurring amino acids, such as selenocysteine, pyrrolysine, *N*-formylmethionine, gamma-carboxyglutamate, hydroxyprolinehypusine, pyroglutamic acid, and selenomethionine. The amino acids referred to herein are described by shorthand designations as follows in Table A:

**TABLE A. Amino Acid Nomenclature**

| **Name** | **3-letter** | **1-letter** |
|---|---|---|
| Alanine | Ala | A |
| Arginine | Arg | R |
| Asparagine | Asn | N |
| Aspartic Acid or Aspartate | Asp | D |
| Cysteine | Cys | C |
| Glutamic Acid or Glutamate | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The phrase "conservative substitution" with regard to a polypeptide, refers to a change in the amino acid composition of the polypeptide that does not substantially alter the function and structure of the overall polypeptide (*see* Creighton, Proteins: Structures and Molecular Properties (W. H. Freeman and Company, New York (2nd ed., 1992))).

As used herein, , the terms "expressed," "expressing," or "expresses" and grammatical variants thereof refer to translation of a polynucleotide or nucleic acid into a polypeptide or protein. The expressed polypeptides or proteins may remain intracellular, become a component of the cell surface membrane or be secreted into an extracellular space.

As used herein, cells which express a significant amount of an extracellular target biomolecule at least one cellular surface are "target positive cells" or "target+ cells" and are cells physically coupled to the specified extracellular target biomolecule.

As used herein, the symbol "α" is shorthand for an immunoglobulin-type binding region capable of binding to the biomolecule following the symbol. The symbol "α" is used to refer to the functional characteristic of an immunoglobulin-type binding region based on its capability of binding to the biomolecule following the symbol.

The symbol "::" means the polypeptide regions before and after it are physically linked together to form a continuous polypeptide.

The term "selective cytotoxicity" with regard to the cytotoxic activity of a cytotoxic protein refers to the relative levels of cytotoxicity between a targeted cell population and a non-targeted bystander cell population, which can be expressed as a ratio of the half-maximal cytotoxic concentration (CD₅₀) for a targeted cell type over the CD₅₀ for an untargeted cell type to show preferentiality of cell killing of the targeted cell type.

For purposes of the present invention, the term "effector" means providing a biological activity, such as cytotoxicity, biological signaling, enzymatic catalysis, subcellular routing, and/or intermolecular binding resulting in the recruitment of a factor(s) and/or allosteric effects.

For purposes of the present invention, the phrase "derived from" means that the polypeptide region comprises amino acid sequences originally found in a protein and which may now comprise additions, deletions, truncations, or other alterations from the original sequence such that overall function and structure are substantially conserved.

For purposes of the present invention, a Shiga toxin effector function is a biological activity conferred by a polypeptide region derived from a Shiga toxin A Subunit. Non-limiting examples of Shiga toxin effector functions include cellular internalization, subcellular routing, catalytic activity, and cytotoxicity. Shiga toxin catalytic activities include, for example, ribosome inactivation, protein synthesis inhibition, N-glycosidase activity, polynucleotide:adenosine glycosidase activity, RNAase activity, and DNAase activity. RIPs can depurinate nucleic acids, polynucleosides, polynucleotides, rRNA, ssDNA, dsDNA, mRNA (and polyA), and viral nucleic acids (Barbieri L et al., Biochem J 286: 1-4 (1992); Barbieri L et al., Nature 372: 624 (1994); Ling J et al., FEBS Lett 345: 143-6 (1994); Barbieri L et al., Biochem J 319: 507-13 (1996); Roncuzzi L, Gasperi-Campani A, FEBS Lett 392: 16-20 (1996); Stirpe F et al., FEBS Lett 382: 309-12 (1996); Barbieri L et al., Nucleic Acids Res 25: 518-22 (1997); Wang P, Tumer N, Nucleic Acids Res 27: 1900-5 (1999); Barbieri L et al., Biochim Biophys Acta 1480: 258-66 (2000); Barbieri L et al., J Biochem 128: 883-9 (2000); Bagga S et al., J Biol Chem 278: 4813-20 (2003); Picard D et al., J Biol Chem 280: 20069-75 (2005)). Some RIPs show antiviral activity and superoxide dismutase activity (Erice A et al., Antimicrob Agents Chemother 37: 835-8 (1993); Au T et al., FEBS Lett 471: 169-72 (2000); Parikh B, Tumer N, Mini Rev Med Chem 4: 523-43 (2004); Sharma N et al., Plant Physiol 134: 171-81 (2004)). Shiga toxin catalytic activities have been observed both *in vitro* and *in vivo.* Assays for Shiga toxin effector activity can measure various activities, such as, *e.g.,* protein synthesis inhibitory activity, depurination activity, inhibition of cell growth, cytotoxicity, supercoiled DNA relaxation activity, and/or nuclease activity.

As used herein, the retention of Shiga toxin effector function refers to a level of Shiga toxin functional activity, as measured by an appropriate quantitative assay with reproducibility comparable to a wild-type Shiga toxin effector region control. For ribosome inhibition, Shiga toxin effector function is exhibiting an IC₅₀ of 10,000 picomolar (pM) or less. For cytotoxicity in a target positive cell kill assay, Shiga toxin effector function is exhibiting a CD₅₀ of 1,000 nanomolar (nM) or less, depending on the cell type and its expression of the appropriate extracellular target biomolecule.

The effectiveness and potency of immunotoxins and ligand-toxin fusions as cytotoxic molecules is influenced by the densities of their target antigen(s) on a target cell surface (*see e.g.* Decket T et al., Blood 103: 2718-26 (2004); Du X et al., Blood 111: 338-43 (2008); Baskar S et al., mAbs 4: 349-61 (2012)), epitope location (Press O et al., J Immunol 141: 4410-7 (1988); Godal A et al., In J Cancer 52: 631-5 (1992); Yazdi P et al., Cancer Res 55: 3763-71 (1995)), rate of internalization of the surface bound cytotoxic molecule *(see e.g.* Du X et al., Cancer Res 68: 6300-5 (2008)), and the intracellular itinerary (Tortorella L et al., PLoS One 7: e47320 (2012)).

The cell surface representation and/or density of a given extracellular target biomolecule may influence the applications for which certain proteins of the invention may be most suitably used. Differences in cell surface representation and/or density of a given target biomolecule between cells may alter the internalization and/or cytotoxicity of a given protein of the invention both quantitatively and qualitatively. The cell surface representation and/or density of a given target biomolecule can vary greatly among target biomolecule positive cells or even on the same cell at different points in the cell cycle or cell differentiation. The total cell surface representation of a given target biomolecule on a particular cell or population of cells may be determined using methods known to the skilled worker, such as the fluorescence-activated cell sorting (FACS) flow cytometry methods.

### Introduction

The present invention solves problems for engineering proteins comprising Shiga-toxin-Subunit-A derived regions linked to heterologous polypeptide regions, such as binding regions for cell targeting, when translocation of the protein to certain intracellular compartment(s) is desired, *e.g.* to the endoplasmic reticulum and/or cytosol. The present invention is based on the unexpected discovery that the addition of KDEL family signal motifs to the carboxyl-terminals of the Shiga-toxin-Subunit-A-derived toxin region within a cytotoxic protein improved cytotoxicity. This discovery contradicted previous research results published in the scientific literature (Jackson M et al., J Cell Sci 112: 467-75 (1999)). As described in more detail in the Examples, the addition of endoplasmic reticulum retention signal motifs to the carboxy-terminals of cytotoxic proteins allowed for the engineering of cell-type specific targeting of more potent Shiga toxin cytotoxicity.

Previously, Shiga toxin A Subunit fusion constructs were shown to be cytotoxic and presumably capable of self-directing their own intracellular routing to deliver an enzymatically active toxin fragment to the cytosol (Al-Jaufy, Infect Immun 62: 956-60 (1994); Al-Jaufy, Infect Immun 63: 3073-8 (1995); Su, Protein Expr Purif 66: 149-57 (2009)). When multiple cytotoxic proteins were created and tested with Shiga toxin derived regions linked to immunoglobulin derived targeting regions, these engineered proteins did not display the expected levels of cytotoxicity (*see,* Examples, *infra*)*.* However, these reduced-cytotoxicity proteins exhibited cell binding and entry, as well as similar *in vitro* enzymatic activities and binding affinities as compared to more cytotoxic variants with the same polypeptide regions linked in a different configuration (*see,* Examples, *infra*; U.S. patent application 61/951,121). This unexpected problem was solved as described below.

As described further in the Examples, the toxicity of Shiga-toxin-Subunit-Abased, cytotoxic proteins was improved by adding KDEL-like signal motifs to their carboxy-terminals. This modification resulted in significantly more potent cell kill results than observed in variants of the cytotoxic proteins lacking KDEL-type signal motifs. However, the addition of the signal motif did not alter either the catalytic activity of the Shiga toxin-derived region or the binding kinetics of the binding region. The addition of endoplasmic reticulum retention and retrieval signal motifs improved the cytotoxicity of these cytotoxic proteins most likely by directing sufficient intracellular routing to effectively deliver the Shiga toxin effector to the cytosol. The present invention provides a specific way of engineering such Shiga toxin derived proteins by adding a carboxy-terminal KDEL signal motif to at least one polypeptide of the protein in order to provide for desired intracellular routing and/or improved cytotoxic.

### I. The General Structure of the Proteins of the Invention

The present invention provides a protein, the protein comprising (a) a binding region for cell targeting, as defined in the claims, (b) a Shiga toxin effector region for providing one or more Shiga toxin effector functions (*e.g*. cellular internalization, intracellular routing, and/or cell killing), as defined in the claims, and (c) a carboxy-terminal endoplasmic reticulum retention/retrieval signal motif for directing subcellular routing, as defined in the claims; wherein the protein is linked to an additional exogenous material for delivery into a cell physically coupled with the extracellular target biomolecule bound by said protein. The linking of cell targeting binding regions with Shiga-toxin-Subunit-A-derived regions enables the engineering of cell-type specific targeting of the potent Shiga toxin cytotoxicity. A protein of the invention comprises a binding region which can bind specifically to at least one extracellular target biomolecule in physical association with a cell, such as a target biomolecule expressed on the surface of a cell. This general structure is modular in that any number of diverse binding regions may be linked to Shiga toxin Subunit A derived effector regions and KDEL-type signal motifs to produce variations of the same general structure.

### A. Binding Regions

The binding region of a protein of the present invention is capable of binding specifically to at least one extracellular target biomolecule. The binding region may comprise one or more various peptidic or polypeptide moieties, such as randomly generated peptide sequences, naturally occurring ligands or derivatives thereof, immunoglobulin derived domains, synthetically engineered scaffolds as alternatives to immunoglobulin domains, and the like. In certain embodiments, a protein of the invention comprises a binding region comprising one or more polypeptides capable of selectively and specifically binding an extracellular target biomolecule.

There are numerous binding regions known in the art that are useful for targeting proteins to specific cell-types via their binding characteristics, such as ligands, monoclonal antibodies, engineered antibody derivatives, and engineered alternatives to antibodies.

According to one specific but non-limiting aspect, the binding region of the protein of the present invention comprises a naturally occurring ligand or derivative thereof that retains binding functionality to an extracellular target biomolecule, commonly a cell surface receptor. For example, various cytokines, growth factors, and hormones known in the art may be used to target the protein to the cell-surface of specific cell types expressing a cognate cytokine receptor, growth factor receptor, or hormone receptor. Certain non-limiting examples of ligands include (alternative names are indicated in parentheses) angiogenin, B-cell activating factors (BAFFs, APRIL), colony stimulating factors (CSFs), epidermal growth factors (EGFs), fibroblast growth factors (FGFs), vascular endothelial growth factors (VEGFs), insulin-like growth factors (IGFs), interferons, interleukins (such as IL-2, IL-6, and IL-23), nerve growth factors (NGFs), platelet derived growth factors, transforming growth factors (TGFs), and tumor necrosis factors (TNFs).

According to certain other embodiments, the binding region comprises a synthetic ligand capable of binding an extracellular target biomolecule (*see e.g.* Liang S et al., J Mol Med 84: 764-73 (2006); Ahmed S et al., Anal Chem 82: 7533-41 (2010); Kaur K et al., Methods Mol Biol 1248: 239-47 (2015)).

According to one specific, but non-limiting aspect, the binding region may comprise an immunoglobulin-type binding region. The term "immunoglobulin-type binding region" as used herein refers to a polypeptide region capable of binding one or more target biomolecules, such as an antigen or epitope. Binding regions may be functionally defined by their ability to bind to target molecules. Immunoglobulin-type binding regions are commonly derived from antibody or antibody-like structures; however, alternative scaffolds from other sources are contemplated within the scope of the term.

Immunoglobulin (Ig) proteins have a structural domain known as an Ig domain. Ig domains range in length from about 70-110 amino acid residues and possess a characteristic Ig-fold, in which typically 7 to 9 antiparallel beta strands arrange into two beta sheets which form a sandwich-like structure. The Ig fold is stabilized by hydrophobic amino acid interactions on inner surfaces of the sandwich and highly conserved disulfide bonds between cysteine residues in the strands. Ig domains may be variable (IgV or V-set), constant (IgC or C-set) or intermediate (IgI or I-set). Some Ig domains may be associated with a complementarity determining region (CDR), also called a "complementary determining region," which is important for the specificity of antibodies binding to their epitopes. Ig-like domains are also found in non-immunoglobulin proteins and are classified on that basis as members of the Ig superfamily of proteins. The HUGO Gene Nomenclature Committee (HGNC) provides a list of members of the Ig-like domain containing family.

An immunoglobulin-type binding region may be a polypeptide sequence of an antibody or antigen-binding fragment thereof wherein the amino acid sequence has been varied from that of a native antibody or an Ig-like domain of a non-immunoglobulin protein, for example by molecular engineering or selection by library screening. Because of the relevance of recombinant DNA techniques and *in vitro* library screening in the generation of immunoglobulin-type binding regions, antibodies can be redesigned to obtain desired characteristics, such as smaller size, cell entry, or other therapeutic improvements. The possible variations are many and may range from the changing of just one amino acid to the complete redesign of, for example, a variable region. Typically, changes in the variable region will be made in order to improve the antigen-binding characteristics, improve variable region stability, or reduce the potential for immunogenic responses.

There are numerous immunoglobulin-type binding regions contemplated as components of the present invention. In certain embodiments, the immunoglobulin-type binding region is derived from an immunoglobulin binding region, such as an antibody paratope capable of binding an extracellular target biomolecule. In certain other embodiments, the immunoglobulin-type binding region comprises an engineered polypeptide not derived from any immunoglobulin domain but which functions like an immunoglobulin binding region by providing high-affinity binding to an extracellular target biomolecule. This engineered polypeptide may optionally include polypeptide scaffolds comprising or consisting essentially of complementary determining regions from immunoglobulins as described herein.

There are also numerous binding regions in the prior art that are useful for targeting polypeptides to specific cell-types via their high-affinity binding characteristics. In certain embodiments, the binding region of the present proteins is selected from the group which includes single-domain antibody domains (sdAbs), nanobodies, heavy-chain antibody domains derived from camelids (V_{H}H fragments), bivalent nanobodies, heavy-chain antibody domains derived from cartilaginous fishes, immunoglobulin new antigen receptors (IgNARs), V_{NAR} fragments, single-chain variable (scFv) fragments, multimerizing scFv fragments (diabodies, triabodies, tetrabodies), bispecific tandem scFv fragments, disulfide stabilized antibody variable (Fv) fragments, disulfide stabilized antigen-binding (Fab) fragments consisting of the V_{L}, V_{H}, C_{L} and C_{H}1 domains, divalent F(ab')2 fragments, Fd fragments consisting of the heavy chain and C_{H}1 domains, single chain Fv-C_{H}3 minibodies, bispecific minibodies, dimeric C_{H}2 domain fragments (C_{H}2D), Fc antigen binding domains (Fcabs), isolated complementary determining region 3 (CDR3) fragments, constrained framework region 3, CDR3, framework region 4 (FR3-CDR3-FR4) polypeptides, small modular immunopharmaceutical (SMIP) domains, scFv-Fc fusions, multimerizing scFv fragments (diabodies, triabodies, tetrabodies), disulfide stabilized antibody variable (Fv) fragments, disulfide stabilized antigen-binding (Fab) fragments consisting of the V_{L}, V_{H}, C_{L} and C_{H}1 domains, bivalent nanobodies, bivalent minibodies, bivalent F(ab')₂ fragments (Fab dimers), bispecific tandem V_{H}H fragments, bispecific tandem scFv fragments, bispecific nanobodies, bispecific minibodies, and any genetically manipulated counterparts of the foregoing that retain its paratope and binding function (*see* Saerens D et al., Curr Opin Pharmacol 8: 600-8 (2008); Dimitrov D, MAbs 1: 26-8 (2009); Weiner L, Cell 148: 1081-4 (2012); Ahmad Z et al., Clin Dev Immunol 2012: 980250 (2012)). There are a variety of binding regions comprising polypeptides derived from the constant regions of immunoglobulins, such as, *e.g.,* engineered dimeric Fc domains, monomeric Fcs (mFcs), scFv-Fcs, V_{H}H-Fcs, C_{H}2 domains, monomeric C_{H}3s domains (mC_{H}3s), synthetically reprogrammed immunoglobulin domains, and/or hybrid fusions of immunoglobulin domains with ligands (Hofer T et al., Proc Natl Acad Sci U. S. A. 105: 12451-6 (2008); Xiao J et al., J Am Chem Soc 131: 13616-13618 (2009); Xiao X et al., Biochem Biophys Res Commun 387: 387-92 (2009); Wozniak-Knopp G et al., Protein Eng Des Sel 23 289-97 (2010); Gong R et al., PLoS ONE 7: e42288 (2012); Wozniak-Knopp G et al., PLoS ONE 7: e30083 (2012); Ying T et al., J Biol Chem 287: 19399-408 (2012); Ying T et al., J Biol Chem 288: 25154-64 (2013); Chiang M et al., J Am Chem Soc 136: 3370-3 (2014); Rader C, Trends Biotechnol 32: 186-97 (2014); Ying T et al., Biochimica Biophys Acta 1844: 1977-82 (2014)).

In accordance with certain other embodiments, the binding region comprises an engineered, alternative scaffold to immunoglobulin domains. Engineered alternative scaffolds are known in the art which exhibit similar functional characteristics to immunoglobulin-derived structures, such as high-affinity and specific binding of target biomolecules, and may provide improved characteristics to immunoglobulin domains, such as, *e.g.,* greater stability or reduced immunogenicity. Generally, alternative scaffolds to immunoglobulins are less than 20 kilodaltons, consist of a single polypeptide chain, lack cysteine residues, and exhibit relatively high thermodynamic stability.

For certain embodiments of the proteins of the present invention, the binding region comprises an alternative scaffold selected from the group which includes engineered, fibronectin-derived, 10^{th} fibronectin type III (10Fn3) domain (monobodies, AdNectins™, or AdNexins™); engineered, tenascin-derived, tenascin type III domain (Centryns™); engineered, ankyrin repeat motif containing polypeptide (DARPins™): engineered, low-density-lipoprotein-receptor-derived, A domain (LDLR-A) (Avimers™); lipocalin (anticalins); engineered, protease inhibitor-derived, Kunitz domain; engineered, Protein-A-derived, Z domain (Affibodies™); engineered, gamma-B crystallin-derived scaffold or engineered, ubiquitin-derived scaffold (Affilins); Sac7d-derived polypeptides (Nanoffitins® or affitins); engineered, Fyn-derived, SH2 domain (Fynomers®); miniproteins; C-type lectin-like domain scaffolds, engineered antibody mimic, and any genetically manipulated counterparts of the foregoing that retains its binding functionality (Wörn A, Plückthun A, J Mol Biol 305: 989-1010 (2001); Xu L et al., Chem Biol 9: 933-42 (2002); Wikman M et al., Protein Eng Des Sel 17: 455-62 (2004); Binz H et al., Nat Biotechnol 23: 1257-68 (2005); Hey T et al., Trends Biotechnol 23 :514-522 (2005); Holliger P, Hudson P, Nat Biotechnol 23: 1126-36 (2005); Gill D, Damle N, Curr Opin Biotech 17: 653-8 (2006); Koide A, Koide S, Methods Mol Biol 352: 95-109 (2007); Byla P et al., J Biol Chem 285: 12096 (2010); Zoller F et al., Molecules 16: 2467-85 (2011)).

For example, numerous alternative scaffolds have been identified which bind to the extracellular receptor HER2 (*see e.g.* Wikman M et al., Protein Eng Des Sel 17: 455-62 (2004); Orlova A et al. Cancer Res 66: 4339-8 (2006); Ahlgren S et al., Bioconjug Chem 19: 235-43 (2008); Feldwisch J et al., J Mol Biol 398: 232-47 (2010); U.S. patents 5,578,482; 5,856,110; 5,869,445; 5,985,553; 6,333,169; 6,987,088; 7,019,017; 7,282,365; 7,306,801; 7,435,797; 7,446,185; 7,449,480; 7,560,111; 7,674,460; 7,815,906; 7,879,325; 7,884,194; 7,993,650; 8,241,630; 8,349,585; 8,389,227; 8,501,909; 8,512,967; 8,652,474; and U.S. patent application 2011/0059090).

Any of the above binding regions may be used as a component of the protein of the present invention as long as the binding region component has a dissociation constant of 10⁻⁵ to 10⁻¹² moles/liter, preferably less than 200 nM, towards an extracellular target biomolecule as described herein.

### Extracellular Target Biomolecules

The binding region of the protein of the invention is capable of binding specifically to an extracellular target biomolecule, preferably which is physically-coupled to the surface of a cell type of interest, such as a cancer cell, tumor cell, plasma cell, infected cell, or host cell harboring an intracellular pathogen.

The term "target biomolecule" refers to a biological molecule, commonly a protein or a protein modified by post-translational modifications, such as glycosylation, which is capable of being bound by a binding region to target a protein to a specific cell-type or location within an organism. Extracellular target biomolecules may include various epitopes, including unmodified polypeptides, polypeptides modified by the addition of biochemical functional groups, and glycolipids (*see e.g.* U.S. Patent 5,091,178; EP 2431743). It is desirable that an extracellular target biomolecule be endogenously internalized or be readily forced to internalize upon interaction with the protein of the invention.

For purposes of the present invention, the term "extracellular" with regard to modifying a target biomolecule refers to a biomolecule that has at least a portion of its structure exposed to the extracellular environment. Extracellular target biomolecules include cell membrane components, transmembrane spanning proteins, cell membrane-anchored biomolecules, cell-surface-bound biomolecules, and secreted biomolecules.

With regard to the present invention, the phrase "physically coupled" when used to describe a target biomolecule means both covalent and/or non-covalent intermolecular interactions that couple the target biomolecule, or a portion thereof, to the outside of a cell, such as a plurality of non-covalent interactions between the target biomolecule and the cell where the energy of each single interaction is on the order of about 1-5 kiloCalories (*e.g.* electrostatic bonds, hydrogen bonds, Van der Walls interactions, hydrophobic forces, etc.). All integral membrane proteins can be found physically coupled to a cell membrane, as well as peripheral membrane proteins. For example, an extracellular target biomolecule might comprise a transmembrane spanning region, a lipid anchor, a glycolipid anchor, and/or be non-covalently associated (*e.g*. via non-specific hydrophobic interactions and/or lipid binding interactions) with a factor comprising any one of the foregoing.

Extracellular target biomolecules of the binding region of the proteins of the invention may include biomarkers over-proportionately or exclusively present on cancer cells, immune cells, and cells infected with intracellular pathogens, such as viruses, bacteria, fungi, prions, or protozoans.

The binding regions of the proteins of the invention may be designed or selected based on numerous criteria, such as the cell-type specific expression of their target biomolecules and/or the physical localization of their target biomolecules with regard to specific cell types. For example, certain proteins of the present invention comprise binding domains capable of binding cell-surface targets which are expressed exclusively by only one cell-type to the cell surface.

The general structure of the proteins of the present invention is modular, in that various, diverse binding regions may be used with the same Shiga toxin effector region to provide for diverse targeting of various extracellular target biomolecules and thus targeting of cytotoxicity, cytostasis, diagnostic agents, and/or exogenous material delivery to various diverse cell types.

### B. Shiga Toxin Effector Regions Derived from A Subunits of Members of the Shiga Toxin Family

For purposes of the present invention, the phrase "Shiga toxin effector region" refers to a polypeptide region derived from a Shiga toxin A Subunit of a member of the Shiga toxin family that is capable of exhibiting at least one Shiga toxin function. Shiga toxin functions include, *e.g.,* cell entry, lipid membrane deformation, directing subcellular routing, avoiding degradation, catalytically inactivating ribosomes, effectuating cytotoxicity, and effectuating cytostatic effects.

A member of the Shiga toxin family refers to any member of a family of naturally occurring protein toxins which are structurally and functionally related, notably toxins isolated from *S. dysenteriae* and *E. coli* (Johannes, Nat Rev Microbiol 8: 105-16 (2010)). For example, the Shiga toxin family encompasses true Shiga toxin (Stx) isolated from *S. dysenteriae* serotype 1, Shiga-like toxin 1 variants (SLT1 or Stx1 or SLT-1 or Slt-I) isolated from serotypes of enterohemorrhagic *E. coli,* and Shiga-like toxin 2 variants (SLT2 or Stx2 or SLT-2) isolated from serotypes of enterohemorrhagic *E. coli.* SLT1 differs by only one residue from Stx, and both have been referred to as Verocytotoxins or Verotoxins (VTs) (O'Brien, Curr Top Microbiol Immunol 180: 65-94 (1992)). Although SLT1 and SLT2 variants are only about 53-60% similar to each other at the amino acid sequence level, they share mechanisms of enzymatic activity and cytotoxicity common to the members of the Shiga toxin family (Johannes, Nat Rev Microbiol 8: 105-16 (2010)). Over 39 different Shiga toxins have been described, such as the defined subtypes Stxla, Stx1c, Stxld, and Stx2a-g (Scheutz F et al., J Clin Microbiol 50: 2951-63 (2012)). Members of the Shiga toxin family are not naturally restricted to any bacterial species because Shiga-toxin-encoding genes can spread among bacterial species via horizontal gene transfer (Strauch E et al., Infect Immun 69: 7588-95 (2001); Zhaxybayeva O, Doolittle W, Curr Biol 21: R242-6 (2011)). As an example of interspecies transfer, a Shiga toxin was discovered in a strain of A. *haemolyticus* isolated from a patient (Grotiuz G et al., J Clin Microbiol 44: 3838-41 (2006)). Once a Shiga toxin encoding polynucleotide enters a new subspecies or species, the Shiga toxin amino acid sequence is presumed to be capable of developing slight sequence variations due to genetic drift and/or selective pressure while still maintaining a mechanism of cytotoxicity common to members of the Shiga toxin family (*see* Scheutz, J Clin Microbiol 50: 2951-63 (2012)).

Shiga toxin effector regions of the protein of the invention may comprise or consist essentially of a polypeptide derived from a Shiga toxin A Subunit dissociated from any form of its native Shiga toxin B Subunit. In addition, the proteins of the present invention do not comprise any polypeptide comprising or consisting essentially of a functional binding domain of a native Shiga toxin B subunit. Rather, the Shiga toxin A Subunit derived regions are functionally associated with heterologous binding regions to effectuate cell targeting.

In certain embodiments, a Shiga toxin effector region of the protein of the invention may comprise or consist essentially of a full length Shiga toxin A Subunit (*e.g.* SLT-1A (SEQ ID NO:1), StxA (SEQ ID NO:2), or SLT-2A (SEQ ID NO:3)), noting that naturally occurring Shiga toxin A Subunits may comprise precursor forms containing signal sequences of about 22 amino acids at their amino-terminals which are removed to produce mature Shiga toxin A Subunits and are recognizable to the skilled worker. In other embodiments, the Shiga toxin effector region of the protein of the invention may comprise or consist essentially of a truncated Shiga toxin A Subunit which is shorter than a full-length Shiga toxin A Subunit.

Shiga-like toxin 1 A Subunit truncations are catalytically active, capable of enzymatically inactivating ribosomes *in vitro,* and cytotoxic when expressed within a cell (LaPointe, J Biol Chem 280: 23310-18 (2005)). The smallest Shiga toxin A Subunit fragment exhibiting full enzymatic activity is a polypeptide composed of residues 1-239 of Slt1A (LaPointe, J Biol Chem 280: 23310-18 (2005)). Although the smallest fragment of the Shiga toxin A Subunit reported to retain substantial catalytic activity was residues 75-247 of StxA (Al-Jaufy, Infect Immun 62: 956-60 (1994)), a StxA truncation expressed *de novo* within a eukaryotic cell requires only up to residue 240 to reach the cytosol and exert catalytic inactivation of ribosomes (LaPointe, J Biol Chem 280: 23310-18 (2005)).

Shiga toxin effector regions may commonly be smaller than the full length A subunit. It is preferred that the Shiga toxin effector region maintain the polypeptide region from amino acid position 77 to 239 (SLT-1A (SEQ ID NO:1) or StxA (SEQ ID NO:2)) or the equivalent in other A Subunits of members of the Shiga toxin family (*e.g.* 77 to 238 of (SEQ ID NO:3)). For example, in certain embodiments of the invention, a Shiga toxin effector region derived from SLT-1A may comprise or consist essentially of amino acids 75 to 251 of SEQ ID NO:1, 1 to 241 of SEQ ID NO:1, 1 to 251 of SEQ ID NO:1, or amino acids 1 to 261 of SEQ ID NO:1. Among certain other embodiments, a Shiga toxin effector region derived from StxA may comprise or consist essentially of amino acids 75 to 251 of SEQ ID NO:2, 1 to 241 of SEQ ID NO:2, 1 to 251 of SEQ ID NO:2, or amino acids 1 to 261 of SEQ ID NO:2. Among certain other embodiments, a Shiga toxin effector region derived from SLT-2 may comprise or consist essentially of amino acids 75 to 251 of SEQ ID NO:3, 1 to 241 of SEQ ID NO:3, 1 to 251 of SEQ ID NO:3, or amino acids 1 to 261 of SEQ ID NO:3.

The invention further provides variants of the proteins of the invention, wherein the Shiga toxin effector region differs from a naturally occurring Shiga toxin A Subunit by up to 1, 2, 3, 4, 5, 6, 7, 8, 9, 10, 15, 20, 25, 30, 35, 40 or more amino acid residues (but by no more than that which retains at least 85%, 90%, 95%, 99% or more amino acid sequence identity). Thus, a polypeptide region derived from an A Subunit of a member of the Shiga toxin family may comprise additions, deletions, truncations, or other alterations from the original sequence as long as at least 85%, 90%, 95%, 99% or more amino acid sequence identity is maintained to a naturally occurring Shiga toxin A Subunit.

Accordingly, the Shiga toxin effector region may comprise or consist essentially of amino acid sequences having at least 55%, 60%, 65%, 70%, 75%, 80%, 85%, 90%, 95%, 97%, 98%, 99%, 99.5% or 99.7% overall sequence identity to a naturally occurring Shiga toxin A Subunit, such as SLT-1A (SEQ ID NO:1), StxA (SEQ ID NO:2), and/or SLT-2A (SEQ ID NO:3).

Optionally, either a full length or a truncated version of the Shiga toxin A Subunit may comprise one or more mutations (*e.g*. substitutions, deletions, insertions or inversions). It is preferred in certain embodiments of the invention that the Shiga toxin effector region has sufficient sequence identity to a naturally occurring Shiga toxin A Subunit to retain cytotoxicity after entry into a cell, either by well-known methods of host cell transformation, transfection, infection or induction, or by internalization mediated by the cell targeting binding region linked with the Shiga toxin effector region. The most critical residues for enzymatic activity and/or cytotoxicity in the Shiga toxin A Subunits have been mapped to the following residue-positions: aspargine-75, tyrosine-77, glutamate-167, arginine-170, and arginine-176 among others (Di, Toxicon 57: 525-39 (2011)). In any one of the embodiments of the invention, the Shiga toxin effector region may preferably but not necessarily maintain one or more conserved amino acids at positions, such as those found at positions 77, 167, 170, and 176 in StxA, SLT-1A, or the equivalent conserved position in other members of the Shiga toxin family which are typically required for cytotoxic activity. The capacity of a cytotoxic protein of the invention to cause cell death, *e.g.* its cytotoxicity, may be measured using any one or more of a number of assays well known in the art.

In certain embodiments of the protein of the invention, one or more amino acid residues may be mutated, inserted, or deleted in order to increase the enzymatic activity of the Shiga toxin effector region. For example, mutating residue-position alanine-231 in Stx1A to glutamate increased its enzymatic activity *in vitro* (Suhan M, Hovde C, Infect Immun 66: 5252-9 (1998)).

In certain embodiments of the protein of the invention, one or more amino acid residues may be mutated or deleted in order to reduce or eliminate catalytic and/or cytotoxic activity of the Shiga toxin effector region. The catalytic and/or cytotoxic activity of the A Subunits of members of the Shiga toxin family may be reduced or eliminated by mutation or truncation. The positions labeled tyrosine-77, glutamate-167, arginine-170, tyrosine-114, and tryptophan-203 have been shown to be important for the catalytic activity of Stx, Stx1, and Stx2 (Hovde C et al., Proc Natl Acad Sci USA 85: 2568-72 (1988); Deresiewicz R et al., Biochemistry 31: 3272-80 (1992); Deresiewicz R et al., Mol Gen Genet 241: 467-73 (1993); Ohmura M et al., Microb Pathog 15: 169-76 (1993); Cao C et al., Microbiol Immunol 38: 441-7 (1994); Suhan, Infect Immun 66: 5252-9 (1998)). Mutating both glutamate-167 and arginine-170 eliminated the enzymatic activity of Slt-I A1 in a cell-free ribosome inactivation assay (LaPointe, J Biol Chem 280: 23310-18 (2005)). In another approach using *de novo* expression of Slt-I A1 in the endoplasmic reticulum, mutating both glutamate-167 and arginine-170 or truncating it to residues 1-239 eliminated Slt-I A1 fragment cytotoxicity at that expression level (LaPointe, J Biol Chem 280: 23310-18 (2005)).

As used herein, the retention of "significant" Shiga toxin effector function refers to a level of Shiga toxin functional activity, as measured by an appropriate quantitative assay with reproducibility comparable to a wild-type Shiga toxin effector polypeptide control. For *in vitro* ribosome inhibition, significant Shiga toxin effector function is exhibiting an IC₅₀ of 300 pM or less depending on the source of the ribosomes (*e.g*. bacteria, archaea, or eukaryote (algae, fungi, plants, or animals)). This is significantly greater inhibition as compared to the approximate IC₅₀ of 100,000 pM for the catalytically inactive SLT-1A 1-251 double mutant (Y77S/E167D). For cytotoxicity in a target positive cell kill assay in laboratory cell culture, significant Shiga toxin effector function is exhibiting a CD₅₀ of 100, 50, or 30 nM or less, depending on the cell line and its expression of the appropriate extracellular target biomolecule. This is significantly greater cytotoxicity to the appropriate target cell line as compared to the SLT-1A component alone, without a cell targeting binding region, which has a CD₅₀ of 100-10,000 nM, depending on the cell line.

For some samples, accurate values for either IC₅₀ or CD₅₀ might be unobtainable due to the inability to collect the required data points for an accurate curve fit. Inaccurate IC₅₀ and/or CD₅₀ values should not be considered when determining significant Shiga toxin effector function activity. Data insufficient to accurately fit a curve as described in the analysis of the data from exemplary Shiga toxin effector function assays, such as, *e.g.,* assays described in the Examples, should not be considered as representative of actual Shiga toxin effector function. For example, theoretically, neither an IC₅₀ or CD₅₀ can be determined if greater than 50% ribosome inhibition or cell death, respectively, does not occur in a concentration series for a given sample.

The failure to detect activity in Shiga toxin effector function may be due to improper expression, polypeptide folding, and/or polypeptide stability rather than a lack of cell entry, subcellular routing, and/or enzymatic activity. Assays for Shiga toxin effector functions may not require much protein of the invention to measure significant amounts of Shiga toxin effector function activity. To the extent that an underlying cause of low or no effector function is determined empirically to relate to protein expression or stability, one of skill in the art may be able to compensate for such factors using protein chemistry and molecular engineering techniques known in the art, such that a Shiga toxin functional effector activity may be restored and measured. As examples, improper cell-based expression may be compensated for by using different expression control sequences; improper polypeptide folding and/or stability may benefit from stabilizing terminal sequences, or compensatory mutations in non-effector regions which stabilize the three-dimensional structure of the protein, etc. When new assays for individual Shiga toxin functions become available, Shiga toxin effector regions or polypeptides may be analyzed for any level of those Shiga toxin effector functions, such as for being within a certain-fold activity of a wild-type Shiga toxin effector polypeptide. Examples of meaningful activity differences are, *e.g.,* Shiga toxin effector regions that have 1000-fold or 100-fold or less the activity of a wild-type Shiga toxin effector polypeptide; or that have 3-fold to 30-fold or more activity compared to a functional knock-down or knockout Shiga toxin effector polypeptide.

Certain Shiga toxin effector functions are not easily measurable, *e.g*. subcellular routing functions. Currently there is no routine, quantitative assay to distinguish whether the failure of a Shiga toxin effector polypeptide to be cytotoxic is due to improper subcellular routing, but at a time when tests are available, then Shiga toxin effector polypeptides may be analyzed for any significant level of subcellular routing as compared to the appropriate wild-type Shiga toxin effector region.

It should be noted that even if the cytotoxicity of a Shiga toxin effector polypeptide is reduced relative to wild-type, in practice, applications using attenuated Shiga toxin effector polypeptides may be equally or more effective than those using wild-type Shiga toxin effector polypeptides because the highest potency variants might exhibit undesirable effects which are minimized in reduced potency variants. Wild-type Shiga toxin effector polypeptides are very potent, being able to kill with only one molecule reaching the cytosol or perhaps 40 molecules being internalized. Shiga toxin effector polypeptides with even considerably reduced Shiga toxin effector functions, such as, *e.g*., subcellular routing or cytotoxicity, as compared to wild-type Shiga toxin effector polypeptides may still be potent enough for practical applications involving targeted cell killing and/or specific cell detection.

### C. Endoplasmic Reticulum Retention/Retrieval Signal Motif of a Member of the KDEL Family

For purposes of the present invention, the phrase "endoplasmic reticulum retention/retrieval signal motif," KDEL-type signal motif, or signal motif refers to any member of the KDEL family capable of functioning within a eukaryotic cell to promote subcellular localization of a protein to the endoplasmic reticulum via KDEL receptors.

The carboxy-terminal lysine-asparagine-glutamate-leucine (KDEL) sequence is a canonical, endoplasmic reticulum retention and retrieval signal motif for soluble proteins in eukaryotic cells and is recognized by KDEL receptors (*see,* Capitani M, Sallese M, FEBS Lett 583: 3863-71 (2009), *for review*)*.* The KDEL family of signal motifs includes many KDEL-like motifs, such as HDEL (SEQ ID NO: 36), RDEL (SEQ ID NO: 38), WDEL (SEQ ID NO: 39), YDEL (SEQ ID NO: 40), HEEL (SEQ ID NO: 42), KEEL (SEQ ID NO: 43), REEL (SEQ ID NO: 44), KFEL (SEQ ID NO: 47), KIEL (SEQ ID NO: 59), DKEL (SEQ ID NO: 60), KKEL (SEQ ID NO: 63), HNEL (SEQ ID NO: 67), HTEL (SEQ ID NO: 68), KTEL (SEQ ID NO: 69), and HVEL (SEQ ID NO: 70), all of which are found at the carboxy-terminals of proteins which are known to be residents of the lumen of the endoplasmic reticulum of organisms throughout multiple phylogenetic kingdoms (Munro S, Pelham H, Cell 48: 899-907 (1987); Raykhel I et al., J Cell Biol 179: 1193-204 (2007)). The KDEL signal motif family includes at least 46 polypeptide variants shown using synthetic constructs (Raykhel, J Cell Biol 179: 1193-204 (2007)). Additional KDEL signal motifs include ALEDEL (SEQ ID NO: 82), HAEDEL (SEQ ID NO: 83), HLEDEL (SEQ ID NO: 84), KLEDEL (SEQ ID NO: 85), IRSDEL (SEQ ID NO: 86), ERSTEL (SEQ ID NO: 87), and RPSTEL (SEQ ID NO: 88) (Alanen H et al., J Mol Biol 409: 291-7 (2011)). A generalized consensus motif representing the majority of KDEL signal motifs has been described as [KRHQSA]-[DENQ]-E-L (Hulo N et al., Nucleic Acids Res 34: D227-30 (2006)).

Proteins containing KDEL family signal motifs are bound by KDEL receptors distributed throughout the Golgi complex and transported to the endoplasmic reticulum by a microtubule-dependent mechanism for release into the lumen of the endoplasmic reticulum (Griffiths G et al., J Cell Biol 127: 1557-74 (1994); Miesenböck G, Rothman J, J Cell Biol 129: 309-19 (1995)). KDEL receptors dynamically cycle between the Golgi complex and endoplasmic reticulum (Jackson M et al., EMBO J. 9: 3153-62 (1990); Schutze M et al., EMBO J. 13: 1696-1705 (1994)).

For purposes of the present invention, the members of the KDEL family include synthetic signal motifs able to function within a eukaryotic cell to promote subcellular localization of a protein to the endoplasmic reticulum via KDEL receptors. In other words, some members of the KDEL family might not occur in nature or have yet to be observed in nature but have or may be constructed and empirically verified using methods known in the art; see *e.g.,* Raykhel I et al., J Cell Biol 179: 1193-204 (2007).

As a component of the proteins of the invention, the KDEL-type signal motif is physically located, oriented, or arranged within the protein such that it is on a carboxy-terminal of a polypeptide.

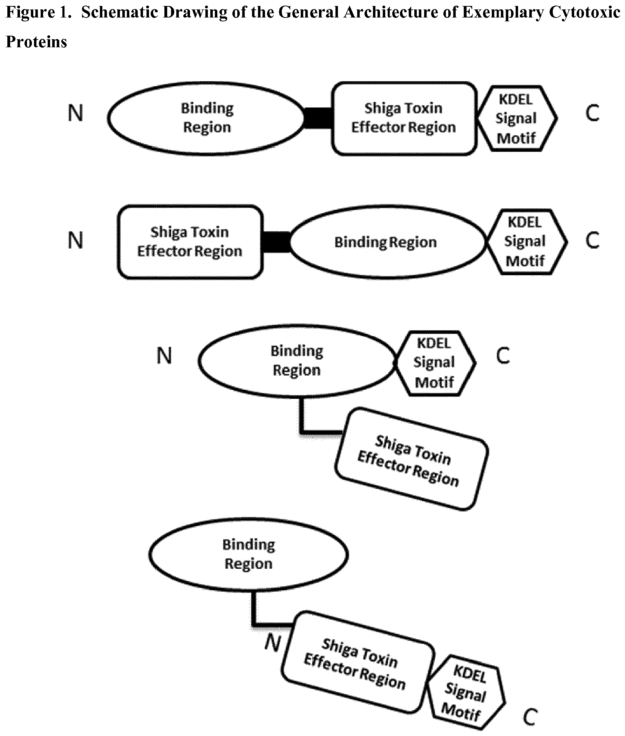

For the purposes of the present invention, the specific order or orientation is not fixed for the Shiga toxin effector region and the binding region in relation to each other or the entire protein (*see e.g.* Figure 1). The components of the proteins of the invention may be arranged in any order provided that the desired activities of the binding region and the Shiga toxin effector region are not eliminated. Desired activities include providing the protein with the ability, *e.g.,* to bind target expressing cells, induce cellular internalization, cause cytostasis, cause cytotoxicity, and/or deliver exogenous materials to the interiors of cells.

In the above embodiments of proteins of the invention, the binding regions, Shiga toxin effector regions (which may be cytotoxic and/or harbor one or more mutations reducing or eliminating catalytic activity and/or cytotoxicity), and endoplasmic reticulum retention/retrieval signal motif may be directly linked to each other and/or suitably linked to each other via one or more intervening polypeptide sequences, such as with one or more linkers well known in the art and/or described herein.

### D. Linkages Connecting Polypeptide Components of the Protein of the Invention and/or Their Subcomponents

Individual polypeptide and/or protein components of the protein of the invention, *e.g*. the binding regions and Shiga toxin effector regions (which may be cytotoxic and/or harbor one or more mutations altering, reducing, or eliminating catalytic activity and/or cytotoxicity), may be suitably linked to each other via one or more linkers well known in the art and/or described herein. Individual polypeptide subcomponents of the binding regions, *e.g.* CDR and/or ABR regions, may be suitably linked to each other via one or more linkers well known in the art and/or described herein (*see e.g.* Weisser N, Hall J, Biotechnol Adv 27: 502-20 (2009); Chen X et al., Adv Drug Deliv Rev 65: 1357-69 (2013)). Polypeptide components of the protein of the invention, *e.g*., multi-chain binding regions, may be suitably linked to each other or other polypeptide components via one or more linkers well known in the art. Peptide components of the protein of the invention, *e.g*., KDEL family endoplasmic reticulum retention/retrieval signal motifs, may be suitably linked to another component via one or more linkers, such as a proteinaceous linker, which are well known in the art.

Suitable linkers are generally those which allow each polypeptide component of the protein of the invention to fold with a three-dimensional structure very similar to the polypeptide components produced individually without any linker or other component. Suitable linkers include single amino acids, peptides, polypeptides, and linkers lacking any of the aforementioned such as, *e.g*., various non-proteinaceous carbon chains, whether branched or cyclic (*see e.g.* Chen X et al., Adv Drug Deliv Rev 65: 1357-69 (2013)).

Suitable linkers may be proteinaceous and comprise one or more amino acids, peptides, and/or polypeptides. Proteinaceous linkers are suitable for both recombinant fusion proteins and chemically linked conjugates. A proteinaceous linker typically has from about 2 to about 50 amino acid residues, such as, *e.g.,* from about 5 to about 30 or from about 6 to about 25 amino acid residues. The length of the linker selected will depend upon a variety of factors, such as, *e.g.,* the desired property or properties for which the linker is being selected (*see e.g.* Chen X et al., Adv Drug Deliv Rev 65: 1357-69 (2013)).

Suitable linkers may be non-proteinaceous, such as, *e.g.* chemical linkers (*see e.g.* Dosio F et al., Toxins 3: 848-83 (2011); Feld J et al., Oncotarget 4: 397-412 (2013)). Various non-proteinaceous linkers known in the art may be used to link binding regions to the Shiga toxin effector regions, such as linkers commonly used to conjugate immunoglobulin-derived polypeptides to heterologous polypeptides. For example, polypeptide regions of the proteins of the present invention may be linked using the functional side chains of their amino acid residues and carbohydrate moieties such as, *e.g.,* a carboxy, amine, sulfhydryl, carboxylic acid, carbonyl, hydroxyl, and/or cyclic ring group. For example, disulfide bonds and thioether bonds may be used to link two or more polypeptides (*see e.g.* Fitzgerald D et al., Bioconjugate Chem 1: 264-8 (1990); Pasqualucci L et al., Haematologica 80: 546-56 (1995)). In addition, non-natural amino acid residues may be used with other functional side chains, such as ketone groups (*see e.g.* Sun S et al., Chembiochem Jul 18 (2014); Tian F et al., Proc Natl Acad Sci USA 111: 1766-71 (2014)). Examples of non-proteinaceous chemical linkers include but are not limited to N-succinimidyl (4-iodoacetyl)-aminobenzoate, *S-*(*N*-succinimidyl) thioacetate (SATA), N-succinimidyl-oxycarbonyl-cu-methyl-a-(2-pyridyldithio) toluene (SMPT), N-succinimidyl 4-(2-pyridyldithio)-pentanoate (SPP), succinimidyl 4-(N-maleimidomethyl) cyclohexane carboxylate (SMCC or MCC), sulfosuccinimidyl (4-iodoacetyl)-aminobenzoate, 4-succinimidyl-oxycarbonyl-α-(2-pyridyldithio) toluene, sulfosuccinimidyl-6-(α-methyl-α-(pyridyldithiol)-toluamido) hexanoate, N-succinimidyl-3-(-2-pyridyldithio)-proprionate (SPDP), succinimidyl 6(3(-(-2-pyridyldithio)-proprionamido) hexanoate, sulfosuccinimidyl 6(3(-(-2-pyridyldithio)-propionamido) hexanoate, maleimidocaproyl (MC), maleimidocaproyl-valine-citrulline-*p*-aminobenzyloxycarbonyl (MC-vc-PAB), 3-maleimidobenzoic acid *N-*hydroxysuccinimide ester (MBS), alpha-alkyl derivatives, sulfoNHS-ATMBA (sulfosuccinimidyl N-[3-(acetylthio)-3-methylbutyryl-beta-alanine]), sulfodicholorphenol, 2-iminothiolane, 3-(2-pyridyldithio)-propionyl hydrazide, Ellman's reagent, dichlorotriazinic acid, and S-(2-thiopyridyl)-L-cysteine (*see e.g.* Thorpe P et al., Eur J Biochem 147: 197-206 (1985); Thorpe P et al., Cancer Res 47: 5924-31 (1987); Thorpe P et al., Cancer Res 48: 6396-403 (1988); Grossbard M et al., Blood 79: 576-85 (1992); Lui C et al., Proc Natl Acad Sci USA 93: 8618-23 (1996); Doronina S et al., Nat Biotechnol 21: 778-84 (2003); Feld J et al., Oncotarget 4: 397-412 (2013)).

Suitable linkers, whether proteinaceous or non-proteinaceous, may include, *e.g.,* protease sensitive, environmental redox potential sensitive, pH sensitive, acid cleavable, photocleavable, and/or heat sensitive linkers (*see e.g.* Dosio F et al., Toxins 3: 848-83 (2011); Chen X et al., Adv Drug Deliv Rev 65: 1357-69 (2013); Feld J et al., Oncotarget 4: 397-412 (2013)).

Proteinaceous linkers may be chosen for incorporation into recombinant fusion proteins of the present invention. For example, the component polypeptides of the proteins of the present invention or their subcomponents may be joined by one or more linkers comprising one or more amino acids, peptides, and/or polypeptides. For recombinant fusion proteins of the invention, linkers typically comprise about 2 to 50 amino acid residues, preferably about 5 to 30 amino acid residues (Argos P, J Mol Biol 211: 943-58 (1990); Williamson M, Biochem J 297: 240-60 (1994); George R, Heringa J, Protein Eng 15: 871-9 (2002); Kreitman R, AAPS J 8: E532-51 (2006)). Commonly, proteinaceous linkers comprise a majority of amino acid residues with polar, uncharged, and/or charged residues, such as, *e.g*., threonines, prolines, glutamines, glycines, and alanines (*see e.g.* Huston J et al. Proc Natl Acad Sci U.S.A. 85: 5879-83 (1988); Pastan I et al., Annu Rev Med 58: 221-37 (2007); Li J et al., Cell Immunol 118: 85-99 (1989); Cumber A et al. Bioconj Chem 3: 397-401 (1992); Friedman P et al., Cancer Res 53: 334-9 (1993); Whitlow M et al., Protein Engineering 6: 989-95 (1993); Siegall C et al., J Immunol 152: 2377-84 (1994); Newton et al. Biochemistry 35: 545-53 (1996); Ladurner et al. J Mol Biol 273: 330-7 (1997); Kreitman R et al., Leuk Lymphoma 52: 82-6 (2011); U.S. 4,894,443). Non-limiting examples of proteinaceous linkers include alanine-serine-glycine-glycine-proline-glutamate (ASGGPE) (SEQ ID NO: 89), valine-methionine (VM), alanine-methionine (AM), AM(G_{2 to 4}S)ₓAM (SEQ ID NO: 90)where G is glycine, S is serine, and x is an integer from 1 to 10.

Proteinaceous linkers may be selected based upon the properties desired. Proteinaceous linkers may be chosen by the skilled worker with specific features in mind, such as to optimize one or more of the fusion molecule's folding, stability, expression, solubility, pharmacokinetic properties, pharmacodynamic properties, and/or the activity of the fused domains in the context of a fusion construct as compared to the activity of the same domain by itself. For example, proteinaceous linkers may be selected based on flexibility, rigidity, and/or cleavability (*see e.g.* Chen X et al., Adv Drug Deliv Rev 65: 1357-69 (2013)). The skilled worker may use databases and linker design software tools when choosing linkers. Certain linkers may be chosen to optimize expression (*see e.g.* Turner D et al., J Immunl Methods 205: 43-54 (1997)). Certain linkers may be chosen to promote intermolecular interactions between identical polypeptides or proteins to form homomultimers or different polypeptides or proteins to form heteromultimers. For example, proteinaceous linkers may be selected which allow for desired non-covalent interactions between polypeptide components of proteins of the invention, such as, *e.g*., interactions related to the formation dimers and other higher order multimers (*see e.g.* U.S. 4,946,778).

Flexible proteinaceous linkers are often greater than 12 amino acid residues long and rich in small, non-polar amino acid residues, polar amino acid residues, and/or hydrophilic amino acid residues, such as, *e.g.,* glycines, serines, and threonines (*see e.g.* Bird R et al., Science 242: 423-6 (1988); Friedman P et al., Cancer Res 53: 334-9 (1993); Siegall C et al., J Immunol 152: 2377-84 (1994)). Flexible proteinaceous linkers may be chosen to increase the spatial separation between components and/or to allow for intramolecular interactions between components. For example, various "GS" linkers are known to the skilled worker and are composed of multiple glycines and/or one or more serines, sometimes in repeating units, such as, *e.g.,* (GₓS)ₙ (SEQ ID NO: 91), (SₓG)ₙ (SEQ ID NO: 92), (GGGGS)ₙ (SEQ ID NO: 93), and (G)ₙ(SEQ ID NO: 94). in which x is 1 to 6 and n is 1 to 30 (*see e.g.* WO 96/06641). Non-limiting examples of flexible proteinaceous linkers include GKSSGSGSESKS (SEQ ID NO: 95), GSTSGSGKSSEGKG (SEQ ID NO: 96), GSTSGSGKSSEGSGSTKG (SEQ ID NO: 97), GSTSGSGKPGSGEGSTKG (SEQ ID NO: 99), EGKSSGSGSESKEF (SEQ ID NO: 100), SRSSG (SEQ ID NO: 101), and SGSSC (SEQ ID NO: 102).

Rigid proteinaceous linkers are often stiff alpha-helical structures and rich in proline residues and/or one or more strategically placed prolines (*see* Chen X et al., Adv Drug Deliv Rev 65: 1357-69 (2013)). Rigid linkers may be chosen to prevent intramolecular interactions between components.

Suitable linkers may be chosen to allow for *in vivo* separation of components, such as, *e.g.,* due to cleavage and/or environment-specific instability (*see* Dosio F et al., Toxins 3: 848-83 (2011); Chen X et al., Adv Drug Deliv Rev 65: 1357-69 (2013)). *In vivo* cleavable proteinaceous linkers are capable of unlinking by proteolytic processing and/or reducing environments often at a specific site within an organism or inside a certain cell type *(see e.g.* Doronina S et al., Bioconjug Chem 17: 144-24 (2006); Erickson H et al., Cancer Res 66: 4426-33 (2006)). *In vivo* cleavable proteinaceous linkers often comprise protease sensitive motifs and/or disulfide bonds formed by one or more cysteine pairs (*see e.g.* Pietersz G et al., Cancer Res 48: 4469-76 (1998); The J et al., J Immunol Methods 110: 101-9 (1998); *see* Chen X et al., Adv Drug Deliv Rev 65: 1357-69 (2013)). *In vivo* cleavable proteinaceous linkers can be designed to be sensitive to proteases that exist only at certain locations in an organism, compartments within a cell, and/or become active only under certain physiological or pathological conditions (such as, *e.g.,* proteases with abnormally high levels, proteases overexpressed at certain disease sites, and proteases specifically expressed by a pathogenic microorganism). For example, there are proteinaceous linkers known in the art which are cleaved by proteases present only intracellularly, proteases present only within specific cell types, and proteases present only under pathological conditions like cancer or inflammation, such as, *e.g*., R-x-x-R motif and AMGRSGGGCAGNRVGSSLSCGGLNLQAM (SEQ ID NO: 103).

In certain embodiments of the proteins of the present invention, a linker may be used which comprises one or more protease sensitive sites to provide for cleavage by a protease present within a target cell. In certain embodiments of the proteins of the invention, a linker may be used which is not cleavable to reduce unwanted toxicity after administration to a vertebrate organism (*see e.g.* Polson A et al., Cancer Res 69: 2358-64 (2009)).

Suitable linkers may include, *e.g*., protease sensitive, environmental redox potential sensitive, pH sensitive, acid cleavable, photocleavable, and/or heat sensitive linkers, whether proteinaceous or non-proteinaceous (*see* Chen X et al., Adv Drug Deliv Rev 65: 1357-69 (2013)).

Suitable cleavable linkers may include linkers comprising cleavable groups which are known in the art such as, *e.g.,* linkers noted by Zarling D et al., J Immunol 124: 913-20 (1980); Jung S, Moroi M, Biochem Biophys Acta 761: 152-62 (1983); Bouizar Z et al., Eur J Biochem 155: 141-7 (1986); Park L et al., J Biol Chem 261: 205-10 (1986); Browning J, Ribolini A, J Immunol 143: 1859-67 (1989); Joshi S, Burrows R, J Biol Chem 265: 14518-25 (1990).

Suitable linkers may include pH sensitive linkers. For example, certain suitable linkers may be chosen for their instability in lower pH environments to provide for dissociation inside a subcellular compartment of a target cell. For example, linkers that comprise one or more trityl groups, derivatized trityl groups, bismaleimideothoxy propane groups, adipic acid dihydrazide groups, and/or acid labile transferrin groups, may provide for release of components of the invention, *e.g.* a polypeptide component, in environments with specific pH ranges (*see e.g.* Welhöner H et al., J Biol Chem 266: 4309-14 (1991); Fattom A et al., Infect Immun 60: 584-9 (1992)). Certain linkers may be chosen which are cleaved in pH ranges corresponding to physiological pH differences between tissues, such as, *e.g.,* the pH of tumor tissue is lower than in healthy tissues (*see e.g.* U.S. 5,612,474).

Photocleavable linkers are linkers that are cleaved upon exposure to electromagnetic radiation of certain wavelength ranges, such as light in the visible range (*see e.g.* Goldmacher V et al., Bioconj Chem 3: 104-7 (1992)). Photocleavable linkers may be used to release a component of a protein of the invention, *e.g.* a polypeptide component, upon exposure to light of certain wavelengths. Non-limiting examples of photocleavable linkers include a nitrobenzyl group as a photocleavable protective group for cysteine, nitrobenzyloxycarbonyl chloride cross-linkers, hydroxypropylmethacrylamide copolymer, glycine copolymer, fluorescein copolymer, and methylrhodamine copolymer (Hazum E et al., Pept Proc Eur Pept Symp, 16th, Brunfeldt K, ed., 105-110 (1981); Senter et al., Photochem Photobiol 42: 231-7 (1985); Yen et al., Makromol Chem 190: 69-82 (1989); Goldmacher V et al., Bioconj Chem 3: 104-7 (1992)). Photocleavable linkers may have particular uses in linking components to form proteins of the invention designed for treating diseases, disorders, and conditions that can be exposed to light using fiber optics. In certain embodiments of the proteins of the invention, a cell-targeting binding region is linked to a Shiga toxin effector region using any number of means known to the skilled worker, including both covalent and noncovalent linkages (*see e.g.* Chen X et al., Adv Drug Deliv Rev 65: 1357-69 (2013); Behrens C, Liu B, MAbs 6: 46-53 (2014)).

In certain embodiments of the proteins of the present invention, the protein comprises a binding region which is a scFv with a linker connecting a heavy chain variable (V_{H}) domain and a light chain variable (V_{L}) domain. There are numerous linkers known in the art suitable for this purpose, such as, *e.g.,* the 15-residue (Gly4Ser)₃ (SEQ ID NO: 104) peptide. Suitable scFv linkers which may be used in forming non-covalent multivalent structures include GGS, GGGS (Gly3Ser or G3S) (SEQ ID NO: 105), GGGGS (Gly4Ser or G4S) (SEQ ID NO: 106), GGGGSGGG (SEQ ID NO: 107), GGSGGGG (SEQ ID NO: 108), GSTSGGGSGGGSGGGGSS (SEQ ID NO: 109), and GSTSGSGKPGSSEGSTKG (SEQ ID NO: 110) (Plückthun A, Pack P, Immunotechnology 3: 83-105 (1997); Atwell J et al., Protein Eng 12: 597-604 (1999); Wu A et al., Protein Eng 14: 1025-33 (2001); Yazaki P et al., J Immunol Methods 253: 195-208 (2001); Carmichael J et al., J Mol Biol 326: 341-51 (2003); Arndt M et al., FEBS Lett 578: 257-61 (2004); Bie C et al., World J Hepatol 2: 185-91 (2010)).

Suitable methods for linkage of components of the proteins of the present invention may be by any method presently known in the art for accomplishing such, as long as the attachment does not substantially impede the binding capability of the binding region, the cellular internalization of the protein, and/or desired toxin effector function(s) of the Shiga toxin effector region as measured by an appropriate assay, including assays as described herein.

### II. Examples of Specific Structural Variations of the Proteins of the Invention

Among certain embodiments of the present invention, the proteins of the invention comprise a binding region derived from an immunoglobulin-type polypeptide selected for specific and high-affinity binding to a surface antigen on the cell surface of a cancer cell, where the antigen is restricted in expression to cancer cells (*see* Glokler J et al., Molecules 15: 2478-90 (2010); Liu Y et al., Lab Chip 9: 1033-6 (2009)). In accordance with other embodiments, the binding region is selected for specific and high-affinity binding to a surface antigen on the cell surface of a cancer cell, where the antigen is over-expressed or preferentially expressed by cancer cells as compared to non-cancer cells. Some representative target biomolecules include, but are not limited to, the following enumerated targets associated with cancers and/or specific immune cell types.

Many immunoglobulin-type binding regions that recognize epitopes associated with cancer cells exist in the prior art, such as binding regions that target (alternative names are indicated in parentheses) annexin AI, B3 melanoma antigen, B4 melanoma antigen, CD2, CD3, CD4, CD20 (B-lymphocyte antigen protein CD20), CD22, CD25 (interleukin-2 receptor IL2R), CD30 (TNFRSF8), CD38 (cyclic ADP ribose hydrolase), CD40, CD44 (hyaluronan receptor), ITGAV (CD51), CD66, CD71 (transferrin receptor), CD73, CD74 (HLA-DR antigens-associated invariant chain), CD79, CD98, endoglin (END or CD105), CD106 (VCAM-1), chemokine receptor type 4 (CDCR-4, fusin, CD184), CD200, insulin-like growth factor 1 receptor (CD221), mucin1 (MUC1, CD227), basal cell adhesion molecule (B-CAM or CD239), CD248 (endosialin or TEM1), tumor necrosis factor receptor 10b (TNFRSF10B, CD262), tumor necrosis factor receptor 13B (TNFRSF13B, TACI, CD276), vascular endothelial growth factor receptor 2 (KDR, CD309), epithelial cell adhesion molecule (EpCAM, CD326), human epidermal growth factor receptor 2 (HER2/Neu/ErbB2/CD340), cancer antigen 15-3 (CA15-3), cancer antigen 19-9 (CA 19-9), cancer antigen 125 (CA125, MUC16), CA242, carcinoembryonic antigen-related cell adhesion molecules (*e.g*. CEACAM3 (CD66d) and CEACAM5), carcinoembryonic antigen protein (CEA), chondroitin sulfate proteoglycan 4 (CSP4, MCSP, NG2), CTLA4, DLL4, epidermal growth factor receptor (EGFR/ErbB1), folate receptor (FOLR), G-28, ganglioside GD2, ganglioside GD3, HLA-Dr10, HLA-DRB, human epidermal growth factor receptor 1 (HER1), Ephrin type-B receptor 2 (EphB2), epithelial cell adhesion molecule (EpCAM), fibroblast activation protein (FAP/seprase), insulin-like growth factor 1 receptor (IGF1R), interleukin 2 receptor (IL-2R), interleukin 6 receptor (IL-6R), integrins alpha-V beta-3 (α_{V}β₃), integrins alpha-V beta-5 (αvβ5), integrins alpha-5 beta-1 (α₅β₁), L6, MPG, melanoma-associated antigen 1 protein (MAGE-1), melanoma-associated antigen 3 (MAGE-3), mesothelin (MSLN), MPG, MS4A, p21, p97, polio virus receptor-like 4 (PVRL4), protease-activated-receptors (such as PARI), prostate-specific membrane antigen protein (PSMA), trophoblast glycoprotein (TPGB), and tumor-associated calcium signal transducers (TACSTDs) (*see e.g.* Lui B et al., Cancer Res 64: 704-10 (2004); Novellino L et al., Cancer Immunol Immunother 54: 187-207 (2005); Bagley R et al., Int J Oncol 34: 619-27 (2009); Gerber H et al., mAbs 1: 247-53 (2009); Beck A et al., Nat Rev Immunol 10: 345-52 (2010); Andersen J et al., J Biol Chem 287: 22927-37 (2012); Nolan-Stevaux O et al., PLoS One 7: e50920 (2012); Rust S et al., Mol Cancer 12: 11 (2013)). This list of target biomolecules is intended to be non-limiting. It will be appreciated by the skilled worker that any desired target biomolecule associated with a cancer cell or other desired cell type may be used to design or select a binding region to be coupled with the Shiga toxin effector region to produce a protein of the invention.

Examples of other target biomolecules which are strongly associated with cancer cells and immunoglobulin-type binding regions known to bind them include BAGE proteins (B melanoma antigens), basal cell adhesion molecules (BCAMs or Lutheran blood group glycoproteins), bladder tumor antigen (BTA), cancer-testis antigen NY-ESO-1, cancer-testis antigen LAGE proteins, CD19 (B-lymphocyte antigen protein CD19), CD21 (complement receptor-2 or complement 3d receptor), CD26 (dipeptidyl peptidase-4, DPP4, or adenosine deaminase complexing protein 2), CD33 (sialic acid-binding immunoglobulin-type lectin-3), CD52 (CAMPATH-1 antigen), CD56 (neural cell adhesion molecule orNCAM), CD133 (prominin-1), CS1 (SLAM family number 7 or SLAMF7), cell surface A33 antigen protein (gpA33), Epstein-Barr virus antigen proteins, GAGE/PAGE proteins (melanoma associated cancer/testis antigens), hepatocyte growth factor receptor (HGFR or c-Met), MAGE proteins, melanoma antigen recognized by T-cells 1 protein (MART-1/MelanA, MARTI), mucins, Preferentially Expressed Antigen of Melanoma (PRAME) proteins, prostate specific antigen protein (PSA), prostate stem cell antigen protein (PSCA), Receptor for Advanced Glycation Endroducts (RAGE), tumor-associated glycoprotein 72 (TAG-72), tyrosine-protein kinase transmembrane receptor (ROR1 or NTRKR1), vascular endothelial growth factor receptors (VEGFRs), and Wilms' tumor antigen.

Examples of other target biomolecules which are strongly associated with cancer cells are carbonic anhydrase IX (CA9/CAIX), claudin proteins (CLDN3, CLDN4), ephrin type-A receptor 3 (EphA3), folate binding proteins (FBP), ganglioside GM2, insulin-like growth factor receptors, integrins (such as CD11a-c), receptor activator of nuclear factor kappa B (RANK), receptor tyrosine-protein kinase erB-3, tumor necrosis factor receptor 10A (TRAIL-R1/DR4), tumor necrosis factor receptor 10B (TRAIL-R2), tenascin C, and CD64 (FcγRI) (*see* Hough C et al., Cancer Res 60: 6281-7 (2000); Thepen T et al., Nat Biotechnol 18: 48-51 (2000); Pastan I et al., Nat Rev Cancer 6: 559-65 (2006); Pastan, Annu Rev Med 58: 221-37 (2007); Fitzgerald D et al., Cancer Res 71: 6300-9 (2011); Scott A et al., Cancer Immun 12: 14-22 (2012)). This list of target biomolecules is intended to be non-limiting.

In addition, there are numerous other examples of contemplated, target biomolecules such as ADAM metalloproteinases (*e.g*. ADAM-9, ADAM-10, ADAM-12, ADAM-15, ADAM-17), ADP-ribosyltransferases (ART1, ART4), antigen F4/80, bone marrow stroma antigens (BST1, BST2), break point cluster region-c-abl oncogene (BCR-ABL) proteins, C3aR (complement component 3a receptors), CD7, CD13, CD14, CD15 (Lewis X or stage-specific embryonic antigen 1), CD23 (FC epsilon RII), CD49d, CD53, CD54 (intercellular adhesion molecule 1), CD63 (tetraspanin), CD69, CD80, CD86, CD88 (complement component 5a receptor 1), CD115 (colony stimulating factor 1 receptor), CD123 (interleukin-3 receptor), CD129 (interleukin 9 receptor), CD183 (chemokine receptor CXCR3), CD191 (CCR1), CD193 (CCR3), CD195 (chemokine receptor CCR5), CD203c, CD225 (interferon-induced transmembrane protein 1), CD244 (Natural Killer Cell Receptor 2B4), CD282 (toll-like receptor 2), CD284 (Toll-like receptor 4), CD294 (GPR44), CD305 (leukocyte-associated immunoglobulin-like receptor 1), ephrin type-A receptor 2 (EphA2), FceRIa, galectin-9, alpha-fetoprotein antigen 17-A1 protein, human aspartyl (asparaginyl) beta-hydroxylase (HAAH), immunoglobulin-like transcript ILT-3, lysophosphatidlglycerol acyltransferase 1 (LPGAT1/IAA0205), lysosome-associated membrane proteins (LAMPs, such as CD107), melanocyte protein PMEL (gp100), myeloid-related protein-14 (mrp-14), programmed death-ligand 1 (PD-L1), receptor tyrosine-protein kinase erbB-3, SART proteins, scavenger receptors (such as CD64 and CD68), Siglecs (sialic acid-binding immunoglobulin-type lectins), syndecans (such as SDC1 or CD138), tyrosinase, tyrosinase-related protein 1 (TRP-1), tyrosinase-related protein 2 (TRP-2), tyrosinase associated antigen (TAA), APO-3, BCMA, CD2, CD3, CD4, CD8, CD18, CD27, CD28, CD29, CD41, CD49, CD90, CD95 (Fas), CD103, CD104, CD134 (OX40), CD137 (4-1BB), CD152 (CTLA-4), chemokine receptors, complement proteins, cytokine receptors, histocompatibility proteins, ICOS, interferon-alpha, interferon-beta, c-myc, osteoprotegerin, PD-1, RANK, TACI, TNF receptor superfamily member (TNF-R1, TNFR-2), Apo2/TRAIL-R1, TRAIL-R2, TRAIL-R3, and TRAIL-R4 (*see,* Cheever M et al., Clin Cancer Res 15: 5323-37 (2009); Scott A et al., Cancer Immun 12: 14 (2012)), for target biomolecules and note the target molecules described therein are non-limiting examples). It will be appreciated by the skilled worker that any desired target biomolecule may be used to design or select a binding region to be coupled with the Shiga toxin effector region to produce a protein of the invention.

In certain embodiments, the binding region comprises or consists essentially of an immunoglobulin-type polypeptide selected for specific and high-affinity binding to the cellular surface of a cell type of the immune system. For example, immunoglobulin-type binding domains are known that bind to CD1, CD2, CD3, CD4, CD5, CD6, CD7, CD8, CD9, CD10, CD11, CD12, CD13, CD14, CD15, CD16, CD17, CD18, CD19, CD20, CD21, CD22, CD23, CD24, CD25, CD26, CD27, CD28, CD29, CD30, CD31, CD33, CD34, CD35, CD36, CD37, CD38, CD40, CD41, CD56, CD61, CD62, CD66, CD95, CD117, CD123, CD235, CD146, CD326, interleukin-2 receptor (IL-2R), receptor activator of nuclear factor kappa B ligand (RANKL, TNFSF11, TRANCE, OPGL, ODF), SLAM-associated protein (SAP), and TNFSF18 (tumor necrosis factor ligand 18 or GITRL).

In certain embodiments, the binding region binds with high-affinity to the target biomolecule which is a chemokine receptor selected from the following CXCR- 1, CXCR-2, CXCR-3 A, CXCR3B, CXCR-4, CXCR-5, CCR-I, CCR-2A, CCR-2B, CCR-3, CCR-4, CCR-5, CCR-6, CCR-7, CCR-8, CCR-9, CCR-10, CX3CR-1, XCR1, CXCR-6, CXCR-7, chemokine binding protein-2 (CCBP2, D6 receptor), and Duffy antigen/chemokine receptor (DARC, Fy glycoprotein, FY, CD234). For more non-limiting target biomolecules, see Table 11 in the Examples below.

In certain embodiments, the binding region comprises or consists essentially of ligand selected for targeting an extracellular receptor. Some representative ligands include, but are not limited to, the following: bone morphogenetic proteins and activin membrane-bound inhibitor BAMB1 (also known as TGFBR), chemokines, B cell-attracting chemokine 1 (BCA-I), breast and kidney-expressed chemokine (BRAK), ClO, chemokine C-C motif ligand 2 (CCL2), CD137L (also known as 4-1BB), cutaneous T-cell attracting chemokine (CTACK), decoy receptor 3 DcR3 (also known as TR6 and TNFRSF6B), epithelial-derived neutrophil-activating protein 78 (ENA-78), eosinophils chemotactic proteins (*e.g*. Eotaxin-1, Eotaxin-2, and Eotaxin-3), exodus-2 (SLC), fractalkine, granulocyte chemotactic protein 2 (GCP-2), growth regulated protein α (GRO-α), hemo filtrate CC chemokine 1 (HCC-I), interleukin-8 (IL-8), interferon-inducible protein 10 (IP-10), interferon-inducible T-cell alpha chemoattractant (I-TAC), liver-expressed chemokine (LEC), lungkine, lymphotactin, macrophage-derived chemokine (MDC), mucosae-associated epithelial chemokine (MEC), gamma interferon-induced monokine (MIG), macrophage inhibitory proteins (*e.g.* MIP-1α, MIP-1β, M1P-1γ, MIP-2, MIP-2α, MIP-2β, MIP-3, MIP-3β, MIP-3α, MIP-4, and MIP-5), monocytes chemotactic proteins (e.g. MCP-I or MCP-1, MCP-2, MCP-3, MCP-4, and MCP-5), platelet basic protein (PBP), platelet factor 4 (PF-4), Regulated on Activation, Normal T-cell Expressed and Secreted (RANTES) protein, stromal derived factors (*e.g.* SDF-1 and SDF-2), single C motif 1-β (SCM- 1 β), thymus and activation-regulated chemokine (TARC), thymus expressed chemokine (TECK), the tumor necrosis factor TWEAK (also known as TNFSF12 and APO3L), and allelic or species variants thereof. For more non-limiting exemplary ligands, see Table 11 in the Examples below.

Among certain embodiments, the proteins of the present invention comprise the Shiga toxin effector region comprising or consisting essentially of amino acids 75 to 251 of SLT-1A (SEQ ID NO: 1), StxA (SEQ ID NO:2), and/or SLT-2A (SEQ ID NO:3). Further embodiments are proteins in which the Shiga toxin effector region comprises or consists essentially of amino acids 1 to 241 of SLT-1A (SEQ ID NO:1), StxA (SEQ ID NO:2), and/or SLT-2A (SEQ ID NO:3). Further embodiments are proteins in which the Shiga toxin effector region comprises or consists essentially of amino acids 1 to 251 of SLT-1A (SEQ ID NO: 1), StxA (SEQ ID NO:2), and/or SLT-2A (SEQ ID NO:3). Further embodiments are proteins in which the Shiga toxin effector region comprises or consists essentially of amino acids 1 to 261 of SLT-1A (SEQ ID NO:1), StxA (SEQ ID NO:2), and/or SLT-2A (SEQ ID NO:3).

In certain embodiments, the proteins of the present invention comprise the immunoglobulin-type binding region comprising or consisting essentially of amino acids 2-245 of SEQ ID NO:4, which exhibits high affinity binding specifically to human HER2. Further embodiments are the proteins comprising any one of the polypeptides shown in SEQ ID NOs: 4-7.

In certain embodiments, the proteins of the present invention comprise the immunoglobulin-type binding region comprising or consisting essentially of amino acids 2-241 of SEQ ID NO:8, which exhibits high affinity binding specifically to human CD38. Further embodiments are the proteins comprising any one of the polypeptides shown in SEQ ID NOs: 8-11.

In certain embodiments, the proteins of the present invention comprise the immunoglobulin-type binding region comprising or consisting essentially of amino acids 2-250 of SEQ ID NO:12, which exhibits high affinity binding specifically to human CD19. Further embodiments are the proteins comprising any one of the polypeptides shown in SEQ ID NOs: 12-15.

In certain embodiments, the proteins of the present invention comprise the immunoglobulin-type binding region comprising or consisting essentially of amino acids 2-251 of SEQ ID NO:16, which exhibits high affinity binding specifically to human CD74. Further embodiments are the proteins comprising any one of the polypeptides shown in SEQ ID NOs: 16-19.

Among certain embodiments of the present invention, the binding region is a single domain immunoglobulin-derived region V_{H}H which exhibits high affinity binding specifically to HER2, such as derived from a single-domain variable region of the camelid antibody (V_{H}H) protein 5F7, as described in U.S. Patent Application Publication 2011/0059090. In certain further embodiments, the proteins of the present invention comprise the immunoglobulin-type binding region comprising or consisting essentially of amino acids 2-119 of SEQ ID NO:20, which exhibits high affinity binding specifically to human HER2. Further embodiments are the proteins comprising any one of the polypeptides shown in SEQ ID NOs: 20-27.

In certain embodiments, the proteins of the present invention comprise the binding region comprising or consisting essentially of amino acids 2-99 of SEQ ID NO:28, which exhibits high affinity binding specifically to human CD20. Further embodiments are the proteins comprising any one of the polypeptides shown in SEQ ID NOs: 28-29.

In certain embodiments, the proteins of the present invention comprise the binding region comprising or consisting essentially of amino acids 2-134 of SEQ ID NO:30, which exhibits high affinity binding specifically to human interleukin-2 receptor (IL-2R). Further embodiments are the proteins comprising any one of the polypeptides shown in SEQ ID NOs: 30-33.

As used herein, the term "heavy chain variable (V_{H}) domain" or "light chain variable (V_{L}) domain" respectively refer to any antibody V_{H} or V_{L} domain (*e.g.* a human V_{H} or V_{L} domain) as well as any derivative thereof retaining at least qualitative antigen binding ability of the corresponding native antibody (*e.g.* a humanized V_{H} or V_{L} domain derived from a native murine V_{H} or V_{L} domain). A V_{H} or V_{L} domain consists of a "framework" region interrupted by the three CDRs or ABRs. The framework regions serve to align the CDRs for specific binding to an epitope of an antigen. From amino-terminus to carboxyl-terminus, both V_{H} and V_{L} domains comprise the following framework (FR) and CDR regions: FR1, CDR1, FR2, CDR2, FR3, CDR3, and FR4. For camlied V_{H}H fragments, IgNARs of cartilaginous fish, V_{NAR} fragments, and derivatives thereof, there is a single heavy chain variable domain comprising the same basic arrangement: FR1, CDR1, FR2, CDR2, FR3, CDR3, and FR4.

The present disclosure provides fragments, variants, and/or derivatives of the polypeptides of the proteins described herein which contain a functional extracellular target biomolecule binding site, and even more preferably capable of binding the target biomolecule with high affinity (*e.g*. as shown by K_{D}). For example, while the invention provides polypeptide sequences that can specifically bind to CD19, CD20, CD38, CD74, and HER2, any binding region that binds to extracellular target biomolecule physically coupled to a cell, expressed on a cell surface, with a dissociation constant of 10⁻⁵ to 10⁻¹² moles per liter, preferably less than 200 nM, may be substituted for use in making proteins of the invention and methods described herein.

Among certain embodiments of the protein of the present invention, the immunoglobulin-type binding region is derived from a nanobody or single domain immunoglobulin-derived region V_{H}H. Generally, nanobodies are constructed from fragments of naturally occurring single, monomeric variable domain antibodies (sdAbs) of the sort found in camelids and cartilaginous fishes (Chondrichthyes). Nanobodies are engineered from these naturally occurring antibodies by truncating the single, monomeric variable domain to create a smaller and more stable molecule, such as, *e.g.* IgNAR, V_{H}H, and V_{NAR} constructs. Due to their small size, nanobodies are able to bind to antigens that are not accessible to whole antibodies. Among certain embodiments of the protein of the present invention, the immunoglobulin-type binding region is derived from a nanobody or single domain immunoglobulin-derived region V_{H}H which exhibits high affinity binding specifically to human HER2 proteins.

### III. General Functions of the Proteins of the Invention

The present invention provides a protein comprising 1) a binding region for cell targeting, as defined in the claims and 2) a Shiga toxin A Subunit effector region, as defined in the claims; and 3) a carboxy-terminal endoplasmic reticulum retention/ retrieval signal motif of a member of the KDEL family as defined in the claims; wherein the protein is linked to an additional exogenous material as defined in the claims. The linking of cell targeting binding regions with Shiga-toxin-Subunit-A-derived regions enables the engineering of cell-type specific targeting of the potent Shiga toxin cytotoxicity. In certain embodiments, the proteins of the invention are capable of binding extracellular target biomolecules associated with the cell surface of particular cell types and entering those cells. Once internalized within a targeted cell type, certain embodiments of the proteins of the invention are capable of routing a cytotoxic Shiga toxin effector polypeptide fragment into the cytosol of the target cell. Once in the cytosol of a targeted cell type, certain embodiments of the cytotoxic proteins of the invention are capable of enzymatically inactivating ribosomes, interfering with cell homeostasis, and eventually killing the cell. This system is modular, in that any number of diverse binding regions can be used to target this potent cytotoxicity or cytostasis to various, diverse cell types. The protein of the invention is used to deliver an additional exogenous material into target cells, such as a detection promoting agent to label the interiors of target cells for diagnostic information collection functions.

### A. Cell Kill via Targeted Shiga Toxin Cytotoxicity

Because members of the Shiga toxin family are adapted to killing eukaryotic cells, proteins designed using Shiga toxin effector regions can show potent cell-kill activity. The A Subunits of members of the Shiga toxin family comprise enzymatic domains capable of killing a eukaryotic cell once in the cell's cytosol. Certain embodiments of the cytotoxic proteins of the invention take advantage of this cytotoxic mechanism but must be capable of getting the Shiga toxin effector region to the cytosol of a targeted cell type. The addition of a KDEL type signal motif improves cytotoxicity of the cytotoxic proteins of the invention most likely by providing sufficient intracellular routing to promote delivery of a Shiga toxin effector polypeptide to the cytosol.

In certain embodiments of the cytotoxic proteins of the present invention, upon contacting a cell physically coupled with an extracellular target biomolecule of the binding region of a cytotoxic protein of the invention, the cytotoxic protein is capable of causing death of the cell. Cell kill may be accomplished using a cytotoxic protein of the invention under varied conditions of target cells, such as an *ex vivo* manipulated target cell, a target cell cultured *in vitro,* a target cell within a tissue sample cultured *in vitro,* or a target cell *in vivo.*

The expression of the target biomolecule need not be native in order for targeted cell killing by a cytotoxic protein of the invention. Cell surface expression of the target biomolecule could be the result of an infection, the presence of a pathogen, and/ or the presence of an intracellular microbial pathogen. Expression of a target biomolecule could be artificial such as, for example, by forced or induced expression after infection with a viral expression vector, *see e.g.* adenoviral, adeno-associated viral, and retroviral systems. An example of inducing expression of a target biomolecule is the upregulation of CD38 expression of cells exposed to retinoids, like all*-trans* retinoic acid and various synthetic retinoids, or any retinoic acid receptor (RAR) agonist (Drach J et al., Cancer Res 54: 1746-52 (1994); Uruno A et al., J Leukoc Biol 90: 235-47 (2011)). In another example, CD20, HER2, and EGFR expression may be induced by exposing a cell to ionizing radiation (Wattenberg M et al., Br J Cancer 110: 1472-80 (2014)).

### B. Selective Cytotoxicity among Cell Types

By targeting the delivery of enzymatically active Shiga toxin regions using high-affinity binding regions to specific cell types, this potent cell-kill activity can be restricted to preferentially killing selected cell types. The present invention provides various cytotoxic proteins with this functional ability.

In certain embodiments, administration of the cytotoxic protein of the present invention to a mixture of cell types, the cytotoxic protein is capable of selectively killing those cells which are physically coupled with a certain extracellular target biomolecule compared to cell types not physically coupled with any extracellular target biomolecule specifically bound by the binding region of that cytotoxic protein. Because members of the Shiga toxin family are adapted for killing eukaryotic cells, cytotoxic proteins designed using Shiga toxin effector regions can show potent cytotoxic activity. By targeting the delivery of enzymatically active Shiga toxin regions to specific cell types using high-affinity binding regions, this potent cell kill activity can be restricted to preferentially killing only those cell types desired to be targeted by their physical association with a target biomolecule specifically bound by chosen binding regions.

In certain embodiments, the cytotoxic protein of the present invention is capable of selectively or preferentially causing the death of a specific cell type within a mixture of two or more different cell types. This enables the targeting of cytotoxic activity to specific cell types with a high preferentiality, such as a 3-fold cytotoxic effect, over "bystander" cell types that do not express the target biomolecule. Alternatively, the expression of the target biomolecule of the binding region may be non-exclusive to one cell type if the target biomolecule is expressed in low enough amounts and/or physically coupled in low amounts with cell types that are not to be targeted. This enables the targeted cell-killing of specific cell types with a high preferentiality, such as a 3-fold cytotoxic effect, over "bystander" cell types that do not express significant amounts of the target biomolecule or are not physically coupled to significant amounts of the target biomolecule.

Levels of extracellular target biomolecules on the surface of cells may be determined using various methods known to the skilled worker, such as, *e.g.,* FACS methods. As used herein, a significant amount of an extracellular target biomolecule expressed at a cellular surface is greater than 10,000, 20,000, 30,000, 40,000, 50,000, 60,000, or 70,000 mean fluorescence intensity (MFI) by FACS analysis depending on the cell type.

In certain further embodiments,administration of the cytotoxic protein of the invention to two populations of cell types which differ with respect to the presence and/or polypeptide sequence of an extracellular target biomolecule, the cytotoxic protein is capable of causing cell death as defined by the half-maximal cytotoxic concentration (CD₅₀) on a population of target cells, whose members express an extracellular target biomolecule of the binding region of the cytotoxic protein, *e.g.,* at a dose at least three-times lower than the CD₅₀ dose of the same cytotoxic protein to a population of cells whose members do not express an extracellular target biomolecule of the binding region of the cytotoxic protein.

In certain embodiments, the cytotoxic activity of a protein of the present invention toward populations of cell types physically coupled with an extracellular target biomolecule is at least 3-fold higher than the cytotoxic activity toward populations of cell types not physically coupled with any extracellular target biomolecule bound specifically by that protein of the invention. Selective cytotoxicity may be quantified in terms of the ratio (a/b) of (a) cytotoxicity towards a population of cells of a specific cell type physically coupled with a target biomolecule of the binding region to (b) cytotoxicity towards a population of cells of a cell type not physically coupled with a target biomolecule of the binding region. In certain embodiments, the cytotoxicity ratio is indicative of selective cytotoxicity which is at least 3-fold, 5-fold, 10-fold, 15-fold, 20-fold, 25-fold, 30-fold, 40-fold, 50-fold, 75-fold, 100-fold, 250-fold, 500-fold, 750-fold, 1000-fold, or higher for populations of cells or cell types physically coupled with a target biomolecule of the binding region compared to populations of cells or cell types not physically coupled with a target biomolecule of the binding region.

This preferential cell-killing function allows a target cell to be killed by certain cytotoxic proteins of the invention under varied conditions and in the presence of non-targeted bystander cells, such as *ex vivo* manipulated mixtures of cell types, *in vitro* cultured tissues with mixtures of cell types, or *in vivo* in the presence of multiple cell types (*e.g. in situ* or in a native location within a multicellular organism).

In certain embodiments, the proteins of the present invention are capable of selectively or preferentially causing the death of a specific cell type within a mixture of two or more different cell types. This enables the targeted cytotoxic activity to specific cell types with a high preferentiality, such as a 3-fold cytotoxic effect, over "bystander" cell types that do not express extracellular target bound specifically by the binding region of that cytotoxic protein of the invention. Alternatively, the expression of an extracellular target biomolecule may be non-exclusive to one cell type if the extracellular target biomolecule is expressed in low enough amounts by cell types that are not to be targeted. This enables the preferential cell-killing of only those cell type(s) expressing the highest amounts of extracellular target biomolecules, such as a 3-fold cytotoxic effect, over "bystander" cell types that do not express significant amounts of the target biomolecule bound specifically by that cytotoxic protein and/or are not physically coupled to significant amounts of extracellularly exposed target biomolecule bound specifically by that cytotoxic protein.

The cytotoxic proteins of the present invention are useful for the elimination of populations of specific cell types. For example, the cytotoxic proteins of the invention are useful for the treatment of certain tumors, cancers, and/or other growth abnormalities by eliminating "target biomolecule+" cells that express elevated levels of target biomolecule at one or more cellular surfaces.

In certain embodiments, the cytotoxic activity of a protein of the present invention toward populations of cell types physically coupled with a certain extracellular target biomolecule is at least 3-fold higher than the cytotoxic activity toward populations of cell types not physically coupled with significant amounts of an extracellular target biomolecule bound specifically by the binding region of that particular protein of the invention. According to the present invention, selective cytotoxicity may be quantified in terms of the ratio (a/b) of (a) cytotoxicity towards a population of cells physically coupled with a significant amount of an extracellular target biomolecule bound by the binding region of the cytotoxic protein of the invention to (b) cytotoxicity towards a population of cells of a cell type not physically coupled with a significant amount of any extracellular target biomolecule bound specifically by the binding region of that particular cyotoxic protein of the invention. In certain embodiments, the cytotoxicity ratio is indicative of selective cytotoxicity which is at least 3-fold, 5-fold, 10-fold, 15-fold, 20-fold, 25-fold, 30-fold, 40-fold, 50-fold, 75-fold, 100-fold, 250-fold, 500-fold, 750-fold, 1000-fold or higher for populations of cells or cell types expressing an extracellular target biomolecule or physically coupled with an extracellular target biomolecule bound by the binding region of the cytotoxic protein of the invention compared to populations of cells or cell types which do not express an extracellular target biomolecule or that are not physically coupled with significant amounts of an extracellular target biomolecule bound specifically the binding region of that particular cytotoxic protein of the invention. For example, upon administration of certain cytotoxic proteins of the present invention to two different populations of cells which differ with respect to the presence and/or polypeptide sequence of an extracellular target biomolecule, the cytotoxic protein of the invention is capable of causing cell death of the cell-types physically coupled with an extracellular target biomolecule bound by the cytotoxic protein's binding region, *e.g.,* at a CD₅₀ that is at least three times less than the CD₅₀ observed for cell types that are not physically coupled with an extracellular target biomolecule bound by the cytotoxic protein's binding region or for cell types that are physically coupled only with forms of that extracellular target biomolecule which comprise sequence variations or mutations which disrupt binding specificity by the binding region of that cytotoxic protein.

In certain embodiments of the cytotoxic proteins of the present invention, administration of the cytotoxic protein to two different populations of cell types, the cytotoxic protein is capable of causing cell death as defined by the half-maximal cytotoxic concentration (CD₅₀) on a first cell population, whose members express a certain target biomolecule at a cellular surface, at a dose at least three-times lower than the CD₅₀ dose of the same cytotoxic protein to a second population of cells whose members do not express that target biomolecule, do not express a significant amount of that target biomolecule, or are not exposing a significant amount of that target biomolecule bound by the cytotoxic protein's binding region.

### C. Delivery of Additional Exogenous Material into the Interior of a Target Cell

The present invention provides a protein as defined in the claims that is linked to an additional exogenous material for delivery into the interiors of target cells, as defined in the claims. The delivery of additional exogenous materials may be used, *e.g.,* for cytotoxic, cytostatic, information gathering, and/or diagnostic functions. Non-toxic variants of the cytotoxic proteins of the invention, or optionally toxic variants, may be used to deliver additional exogenous materials to and/or label the interiors of cells physically coupled with an extracellular target biomolecule of the cytotoxic protein. Various types of cells and/or cell populations which express target biomolecules to at least one cellular surface may be targeted by the proteins of the invention for receiving exogenous materials. The functional components of the protein of the present invention are modular, in that various Shiga toxin effector regions and additional exogenous materials may be linked to various binding regions to provide diverse applications, such as non-invasive *in vivo* imaging of tumor cells.

Because the proteins of the present invention, whether toxic or nontoxic, and catalytically inactive forms thereof, are capable of entering cells physically coupled with an extracellular target biomolecule recognized by its binding region, the protein of the invention is used to deliver additional exogenous materials into the interior of targeted cell types. In one sense, the entire protein is an exogenous material which will enter the cell; thus, the "additional" exogenous materials are heterologous materials linked to but other than the core protein itself.

"Additional exogenous material" as used herein refers to one or more molecules, often not generally present within a native target cell, where the proteins of the present invention may be used to specifically transport such material to the interior of a cell. Non-limiting examples of additional exogenous materials are cytotoxic agents, peptides, polypeptides, proteins, polynucleotides, detection promoting agents, and small molecule chemotherapeutic agents.

In certain embodiments of the proteins of the present invention for delivery of additional exogenous material, the additional exogenous material is a cytotoxic agent, such as, *e.g.,* a small molecule chemotherapeutic agent, cytotoxic antibiotic, alkylating agent, antimetabolite, topoisomerase inhibitor, and/or tubulin inhibitor. Non-limiting examples of cytotoxic agents include aziridines, cisplatins, tetrazines, procarbazine, hexamethylmelamine, vinca alkaloids, taxanes, camptothecins, etoposide, doxorubicin, mitoxantrone, teniposide, novobiocin, aclarubicin, anthracyclines, actinomycin, bleomycin, plicamycin, mitomycin, daunorubicin, epirubicin, idarubicin, dolastatins, maytansines, docetaxel, adriamycin, calicheamicin, auristatins, pyrrolobenzodiazepine, carboplatin, 5-fluorouracil (5-FU), capecitabine, mitomycin C, paclitaxel, 1,3-Bis(2-chloroethyl)-1-nitrosourea (BCNU), rifampicin, cisplatin, methotrexate, and gemcitabine.

In certain embodiments, the additional exogenous material comprises a protein or polypeptide comprising an enzyme. In certain other embodiments, the additional exogenous material is a nucleic acid, such as, *e.g.* a ribonucleic acid that functions as a small inhibiting RNA (siRNA) or microRNA (miRNA). In certain embodiments, the additional exogenous material is an antigen, such as antigens derived from bacterial proteins, viral proteins, proteins mutated in cancer, proteins aberrantly expressed in cancer, or T-cell complementary determining regions. For example, exogenous materials include antigens, such as those characteristic of antigen-presenting cells infected by bacteria, and T-cell complementary determining regions capable of functioning as exogenous antigens.

### D. Information Gathering for Diagnostic Functions

Certain proteins of the invention have uses in the *in vitro* and/or *in vivo* detection of specific cells, cell types, and/or cell populations. In certain embodiments, the cytotoxic proteins described herein are used for both diagnosis and treatment, or for diagnosis alone. When the same cytotoxic protein is used for both diagnosis and treatment, the cytotoxic protein variant which incorporates a detection promoting agent for diagnosis may be rendered non-toxic by catalytic inactivation of a Shiga toxin effector region via one or more amino acid substitutions, including exemplary substitutions described herein. Catalytically inactive forms of the cytotoxic proteins of the invention that are conjugated to detection promoting agents optionally may be used for diagnostic functions, such as for companion diagnostics used in conjunction with a therapeutic regimen comprising the same or a related binding region.

The ability to conjugate detection promoting agents known in the art to various proteins of the invention provides useful compositions for the detection of cancer, tumor, immune, and infected cells. These diagnostic embodiments of the proteins of the present invention may be used for information gathering via various imaging techniques and assays known in the art. For example, diagnostic embodiments of the proteins of the invention may be used for information gathering via imaging of intracellular organelles (*e.g*. endocytotic, Golgi, endoplasmic reticulum, and cytosolic compartments) of individual cancer cells, immune cells, or infected cells in a patient or biopsy sample.

Various types of information may be gathered using the diagnostic embodiments of the proteins of the invention whether for diagnostic uses or other uses. This information may be useful, for example, in diagnosing neoplastic cell types, determining therapeutic susceptibilities of a patient's disease, assaying the progression of antineoplastic therapies over time, assaying the progression of immunomodulatory therapies over time, assaying the progression of antimicrobial therapies over time, evaluating the presence of infected cells in transplantation materials, evaluating the presence of unwanted cell types in transplantation materials, and/or evaluating the presence of residual tumor cells after surgical excision of a tumor mass.

For example, subpopulations of patients might be ascertained using information gathered using the diagnostic variants of the proteins of the present invention, and then individual patients could be categorized into subpopulations based on their unique characteristic(s) revealed using those diagnostic embodiments. For example, the effectiveness of specific pharmaceuticals or therapies might be one type of criterion used to define a patient subpopulation. For example, a non-toxic diagnostic variant of a particular cytotoxic protein of the invention may be used to differentiate which patients are in a class or subpopulation of patients predicted to respond positively to a cytotoxic variant of the same cytotoxic protein of the invention. Accordingly, the present disclosure relates to associated methods for patient identification, patient stratification and diagnosis using cytotoxic proteins described herein and/or their non-toxic variants.

### IV. Variations in the Polypeptide Sequences of the Proteins of the Invention which Maintain Overall Structure and Function

The skilled worker will recognize that variations may be made to the proteins of the present invention (and polynucleotides encoding them) without diminishing their biological activities, *e.g*. by maintaining the overall structure and function of the protein of the invention. For example, some modifications may facilitate expression, purification, pharmacokinetic properties, and/or immunogenicity. Such modifications are well known to the skilled worker and include, for example, a methionine added at the amino terminus to provide an initiation site, additional amino acids placed on either terminus to create conveniently located restriction sites or termination codons, and biochemical affinity tags fused to either terminus to provide for convenient detection and/or purification.

Also contemplated herein is the inclusion of additional amino acid residues at the amino and/or carboxy termini, such as sequences for epitope tags or other moieties. The additional amino acid residues may be used for various purposes including, *e.g.,* to facilitate cloning, expression, post-translational modification, synthesis, purification, detection, and/or administration. Non-limiting examples of epitope tags and moieties are: chitin binding protein domains, enteropeptidase cleavage sites, Factor Xa cleavage sites, FIAsH tags, FLAG tags, green fluorescent proteins (GFP), glutathione-S-transferase moieties, HA tags, maltose binding protein domains, myc tags, polyhistidine tags, ReAsH tags, strep-tags, strep-tag II, TEV protease sites, thioredoxin domains, thrombin cleavage site, and V5 epitope tags.

In certain of the above embodiments, the protein of the present invention is a variant in which there are one or more conservative amino acid substitutions introduced into the polypeptide region(s). As used herein, the term "conservative substitution" denotes that one or more amino acids are replaced by another, biologically similar amino acid residue. Examples include substitution of amino acid residues with similar characteristics, *e.g*. small amino acids, acidic amino acids, polar amino acids, basic amino acids, hydrophobic amino acids and aromatic amino acids (*see,* for example, Table B below). An example of a conservative substitution with a residue normally not found in endogenous, mammalian peptides and proteins is the conservative substitution of an arginine or lysine residue with, for example, ornithine, canavanine, aminoethylcysteine, or another basic amino acid. For further information concerning phenotypically silent substitutions in peptides and proteins (*see e.g.* Bowie J et al., Science 247: 1306-10 (1990)).

In the conservative substitution scheme in Table B below, exemplary conservative substitutions of amino acids are grouped by physicochemical properties - I: neutral, hydrophilic; II: acids and amides; III: basic; IV: hydrophobic; V: aromatic, bulky amino acids, VI hydrophilic uncharged, VII aliphatic uncharged, VIII non-polar uncharged, IX cycloalkenyl-associated, X hydrophobic, XI polar, XII small, XIII turn-permitting, and XIV flexible. For example, conservative amino acid substitutions include the following: 1) S may be substituted for C; 2) M or L may be substituted for F; 3) Y may be substituted for M; 4) Q or E may be substituted for K; 5) N or Q may be substituted for H; and 6) H may be substituted for N.

**TABLE B. Examples of Conservative Amino Acid Substitutions**

| **I** | **II** | **III** | **IV** | **V** | **VI** | **VII** | **VIII** | **IX** | **X** | **XI** | **XII** | **XIII** | **XIV** |
|---|---|---|---|---|---|---|---|---|---|---|---|---|---|
| A | D | H | C | F | N | A | C | F | A | C | A | A | D |
| G | E | K | I | W | Q | G | M | H | C | D | C | C | E |
| P | Q | R | L | Y | S | I | P | W | F | E | D | D | G |
| S | N | | M | | T | L | | Y | G | H | G | E | K |
| T | | | V | | | V | | | H | K | N | G | P |
| | | | | | | | | | I | N | P | H | Q |
| | | | | | | | | | L | Q | S | K | R |
| | | | | | | | | | M | R | T | N | S |
| | | | | | | | | | R | S | V | Q | T |
| | | | | | | | | | T | T | | R | |
| | | | | | | | | | V | | | S | |
| | | | | | | | | | W | | | P | |
| | | | | | | | | | Y | | | T | |

In certain embodiments, a protein of the present disclosure may comprise functional fragments or variants of a polypeptide region that have, at most, 20, 15, 10, 9, 8, 7, 6, 5, 4, 3, 2, or 1 amino acid substitution(s) compared to a polypeptide sequence recited herein, as long as the substituted polypeptide region retains measurable biological activity alone or as a component of a protein described herein. Variants of proteins of the invention as defined in the claims are within the scope of the invention as a result of changing a polypeptide of the protein of the invention by altering one or more amino acids or deleting or inserting one or more amino acids, such as within the binding region or the Shiga toxin effector region, in order to achieve desired properties, such as changed cytotoxicity, changed cytostatic effects, changed immunogenicity, and/or changed serum half-life. A polypeptide of a protein of the invention may further be with or without a signal sequence.

In certain embodiments, a protein of the present invention shares at least 85%, 90%, 95%, 96%, 97%, 98%, 99% or more amino acid sequence identity to any one of the amino acid sequences of a protein recited herein, as long as it retains measurable biological activity, such as cytotoxicity, extracellular target biomolecule binding, enzymatic catalysis, or subcellular routing. The immunoglobulin-type binding region may differ from the amino acid sequences of a protein recited herein, as long as it retains binding functionality to its extracellular target biomolecule. Binding functionality will most likely be retained if the amino acid sequences of the CDRs or ABRs are identical. For example, a protein is within the claim scope that comprises or consists essentially of 85% amino acid identity to a protein recited herein which for the purposes of determining the degree of amino acid identity, the amino acid residues that form the CDRs or ABRs are disregarded. Binding functionality can be determined by the skilled worker using standard techniques.

In certain embodiments, the Shiga toxin effector region may be altered to change its enzymatic activity and/or cytotoxicity as long as the Shiga toxin effector region retains one or more other Shiga toxin effector functions. This change may or may not result in a change in the cytotoxicity of a protein of which the altered Shiga toxin effector region is a component. Possible alterations include mutations to the Shiga toxin effector region selected from the group consisting of: a truncation, deletion, inversion, insertion, rearrangement, and substitution.

The cytotoxicity of the A Subunits of members of the Shiga toxin family may be altered, reduced, or eliminated by mutation or truncation. The positions labeled tyrosine-77, glutamate-167, arginine-170, tyrosine-114, and tryptophan-203 have been shown to be important for the catalytic activity of Stx, Stx1, and Stx2 (Hovde C et al., Proc Natl Acad Sci USA 85: 2568-72 (1988); Deresiewicz R et al., Biochemistry 31: 3272-80 (1992); Deresiewicz R et al., Mol Gen Genet 241: 467-73 (1993); Ohmura M et al., Microb Pathog 15: 169-76 (1993); Cao C et al., Microbiol Immunol 38: 441-7 (1994); Suhan M, Hovde C, Infect Immun 66: 5252-9 (1998)). Mutating both glutamate-167 and arginine-170 eliminated the enzymatic activity of Slt-I A1 in a cell-free ribosome inactivation assay (LaPointe, J Biol Chem 280: 23310-18 (2005)). In another approach using *de novo* expression of Slt-I A1 in the endoplasmic reticulum, mutating both glutamate-167 and arginine-170 eliminated Slt-I A1 fragment cytotoxicity at that expression level (LaPointe, J Biol Chem 280: 23310-18 (2005)). A truncation analysis demonstrated that a fragment of StxA from residues 75 to 268 still retains significant enzymatic activity *in vitro* (Haddad, J Bacteriol 175: 4970-8 (1993)). A truncated fragment of Slt-I A1 containing residues 1-239 displayed significant enzymatic activity *in vitro* and cytotoxicity by *de novo* expression in the cytosol (LaPointe, J Biol Chem 280: 23310-18 (2005)). Expression of a Slt-I A1 fragment truncated to residues 1-239 in the endoplasmic reticulum was not cytotoxic because it could not retrotranslocate to the cytosol (LaPointe, J Biol Chem 280: 23310-18 (2005)).

The most critical residues for enzymatic activity and/or cytotoxicity in the Shiga toxin A Subunits have been mapped to the following residue-positions: aspargine-75, tyrosine-77, glutamate-167, arginine-170, and arginine-176 among others (Di, Toxicon 57: 525-39 (2011)). In particular, a double-mutant construct of Stx2A containing glutamate-E167-to-lysine and arginine-176-to-lysine mutations was completely inactivated; whereas, many single mutations in Stx1 and Stx2 showed a 10-fold reduction in cytotoxicity. Further, truncation of Stx1A to 1-239 or 1-240 reduced its cytotoxicity, and similarly, truncation of Stx2A to a conserved hydrophobic residue reduced its cytotoxicity.

The most critical residues for binding eukaryotic ribosomes and/or eukaryotic ribosome inhibition in the Shiga toxin A Subunit have been mapped to the following residue-positions arginine-172, arginine-176, arginine-179, arginine-188, tyrosine-189, valine-191, and leucine-233 among others (McCluskey A et al., PLoS One 7: e31191 (2012)).

Shiga-like toxin 1 A Subunit truncations are catalytically active, capable of enzymatically inactivating ribosomes *in vitro,* and cytotoxic when expressed within a cell (LaPointe, J Biol Chem 280: 23310-18 (2005)). The smallest Shiga toxin A Subunit fragment exhibiting full enzymatic activity is a polypeptide composed of residues 1-239 of Slt1A (LaPointe, J Biol Chem 280: 23310-18 (2005)). Although the smallest fragment of the Shiga toxin A Subunit reported to retain substantial catalytic activity was residues 75-247 of StxA (Al-Jaufy, Infect Immun 62: 956-60 (1994)), a StxA truncation expressed *de novo* within a eukaryotic cell requires only up to residue 240 to reach the cytosol and exert catalytic inactivation of ribosomes (LaPointe, J Biol Chem 280: 23310-18 (2005)).

In certain embodiments derived from SLT-1A (SEQ ID NO:1) or StxA (SEQ ID NO:2), these changes include substitution of the asparagine at position 75, tyrosine at position 77, tyrosine at position 114, glutamate at position 167, arginine at position 170, arginine at position 176, and/or substitution of the tryptophan at position 203. Examples of such substitutions will be known to the skilled worker based on the prior art, such as asparagine at position 75 to alanine, tyrosine at position 77 to serine, substitution of the tyrosine at position 114 to serine, substitution of the glutamate at position 167 to aspartate, substitution of the arginine at position 170 to alanine, substitution of the arginine at position 176 to lysine, and/or substitution of the tryptophan at position 203 to alanine.

Proteins of the present invention may optionally be conjugated to one or more additional agents, such as therapeutic and/or diagnostic agents known in the art, including such agents as described herein.

### V. Production, Manufacture, and Purification of a Protein of the Invention

The proteins of the present invention may be produced using biochemical engineering techniques well known to those of skill in the art. For example, proteins of the invention may be manufactured by standard synthetic methods, by use of recombinant expression systems, or by any other suitable method. The proteins of the invention may be produced as fusion proteins, chemically coupled conjugates, and/or combinations thereof, such as, *e.g.,* a fusion protein component covalently coupled to one or more components. Thus, the proteins of the invention may be synthesized in a number of ways, including, *e.g*. methods comprising: (1) synthesizing a polypeptide or polypeptide component of a protein of the invention using standard solid-phase or liquid-phase methodology, either stepwise or by fragment assembly, and isolating and purifying the final peptide compound product; (2) expressing a polynucleotide that encodes a polypeptide or polypeptide component of a protein of the invention in a host cell and recovering the expression product from the host cell or host cell culture; or (3) cell-free *in vitro* expression of a polynucleotide encoding a polypeptide or polypeptide component of a protein of the invention, and recovering the expression product; or by any combination of the methods of (1), (2) or (3) to obtain fragments of the peptide component, subsequently joining (*e.g.* ligating) the fragments to obtain the peptide component, and recovering the peptide component.

It may be preferable to synthesize a polypeptide or polypeptide component of a protein of the present invention by means of solid-phase or liquid-phase peptide synthesis. Proteins of the invention may suitably be manufactured by standard synthetic methods. Thus, peptides may be synthesized by, *e.g*. methods comprising synthesizing the peptide by standard solid-phase or liquid-phase methodology, either stepwise or by fragment assembly, and isolating and purifying the final peptide product. In this context, reference may be made to WO 1998/11125 or, *inter alia,* Fields G et al., Principles and Practice of Solid-Phase Peptide Synthesis (Synthetic Peptides, Grant G, ed., Oxford University Press, U.K., 2nd ed., 2002) and the synthesis examples therein.

Proteins of the present invention may be prepared (produced and purified) using recombinant techniques well known in the art. In general, methods for preparing polypeptides by culturing host cells transformed or transfected with a vector comprising the encoding polynucleotide and recovering the polypeptide from cell culture are described in, *e.g.* Sambrook J et al., Molecular Cloning: A Laboratory Manual (Cold Spring Harbor Laboratory Press, NY, U.S., 1989); Dieffenbach C et al., PCR Primer: A Laboratory Manual (Cold Spring Harbor Laboratory Press, N.Y., U.S., 1995). Any suitable host cell may be used to produce a protein of the invention. Host cells may be cells stably or transiently transfected, transformed, transduced or infected with one or more expression vectors which drive expression of a polypeptide of the invention. In addition, a protein of the invention may be produced by modifying the polynucleotide encoding the protein of the invention that result in altering one or more amino acids or deleting or inserting one or more amino acids in order to achieve desired properties, such as changed cytotoxicity, changed cytostatic effects, changed immunogenicity, and/or changed serum half-life.

There are a wide variety of expression systems which may be chosen to produce a protein of the present invention. For example, host organisms for expression of proteins of the invention include prokaryotes, such as *E. coli* and *B. subtilis,* eukaryotic cells, such as yeast and filamentous fungi (like *S*. *cerevisiae, P. pastoris, A. awamori,* and *K. lactis*)*,* algae (like *C*. *reinhardtii*)*,* insect cell lines, mammalian cells (like CHO cells), plant cell lines, and eukaryotic organisms such as transgenic plants (like *A*. *thaliana* and *N. benthamiana*)*.*

Accordingly, the present disclosure also provides methods for producing a protein described herein according to above recited methods and using (i) a polynucleotide encoding part or all of a protein described herein or a polypeptide component thereof, (ii) an expression vector comprising at least one polynucleotide described herein capable of encoding part or all of a protein described herein or a polypeptide component thereof when introduced into a suitable host cell or cell-free expression system, and/or (iii) a host cell comprising a polynucleotide or expression vector described herein.

When a polypeptide or protein is expressed using recombinant techniques in a host cell or cell-free system, it is advantageous to separate (or purify) the desired polypeptide or protein away from other components, such as host cell factors, in order to obtain preparations that are of high purity or are substantially homogeneous. Purification can be accomplished by methods well known in the art, such as centrifugation techniques, extraction techniques, chromatographic and fractionation techniques (*e.g*. size separation by gel filtration, charge separation by ion-exchange column, hydrophobic interaction chromatography, reverse phase chromatography, chromatography on silica or cation-exchange resins such as DEAE and the like, chromatofocusing, and Protein A Sepharose chromatography to remove contaminants), and precipitation techniques (*e.g.* ethanol precipitation or ammonium sulfate precipitation). Any number of biochemical purification techniques may be used to increase the purity of a protein of the invention. In certain embodiments, the proteins of the invention may optionally be purified in homo-multimeric forms (*i.e.* a protein complex of two or more identical proteins) or in hetero-multimeric forms (*i.e.* a protein complex of two or more non-identical proteins).

In the Examples below are descriptions of non-limiting examples of methods that are suitable for producing a protein of the present invention.

### VI. Pharmaceutical and Diagnostic Compositions Comprising a Protein of the Invention

The protein of the invention may be used, alone or in combination with one or more additional therapeutic agents, in a pharmaceutical composition, for treatment or prophylaxis of conditions, diseases, disorders, or symptoms described in further detail below (*e.g.* cancers, malignant tumors, non-malignant tumors, growth abnormalities, immune disorders, and microbial infections). The present invention further provides a pharmaceutical composition comprising a protein of the invention, or a pharmaceutically acceptable salt or solvate thereof, according to the invention, together with at least one pharmaceutically acceptable carrier or excipient, as defined in the claims. In certain embodiments, the pharmaceutical composition of the invention may comprise homo-multimeric and/or hetero-multimeric forms of the proteins of the invention. The pharmaceutical compositions will be useful in methods of treating, ameliorating, or preventing a disease, condition, disorder, or symptom described in further detail below. Each such disease, condition, disorder, or symptom is envisioned to be a separate embodiment with respect to uses of a pharmaceutical composition according to the invention. The disclosure further provides pharmaceutical compositions for use in at least one method of treatment, as described in more detail below.

As used herein, the terms "patient" and "subject" are used interchangeably to refer to any organism, commonly vertebrates such as humans and animals, which presents symptoms, signs, and/or indications of at least one disease, disorder, or condition. These terms include mammals such as the non-limiting examples of primates, livestock animals (*e.g.* cattle, horses, pigs, sheep, goats, *etc.*)*,* companion animals (*e.g.* cats, dogs, *etc.*) and laboratory animals (*e.g.* mice, rabbits, rats, *etc.*)*.*

As used herein, "treat," "treating," or "treatment" and grammatical variants thereof refer to an approach for obtaining beneficial or desired clinical results. The terms may refer to slowing the onset or rate of development of a condition, disorder or disease, reducing or alleviating symptoms associated with it, generating a complete or partial regression of the condition, or some combination of any of the above. For the purposes of this invention, beneficial or desired clinical results include, but are not limited to, reduction or alleviation of symptoms, diminishment of extent of disease, stabilization (*e.g*. not worsening) of state of disease, delay or slowing of disease progression, amelioration or palliation of the disease state, and remission (whether partial or total), whether detectable or undetectable. "Treat," "treating," or "treatment" can also mean prolonging survival relative to expected survival time if not receiving treatment. A subject (*e.g.* a human) in need of treatment may thus be a subject already afflicted with the disease or disorder in question. The terms "treat," "treating," or "treatment" includes inhibition or reduction of an increase in severity of a pathological state or symptoms relative to the absence of treatment, and is not necessarily meant to imply complete cessation of the relevant disease, disorder, or condition. With regard to tumors and/or cancers, treatment includes reductions in overall tumor burden and/or individual tumor size.

As used herein, the terms "prevent," "preventing," "prevention" and grammatical variants thereof refer to an approach for preventing the development of, or altering the pathology of, a condition, disease, or disorder. Accordingly, "prevention" may refer to prophylactic or preventive measures. For the purposes of this invention, beneficial or desired clinical results include, but are not limited to, prevention or slowing of symptoms, progression or development of a disease, whether detectable or undetectable. A subject (*e.g.* a human) in need of prevention may thus be a subject not yet afflicted with the disease or disorder in question. The term "prevention" includes slowing the onset of disease relative to the absence of treatment, and is not necessarily meant to imply permanent prevention of the relevant disease, disorder or condition. Thus "preventing" or "prevention" of a condition may in certain contexts refer to reducing the risk of developing the condition, or preventing or delaying the development of symptoms associated with the condition.

As used herein, an "effective amount" or "therapeutically effective amount" is an amount or dose of a composition (*e.g.* a therapeutic composition or agent) that produces at least one desired therapeutic effect in a subject, such as preventing or treating a target condition or beneficially alleviating a symptom associated with the condition. The most desirable therapeutically effective amount is an amount that will produce a desired efficacy of a particular treatment selected by one of skill in the art for a given subject in need thereof. This amount will vary depending upon a variety of factors understood by the skilled worker, including but not limited to the characteristics of the therapeutic compound (including activity, pharmacokinetics, pharmacodynamics, and bioavailability), the physiological condition of the subject (including age, sex, disease type, disease stage, general physical condition, responsiveness to a given dosage, and type of medication), the nature of the pharmaceutically acceptable carrier or carriers in the formulation, and the route of administration. One skilled in the clinical and pharmacological arts will be able to determine a therapeutically effective amount through routine experimentation, namely by monitoring a subject's response to administration of a compound and adjusting the dosage accordingly (*see e.g.* Remington: The Science and Practice of Pharmacy (Gennaro A, ed., Mack Publishing Co., Easton, PA, U.S., 19th ed., 1995)).

Diagnostic compositions of the present invention comprise a protein of the invention and one or more detection promoting agents, as defined in the claims. Various detection promoting agents are known in the art, such as isotopes, dyes, colorimetric agents, contrast enhancing agents, fluorescent agents, bioluminescent agents, and magnetic agents. These agents may be incorporated into the protein of the invention at any position. The incorporation of the agent may be via an amino acid residue(s) of the protein of the invention or via some type of linkage known in the art, including via linkers and/or chelators. The incorporation of the agent is in such a way to enable the detection of the presence of the diagnostic composition in a screen, assay, diagnostic procedure, and/or imaging technique.

When producing or manufacturing a diagnostic composition of the present invention, a protein of the invention may be directly or indirectly linked to one or more detection promoting agents. There are numerous detection promoting agents known to the skilled worker which can be operably linked to the proteins of the present invention for information gathering methods, such as for diagnostic and/or prognostic applications to diseases, disorders, or conditions of an organism (*see e.g.* Cai W et al., J Nucl Med 48: 304-10 (2007); Nayak T, Brechbiel M, Bioconjug Chem 20: 825-41 (2009); Paudyal P et al., Oncol Rep 22: 115-9 (2009); Qiao J et al., PLoS ONE 6: e18103 (2011); Sano K et al., Breast Cancer Res 14: R61 (2012)). For example, detection promoting agents include image enhancing contrast agents, such as fluorescent dyes (*e.g*. Alexa680, indocyanine green, and Cy5.5), isotopes and radionuclides, such as ¹¹C, ¹³N, ¹⁵O, ¹⁸F, ³²P, ⁵¹Mn, ⁵²mMn, ⁵²Fe, ⁵⁵Co, ⁶²Cu, ⁶⁴Cu, ⁶⁷Cu, ⁶⁷Ga, ⁶⁸Ga, ⁷²As, ⁷³Se, ⁷⁵Br, ⁷⁶Br, ⁸²mRb, ⁸³Sr, ⁸⁶Y, ⁹⁰Y, ⁸⁹Zr, ⁹⁴mTc, ⁹⁴Tc, ⁹⁹mTc, ¹¹⁰In, ¹¹¹In, ¹²⁰I, ¹²³I, ¹²⁴I, ¹²⁵I, ¹³¹I, ¹⁵⁴Gd, ¹⁵⁵Gd, ¹⁵⁶Gd, ¹⁵⁷Gd, ¹⁵⁸Gd, ¹⁷⁷Lu, ¹⁸⁶Re, ¹⁸⁸Re, and ²²³R, paramagnetic ions, such as chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) or erbium (III), metals, such as lanthanum (III), gold (III), lead (II), and bismuth (III), ultrasound contrast enhancing agents, such as liposomes, radiopaque agents, such as barium, gallium, and thallium compounds. Detection promoting agents may be incorporated directly or indirectly by using an intermediary functional group, such as chelators like 2-benzyl DTPA, PAMAM, NOTA, DOTA, TETA, analogs thereof, and functional equivalents of any of the foregoing (*see* Leyton J et al., Clin Cancer Res 14: 7488-96 (2008)).
There are numerous standard techniques known to the skilled worker for incorporating, affixing, and/or conjugating various detection promoting agents to proteins, especially to immunoglobulins and immunoglobulin-derived domains (Wu A, Methods 65: 139-47 (2014)). Similarly, there are numerous imaging approaches known to the skilled worker, such as non-invasive *in vivo* imaging techniques commonly used in the medical arena, for example: computed tomography imaging (CT scanning), optical imaging (including direct, fluorescent, and bioluminescent imaging), magnetic resonance imaging (MRI), positron emission tomography (PET), single-photon emission computed tomography (SPECT) ultrasound, and x-ray computed tomography imaging (*see* Kaur S et al., Cancer Lett 315: 97-111 (2012), *for review*)*.*

### Production or Manufacture of a Pharmaceutical and/or Diagnostic Composition Comprising a Protein of the Invention

Pharmaceutically acceptable salts or solvates of any of the proteins of the present invention are likewise within the scope of the present invention, as defined in the claims.

The term "solvate" in the context of the present invention refers to a complex of defined stoichiometry formed between a solute (*in casu,* a polypeptide compound or pharmaceutically acceptable salt thereof according to the invention) and a solvent. The solvent in this connection may, for example, be water, ethanol or another pharmaceutically acceptable, typically small-molecular organic species, such as, but not limited to, acetic acid or lactic acid. When the solvent in question is water, such a solvate is normally referred to as a hydrate.

Proteins of the present invention, or salts thereof, may be formulated as pharmaceutical compositions prepared for storage or administration, which typically comprise a therapeutically effective amount of a protein of the invention, or a salt thereof, in a pharmaceutically acceptable carrier. The term "pharmaceutically acceptable carrier" includes any of the standard pharmaceutical carriers. Pharmaceutically acceptable carriers for therapeutic use are well known in the pharmaceutical art, and are described, for example, in Remington's Pharmaceutical Sciences (Mack Publishing Co. (A. Gennaro, ed., 1985). As used herein, "pharmaceutically acceptable carrier" includes any and all physiologically acceptable, *i.e.* compatible, solvents, dispersion media, coatings, antimicrobial agents, isotonic, and absorption delaying agents, and the like. Pharmaceutically acceptable carriers or diluents include those used in formulations suitable for oral, rectal, nasal or parenteral (including subcutaneous, intramuscular, intravenous, intradermal, and transdermal) administration. Exemplary pharmaceutically acceptable carriers include sterile aqueous solutions or dispersions and sterile powders for the extemporaneous preparation of sterile injectable solutions or dispersions. Examples of suitable aqueous and nonaqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyloleate. Proper fluidity can be maintained, for example, by the use of coating materials, such as lecithin, by the maintenance of the required particle size in the case of dispersions, and by the use of surfactants. In certain embodiments, the carrier is suitable for intravenous, intramuscular, subcutaneous, parenteral, spinal or epidermal administration (*e.g*. by injection or infusion). Depending on selected route of administration, the protein of the invention or other pharmaceutical component may be coated in a material intended to protect the compound from the action of low pH and other natural inactivating conditions to which the active protein of the invention may encounter when administered to a patient by a particular route of administration.

The formulations of the pharmaceutical compositions of the present invention may conveniently be presented in unit dosage form and may be prepared by any of the methods well known in the art of pharmacy. In such form, the composition is divided into unit doses containing appropriate quantities of the active component. The unit dosage form can be a packaged preparation, the package containing discrete quantities of the preparations, for example, packeted tablets, capsules, and powders in vials or ampoules. The unit dosage form can also be a capsule, cachet, or tablet itself, or it can be the appropriate number of any of these packaged forms. It may be provided in single dose injectable form, for example in the form of a pen. Compositions may be formulated for any suitable route and means of administration. Subcutaneous or transdermal modes of administration may be particularly suitable for therapeutic proteins described herein.

The pharmaceutical compositions of the present invention may also contain adjuvants such as preservatives, wetting agents, emulsifying agents and dispersing agents. Prevention of the presence of microorganisms may be ensured both by sterilization procedures, and by the inclusion of various antibacterial and antifungal agents, for example, paraben, chlorobutanol, phenol sorbic acid, and the like. Isotonic agents, such as sugars, sodium chloride, and the like into the compositions, may also be desirable. In addition, prolonged absorption of the injectable pharmaceutical form may be brought about by the inclusion of agents which delay absorption such as, aluminum monostearate and gelatin.

A pharmaceutical composition of the present invention also optionally includes a pharmaceutically acceptable antioxidant. Exemplary pharmaceutically acceptable antioxidants are water soluble antioxidants such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propylgallate, alpha-tocopherol, and the like; and metal chelating agents, such as citric acid, ethylenediamine tetraacetic acid (EDTA), sorbitol, tartaric acid, phosphoric acid, and the like.

In another aspect, the present disclosure provides pharmaceutical compositions comprising one or a combination of different proteins described herein, or an ester, salt or amide of any of the foregoing, and at least one pharmaceutically acceptable carrier.

Therapeutic compositions are typically sterile and stable under the conditions of manufacture and storage. The composition may be formulated as a solution, microemulsion, liposome, or other ordered structure suitable to high drug concentration. The carrier may be a solvent or dispersion medium containing, for example, water, alcohol such as ethanol, polyol (*e.g.* glycerol, propylene glycol, and liquid polyethylene glycol), or any suitable mixtures. The proper fluidity may be maintained, for example, by the use of a coating such as lecithin, by the maintenance of the required particle size in the case of dispersion and by use of surfactants according to formulation chemistry well known in the art. In certain embodiments, isotonic agents, *e.g*. sugars, polyalcohols such as mannitol, sorbitol, or sodium chloride may be desirable in the composition. Prolonged absorption of injectable compositions may be brought about by including in the composition an agent that delays absorption for example, monostearate salts and gelatin.

Solutions or suspensions used for intradermal or subcutaneous application typically include one or more of: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates; and tonicity adjusting agents such as, *e.g.,* sodium chloride or dextrose. The pH can be adjusted with acids or bases, such as hydrochloric acid or sodium hydroxide, or buffers with citrate, phosphate, acetate and the like. Such preparations may be enclosed in ampoules, disposable syringes or multiple dose vials made of glass or plastic.

Sterile injectable solutions may be prepared by incorporating a protein of the invention in the required amount in an appropriate solvent with one or a combination of ingredients described above, as required, followed by sterilization microfiltration. Dispersions may be prepared by incorporating the active compound into a sterile vehicle that contains a dispersion medium and other ingredients, such as those described above. In the case of sterile powders for the preparation of sterile injectable solutions, the methods of preparation are vacuum drying and freeze-drying (lyophilization) that yield a powder of the active ingredient in addition to any additional desired ingredient from a sterile-filtered solution thereof.

When a therapeutically effective amount of a protein of the invention is designed to be administered by, *e.g*. intravenous, cutaneous or subcutaneous injection, the binding agent will be in the form of a pyrogen-free, parenterally acceptable aqueous solution. Methods for preparing parenterally acceptable protein solutions, taking into consideration appropriate pH, isotonicity, stability, and the like, are within the skill in the art. A preferred pharmaceutical composition for intravenous, cutaneous, or subcutaneous injection will contain, in addition to binding agents, an isotonic vehicle such as sodium chloride injection, Ringer's injection, dextrose injection, dextrose and sodium chloride injection, lactated Ringer's injection, or other vehicle as known in the art. A pharmaceutical composition of the present invention may also contain stabilizers, preservatives, buffers, antioxidants, or other additives well known to those of skill in the art.

As described elsewhere herein, a protein of the present invention or composition thereof (*e.g.* pharmaceutical or diagnostic composition) may be prepared with carriers that will protect the composition against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art (*see e.g.* Sustained and Controlled Release Drug Delivery Systems (Robinson J, ed., Marcel Dekker, Inc., NY, U.S., 1978)).

In certain embodiments, the composition of the present invention (*e.g*. pharmaceutical or diagnostic composition) may be formulated to ensure a desired distribution *in vivo.* For example, the blood-brain barrier excludes many large and/or hydrophilic compounds. To target a therapeutic protein or composition of the invention to a particular *in vivo* location, it can be formulated, for example, in liposomes which may comprise one or more moieties that are selectively transported into specific cells or organs, thus enhancing targeted drug delivery. Exemplary targeting moieties include folate or biotin; mannosides; antibodies; surfactant protein A receptor; p120 catenin and the like.

Pharmaceutical compositions include parenteral formulations designed to be used as implants or particulate systems. Examples of implants are depot formulations composed of polymeric or hydrophobic components such as emulsions, ion exchange resins, and soluble salt solutions. Examples of particulate systems are dendrimers, liposomes, microspheres, microparticles, nanocapsules, nanoparticles, nanorods, nanospheres, polymeric micelles, and nanotubes (*see e.g.* Honda M et al., Int J Nanomedicine 8: 495-503 (2013); Sharma A et al., Biomed Res Int 2013: 960821 (2013); Ramishetti S, Huang L, Ther Deliv 3: 1429-45 (2012)). Controlled release formulations may be prepared using polymers sensitive to ions, such as, *e.g.* liposomes, polaxamer 407, and hydroxyapatite. Particulate and polymer formulations may comprise a plasma membrane permeability altering agent(s), such as, *e.g.,* various peptides and proteins like cytolysins, toxin-derived agents, virus derived agents, synthetic biomimetic peptides, and chemical agents (*see e.g.* Varkouhi et al., J Control Release 151: 220-8 (2011); J Pirie C et al., Mol Cancer Ther 12: 1774-82 (2013)).

### VII. Polynucleotides, Expression Vectors, and Host Cells

The disclosure further provides polynucleotides which encode proteins described herein, or functional portions thereof. The term "polynucleotide" is equivalent to the term "nucleic acids" both of which include polymers of deoxyribonucleic acids (DNAs), polymers of ribonucleic acids (RNAs), analogs of these DNAs or RNAs generated using nucleotide analogs, and derivatives, fragments and homologs thereof. The polynucleotide described herein may be single-, double-, or triple-stranded. Disclosed polynucleotides are specifically disclosed to include all polynucleotides capable of encoding an exemplary protein described herein, for example, taking into account the wobble known to be tolerated in the third position of RNA codons, yet encoding for the same amino acid as a different RNA codon (*see* Stothard P, Biotechniques 28: 1102-4 (2000)).

The discosure provides polynucleotides which encode a protein described herein, or a fragment or derivative thereof. The polynucleotides may include, *e.g.,* nucleic acid sequence encoding a polypeptide at least 50%, 55%, 60%, 65%, 70%, 75%, 80%, 85%, 90%, 95%, 99% or more, identical to a polypeptide comprising one of the amino acid sequences of the protein described herein. The disclosure also provides polynucleotides comprising nucleotide sequences that hybridize under stringent conditions to a polynucleotide which encodes a protein described herein, or a fragment or derivative thereof, or the antisense or complement of any such sequence.

Derivatives or analogs of the polynucleotides (or proteins) described herein include, *inter alia,* polynucleotide (or polypeptide) molecules having regions that are substantially homologous to the polynucleotides or proteins described herein, *e.g.* by at least about 45%, 50%, 70%, 80%, 95%, 98%, or even 99% identity (with a preferred identity of 80-99%) over a polynucleotide or polypeptide sequence of the same size or when compared to an aligned sequence in which the alignment is done by a computer homology program known in the art. An exemplary program is the GAP program (Wisconsin Sequence Analysis Package, Version 8 for UNIX, Genetics Computer Group, University Research Park, Madison, WI, U.S.) using the default settings, which uses the algorithm of Smith T, Waterman M, Adv Appl Math 2: 482-9 (1981). Also included are polynucleotides capable of hybridizing to the complement of a sequence encoding the proteins described herein under stringent conditions (*see e.g.* Ausubel F et al., Current Protocols in Molecular Biology (John Wiley & Sons, New York, NY, U.S., 1993)), and below. Stringent conditions are known to those skilled in the art and may be found in Current Protocols in Molecular Biology (John Wiley & Sons, NY, U.S., Ch. Sec. 6.3.1-6.3.6 (1989)).

The present disclosure further provides expression vectors that comprise the polynucleotides described herein. The polynucleotides capable of encoding the proteins described herein may be inserted into known vectors, including bacterial plasmids, viral vectors and phage vectors, using material and methods well known in the art to produce expression vectors. Such expression vectors will include the polynucleotides necessary to support production of contemplated proteins described herein within any host cell of choice or cell-free expression systems (*e.g.* pTxb1 and pIVEX2.3 described in the Examples below). The specific polynucleotides comprising expression vectors for use with specific types of host cells or cell-free expression systems are well known to one of ordinary skill in the art, can be determined using routine experimentation, or may be purchased.

The term "expression vector," as used herein, refers to a polynucleotide, linear or circular, comprising one or more expression units. The term "expression unit" denotes a polynucleotide segment encoding a polypeptide of interest and capable of providing expression of the nucleic acid segment in a host cell. An expression unit typically comprises a transcription promoter, an open reading frame encoding the polypeptide of interest, and a transcription terminator, all in operable configuration. An expression vector contains one or more expression units. Thus, in the context of the present disclosure, an expression vector encoding a protein described herein comprising a single polypeptide chain (*e.g.* a scFv genetically recombined with a Shiga toxin effector region) includes at least an expression unit for the single polypeptide chain, whereas a protein comprising, *e.g.* two or more polypeptide chains (*e.g.* one chain comprising a V_{L} domain and a second chain comprising a V_{H} domain linked to a toxin effector region) includes at least two expression units, one for each of the two polypeptide chains of the protein. For expression of multi-chain proteins, an expression unit for each polypeptide chain may also be separately contained on different expression vectors (*e.g*. expression may be achieved with a single host cell into which expression vectors for each polypeptide chain has been introduced).

Expression vectors capable of directing transient or stable expression of polypeptides and proteins are well known in the art. The expression vectors generally include, but are not limited to, one or more of the following: a heterologous signal sequence or peptide, an origin of replication, one or more marker genes, an enhancer element, a promoter, and a transcription termination sequence, each of which is well known in the art. Optional regulatory control sequences, integration sequences, and useful markers that can be employed are known in the art.

The term "host cell" refers to a cell which can support the replication or expression of the expression vector. Host cells may be prokaryotic cells, such as *E. coli* or eukaryotic cells (*e.g.* yeast, insect, amphibian, bird, or mammalian cells). Creation and isolation of host cell lines comprising a polynucleotide described herein or capable of producing a protein described herein can be accomplished using standard techniques known in the art.

Proteins of the present disclosure may be variants or derivatives of the proteins described herein that are produced by modifying the polynucleotide encoding a disclosed protein by altering one or more amino acids or deleting or inserting one or more amino acids that may render it more suitable to achieve desired properties, such as more optimal expression by a host cell.

### VIII. Delivery Devices and Kits

In certain embodiments, the disclosure relates to a device comprising one or more compositions of matter described herein, such as a pharmaceutical composition, for delivery to a subject. Thus, a delivery devices comprising one or more compounds described herein may be used to administer to a patient a composition of matter described herein by various delivery methods, including: intravenous, subcutaneous, intramuscular or intraperitoneal injection; oral administration; transdermal administration; pulmonary or transmucosal administration; administration by implant, osmotic pump, cartridge or micro pump; or by other means recognized by a person of skill in the art.

Also within the scope of the invention is a kit as defined in the claims comprising: (i) a protein of the invention, a pharmaceutical composition of the invention or a diagnostic composition of the invention; and (ii) an additional reagent and/or pharmaceutical delivery device; and optionally, packaging and instructions for use. Kits may be useful for drug administration and/or diagnostic information gathering. A kit of the invention may optionally comprise at least one additional reagent (*e.g*., standards, markers and the like). Kits typically include a label indicating the intended use of the contents of the kit. The kit may further comprise reagents and other tools for detecting a cell type (*e.g.* tumor cell) in a sample or in a subject, or for diagnosing whether a patient belongs to a group that responds to a therapeutic strategy which makes use of a protein, composition or related method of the invention as described herein.

### IX. Methods for Using a Protein of the Invention or a Composition Thereof

Generally, it is an object of the invention to provide a protein of the invention or a pharmaceutical composition comprising the same, for use in the prevention and/or treatment of a disease, disorder, or condition in a subject in need thereof, such as certain cancers, tumors, immune disorders, microbial infections, or further pathological conditions mentioned herein. Accordingly, the present disclosure provides methods of using the proteins and pharmaceutical compositions described herein for the targeted killing of cells, for delivering additional exogenous materials into target cells, for labeling of the interiors of target cells, for collecting diagnostic information, and for treating diseases, disorders, and conditions as described herein.

In particular, it is an object of the disclosure to provide such pharmacologically active agents, compositions, and/or methods that have certain advantages compared to the agents, compositions, and/or methods that are currently known in the art. Accordingly, the present disclosure provides methods of using proteins described herein characterized by specified polypeptide sequences and pharmaceutical compositions thereof. For example, any of the polypeptide sequences in SEQ ID NOs: 1-34 may be specifically utilized as a component of the protein used in the following methods.

The present invention provides an *in vitro* method of killing a cell comprising the step of contacting the cell with a protein of the present invention, as defined in the claims. The proteins of the invention can be used to kill a specific cell type upon contacting a cell or cells with one of the claimed compositions of matter. In certain embodiments, a protein of the present invention can be used to kill specific cell types in a mixture of different cell types, such as mixtures comprising cancer cells, infected cells, and/or hematological cells. In certain embodiments, a protein of the present invention can be used to kill cancer cells in a mixture of different cell types. In certain embodiments, a protein of the present invention can be used to kill specific cell types in a mixture of different cell types, such as pre-transplantation tissues. In certain embodiments, a protein of the present invention can be used to kill specific cell types in a mixture of cell types, such as pre-administration tissue material for therapeutic purposes. In certain embodiments, a protein of the present invention can be used to selectively kill cells infected by viruses or microorganisms, or otherwise selectively kill cells expressing a particular extracellular target biomolecule, such as a cell surface biomolecule. The proteins of the invention have varied applications, including, *e.g*., uses in depleting unwanted cell types from tissues either *in vitro* or *in vivo,* uses in modulating immune responses to treat graft-versus-host disease, uses as antiviral agents, uses as anti-parasitic agents, and uses in purging transplantation tissues of unwanted cell types.

In certain embodiments, a protein or pharmaceutical composition of the present invention, alone or in combination with other compounds or pharmaceutical compositions, can show potent cell-kill activity when administered to a population of cells, *in vitro* or *in vivo* in a subject such as in a patient in need of treatment. By targeting the delivery of enzymatically active Shiga toxin regions using high-affinity binding regions to cancer cell types, this potent cell-kill activity can be restricted to specifically and selectively kill certain cell types within an organism, such as certain cancer cells, neoplastic cells, malignant cells, non-malignant tumor cells, or infected cells.

The present disclosure provides a method of killing a cell in a patient in need thereof, the method comprising the step of administering to the patient at least one protein described herein or a pharmaceutical composition thereof.

Certain embodiments of the protein of the invention or pharmaceutical compositions thereof can be used to kill a cancer and/or tumor cell in a patient by targeting an extracellular biomolecule found physically coupled with a cancer and/or tumor cell. The terms "cancer cell" or "cancerous cell" refers to various neoplastic cells which grow and divide in an abnormally accelerated fashion and will be clear to the skilled person. The term "tumor cell" includes both malignant and non-malignant cells (*e.g*. non-cancerous, benign tumor cells, non-cancerous "cancer" stem cells, tumor stem cells, pre-malignant cancer-initiating cells, tumor-initiating cells, or tumorigenic cells all of which can give rise to daughter cells which become malignant tumor and/or cancer cells but are unable to metastasize on their own (*see e.g.* Martinez-Climent J et al., Haematologica 95: 293-302 (2010)). Generally, cancers and/or tumors can be defined as diseases, disorders, or conditions that are amenable to treatment and/or prevention. The cancers and tumors (either malignant or non-malignant) which are comprised of cancer cells and/or tumor cells which may benefit from methods and compositions of the invention will be clear to the skilled person. Neoplastic cells are often associated with one or more of the following: unregulated growth, lack of differentiation, local tissue invasion, angiogenesis, and metastasis.

Certain embodiments of the proteins of the invention or pharmaceutical compositions thereof can be used to kill an immune cell (whether healthy or malignant) in a patient by targeting an extracellular biomolecule found physically coupled with an immune cell.

Certain embodiments of the proteins of the invention or pharmaceutical compositions thereof can be used to kill an infected cell in a patient by targeting an extracellular biomolecule found physically coupled with an infected cell.

Proteins of the invention or pharmaceutical compositions thereof may be utilized for the purposes of purging patient cell populations (*e.g*. bone marrow) of infected, malignant, neoplastic, or otherwise unwanted B-cells and/or T-cells and then reinfusing the B-cell and/or T-cell depleted material into the patient (*see e.g.* van Heeckeren W et al., Br J Haematol 132: 42-55 (2006); Alpdogan O, van den Brink M, Semin Oncol 39: 629-42 (2012)).

Proteins of the invention or pharmaceutical compositions thereof may be utilized for the purposes of *ex vivo* depletion of B-cells and/or T-cells from isolated cell populations removed from a patient. In one non-limiting example, the protein of the invention may be used in a method for prophylaxis of organ and/or tissue transplant rejection wherein the donor organ or tissue is perfused prior to transplant with a cytotoxic protein of the invention or a pharmaceutical composition thereof in order to purge the organ of unwanted donor B-cells and/or T-cells (*see e.g.* Alpdogan O, van den Brink M, Semin Oncol 39: 629-42 (2012)).

Proteins of the invention or pharmaceutical compositions thereof may be utilized for the purposes of depleting B-cells and/or T-cells from a donor cell population as a prophylaxis against graft-versus-host disease, and induction of tolerance, in a patient to undergo a bone marrow and or stem cell transplant.

Certain embodiments of the protein of the present invention or pharmaceutical compositions thereof can be used to kill an infected cell in a patient by targeting an extracellular biomolecule found physically coupled with an infected cell.

Certain embodiments of the protein of the present invention or pharmaceutical compositions thereof can be used to kill a cell(s) of a multicellular parasite. In certain further embodiments, the cell killing occurs while the multicellular parasite is present in a host organism or subject. In certain further embodiments, the protein of the invention may be used to kill a helminth, such as, *e.g.,* a plathelminth, nemathelminth, cestode, mongenean, nematode, and/or trematode.

Additionally, the present invention provides a protein of the invention or a pharmaceutical composition of the invention for use in treating a disease, disorder, or condition in a patient, as recited in the claims. Contemplated diseases, disorders, and conditions that can be treated include cancers, malignant tumors, non-malignant tumors, growth abnormalities, immune disorders, and microbial infections. Administration of a "therapeutically effective dosage" of a protein of the invention may result in a decrease in severity of disease symptoms, an increase in frequency and duration of disease symptom-free periods, or a prevention of impairment or disability due to the disease affliction.

The therapeutically effective amount of a protein of the present invention will depend on the route of administration, the type of mammal being treated, and the physical characteristics of the specific patient under consideration. These factors and their relationship to determining this amount are well known to skilled practitioners in the medical arts. This amount and the method of administration can be tailored to achieve optimal efficacy, and may depend on such factors as weight, diet, concurrent medication and other factors, well known to those skilled in the medical arts. The dosage sizes and dosing regimen most appropriate for human use may be guided by the results obtained by the present invention, and may be confirmed in properly designed clinical trials. An effective dosage and treatment protocol may be determined by conventional means, starting with a low dose in laboratory animals and then increasing the dosage while monitoring the effects, and systematically varying the dosage regimen as well. Numerous factors may be taken into consideration by a clinician when determining an optimal dosage for a given subject. Such considerations are known to the skilled person.

An acceptable route of administration may refer to any administration pathway known in the art, including but not limited to aerosol, enteral, nasal, ophthalmic, oral, parenteral, rectal, vaginal, or transdermal (*e.g*. topical administration of a cream, gel or ointment, or by means of a transdermal patch). "Parenteral administration" is typically associated with injection at or in communication with the intended site of action, including infraorbital, infusion, intraarterial, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal administration.

For administration of a pharmaceutical composition of the invention, the dosage range will generally be from about 0.0001 to 100 milligrams per kilogram (mg/kg), and more usually 0.01 to 5 mg/kg, of the host body weight. Exemplary dosages may be 0.25 mg/kg body weight, 1 mg/kg body weight, 3 mg/kg body weight, 5 mg/kg body weight or 10 mg/kg body weight or within the range of 1-10 mg/kg. An exemplary treatment regime is a once or twice daily administration, or a once or twice weekly administration, once every two weeks, once every three weeks, once every four weeks, once a month, once every two or three months or once every three to 6 months. Dosages may be selected and readjusted by the skilled health care professional as required to maximize therapeutic benefit for a particular patient.

Pharmaceutical compositions of the invention may be administered to the same patient on multiple occasions. Intervals between single dosages can be, for example, 2-5 days, weekly, monthly, every two or three months, every six months, or yearly. Intervals between administrations can also be irregular, based on regulating blood levels or other markers in the subject or patient. Dosage regimens include intravenous administration of 1 mg/kg body weight or 3 mg/kg body weight with the compound administered every two to four weeks for six dosages, then every three months at 3 mg/kg body weight or 1 mg/kg body weight.

A pharmaceutical composition of the present invention may be administered via one or more routes of administration, using one or more of a variety of methods known in the art. As will be appreciated by the skilled worker, the route and/or mode of administration will vary depending upon the desired results. Routes of administration for proteins of the present invention or compositions thereof include, *e.g*. intravenous, intramuscular, intradermal, intraperitoneal, subcutaneous, spinal, or other parenteral routes of administration, for example by injection or infusion. In other embodiments, a protein or pharmaceutical composition of the invention may be administered by a non-parenteral route, such as a topical, epidermal or mucosal route of administration, for example, intranasally, orally, vaginally, rectally, sublingually, or topically.

Proteins or pharmaceutical compositions of the invention may be administered with one or more of a variety of medical devices known in the art. For example, in one embodiment, a pharmaceutical composition of the invention may be administered with a needleless hypodermic injection device. Examples of well-known implants and modules useful in the present invention are in the art, including *e.g*., implantable micro-infusion pumps for controlled rate delivery; devices for administering through the skin; infusion pumps for delivery at a precise infusion rate; variable flow implantable infusion devices for continuous drug delivery; and osmotic drug delivery systems. These and other such implants, delivery systems, and modules are known to those skilled in the art.

A protein or pharmaceutical composition of the present invention may be administered alone or in combination with one or more other therapeutic or diagnostic agents. A combination therapy may include a protein of the invention or pharmaceutical composition thereof combined with at least one other therapeutic agent selected based on the particular patient, disease or condition to be treated. Examples of other such agents include, *inter alia,* a cytotoxic, anti-cancer or chemotherapeutic agent, an anti-inflammatory or anti-proliferative agent, an antimicrobial or antiviral agent, growth factors, cytokines, an analgesic, a therapeutically active small molecule or polypeptide, a single chain antibody, a classical antibody or fragment thereof, or a nucleic acid molecule which modulates one or more signaling pathways, and similar modulating therapeutics which may complement or otherwise be beneficial in a therapeutic or prophylactic treatment regimen.

Treatment of a patient with a protein or pharmaceutical composition of the invention preferably leads to cell death of targeted cells and/or the inhibition of growth of targeted cells. As such, proteins of the invention, and pharmaceutical compositions comprising them, will be useful for treating a variety of pathological disorders in which killing or depleting target cells may be beneficial, such as, *inter alia,* cancers, tumors, other growth abnormalities, immune disorders, and infected cells. The present disclosure provides methods for suppressing cell proliferation, and treating cell disorders, including neoplasia, overactive B-cells, and overactive T-cells.

In certain embodiments, proteins and pharmaceutical compositions of the invention may be used to treat or prevent cancers, tumors (malignant and non-malignant), growth abnormalities, immune disorders, and microbial infections. The above *ex vivo* method can be combined with the above *in vivo* method to provide methods of treating or preventing rejection in bone marrow transplant recipients, and for achieving immunological tolerance.

In certain embodiments, the present disclosure provides methods for treating malignancies or neoplasms and other blood cell associated cancers in a mammalian subject, such as a human, the method comprising the step of administering to a subject in need thereof a therapeutically effective amount of a protein or pharmaceutical composition described herein.

The proteins and pharmaceutical compositions of the invention have varied applications, including, *e.g*., uses in removing unwanted B-cells and/or T-cells, uses in modulating immune responses to treat graft-versus-host diseases, uses as antiviral agents, uses as antimicrobial agents, and uses in purging transplantation tissues of unwanted cell types. The proteins and pharmaceutical compositions of the present invention are commonly anti-neoplastic agents-meaning they are capable of treating and/or preventing the development, maturation, or spread of neoplastic or malignant cells by inhibiting the growth and/or causing the death of cancer or tumor cells.

A protein or pharmaceutical composition of the present invention may be used to treat a B-cell-, plasma cell-, T-cell-, or antibody- mediated disease or disorder, such as for example leukemia, lymphoma, myeloma, Human Immunodeficiency Virus-related diseases, amyloidosis, hemolytic uremic syndrome, polyarteritis nodosa, polyarthritis, septic shock, Crohn's Disease, rheumatoid arthritis, ankylosing spondylitis, psoriatic arthritis, ulcerative colitis, psoriasis, asthma, Sjögren's syndrome, graft-versus-host disease, graft rejection, diabetes, vasculitis, scleroderma, and systemic lupus erythematosus.

In another aspect, certain embodiments of the proteins and pharmaceutical compositions of the present invention are antimicrobial agents - meaning they are capable of treating and/or preventing the acquisition, development, or consequences of microbiological pathogenic infections, such as caused by viruses, bacteria, fungi, prions, or protozoans.

The present disclosure provides a prophylaxis or treatment for diseases or conditions mediated by B-cells and/or T-cells, the prophylaxis or treatment involving administering the protein described herein, or a pharmaceutical composition thereof, to a patient in need thereof for the purpose of killing B-cells and/or T-cells in the patient. This usage is compatible with preparing or conditioning a patient for bone marrow transplantation, stem cell transplantation, tissue transplantation, or organ transplantation, regardless of the source of the transplanted material, *e.g*. human or non-human sources.

The present disclosure provides a bone marrow recipient for prophylaxis or treatment of host-versus-graft disease via the targeted cell-killing of host B-cells, NK cells, and/or T-cells using a protein or pharmaceutical composition described herein (*see e.g.* Sarantopoulos S et al., Biol Blood Marrow Transplant 21: 16-23 (2015)).

The proteins and pharmaceutical compositions of the present invention may be utilized in a method of treating cancer comprising administering to a patient, in need thereof, a therapeutically effective amount of the protein or a pharmaceutical composition of the present invention. In certain embodiments of the methods of the present disclosure, the cancer being treated is selected from the group consisting of: bone cancer (such as multiple myeloma or Ewing's sarcoma), breast cancer, central/peripheral nervous system cancer (such as brain cancer, neurofibromatosis, or glioblastoma), gastrointestinal cancer (such as stomach cancer or colorectal cancer), germ cell cancer (such as ovarian cancers and testicular cancers, glandular cancer (such as pancreatic cancer, parathyroid cancer, pheochromocytoma, salivary gland cancer, or thyroid cancer), head-neck cancer (such as nasopharyngeal cancer, oral cancer, or pharyngeal cancer), hematological cancers (such as leukemia, lymphoma, or myeloma), kidney-urinary tract cancer (such as renal cancer and bladder cancer), liver cancer, lung/pleura cancer (such as mesothelioma, small cell lung carcinoma, or non-small cell lung carcinoma), prostate cancer, sarcoma (such as angiosarcoma, fibrosarcoma, Kaposi's sarcoma, or synovial sarcoma), skin cancer (such as basal cell carcinoma, squamous cell carcinoma, or melanoma), and uterine cancer.

The proteins and pharmaceutical compositions of the present disclosure may be utilized in a method of treating an immune disorder comprising administering to a patient, in need thereof, a therapeutically effective amount of the protein or a pharmaceutical composition described herein. In certain embodiments of the methods of the present disclosure, the immune disorder is related to an inflammation associated with a disease selected from the group consisting of: amyloidosis, ankylosing spondylitis, asthma, Crohn's disease, diabetes, graft rejection, graft-versus-host disease, Hashimoto's thyroiditis, hemolytic uremic syndrome, HIV-related disease, lupus erythematosus, multiple sclerosis, polyarteritis nodosa, polyarthritis, psoriasis, psoriatic arthritis, rheumatoid arthritis, scleroderma, septic shock, Sjögren's syndrome, ulcerative colitis, and vasculitis.

Among certain embodiments of the present invention, the protein of the invention or a pharmaceutical composition of the invention is for use in the treatment or prevention of a cancer, tumor, growth abnormality, immune disorder, and/or microbial infection. For example, immune disorders presenting on the skin of a patient may be treated with such a medicament in efforts to reduce inflammation. In another example, skin tumors may be treated with such a medicament in efforts to reduce tumor size or eliminate the tumor completely.

Certain proteins of the present invention may be used in molecular neurosurgery applications such as immunolesioning and neuronal tracing (*see,* Wiley R, Lappi D, Adv Drug Deliv Rev 55: 1043-54 (2003), *for review*)*.* For example, the targeting domain may be selected or derived from various ligands, such as neurotransmitters and neuropeptides, which target specific neuronal cell types by binding neuronal surface receptors, such as a neuronal circuit specific G-protein coupled receptor. Similarly, the targeting domain may be selected from or derived from antibodies that bind neuronal surface receptors. Because Shiga toxins robustly direct their own retrograde axonal transport, certain cytotoxic proteins of the invention may be used to kill a neuron(s) which expresses the extracellular target at a site of cytotoxic protein injection distant from the cell body (*see* Llewellyn-Smith I et al., J Neurosci Methods 103: 83-90 (2000)). These neuronal cell type specific targeting cytotoxic proteins have uses in neuroscience research, such as for elucidating mechanisms of sensations (*see e.g.* Mishra S, Hoon M, Science 340: 968-71 (2013)), and creating model systems of neurodegenerative diseases, such as Parkinson's and Alzheimer's (*see e.g.* Hamlin A et al., PLoS One e53472 (2013)).

Among certain embodiments of the present disclosure is a method of using a protein, pharmaceutical composition, and/or diagnostic composition described herein to detect the presence of a cell type for the purpose of information gathering regarding diseases, conditions and/or disorders. The method comprises contacting a cell with a diagnostically sufficient amount of a protein described herein to detect the protein by an assay or diagnostic technique. The term "diagnostically sufficient amount" refers to an amount that provides adequate detection and accurate measurement for information gathering purposes by the particular assay or diagnostic technique utilized. Generally, the diagnostically sufficient amount for whole organism *in vivo* diagnostic use will be a non-cumulative dose of between 0.1 mg to 100 mg of the detection promoting agent linked protein per kg of subject per subject. Typically, the amount of protein used in these information gathering methods will be as low as possible provided that it is still a diagnostically sufficient amount. For example, for *in vivo* detection in an organism, the amount of protein administered to a subject will be as low as feasibly possible.

The cell-type specific targeting of proteins of the invention combined with detection promoting agents provides a way to detect and image cells physically coupled with an extracellular target biomolecule of a binding region of the proteins of the invention. Imaging of cells using the proteins of the invention may be performed *in vitro* or *in vivo* by any suitable technique known in the art. For example, the method of using a protein, pharmaceutical composition, or diagnostic composition described herein to detect the presence of a cell type for the purpose of information gathering may be performed on cells *in vivo* within a patient, including on cells *in situ, e.g.* at a disease locus, on cells *in vitro,* and/or in an *ex vivo* setting on cells and tissues removed from an organism, *e.g.* a biopsy material.

Diagnostic information may be collected using various methods known in the art, including whole body imaging of an organism or using *ex vivo* samples taken from an organism. The term sample used herein refers to any number of things, but not limited to, fluids such as blood, urine, serum, lymph, saliva, anal secretions, vaginal secretions, and semen, and tissues obtained by biopsy procedures. For example, various detection promoting agents may be utilized for non-invasive *in vivo* tumor imaging by techniques such as magnetic resonance imaging (MRI), optical methods (such as direct, fluorescent, and bioluminescent imaging), positron emission tomography (PET), single-photon emission computed tomography (SPECT), ultrasound, x-ray computed tomography, and combinations of the aforementioned (*see,* Kaur S et al., Cancer Lett 315: 97-111 (2012), *for review*).

The disclosed method of using a protein, pharmaceutical composition, or diagnostic composition described herein to detect the presence of a target biomolecule positive cell type for the purpose of information gathering may be performed on cells *in vivo* within a patient, on cells *in situ, e.g.* at a disease locus, on cells *in vitro,* and/or in an *ex vivo* setting on cells and tissues removed from an organism, *e.g.* a biopsy material. The detection of specific cells, cell types, and cell populations using a composition described herein may be used for diagnosis and imaging of cells, such as, *e.g.,* tumor, cancer, immune, and infected cells. For example, proteins and diagnostic compositions described herein may be employed to image or visualize a site of possible accumulation of target biomolecule expressing cells in an organism. These methods may be used to identify sites of tumor development or residual tumor cells after a therapeutic intervention.

The disclosed method used to detect the presence of a cell type may be used to gather information regarding diseases, disorders, and conditions, such as, for example bone cancer (such as multiple myeloma or Ewing's sarcoma), breast cancer, central/peripheral nervous system cancer (such as brain cancer, neurofibromatosis, or glioblastoma), gastrointestinal cancer (such as stomach cancer or colorectal cancer), germ cell cancer (such as ovarian cancers and testicular cancers, glandular cancer (such as pancreatic cancer, parathyroid cancer, pheochromocytoma, salivary gland cancer, or thyroid cancer), head-neck cancer (such as nasopharyngeal cancer, oral cancer, or pharyngeal cancer), hematological cancers (such as leukemia, lymphoma, or myeloma), kidney-urinary tract cancer (such as renal cancer and bladder cancer), liver cancer, lung/pleura cancer (such as mesothelioma, small cell lung carcinoma, or non-small cell lung carcinoma), prostate cancer, sarcoma (such as angiosarcoma, fibrosarcoma, Kaposi's sarcoma, or synovial sarcoma), skin cancer (such as basal cell carcinoma, squamous cell carcinoma, or melanoma), uterine cancer, AIDS, amyloidosis, ankylosing spondylitis, asthma, autism, cardiogenesis, Crohn's disease, diabetes, erythematosus, gastritis, graft rejection, graft-versus-host disease, Grave's disease, Hashimoto's thyroiditis, hemolytic uremic syndrome, HIV-related disease, lupus erythematosus, lymphoproliferative disorders, multiple sclerosis, myasthenia gravis, neuroinflammation, polyarteritis nodosa, polyarthritis, psoriasis, psoriatic arthritis, rheumatoid arthritis, scleroderma, septic shock, Sjögren's syndrome, systemic lupus erythematosus, ulcerative colitis, vasculitis, cell proliferation, inflammation, leukocyte activation, leukocyte adhesion, leukocyte chemotaxis, leukocyte maturation, leukocyte migration, neuronal differentiation, acute lymphoblastic leukemia (ALL), T acute lymphocytic leukemia/lymphoma (ALL), acute myelogenous leukemia, acute myeloid leukemia (AML), B-cell chronic lymphocytic leukemia (B-CLL), B-cell prolymphocytic lymphoma, Burkitt's lymphoma (BL), chronic lymphocytic leukemia (CLL), chronic myelogenous leukemia (CML-BP), chronic myeloid leukemia (CML), diffuse large B-cell lymphoma, follicular lymphoma, hairy cell leukemia (HCL), Hodgkin's Lymphoma (HL), intravascular large B-cell lymphoma, lymphomatoid granulomatosis, lymphoplasmacytic lymphoma, MALT lymphoma, mantle cell lymphoma, multiple myeloma (MM), natural killer cell leukemia, nodal marginal B-cell lymphoma, Non-Hodgkin's lymphoma (NHL), plasma cell leukemia, plasmacytoma, primary effusion lymphoma, pro-lymphocytic leukemia, promyelocytic leukemia, small lymphocytic lymphoma, splenic marginal zone lymphoma, T-cell lymphoma (TCL), heavy chain disease, monoclonal gammopathy, monoclonal immunoglobulin deposition disease, myelodusplastic syndromes (MDS), smoldering multiple myeloma, and Waldenstrom macroglobulinemia.

Among certain embodiment of the present disclosure is a method of using a protein, pharmaceutical composition, and/or diagnostic composition to label or detect the interiors of neoplastic cells and/or immune cell types (*see e.g.,* Koyama Y et al., Clin Cancer Res 13: 2936-45 (2007); Ogawa M et al., Cancer Res 69: 1268-72 (2009); Yang L et al., Small 5: 235-43 (2009)). Based on the ability of the proteins and pharmaceutical compositions described herein to enter specific cell types and route within cells via retrograde intracellular transport, the interior compartments of specific cell types are labeled for detection. This can be performed on cells *in situ* within a patient or on cells and tissues removed from an organism, *e.g*. biopsy material.

Diagnostic compositions of the invention may be used to characterize a disease, disorder, or condition as potentially treatable by a related pharmaceutical composition of the invention. Certain compositions of matter described herein may be used to determine whether a patient belongs to a group that responds to a therapeutic strategy which makes use of a compound, composition or related method of the disclosure as described herein or is well suited for using a delivery device of the disclosure.

Diagnostic compositions of the invention may be used after a disease, *e.g.* a cancer, is detected in order to better characterize it, such as to monitor distant metastases, heterogeneity, and stage of cancer progression. The phenotypic assessment of disease disorder or infection can help prognostic and prediction during therapeutic decision making. In disease reoccurrence, certain methods of the disclosure may be used to determine if local or systemic problem.

Diagnostic compositions of the invention may be used to assess responses to therapeutic(s) regardless of the type of therapeutic, *e.g*. small molecule drug, biological drug, or cell-based therapy. For example, certain embodiments of the diagnostics of the invention may be used to measure changes in tumor size, changes in antigen positive cell populations including number and distribution, and/or monitor a different marker than the antigen targeted by a therapy already being administered to a patient (*see e.g.* Smith-Jones P et al., Nat Biotechnol 22: 701-6 (2004); Evans M et al., Proc Natl Acad Sci U.S.A. 108: 9578-82 (2011)).

In certain embodiments, the proteins of the invention or pharmaceutical and/or diagnostic compositions thereof may be used for both diagnosis and treatment, or for diagnosis alone.

The present invention is further illustrated by the following non-limiting examples of selectively cytotoxic proteins comprising Shiga toxin effector regions derived from A Subunits of members of the Shiga toxin family and binding regions capable of binding extracellular target biomolecules physically coupled to specific cell types.

### EXAMPLES

The following examples demonstrate certain embodiments of the present disclosure. The examples were carried out using standard techniques, which are well known and routine to those of skill in the art, except where otherwise described in detail.

The following examples of cytotoxic proteins demonstrate the improved ability of exemplary cytotoxic proteins to selectively kill cells physically coupled with an extracellular target biomolecule of the immunoglobulin-type binding region as compared to their ancestral variants which lacked signal motifs. The exemplary cytotoxic proteins bound to target biomolecules expressed by targeted cell types and entered the targeted cells. The internalized cytotoxic proteins effectively routed their Shiga toxin effector regions to the cytosol to inactivate ribosomes and subsequently caused the apoptotic death of the targeted cells.

First, it was observed that the addition of KDEL family signal motifs to multiple Shiga-toxin-based fusion proteins reduces their cytotoxicity. This is consistent with the scientific literature which never described any example of a Shiga toxin construct that benefits from the presence of a KDEL family signal motif. Then, it was unexpectedly discovered that the addition of a KDEL family signal motif can improve Shiga-toxin-based cytotoxicity of certain constructs. Surprisingly, both the KDEL bearing and non-KDEL bearing variants exhibited similar *in vitro* enzymatic activities, similar cell binding characteristics, and similar cell internalization; however, the KDEL bearing variants of the cytotoxic proteins exhibited improved cytotoxicity. These results contradicted previous findings (*see e.g.* Jackson M et al., J Cell Sci 112: 467-75 (1999)).

One exemplary cytotoxic protein comprises a single-chain, variable fragment, binding region capable of binding HER2 with high affinity combined with a Shiga toxin A Subunit fragment and a carboxy-terminal KDEL motif. This exemplary cytotoxic protein is capable of selectively killing cells that express HER2 on their surface. A second exemplary cytotoxic protein comprises a single-chain, variable fragment, binding region capable of binding CD38 with high affinity combined with a Shiga toxin A Subunit fragment and a carboxy-terminal KDEL motif. This second exemplary cytotoxic protein is capable of selectively killing cells that express CD38 on their surface. A third exemplary cytotoxic protein comprises a single-chain, variable fragment, binding region capable of binding CD19 with high affinity combined with a Shiga toxin A Subunit fragment and a carboxy-terminal KDEL motif. This third exemplary cytotoxic protein is capable of selectively killing cells that express CD 19 on their surface. A fourth exemplary cytotoxic protein comprises a single-chain, variable fragment, binding region capable of binding CD74 with high affinity combined with a Shiga toxin A Subunit fragment and a carboxy-terminal KDEL motif. This fourth exemplary cytotoxic protein is capable of selectively killing cells that express CD74 on their surface. Other exemplary cytotoxic proteins include those with binding regions targeting Epstein-Barr viral antigens, Leishmania antigens, neurotensin receptors, epidermal growth factor receptors, the immune cell receptor CCR5, and the viral protein targets Env and UL18.

### Example 1. A HER2-targeted, cytotoxic protein derived from Shiga-like toxin 1 A Subunit with an endoplasmic reticulum signal motif (αHER2scFv::SLT-1A::KDEL)

The cytotoxic protein of this example αHER2scFv::SLT-1A::KDEL comprises a single-chain, variable fragment, binding region capable of binding HER2 with high affinity combined with a Shiga toxin A Subunit fragment and a carboxy-terminal KDEL motif.

### Construction. Production, and Purification of the Cytotoxic Protein αHER2scFv::SLT-1A::KDEL

First, a Shiga toxin effector region and a binding region were designed or selected. In this example, the Shiga toxin effector region was derived from the A subunit of Shiga-like Toxin 1 (SLT-1A). A polynucleotide was obtained that encoded amino acids 1-251 of SLT-1A (Cheung M et al., Mol Cancer 9: 28 (2010)). An immunoglobulin-type binding region αHER2scFv was derived from trastuzumab (marketed as Herceptin®, Genentech, Inc., South San Francisco, CA, U.S.) and the 4D5 monoclonal antibody (Zhao et al., J Immunol 183: 5563-74 (2009)) such that a single-chain variable fragment (scFv) is created with the two immunoglobulin variable regions (V_{L} and V_{H}) separated by a linker known in the art.

Second, the binding region and Shiga toxin effector region were linked together to form a fusion protein. In this example, a polynucleotide encoding the binding region αHER2scFv comprising amino acids 1-245 of SEQ ID NO:4 was cloned in frame with a polynucleotide encoding a "murine hinge" derived from a murine IgG3 molecule (or other linkers known to the skilled person) and in frame with a polynucleotide encoding Shiga toxin effector region SLT-1A comprising amino acids 1-251 of SEQ ID NO:1. In certain experiments, the full-length coding sequence of the cytotoxic protein of this example began with a polynucleotide encoding a Strep-tag® to facilitate detection and purification. The polynucleotide sequence encoding the cytotoxic protein αHER2scFv::SLT-1A::KDEL of this example was codon optimized for efficient expression in *E. coli* using services from DNA 2.0, Inc. (Menlo Park, CA, U.S.).

Third, a fusion protein was produced by expressing the polynucleotide encoding the cytotoxic protein αHER2scFv::SLT-1A::KDEL (SEQ ID NO:4). Expression of the αHER2scFv::SLT-1A::KDEL cytotoxic protein was accomplished using both bacterial and cell-free, protein translation systems.

In this example of αHER2scFv::SLT-1A::KDEL production by an *E. coli* expression system, the polynucleotide "insert" sequence encoding αHER2scFv::SLT-1A::KDEL was cloned into the pTxb1 vector (New England Biolabs, Ipswich, MA, U.S.) using standard procedures to produce a polynucleotide sequence encoding the cytotoxic protein αHER2scFv::SLT-1A::KDEL ligated in frame to polynucleotide sequences encoding the amino-terminal intein of the vector. The plasmid insert polynucleotide sequence was verified by Sanger sequencing (Functional Biosciences, Madison, WI, U.S.) and transformed into T7 Shuffle® cells (New England Biolabs, Ipswich, MA, U.S.). The αHER2scFv::SLT-1A::KDEL protein was produced and purified according to the IMPACT™ (Intein Mediated Purification with an Affinity Chitin-binding Tag) system manual (New England Biolabs, Ipswich, MA, U.S.). Purification was accomplished using standard techniques known in the art, such as using immobilized targets of the Strep-tag® or the binding region.

In this example of αHER2scFv::SLT-1A::KDEL production by a cell-free, protein translation system, the polynucleotide "insert" sequence encoding αHER2scFv::SLT-1A::KDEL was cloned into the pIVEX2.3 vector with a stop codon directly after the coding region using the In-Fusion® HD Cloning Kit (Clonetech, Mountain View, CA, U.S.) according to the manufacturer's instructions. The plasmid insert polynucleotide sequence was verified by Sanger sequencing (Functional Biosciences, Madison, WI, U.S.). αHER2scFv::SLT-1A::KDEL protein was produced using the rapid translation system 5 Prime™ RTS 100 *E. coli* Disulfide Kit (5 Prime, Gaithersburg, MD, U.S.) according to the manufacturer's instructions. Purification was accomplished using standard techniques known in the art, such as using immobilized targets of the Strep-tag® or the binding region.

### Determining the Maximum Specific Binding (Bₘₐₓ) and Equilibrium Binding Constant (K_{D}) of αHER2scFv::SLT-1A::KDEL Binding Target Cell Types

The binding characteristics of the αHER2scFv::SLT-1A::KDEL protein produced as described above was determined by a fluorescence-based, flow-cytometry assay. Samples containing HER2 positive (+) cells and HER2 negative (-) cells were suspended in phosphate buffered saline (1X PBS) (Hyclone Brand, Fisher Scientific, Waltham, MA, U.S.) containing 1 percent bovine serum albumin (BSA) (Calbiochem, San Diego, CA, U.S.), hereinafter referred to as "IX PBS+1%BSA" and incubated for 1 hour at 4 degrees Celsius (°C) with 100 µL of various dilutions of the αHER2scFv::SLT-1A::KDEL protein to be assayed. The highest concentrations of αHER2scFv::SLT-1A::KDEL protein was selected to lead to saturation of the binding reaction. After the one hour incubation, cell samples were washed twice with 1X PBS+1%BSA. The cell samples were incubated for 1 hour at 4°C with 100 µL of IX PBS+1%BSA containing 0.3 µg of anti-Strep-tag® mAb-FITC (# A01736-100, Genscript, Piscataway, NJ, U.S.).

The cell samples were next washed twice with IX PBS+1%BSA, resuspended in 200 µL of IX PBS and subjected to fluorescence-based, flow cytometry. The mean fluorescence intensity (MFI) data for all the samples was obtained by gating the data using a FITC-only sample as a negative control. Graphs were plotted of MFI versus "concentration of cells" using Prism software (GraphPad Software, San Diego, CA, U.S.). Using the Prism software function of one-site binding [Y = Bₘₐₓ^{∗}X/(K_{D} + X)] under the heading binding-saturation, the Bₘₐₓ and K_{D} were calculated using baseline corrected data. Abs values were corrected for background by subtracting the Abs values measured for wells containing only PBS. Bₘₐₓ is the maximum specific binding reported in MFI. K_{D} is the equilibrium binding constant, reported in nanomolar (nM).

The Bₘₐₓ for αHER2scFv::SLT-1A::KDEL binding to HER2+ cells was measured to be about 110,000 MFI with a K_{D} of about 160 nM (Table 1). This result was relatively similar to the Bₘₐₓ for the αHER2scFv::SLT-1A protein, which lacked a KDEL signal motif, binding to HER2+ cells which was measured to be about 140,000 MFI with a K_{D} of about 180 nM (Table 1). Neither protein was observed to have measurable binding to HER2- negative cells in this assay. This shows that the improved cytotoxicity of the KDEL variant is probably not related to a change in the immunoglobulin-derived domain's target cell binding properties.

**Table 1. Addition of KDEL Had No Significant Effect on Binding Characteristics: Representative values for Bₘₐₓ and K_{D} for αHER2scFv::SLT-1A::KDEL as compared to its ancestor which lacked a KDEL signal motif**

| | | Target Positive Cells | |
|---|---|---|---|
| Cytotoxic Protein | **target biomolecule** | **Bₘₐₓ (MFI)** | **K_{D} (nM)** |
| **αHER2scFv::SLT-1A::KDEL** | HER2 | 111,000 | 158 |
| **αHER2scFv::SLT-1A** | HER2 | 141,000 | 182 |

### Determining the Half-Maximal Inhibitory Concentration (IC₅₀) of αHER2scFv::SLT-1A::KDEL to Eukaryotic Ribosomes In Vitro

The ribosome inactivation capabilities of αHER2scFv::SLT-1A::KDEL was determined in a cell-free, *in vitro* protein translation assay using the TNT® Quick Coupled Transcription/Translation Kit (L1170 Promega, Madison, WI, U.S.). The kit includes Luciferase T7 Control DNA and TNT® Quick Master Mix. The ribosome activity reaction was prepared according to the manufacturer's instructions to create "TNT" reaction mixtures.

A series of 10-fold dilutions of αHER2scFv::SLT-1A::KDEL to be tested was prepared in appropriate buffer and a series of identical TNT reaction mixture components was created for each dilution of αHER2scFv::SLT-1A::KDEL. Each sample in the dilution series of αHER2scFv::SLT-1A::KDEL protein was combined with each of the TNT reaction mixtures along with the Luciferase T7 Control DNA. The test samples were incubated for 1.5 hours at 30°C. After the incubation, Luciferase Assay Reagent (E1483 Promega, Madison, WI, U.S.) was added to all test samples and the amount of luciferase protein translation was measured by luminescence according to the manufacturer instructions. The level of translational inhibition was determined by non-linear regression analysis of log-transformed concentrations of total protein versus relative luminescence units. Using statistical software (GraphPad Prism, San Diego, CA, U.S.), the half maximal inhibitory concentration (IC₅₀) value was calculated for each sample. Then, the data were normalized by calculating the "percent of SLT-1A-only control protein" using the Prism software function of log(inhibitor) vs. response (three parameters) [Y = Bottom + ((Top-Bottom) / (1+10^(X-LogIC50)))] under the heading dose-response-inhibition. The IC₅₀ for experimental proteins and SLT-1A-only control protein were calculated. The percent of SLT-1A-only control protein was calculated by [(IC50 of SLT-1A control protein / IC50 of experimental protein) x 100].

The inhibitory effect of αHER2scFv::SLT-1A::KDEL on cell-free protein synthesis was strong. Dose dependence experiments determined that the IC₅₀ of αHER2scFv::SLT-1A::KDEL on protein synthesis in this cell-free assay was about 70 picomolar (pM) or within 6% of the SLT-1A-only positive control (Table 2). This result was not substantially different from the IC₅₀ for the αHER2scFv::SLT-1A protein, which lacked a KDEL signal motif, which was measured to be within 8% of the SLT-1A-only positive control (Table 2). This shows that the improved cytotoxicity of the KDEL variant is probably not related to a change in the Shiga toxin A Subunit enzymatic activity.

**Table 2. Addition of KDEL Had No Significant Effect on Ribosome Inactivation: Representative half-maximal inhibitory concentration (IC₅₀) for αHER2scFv::SLT-1A::KDEL as compared to its ancestor which lacked a KDEL signal motif**

| Cytotoxic Protein | **IC₅₀ (pM)** | **IC₅₀ of SLT-1A only positive control (pM)** | **Percent of IC₅₀ of SLT-1A control protein** |
|---|---|---|---|
| **αHER2scFv::SLT-1A::KDEL** | 69.0 | 73.0 | 106% |
| **αHER2scFv::SLT-1A** | 28.1 | 30.4 | 108% |

### Determining the Selective Cytotoxicity and Half-Maximal Cytotoxic Concentration (CD₅₀) of αHER2scFv::SLT-1A::KDEL Using a Cell-Kill Assay

The cytotoxicity characteristics of αHER2scFv::SLT-1A::KDEL was determined by the following cell-kill assay. This assay determines the capacity of a cytotoxic protein to kill cells expressing the target biomolecule of cytotoxic protein's binding region as compared to cells that do not express the target biomolecule. Cells were plated (2 x 10³ cells per well) in 20 µL cell culture medium in 384-well plates. The αHER2scFv::SLT-1A::KDEL protein was diluted either 5-fold or 10-fold in a IX PBS and 5 µL of the dilutions were added to the cells. Control wells containing only cell culture medium were used for baseline correction. The cell samples were incubated with αHER2scFv::SLT-1A::KDEL, or just buffer, for 3 days at 37°C and in an atmosphere of 5% carbon dioxide (CO₂). The total cell survival or percent viability was determined using a luminescent readout using the CellTiter-Glo® Luminescent Cell Viability Assay (G7573 Promega Madison, WI, U.S.) according to the manufacturer's instructions. The Percent Viability of experimental wells was calculated using the following equation: (Test RLU - Average Media RLU) / (Average Cells RLU - Average Media RLU) ^{∗} 100. Log polypeptide concentration versus Percent Viability was plotted in Prism (GraphPad Prism, San Diego, CA, U.S.) and log (inhibitor) vs. normalized response (variable slope) analysis was used to determine the half-maximal cytotoxic concentration (CD₅₀) value for αHER2scFv::SLT-1A::KDEL.

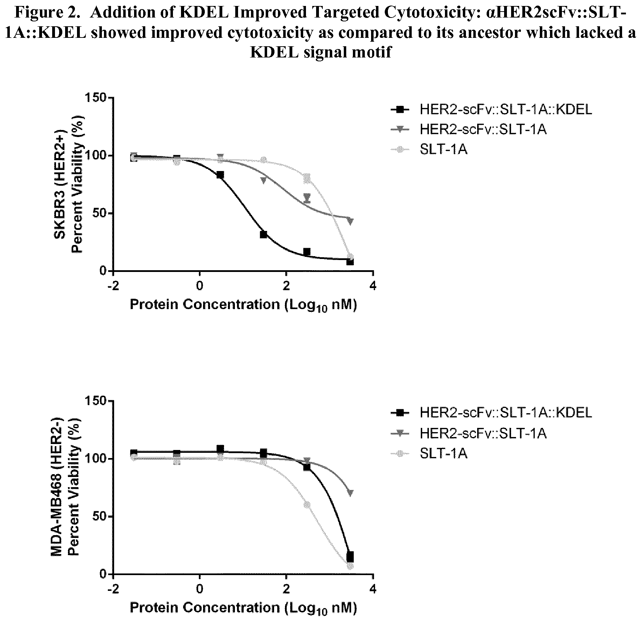

Dose dependence experiments determined that the CD₅₀ of the αHER2scFv::SLT-1A::KDEL protein was measured to be about 0.001 to 0.13 nM for HER2 + cells as compared to 14-37 nM for HER2- cells, depending on the cell line (Table 3; Figure 2). The CD₅₀ of αHER2scFv::SLT-1A::KDEL was over 100 times greater for cells not physically coupled with the extracellular target biomolecule HER2 as compared to cells that were physically coupled with the extracellular target biomolecule HER2, *e.g*. expressed HER2 on their cell surface (Table 3; Figure 2).

**Table 3. Addition of KDEL Improved Cytotoxicity: Representative half-maximal cytotoxic concentrations (CD₅₀) for αHER2scFv::SLT-1A::KDEL as compared to its ancestor which lacked a KDEL signal motif**

| | | **CD₅₀ (nM)** | | |
|---|---|---|---|---|
| **Cell Line** | **HER2 status** | **αHER2scFv::SLT-1A::KDEL** | **αHER2scFv::SLT-1A** | **SLT-1A only negative control** |
| HCC-1954 | positive | 0.1100 | 2.1 | 320 |
| SKBR3 | positive | 0.1300 | 1.3 | 220 |
| BT474 | positive | 0.0010 | 1.6 | 14 |
| MDA-MB-231 | negative | 37.0000 | 1500.0 | 130 |
| MDA-MB-468 | negative | 14.0000 | 546.0 | 230 |

The CD₅₀ of the αHER2scFv::SLT-1A variant lacking a carboxy-terminal KDEL signal motif was measured to be about 1.3-2.1 nM for HER2+ cells (Table 3; Figure 2). Thus, the addition of a KDEL signal motif reduced the CD₅₀ to HER2+ cells by 10-20 fold. Additionally, αHER2scFv::SLT-1A::KDEL killed more total cells than αHER2scFv::SLT-1A as seen in the decreased cell viability at the highest tested concentration (Figure 2). These results exemplify the effect of adding a KDEL signal motif on both cytotoxicity and selective cytotoxicity. The differences in cell-kill for these cytotoxic proteins were not predictable based on the *in vitro* results for either protein synthesis inhibition or target cell-binding characteristics.

### Determining Cell Internalization of αHER2scFv::SLT-1A with and without a KDEL Signal Motif Using Immunofluorescence

The ability of cytotoxic proteins to enter target cells was investigated using standard immunocytochemical techniques known in the art. Briefly, 0.8 x 10⁶ cells of each cell type (SKBR3 (HER2+) and MDA-MB-231 (HER2-)) were harvested and suspended in 50 µL of cell culture medium containing a cocktail of protease inhibitors (*e.g.* P1860 Sigma-Aldrich Co., St. Louis, MO, U.S.) and human Fc receptor protein to reduce non-specific immunofluorescent staining. Next, 100 nM of the cytotoxic protein to be analyzed was added to the cells, and the cells were incubated at 37°C for 1 hour to allow for intoxication to progress. Then, cells were "fixed" and "permeabilized" using the Cytofix/Cytoperm™ Kit (BD Biosciences San Diego, CA, U.S.) according to the manufacturer's instructions. The Shiga toxin effector region was "stained" using a mouse monoclonal antibody (mouse IgG anti-Shiga toxin 1 Subunit A, BEI NR-867 BEI Resources, Manassas, VA, U.S.). The mouse monoclonal antibody localization was then detected with the Alexa Fluor® 555 Monoclonal Antibody Labeling Kit (Life Technologies, Carlsbad, CA, U.S.) according to manufacturer's instructions.

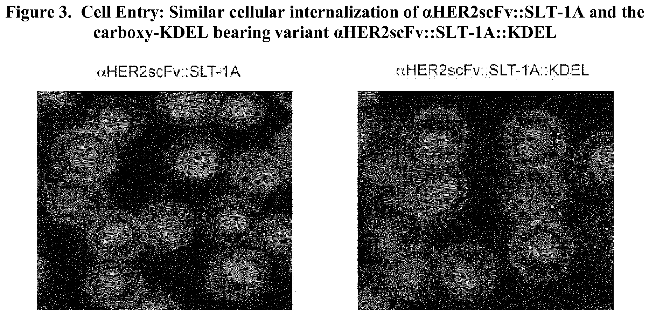

In this assay, cell internalization was observed in HER2+ cells for both αHER2scFv::SLT-1A::KDEL and the variant αHER2scFv::SLT-1A which lacked a KDEL signal motif (Figure 3). No cell internalization was observed for either protein to HER2- cells. This shows the differences in cytotoxicity (Table 3; Figure 2) between the KDEL variant and the ancestral variant between these two variants is probably not related to any significant change in target cell binding and/or cell entry.

### Determining the In Vivo Effects of the Cytotoxic Protein αHER2scFv::SLT-1A Using Animal Models

Animal models are used to determine the *in vivo* effects of the cytotoxic protein αHER2scFv::SLT-1A on HER2+ neoplastic cells. Various mice strains are used to test the effect of the cytotoxic protein after intravenous administration on xenograft tumors in mice resulting from the injection into those mice of human neoplastic cells which express HER2 on their cell surfaces.

### Example 2. A CD38-targeted, cytotoxic protein derived from Shiga-like toxin 1 A Subunit with a carboxy-terminal endoplasmic reticulum signal motif (αCD38scFv::SLT-1A::KDEL)

The cytotoxic protein of this example αCD38scFv::SLT-1A::KDEL comprises a single-chain, variable fragment, binding region capable of binding CD38 with high affinity combined with a Shiga toxin A Subunit fragment and a carboxy-terminal KDEL motif.

### Construction. Production, and Purification of the Cytotoxic Protein αCD38scFv::SLT-1A::KDEL

In this example, the Shiga toxin effector region was derived from the A subunit of Shiga-like Toxin 1 (SLT-1A). A polynucleotide was obtained that encoded amino acids 1-251 of SLT-1A (Cheung M et al., Mol Cancer 9: 28 (2010)). An immunoglobulin-type binding region αCD38scFv was derived from the monoclonal antibody anti-CD38 HB7 (Peng et al., Blood 101: 2557-62 (2003); *see also* GenBank Accession BD376144, National Center for Biotechnology Information, U.S.) such that a single-chain variable fragment (scFv) is created with the two immunoglobulin variable regions (V_{L} and V_{H}) separated by a linker known in the art.

In this example, the binding region and Shiga toxin effector region were linked together to form a fusion protein. In this example, a polynucleotide encoding the binding region αCD38scFv comprising amino acids 1-241 of SEQ ID NO:8 was cloned in frame with a polynucleotide encoding a "murine hinge" derived from a murine IgG3 molecule (or other linkers known to the skilled person) and in frame with a polynucleotide encoding Shiga toxin effector region SLT-1A comprising amino acids 1-251 of SEQ ID NO:1. In certain experiments, the full-length coding sequence of the cytotoxic protein of this example began with a polynucleotide encoding a Strep-tag® to facilitate detection and purification. The polynucleotide sequence encoding the cytotoxic protein αCD38scFv::SLT-1A::KDEL of this example was codon optimized for efficient expression in *E. coli* using services from DNA 2.0, Inc. (Menlo Park, CA, U.S.).

A fusion protein was produced by expressing the polynucleotide encoding the cytotoxic protein αCD38scFv::SLT-1A::KDEL (SEQ ID NO:8). Expression of the αCD38scFv::SLT-1A::KDEL cytotoxic protein was accomplished using both bacterial and cell-free, protein translation systems.

In this example of αCD38scFv::SLT-1A::KDEL production by an *E. coli* expression system, the polynucleotide "insert" sequence encoding αCD38scFv::SLT-1A::KDEL was cloned into the pTxb1 vector (New England Biolabs, Ipswich, MA, U.S.) using standard procedures to produce a polynucleotide sequence encoding the cytotoxic protein αCD38scFv::SLT-1A::KDEL ligated in frame to polynucleotide sequences encoding the amino-terminal intein of the vector. The plasmid insert polynucleotide sequence was verified by Sanger sequencing (Functional Biosciences, Madison, WI, U.S.) and transformed into T7 Shuffle® cells (New England Biolabs, Ipswich, MA, U.S.). The αCD38scFv::SLT-1A::KDEL protein was produced and purified according to the IMPACT™ (Intein Mediated Purification with an Affinity Chitin-binding Tag) system manual (New England Biolabs, Ipswich, MA, U.S.). Purification was accomplished using standard techniques known in the art, such as using immobilized targets of the Strep-tag® or the binding region.

In this example of αCD38scFv::SLT-1A::KDEL production by a cell-free, protein translation system, the polynucleotide "insert" sequence encoding αCD38scFv::SLT-1A::KDEL was cloned into the pIVEX2.3 vector with a stop codon directly after the coding region using the In-Fusion® HD Cloning Kit (Clonetech, Mountain View, CA, U.S.) according to the manufacturer's instructions. The plasmid insert polynucleotide sequence was verified by Sanger sequencing (Functional Biosciences, Madison, WI, U.S.). αCD38scFv::SLT-1A::KDEL protein was produced using the rapid translation system 5 Prime™ RTS 100 *E. coli* Disulfide Kit (5 Prime, Gaithersburg, MD, U.S.) according to the manufacturer's instructions. Purification was accomplished using standard techniques known in the art, such as using immobilized targets of the Strep-tag® or the binding region.

### Determining the Maximum Specific Binding (Bₘₐₓ) and Equilibrium Binding Constant (K_{D}) of αCD38scFv::SLT-1A::KDEL Binding Target Cell Types

The binding characteristics of the αCD38scFv::SLT-1A::KDEL protein produced as described above were determined by a fluorescence-based, flow-cytometry assay. Samples containing CD38 positive (+) cells and CD38 negative (-) cells were suspended in 1X PBS+1%BSA and incubated for 1 hour at 4°C with 100 µL of various dilutions of the αCD38scFv::SLT-1A::KDEL protein to be assayed. The highest concentrations of αCD38scFv::SLT-1A::KDEL protein was selected to lead to saturation of the binding reaction. After the one hour incubation, cell samples were washed twice with IX PBS+1%BSA. The cell samples were incubated for 1 hour at 4°C with 100 µL of IX PBS+1%BSA containing 0.3 µg of anti-Strep-tag® mAb-FITC (# A01736-100, Genscript, Piscataway, NJ, U.S.).

The cell samples were next washed twice with 1X PBS+1%BSA, resuspended in 200 µL of IX PBS and subjected to fluorescence-based, flow cytometry. The mean fluorescence intensity (MFI) data for all the samples was obtained by gating the data using a FITC-only sample as a negative control. Graphs were plotted of MFI versus "concentration of cells" using Prism software (GraphPad Software, San Diego, CA, U.S.). Using the Prism software function of one-site binding [Y = Bₘₐₓ^{∗}X/(K_{D} + X)] under the heading binding-saturation, the Bₘₐₓ and K_{D} were calculated using baseline corrected data. Abs values were corrected for background by subtracting the Abs values measured for wells containing only PBS. Bₘₐₓ is the maximum specific binding reported in MFI. K_{D} is the equilibrium binding constant, reported in nM.

The Bₘₐₓ for αCD38scFv::SLT-1A::KDEL binding to CD38+ cells was measured to be about 100,000 MFI with a K_{D} of about 13 nM (Table 4). This result was similar to the Bₘₐₓ for the αCD38scFv::SLT-1A protein, which lacked a KDEL signal motif, binding to CD38+ cells which was measured to be about 110,000 MFI with a K_{D} of about 17 nM (Table 4). Neither protein bound to CD38- cells. This shows that the improved cytotoxicity of the KDEL variant is probably not related to a change in the immunoglobulin-derived domain's target cell binding properties.

**Table 4. Addition of KDEL Had No Effect on Binding Characteristics: Representative values for Bₘₐₓ and K_{D} for αCD38scFv::SLT-1A::KDEL as compared to its ancestor which lacked a KDEL signal motif**

| | | Target Positive Cells | |
|---|---|---|---|
| Cytotoxic Protein | **target biomolecule** | **Bₘₐₓ (MFI)** | **K_{D} (nM)** |
| **αCD38scFv::SLT-1A::KDEL** | CD38 | 102,000 | 12.5 |
| **αCD38scFv::SLT-1A** | CD38 | 108,000 | 17.0 |

### Determining the Half-Maximal Inhibitory Concentration (IC₅₀) of αCD38scFv::SLT-1A::KDEL to Eukaryotic Ribosomes In Vitro

The ribosome inactivation capabilities of αCD38scFv::SLT-1A::KDEL was determined in a cell-free, *in vitro* protein translation assay using the TNT® Quick Coupled Transcription/Translation Kit (L1170 Promega, Madison, WI, U.S.). The kit includes Luciferase T7 Control DNA and TNT® Quick Master Mix. The ribosome activity reaction was prepared according to the manufacturer's instructions to create "TNT" reaction mixtures.

A series of 10-fold dilutions of αCD38scFv::SLT-1A::KDEL to be tested were prepared in appropriate buffer and a series of identical TNT reaction mixture components were created for each dilution of αCD38scFv::SLT-1A::KDEL. Each sample in the dilution series of αCD38scFv::SLT-1A::KDEL protein was combined with each of the TNT reaction mixtures along with the Luciferase T7 Control DNA. The test samples were incubated for 1.5 hours at 30°C. After the incubation, Luciferase Assay Reagent (E1483 Promega, Madison, WI, U.S.) was added to all test samples and the amount of luciferase protein translation was measured by luminescence according to the manufacturer instructions. The level of translational inhibition was determined by non-linear regression analysis of log-transformed concentrations of total protein versus relative luminescence units. Using statistical software (GraphPad Prism, San Diego, CA, U.S.), the half maximal inhibitory concentration (IC₅₀) value was calculated for each sample. Then, the data were normalized by calculating the "percent of SLT-1A-only control protein" using the Prism software function of log(inhibitor) vs. response (three parameters) [Y = Bottom + ((Top-Bottom) / (1+10^(X-LogIC50)))] under the heading dose-response-inhibition. The IC₅₀ for experimental proteins and SLT-1A-only control protein were calculated. The percent of SLT-1A-only control protein was calculated by [(IC50 of SLT-1A control protein / IC50 of experimental protein) x 100].

The inhibitory effect of αCD38scFv::SLT-1A::KDEL on cell-free protein synthesis was strong. Dose dependence experiments determined that the IC₅₀ of αCD38scFv::SLT-1A::KDEL on protein synthesis in this cell-free assay was about 15 pM or 99% of the SLT-1A-only positive control (Table 5). This result was similar to the IC₅₀ for the αCD38scFv::SLT-1A protein, which lacked a KDEL signal motif, which was measured to be about 15 pM or 101% of the SLT-1A-only positive control (Table 5). This showed the improved cytotoxicity of the KDEL comprising variant is probably not related to a significant change in the Shiga toxin A Subunit enzymatic activity.

**Table 5. Addition of KDEL Had No Effect on Ribosome Inactivation: Representative half-maximal inhibitory concentration (IC₅₀) for αCD38scFv::SLT-1A::KDEL as compared to its ancestor which lacked a KDEL signal motif**

| Cytotoxic Protein | **IC₅₀ (pM)** | **IC₅₀ of SLT-1A only positive control (pM)** | **Percent of IC₅₀ of SLT-1A control protein** |
|---|---|---|---|
| **αCD38scFv::SLT-1A::KDEL** | 15.1 | 15.0 | 99% |
| **αCD38scFv::SLT-1A** | 14.8 | 15.0 | 101% |

### Determining the Selective Cytotoxicity and Half-Maximal Cytotoxic Concentration (CD₅₀) of αCD38scFv::SLT-1A::KDEL Using a Cell-Kill Assay

The cytotoxicity characteristics of αCD38scFv::SLT-1A::KDEL was determined by the following cell-kill assay. This assay determines the capacity of a cytotoxic protein to kill cells expressing the target biomolecule of cytotoxic protein's binding region as compared to cells that do not express the target biomolecule. Cells were plated (2 x 10³ cells per well) in 20 µL cell culture medium in 384-well plates. The αCD38scFv::SLT-1A::KDEL protein was diluted either 5-fold or 10-fold in a IX PBS and 5 µL of the dilutions were added to the cells. Control wells containing only cell culture medium were used for baseline correction. The cell samples were incubated with αCD38scFv::SLT-1A::KDEL, or just buffer, for 3 days at 37°C and in an atmosphere of 5% CO₂. The total cell survival or percent viability was determined using a luminescent readout using the CellTiter-Glo® Luminescent Cell Viability Assay (G7573 Promega Madison, WI, U.S.) according to the manufacturer's instructions. The Percent Viability of experimental wells was calculated using the following equation: (Test RLU - Average Media RLU) / (Average Cells RLU - Average Media RLU) ^{∗} 100. Log polypeptide concentration versus Percent Viability was plotted in Prism (GraphPad Prism, San Diego, CA, U.S.) and log (inhibitor) vs. normalized response (variable slope) analysis was used to determine the half-maximal cytotoxic concentration (CD₅₀) value for αCD38scFv::SLT-1A::KDEL.

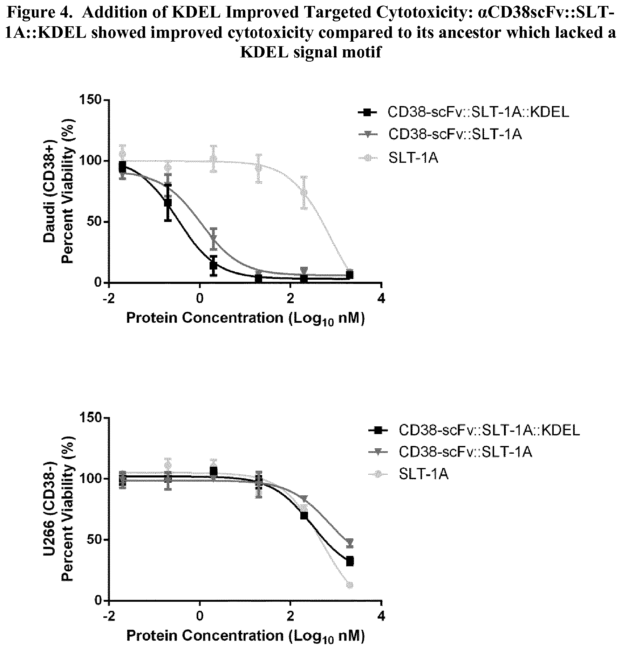

The CD₅₀ of the αCD38scFv::SLT-1A::KDEL protein was measured to be about 0.2-1 nM for CD38 + cells, depending on the cell line, as compared to 300 nM for a CD38- cell line, which was relatively similar to the CD₅₀ for the SLT-1A-only negative control (Table 6; Figure 4). The CD₅₀ of the αCD38scFv::SLT-1A::KDEL was about 300-2000 fold greater for cells not physically coupled with the extracellular target biomolecule CD38 as compared to cells that were physically coupled with the extracellular target biomolecule CD38, *e.g.* expressed CD38 on their cell surface.

Dose dependence experiments determined that the CD₅₀ for the αCD38scFv::SLT-1A, which lacked a KDEL signal motif, was about 0.8-3.2 nM (Table 6; Figure 4), which was 2-4 fold greater (less cytotoxic) than the same protein with a carboxy-terminal KDEL signal motif. This exemplifies the effect of the addition of a KDEL signal motif on both cytotoxicity and selective cytotoxicity. The differences in cell-kill for these cytotoxic proteins were not predictable based on the *in vitro* results for either ribosome inactivation or target cell-binding characteristics.

**Table 6. Addition of KDEL Improved Cytotoxicity: Representative half-maximal cytotoxic concentrations (CD₅₀) for αCD38scFv::SLT-1A::KDEL compared to its ancestor which lacked a KDEL signal motif**

| | | **CD₅₀ (nM)** | | |
|---|---|---|---|---|
| **Cell Line** | **CD38 status** | **αCD38scFv::SLT-1A::KDEL** | **αCD38scFv::SLT-1A** | **SLT-1A only negative control** |
| Daudi | positive | 0.32 | 1.10 | 750 |
| Raji | positive | 0.98 | 3.20 | 1,100 |
| ST486 | positive | 0.18 | 0.75 | 940 |
| BC-1 | positive | 0.59 | 1.10 | 510 |
| U226 | negative | 300.00 | 670.00 | 490 |

### Determining Cell Internalization of SLT-1A::αCD38scFv Using Immunofluorescence

The ability of αCD38scFv::SLT-1, the ancestor of αCD38scFv::SLT-1::KDEL ancestor which lacked a KDEL signal motif, to enter target cells was investigated using standard immunocytochemical techniques known in the art. Briefly, 0.8 x 10⁶ cells of each cell type (Raji (CD38+), Daudi (CD38+), and U266 (CD38-)) were harvested and suspended in 50 µL of cell culture medium containing a cocktail of protease inhibitors (*e.g.* P1860 Sigma-Aldrich Co., St. Louis, MO, U.S.) and human Fc receptor protein to reduce non-specific immunofluorescent staining. Next, 100 nM of the cytotoxic protein to be analyzed was added to the cells, and the cells were incubated at 37°C for 1 hour to allow for intoxication to progress. Then, cells were "fixed" and "permeabilized" using the Cytofix/Cytoperm™ Kit (BD Biosciences San Diego, CA, U.S.) according to the manufacturer's instructions. The Shiga toxin effector region was "stained" using a mouse monoclonal antibody (mouse IgG anti-Shiga toxin 1 Subunit A, BEI NR-867 BEI Resources, Manassas, VA, U.S.). The mouse monoclonal antibody localization was then detected with the Alexa Fluor® 555 Monoclonal Antibody Labeling Kit (Life Technologies, Carlsbad, CA, U.S.) according to manufacturer's instructions.

In this assay, cell surface binding and cell internalization was observed in CD38+ cells for αCD38scFv::SLT-1. No cell internalization was observed for either protein to CD38- cells. This showed the differences in cytotoxicity (Table 6; Figure 4) between the cytotoxic protein KDEL variant and its ancestor was probably not caused by significant changes to the ability of αCD38scFv::SLT-1 to bind target cells and become internalized.

### Determining the In Vivo Effects of the Cytotoxic Protein αCD38scFv::SLT-1::KDEL Using Animal Models

Animal models are used to determine the *in vivo* effects of the cytotoxic protein αCD38scFv::SLT-1::KDEL on CD38+ neoplastic cells and/or immune cells. Various mice strains are used to test the effect of the cytotoxic protein after intravenous administration on xenograft tumors in mice resulting from the injection into those mice of human neoplastic and/or human immune cells which express CD38 on their cell surfaces.

### Example 3. A CD19-targeted, cytotoxic protein derived from the A Subunit of Shiga-like toxin-1 (αCD19scFv::SLT-1A::KDEL)

The cytotoxic protein of this example αCD19scFv::SLT-1A::KDEL comprises a single-chain, variable fragment, binding region capable of binding CD19 with high affinity combined with a Shiga toxin A Subunit fragment and a carboxy-terminal KDEL motif.

### Construction, Production, and Purification of the Cytotoxic Protein αCD19scFv::SLT-1A::KDEL

First, a Shiga toxin effector region and an immunoglobulin-type binding region were designed or selected. In this example, the Shiga toxin effector region was derived from the A subunit of Shiga-like Toxin 1 (SLT-1A). A polynucleotide was obtained that encoded amino acids 1-251 of SLT-1A (Cheung M et al., Mol Cancer 9: 28 (2010). An immunoglobulin-type binding region αCD19scFv was derived from the humanized monoclonal antibody anti-CD 19 4G7 (Peipp M et al., J Immunol Methods 285: 265-80 (2004) and references therein) such that a single-chain variable fragment (scFv) is created with the two immunoglobulin variable regions (V_{L} and V_{H}) separated by a linker known in the art.

Second, the binding region and Shiga toxin effector region were linked together to form a fusion protein. In this example, a polynucleotide encoding the immunoglobulin-type binding region αCD19scFv comprising amino acids 1-250 of SEQ ID NO:12 was cloned in frame with a polynucleotide encoding a linker, such as a "murine hinge" derived from a murine IgG3 molecule (or other linkers known to the skilled person) and in frame with a polynucleotide encoding the Shiga toxin effector region from SLT-1A (amino acids 1-251 of SEQ ID NO:1) fused to a carboxy-terminal KDEL motif. The polynucleotide sequence encoding the cytotoxic protein αCD19scFv::SLT-1A::KDEL of this example was codon optimized for efficient expression in *E. coli* using services from DNA 2.0, Inc. (Menlo Park, CA, U.S.).

A fusion protein was produced by expressing the polynucleotide encoding the cytotoxic protein αCD19scFv::SLT-1A::KDEL (SEQ ID NO:12). Expression of the αCD19scFv::SLT-1A::KDEL cytotoxic protein was accomplished using a bacterial system known in the art.

In this example of αCD19scFv::SLT-1A::KDEL production by an *E. coli* expression system, the polynucleotide "insert" sequence encoding αCD19scFv::SLT-1A::KDEL was cloned into the pTxb1 vector (New England Biolabs, Ipswich, MA, U.S.) using standard procedures to produce a polynucleotide sequence encoding the cytotoxic protein αCD19scFv::SLT-1A::KDEL ligated in frame to polynucleotide sequences encoding the amino-terminal intein of the vector. The plasmid insert polynucleotide sequence was verified by Sanger sequencing (Functional Biosciences, Madison, WI, U.S.) and transformed into T7 Shuffle® cells (New England Biolabs, Ipswich, MA, U.S.). The αCD19scFv::SLT-1A::KDEL protein was produced and purified according to the IMPACT™ (Intein Mediated Purification with an Affinity Chitin-binding Tag) system manual (New England Biolabs, Ipswich, MA, U.S.). Purification was accomplished using standard techniques known in the art, such as affinity chromatography.

### Determining the Half-Maximal Inhibitory Concentration (IC₅₀) of αCD19scFv::SLT-1A::KDEL to Eukaryotic Ribosomes in vitro

The ribosome inactivation capabilities of αCD19scFv::SLT-1A::KDEL was determined in a cell-free, *in vitro* protein translation assay using the TNT® Quick Coupled Transcription/Translation Kit (L1170 Promega, Madison, WI, U.S.). The kit includes Luciferase T7 Control DNA and TNT® Quick Master Mix. The ribosome activity reaction was prepared according to the manufacturer's instructions to create "TNT" reaction mixtures.

A series of 10-fold dilutions of αCD19scFv::SLT-1A::KDEL to be tested was prepared in appropriate buffer and a series of identical TNT reaction mixture components was created for each dilution of αCD19scFv::SLT-1A::KDEL. Each sample in the dilution series of αCD19scFv::SLT-1A::KDEL protein was combined with each of the TNT reaction mixtures along with the Luciferase T7 Control DNA. The test samples were incubated for 1.5 hours at 30°C. After the incubation, Luciferase Assay Reagent (E1483 Promega, Madison, WI, U.S.) was added to all test samples and the amount of luciferase protein translation was measured by luminescence according to the manufacturer instructions. The level of translational inhibition was determined by non-linear regression analysis of log-transformed concentrations of total protein versus relative luminescence units. Using statistical software (GraphPad Prism, San Diego, CA, U.S.), the half maximal inhibitory concentration (IC₅₀) value was calculated for each sample.

The inhibitory effect of αCD19scFv::SLT-1A::KDEL on cell-free protein synthesis was strong. Dose dependence experiments determined that the IC₅₀ of αCD19scFv::SLT-1A::KDEL on protein synthesis in this cell-free assay was about 2.7 pM (Table 7). This result was not substantially different from the IC₅₀ for the protein αCD19scFv::SLT-1A, which lacked a KDEL signal motif, that was measured to be about 3.2 pM or equivalent of the SLT-1A-only positive control (Table 7). This shows that the improved cytotoxicity of the KDEL variant is probably not related to any significant perturbation of Shiga toxin A Subunit enzymatic activity.

**Table 7. Addition of KDEL Had No Significant Effect on Ribosome Inactivation: Representative half-maximal inhibitory concentrations (IC₅₀) for αCD19scFv::SLT-1A::KDEL as compared to αCD19scFv::SLT-1A**

| Cytotoxic Protein | **IC₅₀ (pM)** | **IC₅₀ of SLT-1A only positive control (pM)** |
|---|---|---|
| **αCD19scFv::SLT-1A::KDEL** | 2.7 | 7.9 |
| **αCD19scFv::SLT-1A** | 3.2 | 7.9 |

### Determining the Selective Cytotoxicity and Half-Maximal Cytotoxic Concentration (CD₅₀) of αCD19scFv::SLT-1A::KDEL Using a Cell-Kill Assay

The cytotoxicity characteristics of αCD19scFv::SLT-1A::KDEL was determined by the following cell-kill assay. This assay determines the capacity of a cytotoxic protein to kill cells expressing the target biomolecule of its immunoglobulin-type binding region as compared to the SLT-1A protein that does not possess the binding region. Cells were plated (2 x 10³ cells per well) in 20 µL of cell culture medium in 384-well plates. The αCD19scFv::SLT-1A::KDEL protein was diluted 10-fold in buffer and 5 µL of the dilutions were added to the cells. Control wells containing only cell culture medium were used for baseline correction. The cell samples were incubated with αCD19scFv::SLT-1A::KDEL, or just buffer, for 3 days at 37°C and in an atmosphere of 5% CO₂. The total cell survival or percent viability was determined using a luminescent readout using the CellTiter-Glo® Luminescent Cell Viability Assay (G7573 Promega Madison, WI, U.S.) according to the manufacturer's instructions. The Percent Viability of experimental wells was calculated using the following equation: (Test RLU - Average Media RLU) / (Average Cells RLU - Average Media RLU) ^{∗} 100. Log polypeptide concentration versus Percent Viability was plotted in Prism (GraphPad Prism, San Diego, CA, U.S.) and log (inhibitor) vs. normalized response (variable slope) analysis was used to determine the half-maximal cytotoxic concentration (CD₅₀) value for αCD19scFv::SLT-1A::KDEL.

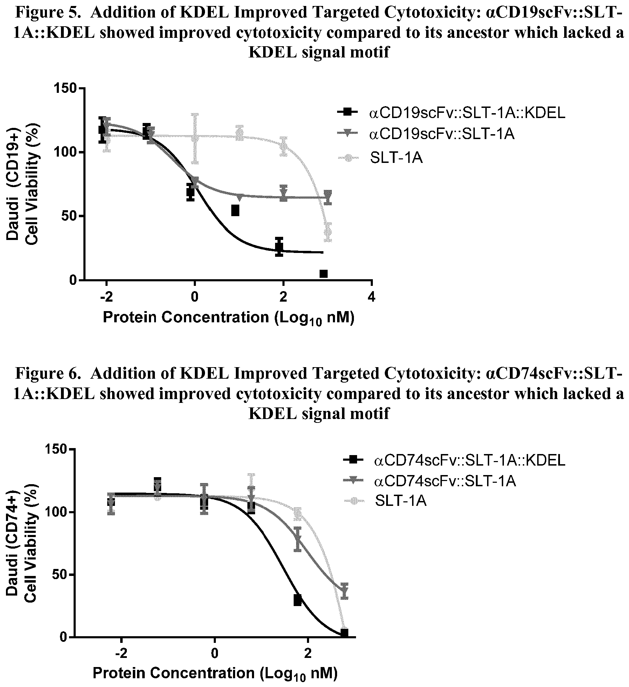

Dose dependence experiments determined that the CD₅₀ of the αCD19scFv::SLT-1A::KDEL protein was about 0.15 nM for CD19 + Daudi cells (Table 8, Figure 5). The CD₅₀ for the SLT-1A-only negative control and the same protein lacking the KDEL motif, αCD19scFv::SLT-1A, could not be accurately measured based on the shape of the curve. These results exemplify the effect of adding a KDEL signal motif on both cytotoxicity and selective cytotoxicity (Table 8, Figure 5). The differences in cell-kill for these cytotoxic proteins were not predictable based on the *in vitro* results for protein synthesis inhibition and are not expected to be predictable based on target cell-binding characteristics.

**Table 8. KDEL Motif Affected Cytotoxicity: Representative half-maximal cytotoxic concentrations (CD₅₀) of αCD19scFv::SLT-1A::KDEL as compared to αCD19scFv::SLT-1A**

| | | **CD₅₀ (nM)** | | |
|---|---|---|---|---|
| **Cell Line** | **CD19 status** | **αCD19scFv::SLT-1A::KDEL** | **αCD19scFv::SLT-1A** | **SLT-1A only negative control** |
| Daudi | positive | 0.15 | NC | NC |

| | | | | |
|---|---|---|---|---|
| * "NC" denotes not calculable. | | | | |

### Determining the In Vivo Effects of the Cytotoxic Protein αCD19scFv::SLT-1A::KDEL Using Animal Models

Animal models are used to determine the *in vivo* effects of the cytotoxic protein αCD19scFv::SLT-1A::KDEL on neoplastic and/or immune cells. Various mice strains are used to test the effect of the cytotoxic protein after intravenous administration on xenograft tumors in mice resulting from the injection into those mice of human neoplastic and/or human immune cells which express CD19 on their cell surfaces.

### Example 4. A CD74-targeted, cytotoxic protein derived from the A Subunit of Shiga-like toxin-1 (αCD74scFv::SLT-1A::KDEL)

A fourth exemplary cytotoxic protein comprises a Shiga toxin A Subunit fragment recombined with a single-chain, variable fragment, binding region capable of binding CD74 with high affinity. This fourth exemplary cytotoxic protein is capable of selectively killing cells that express CD74 on their surface.

### Construction. Production, and Purification of the Cytotoxic Protein αCD74scFv::SLT-1A::KDEL

First, a Shiga toxin effector region and an immunoglobulin-type binding region were designed or selected. In this example, the Shiga toxin effector region was derived from the A subunit of Shiga-like Toxin 1 (SLT-1A). A polynucleotide was obtained that encoded amino acids 1-251 of SLT-1A (Cheung M et al., Mol Cancer 9: 28 (2010). An immunoglobulin-type binding region αCD74scFv was derived from the humanized monoclonal antibody anti-CD74, Milatuzumab (Sapra P et al., Clin Cancer Res 11: 5257-64 (2005) and references therein) such that a single-chain variable fragment (scFv) is created with the two immunoglobulin variable regions (V_{L} and V_{H}) separated by a linker known in the art.

Second, the binding region and Shiga toxin effector region were linked together to form a fusion protein. In this example, a polynucleotide encoding the immunoglobulin-type binding region αCD74scFv comprising amino acids 1-251 of SEQ ID NO:16 was cloned in frame with a polynucleotide encoding a linker, such as a "murine hinge" derived from a murine IgG3 molecule (or other linkers known to the skilled person) and in frame with a polynucleotide encoding the Shiga toxin effector region from SLT-1A (amino acids 1-251 of SEQ ID NO:1) fused to a carboxy-terminal KDEL motif. The polynucleotide sequence encoding the cytotoxic protein αCD74scFv::SLT-1A::KDEL of this example was codon optimized for efficient expression in *E. coli* using services from DNA 2.0, Inc. (Menlo Park, CA, U.S.).

A fusion protein was produced by expressing the polynucleotide encoding the cytotoxic protein αCD74scFv::SLT-1A::KDEL (SEQ ID NO:16). Expression of the αCD74scFv::SLT-1A::KDEL cytotoxic protein was accomplished using a bacterial system known in the art.

In this example of αCD74scFv::SLT-1A::KDEL production by an *E. coli* expression system, the polynucleotide "insert" sequence encoding αCD74scFv::SLT-1A::KDEL was cloned into the pTxb1 vector (New England Biolabs, Ipswich, MA, U.S.) using standard procedures to produce a polynucleotide sequence encoding the cytotoxic protein αCD74scFv::SLT-1A::KDEL ligated in frame to polynucleotide sequences encoding the amino-terminal intein of the vector. The plasmid insert polynucleotide sequence was verified by Sanger sequencing (Functional Biosciences, Madison, WI, U.S.) and transformed into T7 Shuffle® cells (New England Biolabs, Ipswich, MA, U.S.). The αCD74scFv::SLT-1A::KDEL protein was produced and purified according to the IMPACT™ (Intein Mediated Purification with an Affinity Chitin-binding Tag) system manual (New England Biolabs, Ipswich, MA, U.S.). Purification was accomplished using standard techniques known in the art, such as affinity chromatography.

### Determining the Half-Maximal Inhibitory Concentration (IC₅₀) of αCD74scFv::SLT-1A::KDEL to Eukaryotic Ribosomes in vitro

The ribosome inactivation capabilities of αCD74scFv::SLT-1A::KDEL was determined in a cell-free, *in vitro* protein translation assay using the TNT® Quick Coupled Transcription/Translation Kit (L1170 Promega, Madison, WI, U.S.). The kit includes Luciferase T7 Control DNA and TNT® Quick Master Mix. The ribosome activity reaction was prepared according to the manufacturer's instructions to create "TNT" reaction mixtures.

A series of 10-fold dilutions of αCD74scFv::SLT-1A::KDEL to be tested was prepared in appropriate buffer and a series of identical TNT reaction mixture components was created for each dilution of αCD74scFv::SLT-1A::KDEL. Each sample in the dilution series of αCD74scFv::SLT-1A::KDEL protein was combined with each of the TNT reaction mixtures along with the Luciferase T7 Control DNA. The test samples were incubated for 1.5 hours at 30°C. After the incubation, Luciferase Assay Reagent (E1483 Promega, Madison, WI, U.S.) was added to all test samples and the amount of luciferase protein translation was measured by luminescence according to the manufacturer instructions. The level of translational inhibition was determined by non-linear regression analysis of log-transformed concentrations of total protein versus relative luminescence units. Using statistical software (GraphPad Prism, San Diego, CA, U.S.), the half maximal inhibitory concentration (IC₅₀) value was calculated for each sample.

The inhibitory effect of αCD74scFv::SLT-1A::KDEL on cell-free protein synthesis was strong. Dose dependence experiments determined that the IC₅₀ of αCD74scFv::SLT-1A::KDEL on protein synthesis in this cell-free assay was about 3.1 pM (Table 9). This result was not substantially different from the IC₅₀ for the protein αCD74scFv::SLT-1A, which lacks a KDEL motif, and was measured to be about 3.6 pM or equivalent of the SLT-1A-only positive control (Table 9).

**Table 9. KDEL Motif Had No Effect on Ribosome Inactivation: Representative half-maximal inhibitory concentrations (IC₅₀) for αCD74scFv::SLT-1A::KDEL as compared to αCD74scFv::SLT-1A**

| Cytotoxic Protein | **IC₅₀ (pM)** | **IC₅₀ of SLT-1A only positive control (pM)** |
|---|---|---|
| **αCD74scFv::SLT-1A::KDEL** | 3.1 | 7.9 |
| **αCD74scFv::SLT-1A** | 3.6 | 7.9 |

### Determining the Selective Cytotoxicity and Half-Maximal Cytotoxic Concentration (CD₅₀) of αCD74scFv::SLT-1A::KDEL Using a Cell-Kill Assay

The cytotoxicity characteristics of αCD74scFv::SLT-1A::KDEL was determined by the following cell-kill assay. This assay determines the capacity of a cytotoxic protein to kill cells expressing the target biomolecule of its immunoglobulin-type binding region as compared to the SLT-1A protein that does not possess the binding region. Cells were plated (2 x 10³ cells per well) in 20 µL cell culture medium in 384-well plates. The αCD74scFv::SLT-1A::KDEL protein was diluted 10-fold in buffer and 5 µL of the dilutions were added to the cells. Control wells containing only cell culture medium were used for baseline correction. The cell samples were incubated with αCD74scFv::SLT-1A::KDEL, or just buffer, for 3 days at 37°C and in an atmosphere of 5% CO₂. The total cell survival or percent viability was determined using a luminescent readout using the CellTiter-Glo® Luminescent Cell Viability Assay (G7573 Promega Madison, WI, U.S.) according to the manufacturer's instructions. The Percent Viability of experimental wells was calculated using the following equation: (Test RLU - Average Media RLU) / (Average Cells RLU - Average Media RLU) ^{∗} 100. Log polypeptide concentration versus Percent Viability was plotted in Prism (GraphPad Prism, San Diego, CA, U.S.) and log (inhibitor) vs. response (three parameter) analysis was used to determine the half-maximal cytotoxic concentration (CD₅₀) value for αCD74scFv::SLT-1A::KDEL.

Dose dependence experiments determined that the CD₅₀ of the αCD74scFv::SLT-1A::KDEL protein was about 29.9 nM for CD74 + Daudi cells (Table 10, Figure 6). The CD₅₀ for the SLT-1A-only negative control was 2026 nM and the same protein lacking the KDEL motif, αCD74scFv::SLT-1A, was 95.3 nM (Table 10, Figure 6). These results exemplify the effect of adding a KDEL signal motif on both cytotoxicity and selective cytotoxicity. The differences in cell-kill for these cytotoxic proteins were not predictable based on the *in vitro* results for protein synthesis inhibition and are not expected to be predictable based on target cell-binding characteristics.

**Table 10. KDEL motif Affected Cytotoxicity: Representative half-maximal cytotoxic concentrations (CD₅₀) of αCD74scFv::SLT-1A::KDEL as compared to αCD74scFv::SLT-1A**

| | | **CD₅₀ (nM)** | | |
|---|---|---|---|---|
| **Cell Line** | **CD74 status** | **αCD74scFv::SLT-1A::KDEL** | **αCD74scFv::SLT-1A** | **SLT-1A only negative control** |
| Daudi | positive | 29.97 | 95.3 | 2026 |

### Determining the In Vivo Effects of the Cytotoxic Protein αCD74scFv::SLT-1A::KDEL Using Animal Models

Animal models are used to determine the *in vivo* effects of the cytotoxic protein αCD74scFv::SLT-1A::KDEL on neoplastic and/or immune cells. Various mice strains are used to test the effect of the cytotoxic protein after intravenous administration on xenograft tumors in mice resulting from the injection into those mice of human neoplastic and/or immune cells which express CD74 on their cell surfaces.

### Example 5. A HER2-targeted, cytotoxic protein derived from Shiga-like toxin 1 A Subunit (αHER2scFv::SLT-1A::RDEL)

In this example, αHER2scFv::SLT-1A::RDEL was created and tested just as in Example 1, except for the carboxy-terminus ended with the peptide sequence RDEL instead of KDEL. The cell binding characteristics of the cytotoxic protein αHER2scFv::SLT-1A::RDEL was determined by a fluorescence-based flow cytometry assay as described in Example 1. The Bₘₐₓ for the αHER2scFv::SLT-1A::RDEL cytotoxic protein was measured to be about 130,000 MFI with a K_{D} of about 167 nM, whereas there was no meaningful binding to HER2- cells observed in this assay. The inhibitory effect of αHER2scFv::SLT-1A::RDEL on cell-free protein synthesis was strong as shown by the measurement of an IC₅₀ for αHER2scFv::SLT-1A::RDEL on protein synthesis of about 36 pM.

The cytotoxicity characteristics of αHER2scFv::SLT-1A::RDEL are determined by the general cell-kill assay as described above in the previous examples using HER2+ cells. In addition, the selective cytotoxicity characteristics of αHER2scFv::SLT-1A::RDEL are determined by the same general cell-kill assay using HER2- cells as a comparison to the HER2+ cells. The CD₅₀ of the cytotoxic protein of this example is approximately 0.01-100 nM for HER2+ cells depending on the cell line. The CD₅₀ of the cytotoxic protein is approximately 10-10,000 fold greater (less cytotoxic) for cells not expressing HER2 on a cellular surface as compared to cells which do express HER2 on a cellular surface

### Example 6. A cytotoxic protein derived from the A Subunit of Shiga-like toxin-1 and the antibody αEpstein-Barr-antigen

In this example, the Shiga toxin effector region is derived from the A subunit of Shiga-like Toxin 1 (SLT-1A). An immunoglobulin-type binding region αEpstein-Barr-antigen is derived from a monoclonal antibody against an Epstein Barr antigen (Fang C et al., J Immunol Methods 287: 21-30 (2004)), which comprises an immunoglobulin-type binding region capable of binding a human cell infected by the Epstein-Barr virus or a transformed cell expressing an Epstein-Barr antigen. The Epstein-Barr antigen is expressed on multiple cell types, such as cells infected by an Epstein-Barr virus and cancer cells (*e.g*. lymphoma and nasopharnygeal cancer cells). In addition, Epstein-Barr infection is associated with other diseases, *e.g*., multiple sclerosis.

### Construction, Production, and Purification of the Cytotoxic Protein αEpsteinBarr::SLT-1A::KDEL

The immunoglobulin-type binding region αEpstein-Barr-antigen and Shiga toxin effector region are linked together, and a carboxy-terminal KDEL is added to form a protein. For example, a fusion protein is produced by expressing a polynucleotide encoding the αEpstein-Barr-antigen-binding protein αEpsteinBarr::SLT-1A::KDEL. Expression of the αEpsteinBarr::SLT-1A::KDEL cytotoxic protein is accomplished using either bacterial and/or cell-free, protein translation systems as described in the previous examples.

### Determining the In Vitro Characteristics of the Cytotoxic Protein αEpsteinBarr::SLT-1A::KDEL

The binding characteristics of the cytotoxic protein of this example for Epstein-Barr antigen positive cells and Epstein-Barr antigen negative cells is determined by a fluorescence-based, flow-cytometry assay as described above in the previous examples. The Bₘₐₓ for αEpsteinBarr::SLT-1A::KDEL to Epstein-Barr antigen positive cells is measured to be approximately 50,000-200,000 MFI with a K_{D} within the range of 0.01-100 nM, whereas there is no significant binding to Epstein-Barr antigen negative cells in this assay.

The ribosome inactivation abilities of the αEpsteinBarr::SLT-1A::KDEL cytotoxic protein is determined in a cell-free, *in vitro* protein translation as described above in the previous examples. The inhibitory effect of the cytotoxic protein of this example on cell-free protein synthesis is significant. The IC₅₀ of αEpsteinBarr::SLT-1A::KDEL on protein synthesis in this cell-free assay is approximately 0.1-100 pM.

### Determining the Cytotoxicity of the Cytotoxic Protein SLT-1A::αEpsteinBarr::KDEL Using a Cell-Kill Assay

The cytotoxicity characteristics of αEpsteinBarr::SLT-1A::KDEL are determined by the general cell-kill assay as described above in the previous examples using Epstein-Barr antigen positive cells. In addition, the selective cytotoxicity characteristics of αEpsteinBarr::SLT-1A::KDEL are determined by the same general cell-kill assay using Epstein-Barr antigen negative cells as a comparison to the Epstein-Barr antigen positive cells. The CD₅₀ of the cytotoxic protein of this example is approximately 0.01-100 nM for Epstein-Barr antigen positive cells depending on the cell line. The CD₅₀ of the cytotoxic protein is approximately 10-10,000 fold greater (less cytotoxic) for cells not expressing the Epstein-Barr antigen on a cellular surface as compared to cells which do express the Epstein-Barr antigen on a cellular surface.

### Determining the In Vivo Effects of the Cytotoxic Protein αEpsteinBarr::SLT-1A::KDEL Using Animal Models

Animal models are used to determine the *in vivo* effects of the cytotoxic protein αEpsteinBarr::SLT-1A::KDEL on neoplastic cells. Various mice strains are used to test the effect of the cytotoxic protein after intravenous administration on xenograft tumors in mice resulting from the injection into those mice of human neoplastic cells which express Epstein-Barr antigens on their cell surfaces.

### Example 7. A cytotoxic protein derived from the A Subunit of Shiga-like toxin-1 and the antibody αLeishmania-antigen

In this example, the Shiga toxin effector region is derived from the A subunit of Shiga-like Toxin 1 (SLT-1A). An immunoglobulin-type binding region αLeishmania-antigen is derived from an antibody generated, using techniques known in the art, to a cell-surface Leishmania antigen present on human cells harboring an intracellular trypanosomatid protozoa (*see* Berman J, Dwyer, Clin Exp Immunol 44: 342-348 (1981); Kenner J et al., J Cutan Pathol 26: 130-6 (1999); Silveira T et al., Int J Parasitol 31: 1451-8 (2001)).

### Construction, Production, and Purification of the Cytotoxic Protein SLT-1A::αLeishmania::KDEL

The immunoglobulin-type binding region α-Leishmania-antigen and Shiga toxin effector region are linked together, and a carboxy-terminal KDEL is added to form a protein. For example, a fusion protein is produced by expressing a polynucleotide encoding the Leishmania-antigen-binding protein SLT-1A::αLeishmania::KDEL. Expression of the SLT-1A::αLeishmania::KDEL cytotoxic protein is accomplished using either bacterial and/or cell-free, protein translation systems as described in the previous examples.

### Determining the In Vitro Characteristics of the Cytotoxic Protein SLT-1A::αLeishmania::KDEL

The binding characteristics of the cytotoxic protein of this example for Leishmania antigen positive cells and Leishmania antigen negative cells is determined by a fluorescence-based, flow-cytometry assay as described above in the previous examples. The Bₘₐₓ for SLT-1A::αLeishmania::KDEL to Leishmania antigen positive cells is measured to be approximately 50,000-200,000 MFI with a K_{D} within the range of 0.01-100 nM, whereas there is no significant binding to Leishmania antigen negative cells in this assay.

The ribosome inactivation abilities of the SLT-1A::αLeishmania::KDEL cytotoxic protein is determined in a cell-free, *in vitro* protein translation as described above in the previous examples. The inhibitory effect of the cytotoxic protein of this example on cell-free protein synthesis is significant. The IC₅₀ of SLT-1A::αLeishmania::KDEL on protein synthesis in this cell-free assay is approximately 0.1-100 pM.

### Determining the Cytotoxicity of the Cytotoxic Protein SLT-1A::αLeishmania::KDEL Using a Cell-Kill Assay

The cytotoxicity characteristics of SLT-1A::αLeishmania::KDEL are determined by the general cell-kill assay as described above in the previous examples using Leishmania antigen positive cells. In addition, the selective cytotoxicity characteristics of SLT-1A::αLeishmania::KDEL are determined by the same general cell-kill assay using Leishmania antigen negative cells as a comparison to the Leishmania antigen positive cells. The CD₅₀ of the cytotoxic protein of this example is approximately 0.01-100 nM for Leishmania antigen positive cells depending on the cell line. The CD₅₀ of the cytotoxic protein is approximately 10-10,000 fold greater (less cytotoxic) for cells not expressing the Leishmania antigen on a cellular surface as compared to cells which do express the Leishmania antigen on a cellular surface.

### Example 8. A cytotoxic protein derived from the A Subunit of Shiga-like toxin-1 and immunoglobulin-type binding region αNeurotensin-Receptor

In this example, the Shiga toxin effector region is derived from the A subunit of Shiga-like Toxin 1 (SLT-1A). An immunoglobulin-type binding region αNeurotensin-Receptor is derived from the DARPin™ (GenBank Accession: 2P2C_R) or a monoclonal antibody (Ovigne J et al., Neuropeptides 32: 247-56 (1998)) which binds the human neurotensin receptor. The neurotensin receptor is expressed by various cancer cells, such as breast cancer, colon cancer, lung cancer, melanoma, and pancreatic cancer cells.

### Construction. Production, and Purification of the Cytotoxic Protein SLT-1A::αNeurotensinR::KDEL

The immunoglobulin-type binding region αNeurotensinR and Shiga toxin effector region are linked together, and a carboxy-terminal KDEL is added to form a protein. For example, a fusion protein is produced by expressing a polynucleotide encoding the neurotensin-receptor-binding protein SLT-1A::αNeurotensinR::KDEL. Expression of the SLT-1A::αNeurotensinR::KDEL cytotoxic protein is accomplished using either bacterial and/or cell-free, protein translation systems as described in the previous examples.

### Determining the In Vitro Characteristics of the Cytotoxic Protein SLT-1A::αNeurotensinR::KDEL

The binding characteristics of the cytotoxic protein of this example for neurotensin receptor positive cells and neurotensin receptor negative cells is determined by a fluorescence-based, flow-cytometry assay as described above in the previous examples. The Bₘₐₓ for SLT-1A::αNeurotensinR::KDEL to neurotensin receptor positive cells is measured to be approximately 50,000-200,000 MFI with a K_{D} within the range of 0.01-100 nM, whereas there is no significant binding to neurotensin receptor negative cells in this assay.

The ribosome inactivation abilities of the SLT-1A::αNeurotensinR::KDEL cytotoxic protein is determined in a cell-free, *in vitro* protein translation as described above in the previous examples. The inhibitory effect of the cytotoxic protein of this example on cell-free protein synthesis is significant. The IC₅₀ of SLT-1A::αNeurotensinR::KDEL on protein synthesis in this cell-free assay is approximately 0.1-100 pM.

### Determining the Cytotoxicity of the Cytotoxic Protein SLT-1A::αNeurotensinR::KDEL Using a Cell-Kill Assay

The cytotoxicity characteristics of SLT-1A::αNeurotensinR::KDEL are determined by the general cell-kill assay as described above in the previous examples using neurotensin receptor positive cells. In addition, the selective cytotoxicity characteristics of SLT-1A::αNeurotensinR::KDEL are determined by the same general cell-kill assay using neurotensin receptor negative cells as a comparison to the neurotensin receptor positive cells. The CD₅₀ of the cytotoxic protein of this example is approximately 0.01-100 nM for neurotensin receptor positive cells depending on the cell line. The CD₅₀ of the cytotoxic protein is approximately 10-10,000 fold greater (less cytotoxic) for cells not expressing neurotensin receptor on a cellular surface as compared to cells which do express neurotensin receptor on a cellular surface.

### Determining the In Vivo Effects of the Cytotoxic Protein SLT-1A::αNeurotensinR::KDEL Using Animal Models

Animal models are used to determine the *in vivo* effects of the cytotoxic protein SLT-1A::αNeurotensinR::KDEL on neoplastic cells. Various mice strains are used to test the effect of the cytotoxic protein after intravenous administration on xenograft tumors in mice resulting from the injection into those mice of human neoplastic cells which express neurotensin receptors on their cell surfaces.

### Example 9. A cytotoxic protein derived from the A Subunit of Shiga-like toxin-1 and an immunoglobulin-type binding region αEGFR

In this example, the Shiga toxin effector region is derived from the A subunit of Shiga-like Toxin 1 (SLT-1A). The binding region αEGFR is derived from the AdNectin™ (GenBank Accession: 3QWQ_B), the Affibody™ (GenBank Accession: 2KZI_A; U.S. patent 8,598,113), or an antibody, all of which bind one or more human epidermal growth factor receptors. The expression of epidermal growth factor receptors are associated with human cancer cells, such as, *e.g.,* lung cancer cells, breast cancer cells, and colon cancer cells.

### Construction, Production, and Purification of the Cytotoxic Protein SLT-1A::αEGFR::KDEL

The immunoglobulin-type binding region αEGFR and Shiga toxin effector region are linked together, and a carboxy-terminal KDEL is added to form a protein. For example, a fusion protein is produced by expressing a polynucleotide encoding the EGFR binding protein SLT-1A::αEGFR::KDEL. Expression of the SLT-1A::αEGFR::KDEL cytotoxic protein is accomplished using either bacterial and/or cell-free, protein translation systems as described in the previous examples.

### Determining the In Vitro Characteristics of the Cytotoxic Protein SLT-1A::αEGFR::KDEL

The binding characteristics of the cytotoxic protein of this example for EGFR+ cells and EGFR- cells is determined by a fluorescence-based, flow-cytometry assay as described above in the previous examples. The Bₘₐₓ for SLT-1A::αEGFR::KDEL to EGFR+ cells is measured to be approximately 50,000-200,000 MFI with a K_{D} within the range of 0.01-100 nM, whereas there is no significant binding to EGFR- cells in this assay.

The ribosome inactivation abilities of the SLT-1A::αEGFR::KDEL cytotoxic protein is determined in a cell-free, *in vitro* protein translation as described above in the previous examples. The inhibitory effect of the cytotoxic protein of this example on cell-free protein synthesis is significant. The IC₅₀ of SLT-1A::αEGFR::KDEL on protein synthesis in this cell-free assay is approximately 0.1-100 pM.

### Determining the Cytotoxicity of the Cytotoxic Protein SLT-1A::αEGFR::KDEL Using a Cell-Kill Assay

The cytotoxicity characteristics of SLT-1A::αEGFR::KDEL are determined by the general cell-kill assay as described above in the previous examples using EGFR+ cells. In addition, the selective cytotoxicity characteristics of SLT-1A::αEGFR::KDEL are determined by the same general cell-kill assay using EGFR- cells as a comparison to the Leishmania antigen positive cells. The CD₅₀ of the cytotoxic protein of this example is approximately 0.01-100 nM for EGFR+ cells depending on the cell line. The CD₅₀ of the cytotoxic protein is approximately 10-10,000 fold greater (less cytotoxic) for cells not expressing EGFR on a cellular surface as compared to cells which do express EGFR on a cellular surface.

### Determining the In Vivo Effects of the Cytotoxic Protein SLT-1A::αEGFR::KDEL Using Animal Models

Animal models are used to determine the *in vivo* effects of the cytotoxic protein SLT-1A::αEGFR::KDEL on neoplastic cells. Various mice strains are used to test the effect of the cytotoxic protein after intravenous administration on xenograft tumors in mice resulting from the injection into those mice of human neoplastic cells which express EGFR(s) on their cell surfaces.

### Example 10. A cytotoxic protein derived from the A Subunit of Shiga-like toxin-1 and the antibody αCCR5

In this example, the Shiga toxin effector region is derived from the A subunit of Shiga-like Toxin 1 (SLT-1A). An immunoglobulin-type binding region αCCR5 is derived from a monoclonal antibody against human CCR5 (CD195) (Bernstone L et al., Hybridoma 31: 7-19 (2012)). CCR5 is predominantly expressed on T-cells, macrophages, dendritic cells, and microglia. In addition, CCR5 plays a role in the pathogenesis and spread of the Human Immunodeficiency Virus (HIV).

### Construction. Production, and Purification of the Cytotoxic Protein SLT-1A::αCCR5::KDEL

The immunoglobulin-type binding region αCCR5 and Shiga toxin effector region are linked together, and a carboxy-terminal KDEL is added to form a protein. For example, a fusion protein is produced by expressing a polynucleotide encoding the αCCR5-binding protein SLT-1A::αCCRS::KDEL. Expression of the SLT-1A::αCCR5::KDEL cytotoxic protein is accomplished using either bacterial and/or cell-free, protein translation systems as described in the previous examples.

### Determining the In Vitro Characteristics of the Cytotoxic Protein SLT-1A::αCCR5

The binding characteristics of the cytotoxic protein of this example for CCR5+ cells and CCR5- cells is determined by a fluorescence-based, flow-cytometry assay as described above in the previous examples. The Bₘₐₓ for SLT-1A::αCCR5::KDEL to CCR5+ positive cells is measured to be approximately 50,000-200,000 MFI with a K_{D} within the range of 0.01-100 nM, whereas there is no significant binding to CCR5- cells in this assay.

The ribosome inactivation abilities of the SLT-1A::αCCR5::KDEL cytotoxic protein is determined in a cell-free, *in vitro* protein translation as described above in the previous examples. The inhibitory effect of the cytotoxic protein of this example on cell-free protein synthesis is significant. The IC₅₀ of SLT-1A::αCCR5::KDEL on protein synthesis in this cell-free assay is approximately 0.1-100 pM.

### Determining the Cytotoxicity of the Cytotoxic Protein SLT-1A::αCCR5::KDEL Using a Cell-Kill Assay

The cytotoxicity characteristics of SLT-1A::αCCR5::KDEL are determined by the general cell-kill assay as described above in the previous examples using CCR5+ cells. In addition, the selective cytotoxicity characteristics of SLT-1A::αCCR5::KDEL are determined by the same general cell-kill assay using CCR5- cells as a comparison to the CCR5+ cells. The CD₅₀ of the cytotoxic protein of this example is approximately 0.01-100 nM for CCR5+ cells depending on the cell line. The CD₅₀ of the cytotoxic protein is approximately 10-10,000 fold greater (less cytotoxic) for cells not expressing CCR5 on a cellular surface as compared to cells which do express CCR5 on a cellular surface.

### Determining the In Vivo Effects of the Cytotoxic Protein SLT-1A::αCCR5::KDEL Using Animal Models

Animal models are used to determine the *in vivo* effects of the cytotoxic protein SLT-1A::αCCR5::KDEL on depleting T-cells from donor materials (*see* Tsirigotis P et al., Immunotherapy 4: 407-24 (2012)). Non-human primates are used to determine *in vivo* effects of SLT-1A::αCCR5. Graft-versus-host disease is analyzed in rhesus macaques after kidney transplantation when the donated organs are pretreated with SLT-1A::αCCR5::KDEL (*see* Weaver T et al., Nat Med 15: 746-9 (2009)). *In vivo* depletion of peripheral blood T lymphocytes in cynomolgus primates is observed after parenteral administration of different doses of SLT-1A::αCCR5::KDEL. The use of SLT-1A::αCCR5::KDEL to block HIV infection is tested by giving an acute dose of SLT-1A::αCCR5::KDEL to non-human primates in order to severely deplete circulating T-cells upon exposure to a simian immunodeficiency virus (SIV) (*see* Sellier P et al., PLoS One 5: e10570 (2010)).

### Example 11. A cytotoxic protein derived from the A Subunit of Shiga toxin and an anti-Env immunoglobulin domain

In this example, the Shiga toxin effector region is derived from the A subunit of Shiga toxin (Stx-A). An immunoglobulin-type binding region αEnv is derived from existing antibodies that bind HIV envelope glycoprotein (Env), such as GP41, GP120, GP140, or GP160 (*see e.g.* Chen W et al., J Mol Bio 382: 779-89 (2008); Chen W et al., Expert Opin Biol Ther 13: 657-71 (2013); van den Kerkhof T et al., Retrovirology 10: 102 (2013) or from antibodies generated using standard techniques (*see* Prabakaran et al., Front Microbiol 3: 277 (2012)). Envs are HIV surface proteins that are also displayed on the cell surfaces of HIV-infected cells during HIV replication. Although Envs are expressed in infected cells predominantly in endosomal compartments, sufficient amounts of Envs could be present on a cell surface to be targeted by a highly potent cytotoxic protein. In addition, Env-targeting cytotoxic proteins might bind HIV virions and enter newly infected cells during the fusion of virions with a host cell.

Because HIV displays a high rate of mutation, it is preferable to use an immunoglobulin domain that binds a functional constrained part of an Env, such as shown by broadly neutralizing antibodies that bind Envs from multiple strains of HIV (van den Kerkhof T et al., Retrovirology 10: 102 (2013)). Because the Envs present on an infected cell's surface are believed to present sterically restricted epitopes (Chen W et al., J Virol 88: 1125-39 (2014), it is preferable to use binding regions smaller than 100 kD and ideally smaller than 25 kD, such as fragments of sdAbs like V_{H}H domains.

### Construction, Production, and Purification of the Cytotoxic Protein αEnv::SLT-1A::KDEL

The immunoglobulin-type binding region αEnv and Shiga toxin effector region are linked together, and a carboxy-terminal KDEL is added to form a cytotoxic protein. For example, a fusion protein is produced by expressing a polynucleotide encoding the αEnv-binding protein SLT-1A::αEnv::KDEL. Expression of the SLT-1A::αEnv::KDEL cytotoxic protein is accomplished using either bacterial and/or cell-free, protein translation systems as described in the previous examples.

### Determining the In Vitro Characteristics of the Cytotoxic Protein SLT-1A::αEnv::KDEL

The binding characteristics of the cytotoxic protein of this example for Env+ cells and Env- cells is determined by a fluorescence-based, flow-cytometry assay as described above in the previous examples. The Bₘₐₓ for SLT-1A::αEnv::KDEL to Env+ positive cells is measured to be approximately 50,000-200,000 MFI with a K_{D} within the range of 0.01-100 nM, whereas there is no significant binding to Env- cells in this assay.

The ribosome inactivation abilities of the SLT-1A::αEnv::KDEL cytotoxic protein is determined in a cell-free, *in vitro* protein translation as described above in the previous examples. The inhibitory effect of the cytotoxic protein of this example on cell-free protein synthesis is significant. The IC₅₀ of SLT-1A::αEnv::KDEL on protein synthesis in this cell-free assay is approximately 0.1-100 pM.

### Determining the Cytotoxicity of the Cytotoxic Protein SLT-1A::αEnv::KDEL Using a Cell-Kill Assay

The cytotoxicity characteristics of SLT-1A::αEnv::KDEL are determined by the general cell-kill assay as described above in the previous examples using Env+ cells. In addition, the selective cytotoxicity characteristics of SLT-1A::αEnv::KDEL are determined by the same general cell-kill assay using Env- cells as a comparison to the Env+ cells. The CD₅₀ of the cytotoxic protein of this example is approximately 0.01-100 nM for Env+ cells depending on the cell line and/or the HIV strain used to infect the cells to make them Env+. The CD₅₀ of the cytotoxic protein is approximately 10-10,000 fold greater (less cytotoxic) for cells not expressing Env on a cellular surface as compared to cells which do express Env on a cellular surface.

### Determining the In Vivo Effects of the Cytotoxic Protein SLT-1A::αEnv::KDEL Using Animal Models

The use of SLT-1A::αEnv::KDEL to inhibit HIV infection is tested by administering SLT-1A::αEnv::KDEL to simian immunodeficiency virus (SIV) infected non-human primates (*see* Sellier P et al., PLoS One 5: e10570 (2010)).

### Example 12. A cytotoxic protein derived from the A Subunit of Shiga-like toxin-1 and the antibody αUL18

In this example, the Shiga toxin effector region is derived from the A subunit of Shiga-like Toxin 1 (SLT-1A). An immunoglobulin-type binding region αUL18 is derived from an antibody generated, using techniques known in the art, to the cell-surface cytomegalovirus protein UL18, which is present on human cells infected with cytomegalovirus (Yang Z, Bjorkman P, Proc Natl Acad Sci USA 105: 10095-100 (2008)). The human cytomegalovirus infection is associated with various cancers and inflammatory disorders.

### Construction. Production, and Purification of the Cytotoxic Protein SLT-1A::αUL18::KDEL

The immunoglobulin-type binding region αUL18 and Shiga toxin effector region are linked together, and a carboxy-terminal KDEL is added to form a protein. For example, a fusion protein is produced by expressing a polynucleotide encoding the αUL18-binding protein SLT-1A::αUL18::KDEL. Expression of the SLT-1A::αUL18::KDEL cytotoxic protein is accomplished using either bacterial and/or cell-free, protein translation systems as described in the previous examples.

### Determining the In Vitro Characteristics of the Cytotoxic Protein SLT-1A::αUL18::KDEL

The binding characteristics of the cytotoxic protein of this example for cytomegalovirus protein UL18 positive cells and cytomegalovirus protein UL18 negative cells is determined by a fluorescence-based, flow-cytometry assay as described above in the previous examples. The Bₘₐₓ for SLT-1A::αUL18::KDEL to cytomegalovirus protein UL18 positive cells is measured to be approximately 50,000-200,000 MFI with a K_{D} within the range of 0.01-100 nM, whereas there is no significant binding to cytomegalovirus protein UL18 negative cells in this assay.

The ribosome inactivation abilities of the SLT-1A::αUL18::KDEL cytotoxic protein is determined in a cell-free, *in vitro* protein translation as described above in the previous examples. The inhibitory effect of the cytotoxic protein of this example on cell-free protein synthesis is significant. The IC₅₀ of SLT-1A::αUL18::KDEL on protein synthesis in this cell-free assay is approximately 0.1-100 pM.

### Determining the Cytotoxicity of the Cytotoxic Protein SLT-1A::αUL18::KDEL Using a Cell-Kill Assay

The cytotoxicity characteristics of SLT-1A::αUL18::KDEL are determined by the general cell-kill assay as described above in the previous examples using cytomegalovirus protein UL18 positive cells. In addition, the selective cytotoxicity characteristics of SLT-1A::αUL18::KDEL are determined by the same general cell-kill assay using cytomegalovirus protein UL18 negative cells as a comparison to the cytomegalovirus protein UL18 positive cells. The CD₅₀ of the cytotoxic protein of this example is approximately 0.01-100 nM for cytomegalovirus protein UL18 positive cells depending on the cell line. The CD₅₀ of the cytotoxic protein is approximately 10-10,000 fold greater (less cytotoxic) for cells not expressing the cytomegalovirus protein UL18 on a cellular surface as compared to cells which do express the cytomegalovirus protein UL18 on a cellular surface.

### Example 13. A cytotoxic protein derived from the A Subunit of Shiga-like toxin-1 and the antibody αhelminth-intestinal-antigen

In this example, the Shiga toxin effector region is derived from the A subunit of Shiga-like Toxin 1 (SLT-1A). An immunoglobulin-type binding region αhelminth-intestinal-antigen is derived from an antibody generated, using techniques known in the art, to the helminth ortholog of a human transferrin receptor (*see e.g.* the nematode gene gcp-2.1 UniProt G8JYE4_CAEEL; Rosa B et al., Mol Cell Proteomics M114.046227 (2015)).

### Construction, Production, and Purification of the Cytotoxic Protein αHelminth-Intestinal-Antigen::SLT-1A::KDEL

The immunoglobulin-type binding region αhelminth-intestinal-antigen and Shiga toxin effector region are linked, and a carboxy-terminal KDEL is added to form a protein. For example, a fusion protein is produced by expressing a polynucleotide encoding the αhelminth-intestinal-antigen-binding protein αhelminth-intestinal-antigen::SLT-1A::KDEL. Expression of the αhelminth-intestinal-antigen::SLT-1A::KDEL cytotoxic protein is accomplished using either bacterial and/or cell-free, protein translation systems as described in the previous examples.

### Determining the In Vitro Characteristics of the Cytotoxic Protein αHelminth-Intestinal-Antigen::SLT-1A::KDEL

The binding characteristics of the cytotoxic protein of this example is determined by a molecular binding assay known in the art using a purified recombinant target protein. The K_{D} for αhelminth-intestinal-antigen::SLT-1A::KDEL to target protein is measured to be approximately 100 nM, whereas there is no significant binding to a negative control protein (*e.g.* purified, recombinant, human transferrin receptor) in this assay.

The ribosome inactivation abilities of the αhelminth-intestinal-antigen::SLT-1A::KDEL cytotoxic protein is determined in a cell-free, *in vitro* protein translation as described above in the previous examples. The inhibitory effect of the cytotoxic protein of this example on cell-free protein synthesis is significant. The IC₅₀ of αhelminth-intestinal-antigen::SLT-1A::KDEL on protein synthesis in this cell-free assay is approximately 0.1-100 pM.

### Determining the Toxicity of the Cytotoxic Protein αHelminth-Intestinal-Antigen::SLT-1A::KDEL Using Helminths

The toxicity of αhelminth-intestinal-antigen::SLT-1A::KDEL to helminths is determined using model helminths (*see e.g.* Iatsenko I et al., Toxins 2050-63 (2014)). The helminth can be administered purified αhelminth-intestinal-antigen::SLT-1A::KDEL by soaking or alternatively by feeding the helminth with bacteria expressing the SLT-1A::αhelminth-intestinal-antigen fusion protein.

In addition, laboratory animals harboring helminths and/or displaying helminth related diseases are administered αhelminth-intestinal-antigen::SLT-1A::KDEL and monitored for reduction or elimination of helminths and/or associated symptoms of parasitic helminth(s).

### Example 14. Cytotoxic proteins targeting various cell types

In this example, the Shiga toxin effector region is derived from the A subunit of Shiga-like Toxin 1 (SLT-1A), Shiga toxin (StxA), and/or Shiga-like Toxin 2 (SLT-2A). A binding region is derived from the molecules chosen from column 1 of Table 11 and which binds the extracellular target biomolecule indicated in column 2 of Table 11. The exemplary proteins of this example are created with a carboxy-terminal KDEL-type signal motif using techniques known in the art and optionally linked with a detection promoting agent(s). The exemplary proteins of this example are tested as described in the previous examples using cells expressing the appropriate extracellular target biomolecules. The exemplary proteins of this example may be used, *e.g.,* to labeling subcellular compartments of target cells and to diagnose and treat diseases, conditions, and/or disorders indicated in column 3 of Table 11.

**Table 11. Various Binding Regions for Cell Targeting of Cytotoxic Proteins**

| Source of binding region | Extracellular target | Application(s) |
|---|---|---|
| alemtuzumab | CD52 | B-cell cancers, such as lymphoma and leukemia, and B-cell related immune disorders, such as autoimmune disorders |
| basiliximab | CD25 | T-cell disorders, such as prevention of organ transplant rejections, and some B-cell lineage cancers |
| brentuximab | CD30 | hematological cancers, B-cell related immune disorders, and T-cell related immune disorders |
| catumaxomab | EpCAM | various cancers, such as ovarian cancer, malignant ascites, gastric cancer |
| cetuximab | EGFR | various cancers, such as colorectal cancer and head and neck cancer |
| daclizumab | CD25 | B-cell lineage cancers and T-cell disorders, such as rejection of organ transplants |
| daratumumab | CD38 | hematological cancers, B-cell related immune disorders, and T-cell related immune disorders |
| dinutuximab | ganglioside GD2 | Various cancers, such as breast cancer, myeloid cancers, and neuroblastoma |
| efalizumab | LFA-1 (CD11a) | autoimmune disorders, such as psoriasis |
| ertumaxomab | HER2/neu | various cancers and tumors, such as breast cancer and colorectal cancer |
| gemtuzumab | CD33 | myeloid cancer or immune disorder |
| ibritumomab | CD20 | B-cell cancers, such as lymphoma and leukemia, and B-cell related immune disorders, such as autoimmune disorders |
| ipilimumab | CD152 | T-cell related disorders and various cancers, such as leukemia, melanoma |
| muromonab | CD3 | prevention of organ transplant rejections |
| natalizumab | integrin α4 | autoimmune disorders, such as multiple sclerosis and Crohn's disease |
| obinutuzumab | CD20 | B-cell cancers, such as lymphoma and leukemia, and B-cell related immune disorders, such as autoimmune disorders |
| ocaratuzumab | CD20 | B-cell cancers, such as lymphoma and leukemia, and B-cell related immune disorders, such as autoimmune disorders |
| ocrelizumab | CD20 | B-cell cancers, such as lymphoma and leukemia, and B-cell related immune disorders, such as autoimmune disorders |
| ofatumumab | CD20 | B-cell cancers, such as lymphoma and leukemia, and B-cell related immune disorders, such as autoimmune disorders |
| palivizumab | F protein of respiratory syncytial virus | treat respiratory syncytial virus |
| panitumumab | EGFR | various cancers, such as colorectal cancer and head and neck cancer |
| pertuzumab | HER2/neu | various cancers and tumors, such as breast cancer and colorectal cancer |
| pro 140 | CCR5 | HIV infection and T-cell disorders |
| ramucirumab | VEGFR2 | various cancers and cancer related disorders, such as solid tumors |
| rituximab | CD20 | B-cell cancers, such as lymphoma and leukemia, and B-cell related immune disorders, such as autoimmune disorders |
| tocilizumab or atlizumab | IL-6 receptor | autoimmune disorders, such as rheumatoid arthritis |
| tositumomab | CD20 | B-cell cancers, such as lymphoma and leukemia, and B-cell related immune disorders, such as autoimmune disorders |
| trastuzumab | HER2/neu | various cancers and tumors, such as breast cancer and colorectal cancer |
| ublituximab | CD20 | B-cell cancers, such as lymphoma and leukemia, and B-cell related immune disorders, such as autoimmune disorders |
| vedolizumab | integrin α4β7 | autoimmune disorders, such as Crohn's disease and ulcerative colitis |
| CD20 binding antibodies and scFv(s) | CD20 | B-cell cancers, such as lymphoma and leukemia, and B-cell related immune disorders, such as autoimmune disorders (*see e.g.* Geng S et al., Cell Mol Immunol 3: 439-43 (2006); Olafesn T et al., Protein Eng Des Sel 23: 243-9 (2010)) |
| CD22 binding scFv(s) | CD22 | B-cell cancers or B-cell related immune disorders (*see e.g.* Kawas S et al., MAbs 3: 479-86 (2011)) |
| CD25 binding scFv(s) | CD25 | various cancers of the B-cell lineage and immune disorders related to T-cells (*see e.g.* Muramatsu H et al., Cancer Lett 225: 225-36 (2005)) |
| CD30 binding monoclonal antibody(s) | CD30 | B-cell cancers or B-cell/T-cell related immune disorders (*see e.g.* Klimka A et al., Br J Cancer 83: 252-60 (2000)) |
| CD33 binding monoclonal antibody(s) | CD33 | myeloid cancer or immune disorder (*see e.g.* Benedict C et al., J Immunol Methods 201: 223-31 (1997)) |
| CD38 binding immunoglobulin domains | CD38 | hematological cancers, B-cell related immune disorders, and T-cell related immune disorders (*see e.g.* U.S. patent 8,153,765) |
| CD40 binding scFv(s) | CD40 | various cancers and immune disorders (*see e.g.* Ellmark P et al., Immunology 106: 456-63 (2002)) |
| CD52 binding monoclonal antibody(s) | CD52 | B-cell cancers, such as lymphoma and leukemia, and B-cell related immune disorders, such as autoimmune disorders (*see e.g.* U.S. Patent 7,910,104 B2) |
| CD56 binding monoclonal antibody(s) | CD56 | immune disorders and various cancers, such as lung cancer, Merkel cell carcinoma, myeloma (*see e.g.* Shin J et al., Hybridoma 18: 521-7 (1999)) |
| CD79 binding monoclonal antibody(s) | CD79 | B-cell cancers or B-cell related immune disorders (*see e.g.* Zhang L et al., Ther Immunol 2: 191-202 (1995)) |
| CD133 binding monoclonal antibodies and scFv(s) | CD133 | various cancers, hematologic malignancies, and immune disorders (*see e.g.* Bidlingmaier S et al., J Mol Med 86: 1025-32 (2008); Pavlon L et al., J Microsc 231: 374-83 (2008); Rappa G et al., Stem Cells 26: 3008-17 (2008); Swaminathan S et al., J Immunol Methods 361: 110-5 (2010); Wang J et al., Hybridoma 29: 241-9 (2010); Zhu X et al., Mol Cancer Ther 9: 2131-41 (2010); Xia J et al., Sci Rep 3: 3320 (2013)) |
| CD248 binding scFv(s) | CD248 | various cancers, such as inhibiting angiogenesis (*see e.g.* Zhao A et al., J Immunol Methods 363: 221-32 (2011)) |
| EpCAM binding monoclonal antibody(s) | EpCAM | various cancers, such as ovarian cancer, malignant ascites, gastric cancer (*see e.g.* Schanzer J et al., J Immunother 29: 477-88 (2006)) |
| PSMA binding monoclonal antibody(s) | PSMA | prostate cancer (*see e.g.* Frigerio B et al., Eur J Cancer 49: 2223-32 (2013)) |
| Eph-B2 binding monoclonal antibody(s) | Eph-B2 | various cancers such as colorectal cancer and prostate cancer (*see e.g.* Abéngozar M et al., Blood 119: 4565-76 (2012)) |
| Endoglin binding monoclonal antibody(s) | Endoglin | various cancers, such as breast cancer and colorectal cancers (*see e.g.* Völkel T et al., Biochim Biophys Res Acta 1663: 158-66 (2004)) |
| FAP binding monoclonal antibody(s) | FAP | various cancers, such as sarcomas and bone cancers (*see e.g.* Zhang J et al., FASEB J 27: 581-9 (2013)) |
| CEA binding antibody(s) and scFv(s) | CEA | various cancers, such as gastrointestinal cancer, pancreatic cancer, lung cancer, and breast cancer (*see e.g.* Neumaier M et al., Cancer Res 50: 2128-34 (1990); Pavoni E et al., BMC Cancer 6: 4 (2006); Yazaki P et al., Nucl Med Biol 35: 151-8 (2008); Zhao J et al., Oncol Res 17: 217-22 (2008)) |
| CD24 binding monoclonal antibody(s) | CD24 | various cancers, such as bladder cancer (*see e.g.* Kristiansen G et al., Lab Invest 90: 1102-16 (2010)) |
| LewisY antigen binding scFv(s) | LewisY antigens | various cancers, such as cervical cancer and uterine cancer (*see e.g.* Power B et al., Protein Sci 12: 734-47 (2003); monoclonal antibody BR96 Feridani A et al., Cytometry 71: 361-70 (2007)) |
| adalimumab | TNF-α | various cancers and immune disorders, such as Rheumatoid arthritis, Crohn's Disease, Plaque Psoriasis, Psoriatic Arthritis, Ankylosing Spondylitis, Juvenile Idiopathic Arthritis, Hemolytic disease of the newborn |
| afelimomab | TNF-α | various cancers and immune disorders |
| ald518 | IL-6 | various cancers and immune disorders, such as rheumatoid arthritis |
| anrukinzumab or ima-638 | IL-13 | various cancers and immune disorders |
| briakinumab | IL-12, IL-23 | various cancers and immune disorders, such as psoriasis, rheumatoid arthritis, inflammatory bowel diseases, multiple sclerosis |
| brodalumab | IL-17 | various cancers and immune disorders, such as inflammatory diseases |
| canakinumab | IL-1 | various cancers and immune disorders, such as rheumatoid arthritis |
| certolizumab | TNF-α | various cancers and immune disorders, such as Crohn's disease |
| fezakinumab | IL-22 | various cancers and immune disorders, such as rheumatoid arthritis, psoriasis |
| ganitumab | IGF-I | various cancers |
| golimumab | TNF-α | various cancers and immune disorders, such as rheumatoid arthritis, psoriatic arthritis, ankylosing spondylitis |
| infliximab | TNF-α | various cancers and immune disorders, such as rheumatoid arthritis, ankylosing spondylitis, psoriatic arthritis, psoriasis, Crohn's disease, ulcerative colitis |
| ixekizumab | IL-17A | various cancers and immune disorders, such as autoimmune diseases |
| mepolizumab | IL-5 | various immune disorders and cancers, such as B-cell cancers |
| nerelimomab | TNF-α | various cancers and immune disorders |
| olokizumab | IL6 | various cancers and immune disorders |
| ozoralizumab | TNF-α | inflammation |
| perakizumab | IL17A | various cancers and immune disorders, such as arthritis |
| placulumab | human TNF | various immune disorders and cancers |
| sarilumab | IL6 | various cancers and immune disorders, such as rheumatoid arthritis, ankylosing spondylitis |
| siltuximab | IL-6 | various cancers and immune disorders |
| sirukumab | IL-6 | various cancers and immune disorders, such as rheumatoid arthritis |
| tabalumab | BAFF | B-cell cancers |
| ticilimumab or tremelimumab | CTLA-4 | various cancers |
| tildrakizumab | IL23 | immunologically mediated inflammatory disorders |
| tnx-650 | IL-13 | various cancers and immune disorders, such as B-cell cancers |
| tocilizumab or atlizumab | IL-6 receptor | various cancers and immune disorders, such as rheumatoid arthritis |
| ustekinumab | IL-12, IL-23 | various cancers and immune disorders, such as multiple sclerosis, psoriasis, psoriatic arthritis |
| Various growth factors: VEGF, EGF1, EGF2, FGF | VEGFR, EGFR, FGFR | various cancer, such as breast cancer and colon cancer, and to inhibit vascularization |
| Various cytokines: IL-2, IL-6, IL-23, CCL2, BAFFs, TNFs, RANKL | IL-2R, IL-6R, IL-23R, CD80/CD86, TNFRSF13/TNF RSF17, TNFR | various immune disorders and cancers |
| Broadly neutralizing antibodies identified from patient samples | Influenza surface antigens (*e.g.* hemaglutinins and matrix protein 2) | viral infections (*see e.g.* Prabakaran et al., Front Microbiol 3: 277 (2012)) |
| Broadly neutralizing antibodies identified from patient samples | Coronavirus surface antigens | viral infections (*see e.g.* Prabakaran et al., Front Microbiol 3: 277 (2012)) |
| Various antibodies | Filovirus surface antigens (*e.g*. VP35, VP40, and glycoprotein) | viral infections (*see e.g.* Olinger G et al., Proc Natl Acad Sci USA 109: 18030-5 (2012); Pettitt J et al., Sci Transl Med 5: 199ra113 (2013); Stahelin R, Expert Opin Ther Targets 18: 115-20 (2014); Becquart P et al., PLoS One 9: e96360 (2014); Stahelin R, Fron Microbiol 5: 300 (2014); Tran E et al., J Virol 88: 10958-62 (2014); Murin C et al., Proc Natl Acad Sci USA 111: 17182-7 (2014)) |
| Broadly neutralizing antibodies identified from patient samples | Henipavirus surface antigens | viral infections (*see e.g.* Prabakaran et al., Front Microbiol 3: 277 (2012)) |
| Various antibodies including broadly neutralizing antibodies and scFvs | HIV surface antigens (*e.g.* matrix protein Map 17) | viral infections (*see e.g.* Kitidee K et al., BMC Biotechnol 10: 80 (2010); Yu L, Guan Y, Front Immunol 5: 250 (2014)) |
| Broadly neutralizing antibodies identified from patient samples | Influenza surface antigens (*e.g.* hemaglutinins and matrix protein 2) | viral infections (*see e.g.* Prabakaran et al., Front Microbiol 3: 277 (2012)) |

The present invention is defined in the appended claims. While some embodiments of the invention have been described by way of illustration, it will be apparent that the invention may be put into practice with many modifications, variations and adaptations, and with the use of numerous equivalents or alternative solutions that are within the scope of persons skilled in the art.

| Sequence Listing | | |
|---|---|---|
| ID Number | Text Description | Biological Sequence |
| SEQ ID NO:1 | Shiga-like toxin 1 Subunit A (SLT-1A) | |
| SEQ ID NO:2 | Shiga toxin Subunit A | |
| SEQ ID NO:3 | Shiga-like toxin 2 Subunit A (SLT-2A) | |
| SEQ ID NO:4 | αHER2scFv::SLT-1A::KDEL variant 1 | |
| | | |
| SEQ ID NO:5 | αHER2scFv::SLT-1A::KDEL variant 2 | |
| SEQ ID NO:6 | αHER2scFv::SLT-1A::KDEL variant 3 | |
| SEQ ID NO:7 | αHER2scFv::SLT-1A::KDEL variant 4 | |
| | | |
| SEQ ID NO:8 | αCD38scFv::SLT-1A::KDEL variant 1 | |
| SEQ ID NO:9 | αCD38scFv::SLT-1A::KDEL variant 2 | |
| SEQ ID NO:10 | αCD38scFv::SLT-1A::KDEL variant 3 | |
| | | |
| SEQ ID NO:11 | αCD38scFv::SLT-1A::KDEL variant 4 | |
| SEQ ID NO:12 | αCD 19scFv:: SLT-1A::KDEL variant 1 | |
| SEQ ID NO:13 | αCD 19scFv:: SLT-1A::KDEL variant 2 | |
| | | |
| SEQ ID NO:14 | αCD 19scFv:: SLT-1A::KDEL variant 3 | |
| SEQ ID NO:15 | αCD 19scFv:: SLT-1A::KDEL variant 4 | |
| SEQ ID NO:16 | αCD74scFv::SLT-1A::KDEL variant 1 | |
| | | |
| SEQ ID NO:17 | αCD74scFv::SLT-1A::KDEL variant 2 | |
| SEQ ID NO:18 | αCD74scFv::SLT-1A::KDEL variant 3 | |
| SEQ ID NO:19 | αCD74scFv::SLT-1A::KDEL variant 4 | |
| | | |
| SEQ ID NO:20 | αHER2-V_{H}H::SLT-1A::KDEL variant 1 | |
| SEQ ID NO:21 | αHER2-V_{H}H::SLT-1A::KDEL variant 2 | |
| SEQ ID NO:22 | αHER2-V_{H}H::SLT-1A::KDEL variant 3 | |
| SEQ ID NO:23 | αHER2-V_{H}H::SLT-1A::KDEL variant 4 | |
| | | |
| SEQ ID NO:24 | αHER2-V_{H}H::StxA: :KDEL variant 1 | |
| SEQ ID NO:25 | αHER2-V_{H}H::StxA: :KDEL variant 2 | |
| SEQ ID NO:26 | αHER2-V_{H}H::StxA: :KDEL variant 3 | |
| SEQ ID NO:27 | αHER2-V_{H}H::StxA: :KDEL variant 4 | |
| | | |
| SEQ ID NO:28 | αCD20-FN3::SLT-1A::KDEL | |
| SEQ ID NO:29 | StxA::αCD20-FN3::KDEL | |
| SEQ ID NO:30 | IL-2::SLT-1A::KDEL variant 1 | |
| SEQ ID NO:31 | IL-2::SLT-1A::KDEL variant 2 | |
| | | |
| SEQ ID NO:32 | IL-2::StxA::KDEL variant 1 | |
| SEQ ID NO:33 | IL-2::StxA::KDEL variant 2 | |

### CLAUSES

## Claims

1. A protein comprising:
a) a binding region capable of specifically binding at least one extracellular target biomolecule,
b) a Shiga toxin A Subunit effector region comprising a polypeptide capable of exhibiting at least one Shiga toxin function selected from the group consisting of: cellular internalization, subcellular routing, and cytotoxicity; wherein the polypeptide comprises or consists of an amino acid sequence that is at least 85% identical to an amino acid sequence selected from:
(i) amino acids 75 to 251 of SEQ ID NO:1, SEQ ID NO:2, or SEQ ID NO:3;
(ii) amino acids 1 to 241 of SEQ ID NO:1, SEQ ID NO:2, or SEQ ID NO:3;
(iii) amino acids 1 to 251 of SEQ ID NO:1, SEQ ID NO:2, or SEQ ID NO: 3; and
(iv) amino acids 1 to 261 of SEQ ID NO:1, SEQ ID NO:2, or SEQ ID NO:3; and
c) a carboxy-terminal endoplasmic reticulum retention/retrieval signal motif of a member of the KDEL family; wherein the protein is linked to an additional exogenous material for delivery into a cell said cell being physically coupled with the extracellular target biomolecule of said protein.

2. The protein of claim 1, wherein the additional exogenous material is selected from any one of: peptide, protein, cytotoxic agent, detection promoting agent, small molecule chemotherapeutic agent, and polynucleotide.

3. The protein of claim 2, wherein the additional exogenous material is a peptide or protein comprising an antigen; such as wherein the antigen is derived from a bacterial protein, a viral protein, a protein mutated or aberrantly expressed in cancer, or a T-cell complementary determining region.

4. The protein of any one of claims 1-3, wherein the binding region comprises a polypeptide selected from the group consisting of:
single-domain antibody fragment, single-chain variable fragment, antibody variable fragment, Fd fragment, antigen-binding fragment, fibronectin-derived 10^{th} fibronectin type III domain, tenascin type III domain, ankyrin repeat motif domain, low-density-lipoprotein-receptor-derived A-domain, lipocalin, Kunitz domain, Protein-A-derived Z domain, gamma-B crystallin-derived domain, ubiquitin-derived domain, Sac7d-derived polypeptide, Fyn-derived SH2 domain, miniprotein, and C-type lectin-like domain scaffold.

5. The protein of any one of claims 1-4, wherein the binding region is capable of binding to an extracellular target biomolecule selected from the group consisting of:
CD20, CD22, CD40, CD74, CD79, CD25, CD30, HER2/neu/ErbB2, EGFR, EpCAM, EphB2, prostate-specific membrane antigen, Cripto, CDCP1, endoglin, fibroblast activation protein, Lewis-Y, CD19, CD21, CS1/ SLAMF7, CD33, CD52, CD133, gpA33, mucin, TAG-72, tyrosine-protein kinase transmembrane receptor, carbonic anhydrase IX, folate binding protein, ganglioside GD2, ganglioside GD3, ganglioside GM2, ganglioside Lewis-Y2, VEGFR, Alpha Vbeta3, Alpha5beta1, ErbB1/EGFR, Erb3, c-MET, IGF1R, EphA3, TRAIL-R1, TRAIL-R2, RANK, tenascin, CD64, mesothelin, BRCA1, MART-1/MelanA, gp100, tyrosinase, TRP-1, TRP-2, MAGE-1, MAGE-3, GAGE-1/2, BAGE, RAGE, NY-ESO-1, CDK-4, beta-catenin, MUM-1, caspase-8, KIAA0205, HPVE6, SART-1, PRAME, carcinoembryonic antigen, prostate specific antigen, prostate stem cell antigen, human aspartyl (asparaginyl) beta-hydroxylase, EphA2, HER3/ErbB-3, MUC1, tyrosinase associated antigen, HPV-E7, Epstein-Barr virus antigen, Bcr-Abl, alpha-fetoprotein antigen, 17-A1, bladder tumor antigen, CD38, CD15, CD23, CD53, CD88, CD129, CD183, CD191, CD193, CD244, CD294, CD305, C3AR, FceRIa, galectin-9, PD-L1, mrp-14, Siglec-8, Siglec-10, CD49d, CD13, CD44, CD54, CD63, CD69, CD123, TLR4, FceRIa, IgE, CD107a, CD203c, CD14, CD68, CD80, CD86, CD105, CD115, F4/80, ILT-3, galectin-3, CD11a-c, GITRL, MHC class I molecule, MHC class II molecule, CD284-TLR4, CD107-Mac3, CD195-CCR5, HLA-DR, CD16/32, CD282-TLR2, CD11c, and any immunogenic fragment of any of the foregoing.

6. The protein of any one of claims 1-5, wherein the binding region comprises or consists of amino acids 2-241 of any one of SEQ ID NOs: 4-16.

7. The protein of any one of claims 1-6, wherein the carboxy-terminal endoplasmic reticulum retention/retrieval signal motif is selected from the group consisting of:
KDEL (SEQ ID NO:34), HDEF (SEQ ID NO:35), HDEL (SEQ ID NO:36), RDEF (SEQ ID NO:37), RDEL (SEQ ID NO:38), WDEL (SEQ ID NO:39), YDEL (SEQ ID NO:40), HEEF (SEQ ID NO:41), HEEL (SEQ ID NO:42), KEEL (SEQ ID NO:43), REEL (SEQ ID NO:44), KAEL (SEQ ID NO:45), KCEL (SEQ ID NO:46), KFEL (SEQ ID NO:47), KGEL (SEQ ID NO:48), KHEL (SEQ ID NO:49), KLEL (SEQ ID NO:50), KNEL (SEQ ID NO:51), KQEL (SEQ ID NO:52), KREL (SEQ ID NO:53), KSEL (SEQ ID NO:54), KVEL (SEQ ID NO:55), KWEL (SEQ ID NO:56), KYEL (SEQ ID NO:57), KEDL (SEQ ID NO:58), KIEL (SEQ ID NO:59), DKEL (SEQ ID NO:60), FDEL (SEQ ID NO:61), KDEF (SEQ ID NO:62), KKEL (SEQ ID NO:63), HADL (SEQ ID NO:64), HAEL (SEQ ID NO:65), HIEL (SEQ ID NO:66), HNEL (SEQ ID NO:67), HTEL (SEQ ID NO:68), KTEL (SEQ ID NO:69), HVEL (SEQ ID NO:70), NDEL (SEQ ID NO:71), QDEL (SEQ ID NO:72), REDL (SEQ ID NO:73), RNEL (SEQ ID NO:74), RTDL (SEQ ID NO:75), RTEL (SEQ ID NO:76), SDEL (SEQ ID NO:77), TDEL (SEQ ID NO:78), SKEL (SEQ ID NO:79), STEL (SEQ ID NO:80), and EDEL (SEQ ID NO:81).

8. The protein of any one of claims 1-7, wherein the Shiga toxin effector region comprises or consists of a sequence that is at least 95% identical to:
(i) amino acids 75 to 251 of SEQ ID NO:1, SEQ ID NO:2, or SEQ ID NO:3;
(ii) amino acids 1 to 241 of SEQ ID NO:1, SEQ ID NO:2, or SEQ ID NO:3;
(iii) amino acids 1 to 251 of SEQ ID NO:1, SEQ ID NO:2, or SEQ ID NO:3; or
(iv) amino acids 1 to 261 of SEQ ID NO:1, SEQ ID NO:2, or SEQ ID NO:3.

9. The protein of any one of claims 1-8, wherein the Shiga toxin effector region comprises a mutation relative to a naturally occurring A Subunit of a member of the Shiga toxin family which changes the enzymatic activity of the Shiga toxin effector region, the mutation selected from at least one amino acid residue deletion, insertion, or substitution; wherein the naturally occurring A Subunit of a member of the Shiga toxin family comprises or consists of the amino acid sequence of any one of SEQ ID NOs: 1-3.

10. The protein of claim 9, wherein the mutation reduces or eliminates cytotoxicity of the Shiga toxin effector region.

11. The protein of any one of claims 1-8, wherein the Shiga toxin effector region comprises or consists of:
(i) amino acids 75 to 251 of SEQ ID NO:1, SEQ ID NO:2, or SEQ ID NO:3;
(ii) amino acids 1 to 241 of SEQ ID NO:1, SEQ ID NO:2, or SEQ ID NO:3;
(iii) amino acids 1 to 251 of SEQ ID NO:1, SEQ ID NO:2, or SEQ ID NO:3; or
(iv) amino acids 1 to 261 of SEQ ID NO:1, SEQ ID NO:2, or SEQ ID NO:3.

12. The protein of any one of claims 1-11, whereby upon administration of the protein to a cell physically coupled with said extracellular target biomolecule, the protein is capable of causing death of the cell;
optionally whereby upon administration of the protein to a first population of cells whose members are physically coupled with said extracellular target biomolecule, and a second population of cells whose members are not physically coupled with said extracellular target biomolecule, the cytotoxic effect of the protein to members of said first population of cells relative to members of said second population of cells is at least 3-fold greater.

13. The protein of any one of claims 1-12 in the form of a homomultimer or heteromultimer.

14. The protein of any one of claims 1-13 in the form of a pharmaceutically acceptable salt or solvate.

15. A pharmaceutical composition comprising the protein of any one of claims 1-14 and at least one pharmaceutically acceptable excipient or carrier.

16. The pharmaceutical composition of claim 15, wherein the at least one pharmaceutically acceptable carrier includes a solvent, dispersion medium, coating such as lecithin, antimicrobial agent, isotonic agent, or absorption delaying agent; optionally wherein the antimicrobial agent is an antibacterial or antifungal agent.

17. A diagnostic composition comprising: the protein of any one of claims 1-14 and a detection promoting agent.

18. A kit comprising:
(i) the protein of any one of claims 1-14, the pharmaceutical composition of claim 15 or claim 16, or the diagnostic composition of claim 17; and
(ii) an additional reagent and/or pharmaceutical delivery device.

19. An *in vitro* method of delivering an exogenous material into a cell, the method comprising the step of contacting a cell with the protein of any one of claims 1-14, the pharmaceutical composition of claim 15 or claim 16, or the diagnostic composition according to claim 17, wherein said cell is physically coupled with the extracellular target biomolecule bound by said protein.

20. An *in vitro* method of killing a cell, comprising the step of contacting the cell with the protein of any one of claims 1-14.

21. A protein according to any one of claims 1-14 or a pharmaceutical composition according to claim 15 or claim 16, for use in treating or preventing a disease, disorder or condition in a subject in need thereof.

22. A protein according to any one of claims 1-14 or a pharmaceutical composition according to claim 15 or claim 16, for use in treating or preventing a disease, disorder, or condition selected from the group consisting of: cancer, tumor, growth abnormality, immune disorder, and microbial infection.

23. A protein according to any one of claims 1-14 or a pharmaceutical composition according to claim 15 or claim 16, for use according to claim 22,
wherein the cancer is selected from the group consisting of:
bone cancer, breast cancer, central/peripheral nervous system cancer, gastrointestinal cancer, germ cell cancer, glandular cancer, head-neck cancer, hematological cancer, kidney-urinary tract cancer, liver cancer, lung/pleura cancer, prostate cancer, sarcoma, skin cancer, and uterine cancer;
or wherein the immune disorder is associated with a disease selected from the group consisting of:
amyloidosis, ankylosing spondylitis, asthma, Crohn's disease, diabetes, graft rejection, graft-versus-host disease, Hashimoto's thyroiditis, hemolytic uremic syndrome, HIV-related disease, lupus erythematosus, multiple sclerosis, polyarteritis nodosa, polyarthritis, psoriasis, psoriatic arthritis, rheumatoid arthritis, scleroderma, septic shock, Sjögren's syndrome, ulcerative colitis, and vasculitis.

24. A protein according to any one of claims 1-14, a pharmaceutical composition according to claim 15 or claim 16, or a diagnostic composition according to claim 17, for use in the *in vivo* diagnosis, prognosis, or characterization of a disease, disorder, or condition.

25. Use of the protein of any one of claims 1-14, the pharmaceutical composition of claim 15 or claim 16, or the diagnostic composition of claim 17, in the *in vitro* diagnosis of a disease, disorder, or condition.

## Patentansprüche

1. Protein, umfassend:
a) eine Bindungsregion, die zur spezifischen Bindung von mindestens einem extrazellulären Zielbiomolekül fähig ist,
b) eine Effektorregion von Shigatoxin-Untereinheit A, umfassend ein Polypeptid, bei dem sich mindestens eine Funktion des Shigatoxins aufweisen lässt, die aus der Gruppe ausgewählt ist, bestehend aus: zellulärer Internalisierung, subzellulärem Routing und Cytotoxizität, wobei das Polypeptid eine Aminosäuresequenz umfasst oder aus einer Aminosäuresequenz besteht, die zu mindestens 85 % mit einer Aminosäuresequenz identisch ist, die ausgewählt ist aus:
i) Aminosäuren 75 bis 251 von SEQ ID NO: 1, SEQ ID NO: 2 oder SEQ ID NO: 3;
ii) Aminosäuren 1 bis 241 von SEQ ID NO: 1, SEQ ID NO: 2 oder SEQ ID NO: 3;
iii) Aminosäuren 1 bis 251 von SEQ ID NO: 1, SEQ ID NO: 2 oder SEQ ID NO: 3; und
iv) Aminosäuren 1 bis 261 von SEQ ID NO: 1, SEQ ID NO: 2 oder SEQ ID NO: 3; und
c) ein carboxyterminales Retentions-/Retrieval-Signalmotiv im endoplasmatischen Retikulum eines Mitglieds der KDEL-Familie;
wobei das Protein mit einem zusätzlichen exogenen Material zur Abgabe in eine Zelle verknüpft ist, wobei die genannte Zelle mit dem extrazellulären Zielbiomolekül des genannten Proteins physikalisch gekoppelt ist.

2. Protein nach Anspruch 1, wobei das zusätzliche exogene Material ausgewählt ist aus einem von: einem Peptid, Protein, cytotoxischen Mittel, Nachweis fördernden Mittel, kleinmoleküligen chemotherapeutischen Mittel und Polynukleotid.

3. Protein nach Anspruch 2, wobei das zusätzliche exogene Material ein Peptid oder Protein, umfassend ein Antigen ist; zum Beispiel wobei das Antigen hergeleitet ist von einem Bakterienprotein, einem Virusprotein, einem mutierten oder aberrant exprimierten Protein bei Krebs, oder einer T-Zelle komplementär bestimmenden Region.

4. Protein nach einem der Ansprüche 1 bis 3, wobei die Bindungsregion ein Polypeptid umfasst, das aus der Gruppe ausgewählt ist, bestehend aus:
einem Einzeldomänen-Antikörperfragment, einem variablen Einzelkettenfragment, einem variablen Antikörperfragment, einem Fd-Fragment, einem Antigenbindenden Fragment, einer von Fibronektin abgeleiteten zehnten Fibronektindomäne vom Typ III, einer Tenascindomäne vom Typ III, einer Ankyrin-Repeatmotiv-Domäne, einer vom Low-Density-Lipoprotein-Rezeptor abgeleiteten A-Domäne, einem Lipocalin, einer Kunitz-Domäne, einer von Protein A abgeleiteten Z-Domäne, einer von Gamma-B-Kristallin abgeleiteten Domäne, einer von Ubiquitin abgeleiteten Domäne, einem von Sac7d abgeleiteten Polypeptid, einer von Fyn abgeleiteten SH2-Domäne, einem Miniprotein und einem C-Typ-Lektin-ähnlichen Domänengerüst.

5. Protein nach einem der Ansprüche 1 bis 4, wobei die Bindungsregion zur Bindung an ein extrazelluläres Zielbiomolekül fähig ist, das aus der Gruppe ausgewählt ist, bestehend aus:
CD20, CD22, CD40, CD74, CD79, CD25, CD30, HER2/neu/ErbB2, EGFR, EpCAM, EphB2, dem Prostataspezifischen Membranantigen, Cripto, CDCP1, Endoglin, Fibroblasten-aktiviertem Protein, Lewis-Y, CD19, CD21, CS1/SLAMF7, CD33, CD52, CD133, gpA33, Mucin, TAG-72, einem Tyrosin-Proteinkinase-Transmembranrezeptor, Carbonsäureanhydrase IX, einem Folat-bindenden Protein, Gangliosid GD2, Gangliosid GD3, Gangliosid GM2, Gangliosid-Lewis-Y2, VEGFR, alphavbeta3, alpha5beta1, ErbB1/EGFR, Erb3, c-MET, IGF1R, EphA3, TRAIL-Rl, TRAIL-R2, RANK, Tenascin, CD64, Mesothelin, BRCA1, MART-1/MelanA, gp100, Tyrosinase, TRP-1, TRP-2, MAGE-1, MAGE-3, GAGE-1/2, BAGE, RAGE, NY-ESO-1, CDK-4, Beta-Catenin, MUM-1, Caspase-8, KIAA0205, HPVE6, SART-1, PRAME, Carcinoembryonalem Antigen, Prostataspezifischem Antigen, Prostata-Stammzellen-Antigen, humaner Aspartyl-(Asparaginyl)-beta-Hydroxylase, EphA2, HER3/ErbB-3, MUC1, einem Tyrosinase-assoziierten Antigen, HPV-E7, einem Epstein-Barr-Virus-Antigen, Bcr-Abl, einem alpha-Fetoprotein-Antigen, 17-A1, einem Blasentumor-Antigen, CD38, CD15, CD23, CD53, CD88, CD129, CD183, CD191, CD193, CD244, CD294, CD305, C3AR, FceR1a, Galectin-9, PD-L1, mrp-14, Siglec-8, Siglec-10, CD49d, CD13, CD44, CD54, CD63, CD69, CD123, TLR4, FceR1a, IgE, CD107a, CD203c, CD14, CD68, CD80, CD86, CD105, CD115, F4/80, ILT-3, Galectin-3, CD11a-c, GITRL, MHC-Klasse-I-Molekül, MHC-Klasse-II-Molekül, CD284-TLR4, CD107-Mac3, CD195-CCR5, HLA-DR, CD16/32, CD282-TLR2, CD11c und jedwedem immunogenen Fragment von jedwedem des Vorstehenden.

6. Protein nach einem der Ansprüche 1 bis 5, wobei die Bindungsregion Aminosäuren 2 bis 241 von jedweder einen der SEQ ID NOs: 4 bis 16 umfasst oder daraus besteht.

7. Protein nach einem der Ansprüche 1 bis 6, wobei das carboxyterminale Retentions-/Retrieval-Signalmotiv im endoplasmatischen Retikulum aus der Gruppe ausgewählt ist, bestehend aus:
KDEL (SEQ ID NO: 34), HDEF (SEQ ID NO: 35), HDEL (SEQ ID NO: 36), RDEF (SEQ ID NO: 37), RDEL (SEQ ID NO: 38), WDEL (SEQ ID NO: 39), YDEL (SEQ ID NO: 40), HEEF (SEQ ID NO: 41), HEEL (SEQ ID NO: 42), KEEL (SEQ ID NO: 43), REEL (SEQ ID NO: 44), KAEL (SEQ ID NO: 45), KCEL (SEQ ID NO: 46), KFEL (SEQ ID NO: 47), KGEL (SEQ ID NO: 48), KHEL (SEQ ID NO: 49), KLEL (SEQ ID NO: 50), KNEL (SEQ ID NO: 51), KQEL (SEQ ID NO: 52), KREL (SEQ ID NO: 53), KSEL (SEQ ID NO: 54), KVEL (SEQ ID NO: 55), KWEL (SEQ ID NO: 56), KYEL (SEQ ID NO: 57), KEDL (SEQ ID NO: 58), KIEL (SEQ ID NO: 59), OKEL (SEQ ID NO: 60), FDEL (SEQ ID NO: 61), KDEF (SEQ ID NO: 62), KKEL (SEQ ID NO: 63), HADL (SEQ ID NO: 64), HAEL (SEQ ID NO: 65), HIEL (SEQ ID NO: 66), HNEL (SEQ ID NO: 67), HTEL (SEQ ID NO: 68), KTEL (SEQ ID NO: 69), HVEL (SEQ ID NO: 70), NDEL (SEQ ID NO: 71), QDEL (SEQ ID NO: 72), REDL (SEQ ID NO: 73), RNEL (SEQ ID NO: 74), RTDL (SEQ ID NO: 75), RTEL (SEQ ID NO: 76), SDEL (SEQ ID NO: 77), TDEL (SEQ ID NO: 78), SKEL (SEQ ID NO: 79), STEL (SEQ ID NO: 80) und EDEL (SEQ ID NO: 81).

8. Protein nach einem der Ansprüche 1 bis 7, wobei die Shigatoxin-Effektorregion eine Sequenz umfasst oder daraus besteht, die zu mindestens 95 % identisch ist mit:
i) Aminosäuren 75 bis 251 von SEQ ID NO: 1, SEQ ID NO: 2 oder SEQ ID NO: 3;
ii) Aminosäuren 1 bis 241 von SEQ ID NO: 1, SEQ ID NO: 2 oder SEQ ID NO: 3;
iii) Aminosäuren 1 bis 251 von SEQ ID NO: 1, SEQ ID NO: 2 oder SEQ ID NO: 3; oder
iv) Aminosäuren 1 bis 261 von SEQ ID NO: 1, SEQ ID NO: 2 oder SEQ ID NO: 3.

9. Protein nach einem der Ansprüche 1 bis 8, wobei die Shigatoxin-Effektorregion eine Mutation relativ zu einer natürlich vorkommenden Untereinheit A eines Mitglieds der Shigatoxin-Familie umfasst, welche die enzymatische Aktivität der Shigatoxin-Effektorregion verändert, wobei die Mutation ausgewählt ist aus mindestens einer Deletion, Insertion oder Substitution eines Aminosäurerests; wobei die natürlich vorkommende Untereinheit A eines Mitglieds der Shigatoxin-Familie die Aminosäuresequenz von jedweder einen von SEQ ID NOs: 1 bis 3 umfasst oder daraus besteht.

10. Protein nach Anspruch 9, wobei die Mutation die Cytotoxizität der Shigatoxin-Effektorregion reduziert oder eliminiert.

11. Protein nach einem der Ansprüche 1 bis 8, wobei die Shigatoxin-Effektorregion Folgendes umfasst oder daraus besteht:
i) Aminosäuren 75 bis 251 von SEQ ID NO: 1, SEQ ID NO: 2 oder SEQ ID NO: 3;
ii) Aminosäuren 1 bis 241 von SEQ ID NO: 1, SEQ ID NO: 2 oder SEQ ID NO: 3;
iii) Aminosäuren 1 bis 251 von SEQ ID NO: 1, SEQ ID NO: 2 oder SEQ ID NO: 3; oder
iv) Aminosäuren 1 bis 261 von SEQ ID NO: 1, SEQ ID NO: 2 oder SEQ ID NO: 3.

12. Protein nach einem der Ansprüche 1 bis 11, wobei nach Verabreichung des Proteins an eine mit dem genannten extrazellulären Zielbiomolekül physikalisch gekoppelte Zelle das Protein zur Verursachung des Todes der Zelle fähig ist;
gegebenenfalls, wobei nach Verabreichung des Proteins an eine erste Population von Zellen, deren Mitglieder mit dem genannten extrazellulären Zielbiomolekül physikalisch gekoppelt sind, und eine zweite Population von Zellen, deren Mitglieder mit dem genannten extrazellulären Zielbiomolekül nicht physikalisch gekoppelt sind, die cytotoxische Wirkung des Proteins auf Mitglieder der genannten ersten Population von Zellen relativ zu Mitgliedern der genannten zweiten Population von Zellen zumindest um das Dreifache größer ist.

13. Protein nach einem der Ansprüche 1 bis 12 in der Form eines Homomultimers oder Heteromultimers.

14. Protein nach einem der Ansprüche 1 bis 13 in der Form eines pharmazeutisch verträglichen Salzes oder Solvats.

15. Pharmazeutische Zusammensetzung, umfassend das Protein nach einem der Ansprüche 1 bis 14 und mindestens einen pharmazeutisch verträglichen Hilfsstoff oder Träger.

16. Pharmazeutische Zusammensetzung nach Anspruch 15, wobei der mindestens eine pharmazeutisch verträgliche Träger Folgendes einschließt: ein Lösungsmittel, ein Dispersionsmedium, eine Beschichtung, wie zum Beispiel Lecithin, ein antimikrobielles Mittel, ein isotonisches Mittel oder ein Absorptionsverzögerungsmittel; gegebenenfalls wobei das antimikrobielle Mittel ein antibakterielles oder antifungales Mittel ist.

17. Diagnostische Zusammensetzung, umfassend: das Protein nach einem der Ansprüche 1 bis 14 und ein Nachweis förderndes Mittel.

18. Kit, umfassend:
i) das Protein nach einem der Ansprüche 1 bis 14, die pharmazeutische Zusammensetzung nach Anspruch 15 oder Anspruch 16, oder die diagnostische Zusammensetzung nach Anspruch 17, und
ii) ein zusätzliches Reagenz und/oder eine pharmazeutische Abgabevorrichtung.

19. In-vitro-Verfahren zur Abgabe eines exogenen Materials in eine Zelle, wobei das Verfahren den Schritt des Inkontaktbringens einer Zelle mit dem Protein nach einem der Ansprüche 1 bis 14, die pharmazeutische Zusammensetzung nach Anspruch 15 oder Anspruch 16, oder die diagnostische Zusammensetzung nach Anspruch 17 umfasst, wobei die genannte Zelle mit dem extrazellulären Zielbiomolekül, gebunden durch das genannte Protein, physikalisch gekoppelt ist.

20. In-vitro-Verfahren zum Abtöten einer Zelle, umfassend den Schritt des Inkontaktbringens der Zelle mit dem Protein nach einem der Ansprüche 1 bis 14.

21. Protein nach einem der Ansprüche 1 bis 14 oder eine pharmazeutische Zusammensetzung nach Anspruch 15 oder Anspruch 16, zur Verwendung bei der Behandlung oder Prävention einer Erkrankung, einer Störung oder eines Zustands bei einem Patienten mit Bedarf daran.

22. Protein nach einem der Ansprüche 1 bis 14 oder eine pharmazeutische Zusammensetzung nach Anspruch 15 oder Anspruch 16, zur Verwendung bei der Behandlung oder Prävention einer Erkrankung, einer Störung oder einem Zustand, ausgewählt aus der Gruppe, bestehend aus Krebs, einem Tumor, einer Wachstumsanomalie, einer Immunstörung und einer mikrobiellen Infektion.

23. Protein nach einem der Ansprüche 1 bis 14 oder eine pharmazeutische Zusammensetzung nach Anspruch 15 oder Anspruch 16, zur Verwendung nach Anspruch 22,
wobei der Krebs aus der Gruppe ausgewählt ist, bestehend aus:
Knochenkrebs, Brustkrebs, Krebs des zentralnervösen/periphernervösen Systems, Gastrointestinalkrebs, Keimzellenkrebs, Drüsenkrebs, Kopf- und Halskrebs, hämatologischem Krebs, Krebs der Niere sowie der Harnwege, Leberkrebs, Lungen-/Pleurakrebs, Prostatakrebs, einem Sarkom, Hautkrebs und Uteruskrebs;
oder wobei die Immunstörung mit einer Erkrankung assoziiert ist, die aus der Gruppe ausgewählt ist, bestehend aus:
Amyloidose, ankylosierender Spondylitis, Asthma, Morbus Crohn, Diabetes, Graft-Abstoßung, Graft-versus-Host-Erkrankung, Hashimoto-Thyreoiditis, dem hämolytischurämischen Syndrom, einer HIV-bedingten Erkrankung, Lupus erythematodes, Multipler Sklerose, Polyarteriitis nodosa, Polyarthritis, Psoriasis, Psoriasis-Arthritis, rheumatoider Arthritis, Sklerodermie, septischem Schock, dem Sjögren-Syndrom, ulzerativer Kolitis und Vaskulitis.

24. Protein nach einem der Ansprüche 1 bis 14, eine pharmazeutische Zusammensetzung nach Anspruch 15 oder Anspruch 16, oder eine diagnostische Zusammensetzung nach Anspruch 17 zur Verwendung bei der *In-vivo*-Diagnose, Prognose oder Charakterisierung einer Erkrankung, einer Störung oder eines Zustands.

25. Verwendung des Proteins nach einem der Ansprüche 1 bis 14, der pharmazeutischen Zusammensetzung nach Anspruch 15 oder Anspruch 16, oder der diagnostischen Zusammensetzung nach Anspruch 17, bei der In-vitro-Diagnose einer Erkrankung, einer Störung oder eines Zustands.

## Revendications

1. Protéine comprenant :
a) une région de liaison capable de se lier spécifiquement à au moins une biomolécule cible extracellulaire,
b) une région effectrice de sous-unité A d'une shiga-toxine comprenant un polypeptide capable de présenter au moins une fonction de shiga-toxine sélectionnée dans le groupe constitué d'une internalisation cellulaire, d'un routage subcellulaire, et d'une cytotoxicité, le polypeptide comprenant ou consistant en une séquence d'acides aminés qui est au moins 85 % identique à une séquence d'acides aminés sélectionnée parmi :
(i) acides aminés 75 à 251 de la SÉQ. ID n° 1, de la SÉQ. ID n° 2, ou de la SÉQ. ID n° 3 ;
(ii) acides aminés 1 à 241 de la SÉQ. ID n° 1, de la SÉQ. ID n° 2, ou de la SÉQ. ID n° 3 ;
(iii) acides aminés 1 à 251 de la SÉQ. ID n° 1, de la SÉQ. ID n° 2, ou de la SÉQ. ID n° 3 ; et
(iv) acides aminés 1 à 261 de la SÉQ. ID n° 1, de la SÉQ. ID n° 2, ou de la SÉQ. ID n° 3 ; et
c) un motif signal de rétention/récupération du réticulum endoplasmique à terminaisons carboxy d'un membre de la famille KDEL ;
la protéine étant liée à un matériau exogène additionnel destiné à être introduit dans une cellule, ladite cellule étant physiquement couplée à la biomolécule cible extracellulaire de ladite protéine.

2. Protéine selon la revendication 1, dans laquelle le matériau exogène additionnel est sélectionné parmi l'un quelconque de : peptide, protéine, agent cytotoxique, agent favorisant la détection, agent de chimiothérapie à petites molécules, et polynucléotide.

3. Protéine selon la revendication 2, dans laquelle le matériau exogène additionnel est un peptide ou une protéine comprenant un antigène ; par exemple dans laquelle l'antigène est dérivé d'une protéine bactérienne, d'une protéine virale, d'une protéine mutée ou exprimée de façon anormale dans un cancer, ou d'une région déterminant la complémentarité de cellules T.

4. Protéine selon l'une quelconque des revendications 1 à 3, dans laquelle la région de liaison comprend un polypeptide sélectionné dans le groupe constitué de :
fragment d'anticorps à un seul domaine, fragment variable à chaîne unique, fragment variable d'anticorps, fragment Fd, fragment de liaison à un antigène, 10^{ème} domaine fibronectine de type III dérivé de fibronectine, domaine ténascine de type III, domaine de motif de répétition ankyrine, domaine A dérivé de récepteur de lipoprotéines à faible densité, lipocaline, domaine de Künitz, domaine Z dérivé de protéine A, domaine dérivé de cristalline gamma-B, domaine dérivé de l'ubiquitine, polypeptide dérivé de Sac7d, domaine SH2 dérivé de Fyn, miniprotéine, et échafaudage de domaine lectine-like de type C.

5. Protéine selon l'une quelconque des revendications 1 à 4, dans laquelle la région de liaison est capable de se lier à une biomolécule cible extracellulaire sélectionnée dans le groupe constitué de :
CD20, CD22, CD40, CD74, CD79, CD25, CD30, HER2/neu/ERbB2, EGFR, EpCAM, EphB2, antigène membranaire spécifique de la prostate, Cripto, CDCP1, endogline, protéine d'activation des fibroblastes, Lewis-Y, CD19, CD21, CS1/SLAMF7, CD33, CD52, CD133, gpA33, mucine, TAG-72, récepteur transmembranaire à activité protéine tyrosine kinase, anhydrase carbonique IX, protéine liant les folates, ganglioside GD2, ganglioside GD3, ganglioside GM2, ganglioside Lewis-Y2, VEGFR, alphaV bêta3, AlphaV bêta1, ErbB1/EGFR, Erb3, c-MET, IGF1 R, EphA3, TRAIL-R1, TRAIL-R2, RANK, ténascine, CD64, mésothéline, BRCA1, MART-1/MelanA, gp100, tyrosinase, TRP-1, TRP-2, MAGE-1, MAGE-3, GAGE-1/2, BAGE, RAGE, NY-ESO-1, CDK-4, bêta-caténine, MUM-1, caspase-8, KIAA0205, HPVE6, SART-1, PRAME, antigène carcinoembryonnaire, antigène spécifique de la prostate, antigène des cellules souches prostatiques, aspartyl(asparaginyl)bêta-hydroxylase humaine, EphA2, HER3/ErbB-3, MUC1, antigène associé à la tyrosinase, HPV-E7, antigène du virus d'Epstein-Barr, Bcr-Abl, antigène de l'alpha-fœtoprotéine, 17-A1, antigène tumoral de la vessie, CD38, CD15, CD23, CD53, CD88, CD129, CD183, CD191, CD193, CD244, CD294, CD305, C3AR, FceRla, galectine-9, PD-L 1, mrp-14, Siglec-8, Siglec-10, CD49d, CD13, CD44, CD54, CD63, CD69, CD123, TLR4, FceRIa, IgE, CD107a, CD203c, CD14, CD68, CD80, CD86, CD105, CD115, F4/80, ILT-3, galectine-3, CD11a-c, GITRL, molécule MHC Classe I, molécule MHC Classe II, CD284-TLR4, CD107-Mac3, CD195-CCR5, HLA-DR, CD16/32, CD282-TLR2, CD11c, et tout fragment immunogène de l'une quelconque des molécules susmentionnées.

6. Protéine selon l'une quelconque des revendications 1 à 5, dans laquelle la région de liaison comprend ou consiste en les acides aminés 2 à 241 de l'une quelconque des SÉQ. ID n^{os} 4 à 16.

7. Protéine selon l'une quelconque des revendications 1 à 6, dans laquelle le motif signal de rétention/récupération du réticulum endoplasmique à terminaisons carboxy est sélectionné dans le groupe constitué de :
KDEL (SÉQ. ID n° 34), HDEF (SÉQ. ID n° 35), HDEL (SÉQ. ID n° 36), RDEF (SÉQ. ID n° 37), RDEL (SÉQ. ID n° 38), WDEL (SÉQ. ID n° 39), YDEL (SÉQ. ID n° 40), HEEF (SÉQ. ID n° 41), HEEL (SÉQ. ID n° 42), KEEL (SÉQ. ID n° 43), REEL (SÉQ. ID n° 44), KAEL (SÉQ. ID n° 45), KCEL (SÉQ. ID n° 46), KFEL (SÉQ. ID n° 47), KGEL (SÉQ. ID n° 48), KHEL (SÉQ. ID n° 49), KLEL (SÉQ. ID n° 50), KNEL (SÉQ. ID n° 51), KQEL (SÉQ. ID n° 52), KREL (SÉQ. ID n° 53), KSEL (SÉQ. ID n° 54), KVEL (SÉQ. ID n° 55), KWEL (SÉQ. ID n° 56), KYEL (SÉQ. ID n° 57), KEDL (SÉQ. ID n° 58), KIEL (SÉQ. ID n° 59), DKEL (SÉQ. ID n° 60), FDEL (SÉQ. ID n° 61), KDEF (SÉQ. ID n° 62), KKEL (SÉQ. ID n° 63), HADL (SÉQ. ID n° 64), HAEL (SÉQ. ID n° 65), HIEL (SÉQ. ID n° 66), HNEL (SÉQ. ID n° 67), HTEL (SÉQ. ID n° 68), KTEL (SÉQ. ID n° 69), HVEL (SÉQ. ID n° 70), NDEL (SÉQ. ID n° 71), QDEL (SÉQ. ID n° 72), REDL (SÉQ. ID n° 73), RNEL (SÉQ. ID n° 74), RTDL (SÉQ. ID n° 75), RTEL (SÉQ. ID n° 76), SDEL (SÉQ. ID n° 77), TDEL (SÉQ. ID n° 78), SKEL (SÉQ. ID n° 79), STEL (SÉQ. ID n° 80), et EDEL (SÉQ. ID n° 81).

8. Protéine selon l'une quelconque des revendications 1 à 7, dans laquelle la région effectrice de shiga-toxine comprend ou consiste en une séquence qui est au moins 95 % identique à :
(i) acides aminés 75 à 251 de la SÉQ. ID n° 1, de la SÉQ. ID n° 2, ou de la SÉQ. ID n° 3 ;
(ii) acides aminés 1 à 241 de la SÉQ. ID n° 1, de la SÉQ. ID n° 2, ou de la SÉQ. ID n° 3 ;
(iii) acides aminés 1 à 251 de la SÉQ. ID n° 1, de la SÉQ. ID n° 2, ou de la SÉQ. ID n° 3 ; ou
(iv) acides aminés 1 à 261 de la SÉQ. ID n° 1, de la SÉQ. ID n° 2, ou de la SÉQ. ID n° 3.

9. Protéine selon l'une quelconque des revendications 1 à 8, dans laquelle la région effectrice de shiga-toxine comprend une mutation par rapport à une sous-unité A naturelle d'un membre de la famille des shiga-toxines qui modifie l'activité enzymatique de la région effectrice de shiga-toxine, la mutation étant sélectionnée parmi au moins une délétion, une insertion, ou une substitution de résidu acide aminé ;
la sous-unité A naturelle d'un membre de la famille des shiga-toxines comprenant ou consistant en la séquence d'acides aminés correspondant à l'une quelconque des SÉQ. ID n^{os} 1 à 3.

10. Protéine selon la revendication 9, dans laquelle la mutation réduit ou élimine la cytotoxicité de la région effectrice de shiga-toxine.

11. Protéine selon l'une quelconque des revendications 1 à 8, dans laquelle la région effectrice de shiga-toxine comprend ou consiste en :
(i) acides aminés 75 à 251 de la SÉQ. ID n° 1, de la SÉQ. ID n° 2, ou de la SÉQ. ID n° 3 ;
(ii) acides aminés 1 à 241 de la SÉQ. ID n° 1, de la SÉQ. ID n° 2, ou de la SÉQ. ID n° 3 ;
(iii) acides aminés 1 à 251 de la SÉQ. ID n° 1, de la SÉQ. ID n° 2, ou de la SÉQ. ID n° 3 ; ou
(iv) acides aminés 1 à 261 de la SÉQ. ID n° 1, de la SÉQ. ID n° 2, ou de la SÉQ. ID n° 3.

12. Protéine selon l'une quelconque des revendications 1 à 11, **caractérisée en ce que**, lorsque la protéine est administrée à une cellule physiquement couplée à ladite biomolécule cible extracellulaire, la protéine est capable de causer la mort de la cellule ;
optionnellement dans laquelle, lorsque la protéine est administrée à une première population de cellules dont les membres sont physiquement couplés à ladite biomolécule cible extracellulaire, et à une deuxième population de cellules dont les membres ne sont pas physiquement couplés à ladite biomolécule cible extracellulaire, l'effet cytotoxique de la protéine sur les membres de ladite première population de cellules relativement aux membres de ladite deuxième population de cellules est au moins 3 fois supérieur.

13. Protéine selon l'une quelconque des revendications 1 à 12, sous la forme d'un homomultimère ou d'un hétéromultimère.

14. Protéine selon l'une quelconque des revendications 1 à 13, sous la forme d'un sel ou d'un solvate pharmaceutiquement acceptable.

15. Composition pharmaceutique comprenant la protéine selon l'une quelconque des revendications 1 à 14 et au moins un excipient ou support pharmaceutiquement acceptable.

16. Composition pharmaceutique selon la revendication 15, dans laquelle ledit au moins un support pharmaceutiquement acceptable comprend un solvant, un milieu de dispersion, un enrobage tel que la lécithine, un agent antimicrobien, un agent isotonique, ou un agent retardant d'absorption ; optionnellement dans laquelle l'agent antimicrobien est un agent antibactérien ou antifongique.

17. Composition diagnostique comprenant la protéine selon l'une quelconque des revendications 1 à 14 et un agent favorisant la détection.

18. Trousse comprenant :
(i) la protéine selon l'une quelconque des revendications 1 à 14, la composition pharmaceutique selon la revendication 15 ou la revendication 16, ou la composition diagnostique selon la revendication 17 ; et
(ii) un réactif et/ou un dispositif d'administration pharmaceutique additionnel.

19. Procédé *in vitro* d'apport d'un matériau exogène dans une cellule, le procédé comprenant l'étape de mise en contact d'une cellule avec la protéine selon l'une quelconque des revendications 1 à 14, la composition pharmaceutique selon la revendication 15 ou la revendication 16, ou la composition diagnostique selon la revendication 17, dans lequel ladite cellule est couplée physiquement à la biomolécule cible extracellulaire liée par ladite protéine.

20. Procédé *in vitro* de destruction d'une cellule, comprenant l'étape de mise en contact de la cellule avec la protéine selon l'une quelconque des revendications 1 à 14.

21. Protéine selon l'une quelconque des revendications 1 à 14, ou composition pharmaceutique selon la revendication 15 ou la revendication 16, destinée à une utilisation dans le traitement ou la prévention d'une maladie, d'une affection ou d'une pathologie chez un patient qui en a besoin.

22. Protéine selon l'une quelconque des revendications 1 à 14, ou composition pharmaceutique selon la revendication 15 ou la revendication 16, destinée à une utilisation dans le traitement ou la prévention d'une maladie, d'une affection ou d'une pathologie sélectionnée dans le groupe constitué d'un cancer, d'une tumeur, d'une anomalie de la croissance, d'un trouble du système immunitaire, et d'une infection microbienne.

23. Protéine selon l'une quelconque des revendications 1 à 14, ou composition pharmaceutique selon la revendication 15 ou la revendication 16, destinée à une utilisation selon la revendication 22,
le cancer étant sélectionné dans le groupe constitué de :
cancer des os, cancer du sein, cancer du système nerveux central/périphérique, cancer gastro-intestinal, cancer des cellules germinales, cancer glandulaire, cancer des voies aérodigestives supérieures, cancer hématologique, cancer du rein/des voies urinaires, cancer du foie, cancer du poumon/pleural, cancer de la prostate, sarcome, cancer de la peau et cancer utérin ;
ou le trouble du système immunitaire étant associé à une maladie sélectionnée dans le groupe constitué de :
amyloïdose, spondylarthrite ankylosante, asthme, maladie de Crohn, diabète, rejet de greffe, maladie du greffon contre l'hôte, thyroïdite de Hashimoto, syndrome hémolytique et urémique, maladie liée au VIH, lupus érythémateux, sclérose en plaques, polyartérite noueuse, polyarthrite, psoriasis, arthrite psoriasique, polyarthrite rhumatoïde, sclérodermie, choc septique, syndrome de Sjögren, colite ulcéreuse, et vascularite.

24. Protéine selon l'une quelconque des revendications 1 à 14, composition pharmaceutique selon la revendication 15 ou la revendication 16, ou composition diagnostique selon la revendication 17, destinée à une utilisation dans le diagnostic *in vivo,* le pronostic, ou la caractérisation d'une maladie, d'une affection, ou d'une pathologie.

25. Utilisation de la protéine selon l'une quelconque des revendications 1 à 14, de la composition pharmaceutique selon la revendication 15 ou la revendication 16, ou de la composition diagnostique selon la revendication 17, dans le diagnostic *in vitro* d'une maladie, d'une affection, ou d'une pathologie.
